# EUROPEAN PATENT APPLICATION

(11) **EP 4 729 125 A2**
(43) Date of publication of application: **22.04.2026**
(21) Application number: 25227044.2
(22) Date of filing: 21.02.2020
(51) Int. Cl.: A61P 31/18

(54) **OPTIMIZED VACCINE COMPOSITIONS AND METHODS FOR MAKING THE SAME**

(30) Priority: 21.02.2019 US 201962808760 P; 13.03.2019 US 201962817902 P
(62) Divisional of application: 20759003.5
(71) Applicant: Centivax, Inc., South San Francisco, CA 94080 (US)
(72) Inventor: GLANVILLE, Jacob, South San Francisco, 94080 (US); IVES, Sarah, South San Francisco, 94080 (US); IZHAKY, Michal, South San Francisco, 94080 (US); YOUSSEF, Sawsan, South San Francisco, 94080 (US); BÜRCKERT, Jean-Philippe, South San Francisco, 94080 (US)
(74) Representative: J A Kemp LLP

(57) **Abstract**

Described herein are compositions of and methods of making vaccines which can provide broad serological reactivity an inverse dose response, and a swarm effect.

## Description

### CROSS-REFERENCE

This application claims the benefit of U.S. Provisional Application No. 62/808,760, filed February 21, 2019, and U.S. Provisional Application No. 62/817,902, filed March 13, 2019, each of which is incorporated herein by reference in its entirety.

### BACKGROUND

Pathogenic agents such as, infectious bacteria, parasites, fungi, viruses, and cancers, have evolved various strategies to evade detection and neutralization by host immune response. Such strategies can often undermine and complicate the development of successful vaccines towards these pathogens. Rapid mutations can complicate vaccine design. Vaccination can enable a host to generate protective antibodies prior to infection. This can be accomplished by exposing the immune system of a host to an antigen or antigens of a pathogen or pathogens. When this is done for a relatively slowly mutating vaccine, a multi-year window for a single vaccine to match and provide protection can be afforded, and a vaccine can be successful.

Vaccination against rapidly mutating pathogens can provide limited protection, for example, because the immune system response against the vaccine can become obsolete. In some cases, the vaccine can become obsolete as antigens change over time. This can result in the requirement for a new vaccine on a periodic or semi-periodic basis, such as every year. In some cases, protection provided by such vaccines can vary, and some vaccines can for some periods or years provide poor protection. This can be due to incorrect predictions of viral strains likely to be in circulation the following season. In some cases, such as for the common cold, such rapid mutation can prevent the effectiveness of a vaccine.

### SUMMARY

Provided in this disclosure are vaccines comprising a set of antigens that are representative of at least 60% of fifth order clades of a microbe.

Also provided in this disclosure are vaccines comprising a set of antigens of a microbe wherein the smallest pairwise edit distance between two of the antigens is at least 10% of the average size of the antigens and the largest pairwise edit distance between two of the antigens is no more than 98% of the average size of the antigens.

Also provided in this disclosure are vaccines comprising a set of antigens that are representative of at least 60% of all operational taxonomic units (OTUs) of the microbe.

In some embodiments, an antigen is representative of a clade if its sequence is up to 40% of the average size of antigens of the represented clade different than other members of the represented clade. In some embodiments, an antigen is representative of a clade if the edit distance is up to 5% of the size of another strain of the represented clade. In some embodiments, an antigen is representative of a clade if its sequence is up to 20% of the average size of antigens of the represented clade different than at least 95%, 96%, 97%, 98%, or 99% of all strains of the represented clade. In some embodiments, an antigen is representative of a clade if its sequence is up to 10% of the average size of antigens of the represented clade different than at least 95%, 96%, 97%, 98%, or 99% of all strains of the represented clade. In some embodiments, an antigen is representative of a clade if the edit distance is up to 5% of the size of 95%, 96%, 97%, 98%, or 99% of all strains of the represented clade. In some embodiments, an antigen is representative of a clade if its sequence is up to 25 edit distance from all strains of the represented clade. In some embodiments, an antigen is representative of a clade if its sequence is up to 100 edit distance from all strains of the represented clade. In some embodiments, an antigen is representative of a clade if its sequence is present in the clade.

Also provided in this disclosure are vaccine compositions comprising a set of antigens, wherein the antigens are derived from a library of variants of a microbe wherein: a) two antigens of the set with the greatest edit distance have an edit distance S; b) two antigens of the library with the greatest edit distance have an edit distance L; and c) S is at least 60% of L.

Also provided in this disclosure are vaccine compositions comprising at least 4 influenza virus hemagglutinin antigen polypeptides, each represented by a sequence that is at least 20% identical, but not more than 95% identical, to the other influenza virus hemagglutinin antigen polypeptides, wherein each polypeptide comprises an antigen that is at least 90% identical among the 4 influenza virus hemagglutinin antigen polypeptides.

Also provided in this disclosure are vaccine compositions comprising at least 4 influenza virus neuraminidase antigen polypeptides, each represented by a sequence that is at least 20% identical, but not more than 95% identical, to the other influenza virus neuraminidase antigen polypeptides, wherein each polypeptide comprises an antigen that is at least 90% identical among the 4 influenza virus neuraminidase antigen polypeptides.

Also provided in this disclosure are vaccine compositions comprising at least 4 HIV gp160 antigen polypeptides, each represented by a sequence that is at least 20% identical, but not more than 95% identical, to the other HIV gp160 antigen polypeptides, wherein each polypeptide comprises an antigen that is at least 90% identical among the 4 HIV gp160 antigen polypeptides.

Also provided in this disclosure are vaccine compositions comprising at least 4 HIV gp120 antigen polypeptides, each represented by a sequence that is at least 20% identical, but not more than 95% identical, to the other HIV gp120 antigen polypeptides, wherein each polypeptide comprises an antigen that is at least 90% identical among the 4 HIV gp120 antigen polypeptides.

Also provided in this disclosure are vaccine compositions comprising at least 4 HIV gp41 antigen polypeptides, each represented by a sequence that is at least 20% identical, but not more than 95% identical, to the other HIV gp41 antigen polypeptides, wherein each polypeptide comprises an antigen that is at least 90% identical among the 4 HIV gp41 antigen polypeptides.

Also provided in this disclosure are vaccine compositions comprising a set of antigens that activate an immune response in a subject to at least 6 strains identified in Table 1.

In some embodiments, the immune response is detectable using head-specific antibodies in a hemagglutinin inhibition assay. In some embodiments, the immune response is at least 2 fold greater using the antigens than H1N1+H3N2+HAB, when tested using the hemagglutinin inhibition assay. In some embodiments, the immune response is at least 10 fold greater using the antigens than H1N1+H3N2+HAB, when tested using the hemagglutinin inhibition assay. In some embodiments, the immune response is at least 100 fold greater using the antigens than H1N1+H3N2+HAB, when tested using the hemagglutinin inhibition assay.

In some embodiments, the microbe is a bacterium. In some embodiments, the microbe is a virus. In some embodiments, the virus is influenza. In some embodiments, the influenza is type A. In some embodiments, the influenza is type B. In some embodiments, the type A influenza is H1N1, H1N2, H3N1, H3N2, or H2N3. In some embodiments, the type A influenza is H1N1. In some embodiments, the type A influenza is H3N2. In some embodiments, the virus is human immunodeficiency virus (HIV). In some embodiments, the HIV is HIV-1. In some embodiments, the HIV-1 is of subclade A, subclade B, or subclade C.

In some embodiments, the antigen is a broadly neutralizing antigen of surface exposed residues adjacent in tertiary space. In some embodiments, the broadly neutralizing antigen is in a stem of hemagglutinin. In some embodiments, the broadly neutralizing antigen is in a head of hemagglutinin. In some embodiments, the broadly neutralizing antigen is in a neuraminidase. In some embodiments, the broadly neutralizing antigen is in a gp160. In some embodiments, the broadly neutralizing antigen is in a gp120. In some embodiments, the broadly neutralizing antigen is in a gp41.

In some embodiments, the antigen is a broadly conserved fragment of hemagglutinin. In some embodiments, the antigen is a broadly conserved fragment of a head of hemagglutinin. In some embodiments, the antigen is a broadly conserved fragment of a stem of hemagglutinin. In some embodiments, the antigen is a broadly conserved fragment of neuraminidase. In some embodiments, the antigen is a broadly conserved fragment of gp160.

In some embodiments, the vaccine comprises at least one antigen selected from SEQ ID NOs:1-87. In some embodiments, the vaccine comprises at least one antigen that is at least 60% identical to any one of SEQ ID NOs: 1-87. In some embodiments, the vaccine comprises at least one antigen that is at least 70% identical to any one of SEQ ID NOs: 1-87. In some embodiments, the vaccine comprises at least one antigen that is at least 80% identical to any one of SEQ ID NOs:1-87. In some embodiments, the vaccine comprises at least one antigen that is at least 90% identical to any one of SEQ ID NOs: 1-87. In some embodiments, the vaccine comprises at least one antigen selected from SEQ ID NOs:88-127. In some embodiments, the vaccine comprises at least one antigen that is at least 60% identical to any one of SEQ ID NOs:88-127. In some embodiments, the vaccine comprises at least one antigen that is at least 70% identical to any one of SEQ ID NOs:88-127. In some embodiments, the vaccine comprises at least one antigen that is at least 80% identical to any one of SEQ ID NOs:88-127. In some embodiments, the vaccine comprises at least one antigen that is at least 90% identical to any one of SEQ ID NOs:88-127. In some embodiments, the vaccine comprises at least one antigen selected from SEQ ID NOs: 128-171. In some embodiments, the vaccine comprises at least one antigen that is at least 60% identical to any one of SEQ ID NOs:128-171. In some embodiments, the vaccine comprises at least one antigen that is at least 70% identical to any one of SEQ ID NOs: 128-171. In some embodiments, the vaccine comprises at least one antigen that is at least 80% identical to any one of SEQ ID NOs: 128-171. In some embodiments, the vaccine comprises at least one antigen that is at least 90% identical to any one of SEQ ID NOs: 128-171. In some embodiments, the vaccine comprises at least one antigen selected from SEQ ID NOs: 172-267. In some embodiments, the vaccine comprises at least one antigen that is at least 60% identical to any one of SEQ ID NOs: 172-267. In some embodiments, the vaccine comprises at least one antigen that is at least 70% identical to any one of SEQ ID NOs:172-267. In some embodiments, the vaccine comprises at least one antigen that is at least 80% identical to any one of SEQ ID NOs: 172-267. In some embodiments, the vaccine comprises at least one antigen that is at least 90% identical to any one of SEQ ID NOs:172-267.

In some embodiments, the set of antigens comprise at least 5, 6, 7, 8, 9, 10, 15, 20, 30, 40, 50, 60, 70, 80, 90, 100, 200, 300, 400, 500, 600, 700, 800, 900 or 1000 different antigens. In some embodiments, the set of antigens comprises at least 30 antigens. In some embodiments, the set of antigens comprises at least 50 antigens. In some embodiments, the antigens have an average edit distance from each of the other antigens that is at least 5% of the average size of antigens in the clade.

In some embodiments, a smallest pairwise edit distance between two or more of the antigens in the set is no more than 1, and the largest pairwise edit distance is at least 5, 10, 20, 30, 40, 50, 60, 70, 80, 90, or 100. In some embodiments, a smallest pairwise edit distance between two or more antigens in the set is no more than 5% of the size of the antigens, and the largest pairwise edit distance is at least 75% of the size of the antigens.

In some embodiments, the clades are clades of a phylogenetic tree is a neighbor joining clustering tree or maximum parsimony tree. In some embodiments, a first order clade is a clade that is not subsumed in whole or in part within another higher level clade. In some embodiments, each X-order clade is phylogenetically below X-1 number branch nodes in a phylogenetic tree of the microbe. In some embodiments, the vaccine comprises antigens that are representative of at least 60% of each of third, fourth, fifth, sixth, seventh, eighth, ninth, tenth, eleventh, twelfth, thirteenth, fourteenth, or fifteenth order clades of the microbe. In some embodiments, a represented clade is a clade Y that is subsumed by a clade Y-1. In some embodiments, a representative clade is a node-based clade. In some embodiments, a representative clade is a stem-based clade. In some embodiments, a representative clade is apomorphy-based clade. In some embodiments, an average number of branches between each of the antigens in the set is at least 1, 3, 5, or 10. In some embodiments, a smallest number of branches between any two antigens in the set is no more than 1 and a largest number of branches between any two antigens in the set is at least 5, 10, or 15.

In some embodiments, each OTU comprises sequences that are at least 95% homologous of one another. In some embodiments, each OTU comprises of at least 3 different sequences.

In some embodiments, each of the antigens in the set shares at least 90%, 95, or 99% sequence identity to at least one other antigen in the set. In some embodiments, each of the antigens in the set shares at least 90%, 95, or 99% % sequence identity to at least one other antigen in the set over a length of at least 100 amino acids. In some embodiments, each of the antigens in the set differs from each of the other antigens in the set by at least 5% sequence identity. In some embodiments, each of the antigens in the set differs from each of the other antigens in the set by no more than 75% sequence identity.

In some embodiments, each antigen is a peptide comprising at least 10, 15, 20, 25, 30, 35, 40, 45, 50, 55, 60, 65, 70, 75, 80, 85, 90, 85, 100, 110, or 120 amino acids. In some embodiments, the antigens are selected from Table 2, Table 3, Table 4, Table 5, or a fragment or homologue thereof. In some embodiments, the vaccine comprises 2 or more antigens from Table 2, Table 3, Table 4, Table 5, or fragments or homologues thereof. In some embodiments, the vaccine comprises 3 or more antigens from Table 2, Table 3, Table 4, Table 5, or fragments or homologues thereof. In some embodiments, the vaccine comprises 5 or more antigens from Table 2, Table 3, Table 4, Table 5, or fragments or homologues thereof. In some embodiments, the vaccine comprises 10 or more antigens from Table 2, Table 3, Table 4, Table 5, or fragments or homologues thereof. In some embodiments, the fragment comprises at most 10, 15, 20, 25, 30, 35, 40, 45, 50, 55, 60, 65, 70, 75, 80, 85, 90, 95, 100, 110, or 120 amino acids. In some embodiments the fragment comprises at least 10, 15, 20, 25, 30, 35, 40, 45, 50, 55, 60, 65, 70, 75, 80, 85, 90, 95, 100, 110, or 120 amino acids. In some embodiments the homologue comprises a sequence having at least 90% sequence identity to the antigen of Table 1.

In some embodiments the library comprises at least 1x10⁴, 1x10⁵, 1x10⁶ different variants of the microbe. In some embodiments the library comprises at least 90% of all known sequences of the microbe.

Also provided in this disclosure are pharmaceutical compositions comprising the vaccine composition of any of the preceding claims and a pharmaceutically acceptable diluent, adjuvant, excipient, or any combinations thereof.

In some embodiments the vaccine is in a form of an aerosol formulation. In some embodiments the vaccine is in a form of an injectable formulation.

In some embodiments each of the antigens is at a concentration that alone does not provide a significant prophylactic immune response to the broadly neutralizing antigen in a subject; and collectively, the antigens have a combined concentration that provides an immune response to the broadly neutralizing antigen in the subject. In some embodiments the subject is a bird. In some embodiments the subject is a mammal. In some embodiments the subject is a human. In some embodiments the subject is a pig.

Also provided in this disclosure are virus like particles (VLPs) comprising the vaccine composition of any of the preceding claims.

Also provided in this disclosure are recombinant expression vectors comprising a nucleic acid molecule encoding: a) a set of antigens that are representative of at least 60% of each of first order clade and second order clades of a microbe; b) set of antigens wherein the smallest pairwise edit distance between two of the antigens is no more than 25 and the largest pairwise edit distance between two of the antigens is at least 300; c) set of antigens that are representative of at least 60% of all operational taxonomic units (OTUs) of the microbe; or d) set of antigens that are representative of at least 60% of all operational taxonomic units (OTUs) of the microbe.

Also provided in this disclosure are recombinant expression vector comprising a nucleic acid molecule encoding: a) a set of antigens that are representative of at least 60% of each of first order clade and second order clades of a microbe; b) a set of antigens wherein the smallest pairwise edit distance between two of the antigens is no more than 5% of the size of the antigens, and the largest pairwise edit distance is at least 75% of the size of the antigen; c) a set of antigens that are representative of at least 60% of all operational taxonomic units (OTUs) of the microbe; or d) a set of antigens that are representative of at least 60% of all operational taxonomic units (OTUs) of the microbe.

Also provided in this disclosure are methods for treating or reducing the likelihood of an infection in a subject, comprising administrating to the subject a vaccine composition of any of the preceding claims.

Also provided in this disclosure are methods for treating or reducing the likelihood of a flu infection in a subject, comprising administrating to the subject a vaccine composition that results in an immune activation effective against seasonal flu for at least 3, 4, 5, 6, 7, 8, 9, or 10 years.

In some methods, the subject is a human. In some methods, the subject is a domesticated animal.

Also provided in this disclosure are methods for making a vaccine composition, comprising: selecting a set of antigens that are (a) representative of at least 60% of each of first order clade and second order clades of a microbe; (b) set of antigens wherein the smallest pairwise edit distance between two of the antigens is no more than 5% of the size of the antigens, and the largest pairwise edit distance is at least 75% of the size of the antigens; (c) representative of at least 60% of all operational taxonomic units (OTUs) of the microbe; or (d) representative of at least 60% of all operational taxonomic units (OTUs) of the microbe.

In some embodiments, the method further comprises obtaining a plurality of antigen sequences from a library of strains of the microbe; and aligning the plurality of antigen sequences to create a phylogenetic tree of the antigen..

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
**FIG. 1** illustrates examples of what is and is not a clade on a phylogenic tree.
**FIG. 2** illustrates examples of three types of clades: a node-based clade, a branch-based clade, and an apomorphy-based clade.
**FIG. 3** illustrates clade order in a phylogenic tree, with a third order clade being subsumed by a second order clade, which is subsumed by a first order clade.
**FIG. 4** illustrates a model of the influenza antigen-antibody interaction space.
**FIG. 5** depicts the probabilities of conserved epitopes by categories (i.e., universal, 90% of strains, all H1 strains, all H3 strains, 90% of all H1 strains, and 90% of all H3 strains).
**FIG. 6** illustrates a concentration conservation coupling vaccination strategy that can shift epitope conservation distribution
**FIG. 7** illustrates ELISA signals indicating a swarm effect observed on inoculating pigs with a vaccine as described herein.
**FIG. 8** illustrates ELISA signal per HA class after inoculating a cohort of pigs three times with a vaccine as described herein.
**FIG. 9** illustrates ELISA signal and foldchange after inoculating a cohort of pigs three times with a vaccine as described herein.
**FIG. 10** illustrates a heatmap showing serum titers after a second boost of a vaccine composition on a panel of antigen variants for each member of each cohort.
**FIG. 11** illustrates a peripheral B cell repertoire occupied by top clonotypes separated by isotype.
**FIG. 12** illustrates a sequence alignment of broadly neutralizing pig monoclonal antibody 9C5 isolated through phage display.
**FIG. 13** illustrates minimum antibody concentrations of three broadly neutralizing human (CR9114, F10, and C05) and one broadly neutralizing pig antibody (9C5) sufficient for *in-vitro* neutralization of a panel of 5 influenza A strains.
**FIG. 14** illustrates data showing a conservation concentration coupling approach can yield serum with improved strain coverage and neutralization breadth compared to a bivalent control.

### DETAILED DESCRIPTION

Rapidly evolving pathogens can pose a fundamental challenge to vaccine design, as mutations in such pathogens can render previous vaccine responses obsolete. For example, annual influenza vaccine redesigns can attempt to anticipate evolved influenza variants that may be in common circulation in the following year. Such attempts can often be unsuccessful. Conserved epitopes on the influenza coat proteins which have been identified can be missed by the antibodies elicited by most vaccine recipients.

Provided herein are immune-generating compositions (e.g., vaccines) that can elicit responses against one or more pathogens. In some cases, vaccines provided herein incorporate antigens that can elicit an immune response that can yield broadly neutralizing antibodies. The present disclosure also provides methods for using and making vaccines as provided herein.

Broadly neutralizing antibodies can provide for "universal" or longer-term vaccines. Such broadly neutralizing antibodies can be against a broadly conserved residue, for example, a residue of an antigen. A broadly conserved antigen can be conserved over multiple strains of a microbe. Microbes, even rapidly mutating pathogens, can comprise broadly conserved antigens. Antibodies that can identify and/or bind these conserved antigens or sets of antigens can confer protection against many strains of a given virus.

Since non-conserved residues can be immunodominant because they can be more abundant (e.g., vastly more abundant) than broadly conserved residues, this disclosure provides an approach that can shift the distribution of abundance of the residues of antigens to favor responses to more conserved antigens. By incorporating broadly conserved antigens representing a spectrum of variations of a microbe (e.g., mutants, different strains, or different clades) into vaccines, immunity can be conferred to a subject to many or substantially all variations of a microbe.

### I. Microbes

A vaccine composition as described herein can elicit an immune response against a microbe, or against a strain or subset of strains of a microbe. In some cases, such vaccines can provide broad immunity (immunity not limited to a particular strain) to a plurality of strains of a microbe. Such vaccines can comprise a set of antigens, which can comprise several or many different antigens from several or many different strains of a microbe.

A microbe can be an agent that a subject can be vaccinated against. In some cases, a microbe can be pathogenic, or can lead to a disease, a condition, or death. A microbe can be a bacterium, a virus, a fungus, a parasite, or a derivative thereof, such as a nucleic acid, protein, toxin, or peptide secreted by or isolated from a microbe.

A bacterium can be a single-celled prokaryotic microorganism. A bacterium can be a gram positive bacterium or a gram negative bacterium. A bacterium can be infectious.

A fungus can be a spore producing organism. A fungus can cause disease in a subject on the skin, in the lungs, in the blood, or another organ, tissue, or biological fluid. In some cases, a fungus can be from the genus *Aspergillus, Blastomyces, Candida, Coccidioides,* or *Cryptococcus* (e.g., *C. neoformans* and *C. gattii*)*.* Other suitable fungi are also within the scope of the present disclosure.

A parasite can be a protozoa, a helminth, an ectoparasite, or any other suitable parasite. In some cases, a parasite can be a *Plasmodium* parasite. In some cases, a parasite can be *a Plasmodium* parasite or a *Trypanosoma* parasite. In the case of a *Plasmodium* parasite, the vaccine might be against a malaria parasite. In the case of a *Trypanosoma* parasite, the vaccine might be against a Chagas parasite, a Sleeping Sickness parasite, or a Leishmaniasis parasite.

A protozoa can be a one-celled organism that can live and/or multiply in the blood or tissue of a subject. Examples of protozoa can include, but are not limited to, Sarcodina (e.g., *Entamoeba*), mastigophore (e.g., *Giardia* and *Leishmania*), ciliophoran (e.g., *Balantidium*), and sporozoan (e.g., *Plasmodium, Cryptosporidium,* etc.).

A helminth can be a multicellular organism. Helminths can be divided into three groups: platyhelminths, acanthocephalins, and nematodes. Examples of helminths can include, but are not limited to, parasitic flatworms, flukes, tapeworms, thorny-headed worms, roundworms, and pinworms. Helminths can live in a gastrointestinal tract, blood, lymphatic system, or other tissue.
An ectoparasite can be an organism that can live on the surface of a subject, and in some cases, can attach or burrow into the skin of a subject. Examples of ectoparasites can include ticks, fleas, lice, and mites.

In some instances, a virus can be a lentivirus, a flavivirus, a filovirus, a coronavirus, or a paramyxovirus. In the case of a lentivirus, the vaccine might be against a human immunodeficiency (HIV) virus. In the case of a flavivirus, the vaccine might be against a Dengue virus, a Zika virus, or a West Nile virus. In the case of a filovirus, the vaccine might be against an Ebola virus, a Marburg virus, or a Ravn virus. In the case of a coronavirus, the vaccine might be against a Middle East Respiratory Syndrome (MERS) virus, a Severe Acute Respiratory Syndrome (SARS) virus, or a novel coronavirus (e.g., 2019-nCoV). In the case of a paramyxovirus, the vaccine might be against a Respiratory Syncytial Virus (RSV) or a Nipah virus.

A vaccine can be against a flu virus of any type, such as, but not limited to, any one or more of the flu viruses described herein. A flu virus can be influenza type A, influenza type B, influenza type C, or influenza type D. Influenza A viruses can be divided into subtypes based on two proteins on the surface of the virus: hemagglutinin (HA) and neuraminidase (NA). Different flu strains can have different subtypes of hemagglutinin, neuraminidase, or both. There can be at least 198 predicted influenza A subtype combinations. Influenza B viruses can be further classified into lineages, which can include, for example, B/Yamagata and B/Victoria.

If a virus is a type A flu virus, it may be of any subtype, including, but not limited to, H1N1, H1N2, H1N3, H2N2, H3N2, H3N8, H4N2, H4N4, H4N6, H4N8, H5N1, H5N2, H5N3, H5N8, H6N1, H6N4, H6N5, H6N6, H6N8, H7N1, H7N2, H7N3, H7N7, H7N8, H7N9, H8N4, H9N2, H9N5, H9N8, H10N3, H10N4, H10N7, H10N8, H10N9, H11N2, H11N6, H11N9, H12N1, H12N3, H12N5, H13N6, H13N8, H14N5, H15N2, H15N8, H16N3, H17N, H18N11, or a variant thereof. If a virus is a type B flu virus, it can be any strain of type B flu virus or a variant thereof. A non-limiting list of flu viruses is included in Table 1.

A flu virus can be of a strain listed in Table 1. Some vaccines can comprise a set of antigens that can activate an immune response in a subject to at least 80% of strains in Table 1. In some instances, a vaccine can comprise a set of antigens that can activate an immune response in a subject to at least 10%, at least 15%, at least 20%, at least 25%, at least 30%, at least 40%, at least 45%, at least 50%, at least 55%, at least 60%, at least 65%, at least 70%, at least 75%, at least 80%, at least 85%, at least at least 90%, at least at least 95%, at least at least 99%, or 100% of the strains in Table 1.

**Table 1: Flu virus strains**

| | |
|---|---|
| | A/Albany/12/1951 |
| | A/Beij ing/22808/2009 |
| | A/Beijing/262/1995 |
| | A/Brevig Mission/1/1918 |
| | A/Brisbane/59/2007 |
| | A/California/04/2009 |
| | A/California/06/2009 |
| | A/California/07/2009 |
| | A/Chile/1/1983 |
| | A/England/195/2009 |
| | A/England/42/1972 |
| | A/New Caledonia/20/1999 |
| H1N1 | A/New York/06/2009 |
| | A/New York/1/1918 |
| | A/New York/18/2009 |
| | A/New Jersey/8/1976 |
| | A/Ohio/07/2009 |
| | A/Ohio/UR06-0091/2007 |
| | A/Puerto Rico/8/1934 |
| | A/Puerto Rico/8/34/Mount Sinai |
| | A/Solomon Islands/3/2006 |
| | A/swine/Belgium/1/1998 |
| | A/Swine/Wisconsin/136/1997 |
| | A/Taiwan/01/1986 |
| | A/Texas/05/2009 |
| | A/Texas/36/1991 |
| | A/USSR/90/1977 |
| | A/US SR/92/1977 |
| | A/WSN/1933 |
| H1N2 | A/swine/Guangxi/13/2006 |
| H1N3 | A/duck/NZL/160/1976 |
| H2N2 | A/Ann Arbor/6/1960 |
| | A/Canada/720/2005 |
| | A/Guiyang/1/1957 |
| | A/Japan/305/1957 |
| H3N2 | A/Aichi/2/1968 |
| | A/Babol/36/2005 |
| | A/Brisbane/10/2007 |
| | A/California/7/2004 |
| | A/Chiang Rai/277/2011 |
| | A/Christchurch/4/1985 |
| | A/Fujian/411/2002 |
| | A/Guangdong-Luohu/ 1256/2009 |
| | A/Hong Kong/1/1968 |
| | A/Hong Kong/CUHK31987/2011 |
| | A/Indiana/07/2012 |
| | A/Memphis/1/68 |
| | A/Moscow/10/1999 |
| | A/New York/55/2004 |
| | A/Perth/16/2009 |
| | A/reassortant/IVR-155 |
| | A/Sydney/5/1997 |
| | A/Texas/50/2012 |
| | A/Victoria/208/2009 |
| | A/Victoria/210/2009 |
| | A/Victoria/3/1975 |
| | A/Victoria/361/2011 |
| | A/Wisconsin/15/2009 |
| | A/Wisconsin/67/X-161/2005 |
| | A/Wyoming/03/2003 |
| | A/X-31 |
| H3N8 | A/canine/New York/145353/2008 |
| | A/equine/Gansu/7/2008 |
| H4N2 | A/duck/Hunan/8-19/2009 |
| H4N4 | A/mallard duck/Alberta/299/1977 |
| H4N6 | A/mallard/Ohio/657/2002 |
| | A/Swine/Ontario/01911-1/99 |
| H4N8 | A/chicken/Alabama/1/1975 |
| H5N1 | A/Anhui/1/2005 |
| | A/bar-headed goose/Qinghai/14/2008 |
| | A/bar-headed goose/Qinghai/1A/2005 |
| | A/barnswallow/Hong Kong/D10-1161/2010 |
| | A/Cambodia/R0405050/2007 |
| | A/Cambodia/S1211394/2008 |
| | A/chicken/Egypt/2253-1/2006 |
| | A/chicken/India/NIV33487/2006 |
| | A/chicken/Jilin/9/2004 |
| | A/chicken/VietNam/NCVD-016/2008 |
| | A/chicken/Yamaguchi/7/2004 |
| | A/Common magpie/Hong Kong/2256/2006 |
| | A/common magpie/Hong Kong/5052/2007 |
| | A/Duck/Hong Kong/p46/97 |
| | A/duck/Hunan/795/2002 |
| | A/duck/Laos/3295/2006 |
| | A/Egypt/2321-NAMRU3/2007 |
| | A/Egypt/3300-NAMRU3/2008 |
| | A/Egypt/N05056/2009 |
| | A/goose/Guangdong/1/96 |
| | A/goose/Guiyang/3 3 7/2006 |
| | A/Hong Kong/213/03 |
| | A/Hong Kong/483/97 |
| | A/Hubei/1/2010 |
| | A/Hubei/2011 |
| | A/Hubei/2011-CDC |
| | A/Indonesia/5/2005 |
| | A/Japanese white-eye/Hong Kong/1038/2006 |
| | A/Thailand/1(KAN-1)/2004 |
| | A/turkey/Turkey/1/2005 |
| | A/Vietnam/UT31413II/2008 |
| | A/whooper swan/Mongolia/244/2005 |
| | A/Xinjiang/1/2006 |
| H5N2 | A/American green-winged teal/California/HKWF609/07 |
| | A/ostrich/South Africa/AI1091/2006 |
| H5N3 | A/duck/Hokkaido/167/2007 |
| H5N8 | A/breeder duck/Korea/Gochang1/2014 |
| | A/broiler duck/Korea/Buan2/2014 |
| | A/duck/Jiangsu/k1203/2010 |
| | A/duck/NY/191255-59/2002 |
| | A/duck/Zhejiang/6D18/2013 |
| | A/duck/Zhejiang/W24/2013 |
| | A/turkey/Ireland/1378/1983 |
| H5N9 | A/chicken/Italy/22A/1998 |
| H6N1 | A/northern shoveler/California/HKWF115/2007 |
| H6N4 | A/chicken/HongKong/17/77 |
| H6N5 | A/shearwater/Australia/1/1973 |
| H6N6 | A/duck/Eastern China/11/2009 |
| H6N8 | A/mallard/Ohio/217/1998 |
| H7N1 | A/turkey/Italy/4602/99 |
| H7N2 | A/ruddy turnstone/New Jersey/563/2006 |
| H7N3 | A/chicken/SK/HR-00011/2007 |
| | A/turkey/Italy/214845/2002 |
| H7N7 | A/chicken/Netherlands/1/03 |
| | Alequine/Kentucky/1a/1975 |
| | A/Netherlands/219/2003 |
| H7N8 | A/mallard/Netherlands/33/2006 |
| H7N9 | A/Anhui/1/2013 |
| | A/Anhui/PA-1/2013 |
| | A/chicken/Zhejiang/DTID-ZJU01/2013 |
| | A/Hangzhou/1/2013 |
| | A/Hangzhou/3/2013 |
| | A/Huzhou/10/2013 |
| | A/Pigeon/Shanghai/S 1069/2013 |
| | A/Shanghai/1/2013 |
| | A/Shanghai/4664T/2013 |
| | A/Shanghai/Patient3/2013 |
| | A/Zhejiang/1/2013 |
| | A/Zhejiang/DTID-ZJU10/2013 |
| H8N4 | A/pintail duck/ Alberta/114/1979 |
| H9N2 | A/brambling/Beijing/16/2012 |
| | A/Chicken/Hong Kong/G9/1997 |
| | A/duck/Hong Kong/448/78 |
| | A/Guinea fowl/Hong Kong/WF 10/99 |
| | A/Hong Kong/1073/99 |
| | A/Hong Kong/2108/2003 |
| | A/Hong Kong/3239/2008 |
| | A/Hong Kong/35820/2009 |
| H9N5 | A/shorebird/DE/261/2003 |
| H9N8 | A/chicken/Korea/164/04 |
| H10N3 | A/duck/Hong Kong/786/1979 |
| | A/duck/Hunan/S11205/2012 |
| | A/mallard/Minnesota/Sg-00194/2007 |
| H10N4 | A/mink/Sweden/3900/1984 |
| H10N7 | A/blue-winged teal/Louisiana/Sg-00073/2007 |
| H10N8 | A/duck/Guangdong/E1/2012 |
| | A/Jiangxi-Donghu/346/2013 |
| H10N9 | A/duck/Hong Kong/562/1979 |
| | A/duck/HongKong/562/1979 |
| H11N2 | A/duck/Yangzhou/906/2002 |
| | A/thick-billed murre/Newfoundland/031/2007 |
| H11N6 | A/duck/England/1/1956 |
| H11N9 | A/mallard/Alberta/294/1977 |
| H12N1 | A/mallard duck/Alberta/342/1983 |
| H12N3 | A/bar headed goose/Mongolia/143/2005 |
| H12N5 | A/green-winged teal/ALB/199/1991 |
| H13N6 | A/black-headed gull/Sweden/1/1999 |
| H13N8 | A/black-headed gull/Netherlands/1/00 |
| H14N5 | A/Mallard/Astrakhan(Gurj ev)/263/1982 |
| H15N2 | A/Australian shelduck/Western Australia/1756/1983 |
| H15N8 | A/duck/AUS/341/1983 |
| H16N3 | A/black-headed gull/Sweden/5/99 |
| H17N10 | A/little yellow-shouldered bat/Guatemala/164/2009 |
| H18N11 | A/flat-faced bat/Peru/033/2010 |
| Influenza B | B/Brisbane/3/2007 |
| | B/Brisbane/60/2008 |
| | B/Florida/07/2004 |
| | B/Florida/4/2006 |
| | B/Hong Kong/05/1972 |
| | B/Malaysia/2506/2004 |
| | B/Massachusetts/03/2010 |
| | B/Ohio/O 1/2005 |
| | B/PHUKET/3073/2013 |
| | B/Utah/02/20 12 |
| | B/Victoria/02/1987 |
| | B/Victoria/504/2000 |
| | B/Wisconsin/01/2012 |
| | B/Yamagata/16/1988 |

A flu vaccine can comprise 2, 3, 4, 5, 6, 7, 8, 9, 10, 11, 12, 13, 14, 15, or more antigens per hemagglutinin antigen class (i.e., H1, H2, H3, etc.)

A vaccine can be against an HIV virus of any species, for example, HIV-1 and HIV-2. HIV-1 can be of group M (main), group O (outlier), or group N (non-M or O). HIV-1 group M can be of subtype A, B, C, D, F, G, H, J, or another subtype. HIV-2 can be of subtype A or subtype B. In some cases, a vaccine against HIV can be against one of HIV-1 or HIV-2. In some cases, a vaccine against HIV can be against both HIV-1 and HIV-2.

A vaccine can be against a variant of HIV or in some cases against simian immunodeficiency virus (SIV). In some cases, a vaccine against HIV can also be against SIV. In some cases, a vaccine against HIV can also offer protection against a variant of SIV.

An HIV virus can be of a strain listed in **FIGs. 26-34.** Some vaccines can comprise a set of antigens that can activate an immune response in a subject to at least 80% of strains in **FIGs. 26-34.** In some instances, a vaccine can comprise a set of antigens that can activate an immune response in a subject to at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least at least 90%, at least 95%, at least 99%, or 100% of the strains in **FIGs. 26-34.**

A microbe can have variants. A variant of a microbe can comprise a microbe having a variation in its genome, a microbe having a variation in its post-translational modification, a microbe having a variation in its epigenome, a microbe having a variation in its protein expression, a microbe having a variation in its RNA expression, a microbe having a variation in its antigen content, or a combination thereof. In some cases, a microbe having a variation in its antigen content can be of particular interest.

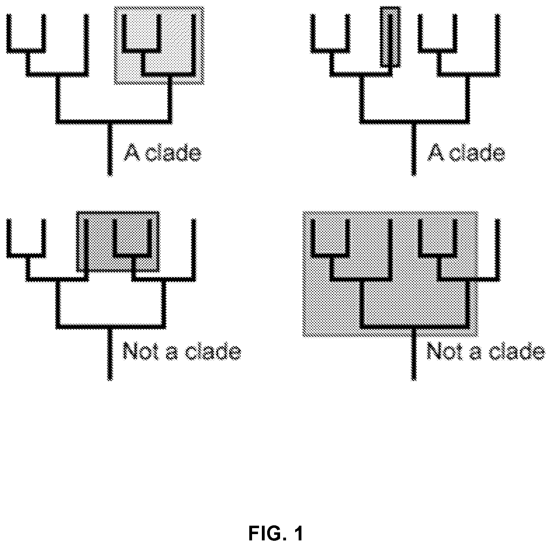

A microbe can have a plurality of variants which can be represented using a phylogenic tree. A phylogenic tree can be a model or a tool, which can be used to explore relationships. In some cases, a phylogenetic tree can be a diagrammatic representation of phylogeny, or evolutionary history, in the sense of a minimally connected graph. A phylogenic tree can comprise a number of clades, wherein a clade can be a grouping of nested branches. A clade can be a grouping that includes a common ancestor and all the descendants (living and extinct) of that ancestor. Examples of clades in a phylogenic tree are provided in **FIG. 1****.** Examples of groupings in a phylogenetic tree that do not comprise clades are also provided in **FIG. 1****.** A vaccine can comprise antigens that are representative of clades of a microbe.

A group of microbes can be divided into clades, or groups in a phylogenetic tree. In some cases, a phylogenetic tree can be an organization of microbes that can indicate how closely related individual microbes are to one another.

Clades can be arranged based on similarity of one or more sequences of antigens of the microbes. Clades and subclades on a phylogenetic tree can be shown as groups that have similar genetic changes, such as nucleotide or amino acid changes, and have a single common ancestor (e.g., a common node or branch).

Clades that are genetically different can be antigenically different. Antigenically different clades can refer to clades whose members have differences that can impact immunity in a subject.

A clade can be a clade of a phylogenic tree which can be a neighbor-joining clustering tree or a maximum parsimony tree. In some cases, a phylogenic tree can be modeled by a Bayesian model, a maximum likelihood model, a weighted pair group method with arithmetic mean (WPGMA), or an unweighted pair group method with arithmetic mean (UPGMA). A phylogenetic tree as provided herein can also be modeled by other suitable statistical models. A neighbor-joining clustering tree can be created using an agglomerative clustering method. A maximum parsimony tree can be created to minimize the total number of character state changes.

A clade can comprise nodes and branches. A node can be a point or vertex on a phylogenetic tree that can represent the split of one lineage of a microbe to form two or more lineages (e.g., an internal node within a phylogenetic tree) or the lineage at the present time (e.g., a terminal node). A branch can be a line on a phylogenetic tree that can be used to represent a lineage, whether ancestral or terminal.

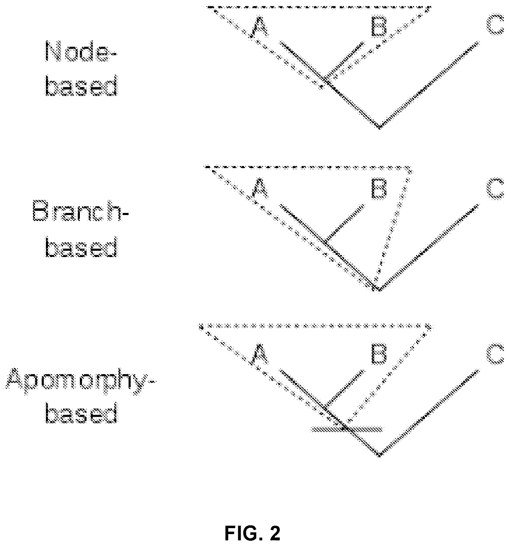

A clade can be a node-based clade, a branch-based clade, or an apomorphy-based clade. A node-based clade can be a clade originating from a particular node on a phylogenic tree and can encompass that node and the branches and nodes descending from that node. An example of a node-based clade is illustrated in the top panel of **FIG. 2****.** A branch-based clade can be a clade originating from a particular branch on a phylogenic tree and can encompass that branch and the branches and nodes descending from that branch. An example of a branch-based clade is illustrated in the middle panel of **FIG. 2****.** In some cases, a clade can be an apomorphy-based clade. An apomorphy-based clade can be a clade originating from an ancestor in which a particular character state, for example, a sequence or structure feature originated. An example of an apomorphy-based clade is illustrated in the bottom panel of **FIG. 2****.** The horizontal line in the apomorphy-based clade illustrated in **FIG. 2** indicates where a particular character state occurred.

In some cases, a clade can be a clade of a phylogenic tree which can be a rooted tree, an unrooted tree, or a bifurcating tree. A phylogenic tree can be a rooted tree if any unique node can correspond to the most recent common ancestor of all entities on the tree. A phylogenic tree can be an unrooted tree if it can be created without making any substantial assumptions about ancestry. A phylogenic tree can be a bifurcating tree if each node has exactly two descendants.

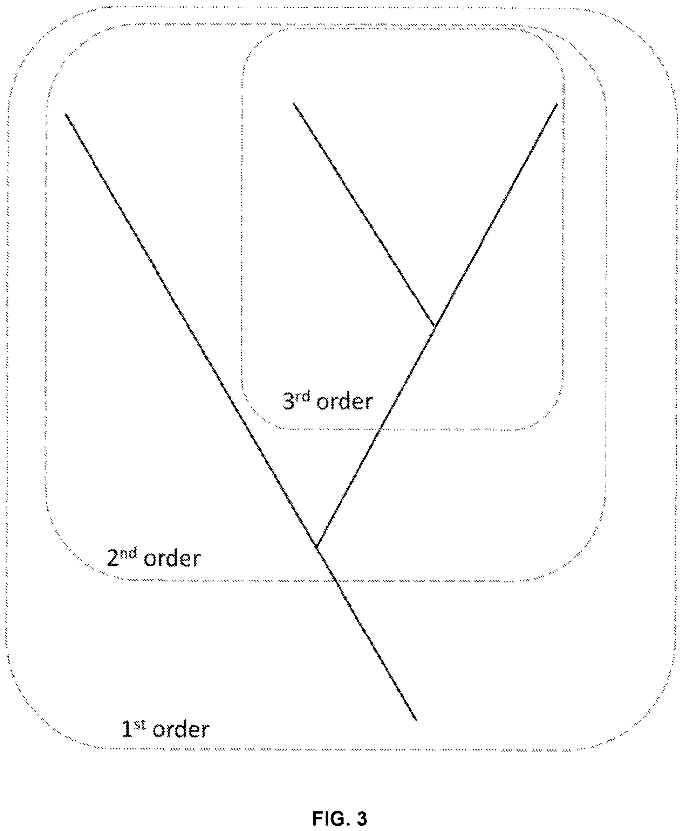

A clade can be described by its order. The order of a clade can be a description of how many clades it subsumes. A clade of order X can subsume clades of order X+1. For example, a clade of order 1 can subsume clades of order 2, 3, 4, etc., and a clade of order 2 can subsume clades of order 3, 4, 5, etc.. An illustration of a first order clade, a second order clade, and a third order clade in the same phylogenic tree is provided in **FIG. 3****.** Here, the first order clade subsumes the second order clade, and the second order clade subsumes the third order clade.

Likewise, a clade of order Y can be subsumed by a clade of order Y-1. For example, a clade of order 5 can be subsumed by a clade of order 4, a clade of order 6 can be subsumed by a clade of order 5, and a clade of order 7 can be subsumed by a clade of order 6, etc.

If a clade is an X-order clade, then the X-order clade may be phylogenetically below X-1 branch nodes in a phylogenic tree of the microbe. For example, a clade of order 5 can be phylogenetically below 4 branch nodes, a clade of order 6 can be phylogenetically below 5 branch nodes, and a clade of order 7 can be phylogenetically below 6 branch nodes.

A clade of a microbe can be first order, second order, third order, fourth order, fifth order, sixth order, seventh order, eighth order, ninth order, tenth order, eleventh order, twelfth order, thirteenth order, fourteenth order, fifteenth order, or any other suitable order. If a clade is a first order clade, then that clade can be not be subsumed in whole or in part within another higher level clade.

For example, flu subtypes (e.g., influenza type A) can be broken down into different genetic clades and sub-clades. A flu clade can be a subdivision of flu viruses based on the similarity of a gene sequence of the flu viruses (e.g., hemagglutinin sequences).

As another example, HIV subtypes (e.g., HIV-1 and/or HIV-2) can be broken down into different genetic clades and sub-clades. An HIV clade can be a subdivision of HIV viruses based on the similarity of a gene sequence of the HIV viruses (e.g., the *env* gene, which encodes the gp160 protein).

### II. Antigens

A vaccine composition as described herein can comprise antigens. Antigens in a vaccine composition can be of a microbe or a strains of a microbe that the vaccine composition can elicit an immune response against.

An antigen can be a molecule that can be recognized by the immune system, which can include antibodies, B cells and/or T cells. For example, an antigen can be a foreign substance that can induce an immune response. In some cases, the induced immune response can comprise the production of antibodies. In some cases, an antibody can recognize, attach to, or bind to an antigen.

An antigen can be any antigenic determinant on an immunogen, for example, any primary immunogen, to which an antibody binds through an antigenic binding site. Determinants or antigenic determinants on an antigen generally consist of chemically active surface groupings of molecules such as amino acids or sugar side chains and generally have specific three-dimensional structural characteristics, as well as specific charge characteristics.

An antigen can be a neoantigen, an autoantigen, an endogenous antigen, an exogenous antigen, a viral antigen, a bacterial antigen, a fungal antigen, a parasitic antigen, a toxin, or a tumor antigen. Some vaccines can comprise antigens from one source, while some vaccines can comprise antigens from two, three, four, or more sources.

A vaccine can comprise an antigen of a strain in Table 1. Some vaccines can comprise one or more homologues of one or more antigens of strains in Table 1. In some cases, such a homologue can comprise at least 80%, at least 85%, at least 90%, at least 95%, or at least 99% sequence identity to an antigen in Table 1.

An antigen can be a flu antigen. In some cases, a flu antigen can be hemagglutinin or neuraminidase. In some cases, a flu antigen can be a fragment of, a derivative of, or a modified hemagglutinin or neuraminidase. In certain cases, a vaccine can comprise a combination of hemagglutinin and neuraminidase, or fragments, derivatives, or modified versions thereof. In various cases, an antigen can be a hemagglutinin-neuraminidase combined protein, or a fragment, derivative, or modified version thereof.

Hemagglutinin can be a homotrimeric glycoprotein that can be found on the surface of a flu virus. Hemagglutinin can be a class 1 fusion protein, and can have multifunctional activity as an attachment factor and/or a membrane fusion protein. In some cases, hemagglutinin can play a role in binding a flu virus to sialic acid on the surface of a target or host cell. After this binding, a flu virus can be internalized. In some cases, hemagglutinin can play a role in the fusion of the viral envelope of a flu virus with a late endosomal membrane in a low pH (e.g., 5.0-5.5) environment.

Hemagglutinin can be of a type A or a type B flu virus. Hemagglutinin can have a structure comprising a head and a stem, and can comprise three identical monomers. A monomer can comprise an intact HA0 single polypeptide chain with HA1 and HA2 regions that can be linked by two disulfide bridges. An HA2 region can have an alpha helical coiled coil structure, and can sit on top of an HA1 region. An HA1 region can be a small globular domain that can comprise a mix of α/β structures.

A vaccine composition can comprise a head of hemagglutinin, a stem of hemagglutinin, or a combination or fragment thereof. In some cases, a vaccine composition can comprise a broadly conserved fragment of hemagglutinin, a broadly conserved fragment of a head of hemagglutinin, a broadly conserved fragment of a stem of hemagglutinin, or a combination thereof. A broadly conserved sequence can be an amino acid sequence or a nucleic acid sequence that can be identical or similar across species or strains of a microbe.

A vaccine composition can comprise an HA0 region, an HA1, an HA2, region, or a combination or fragment thereof. A hemagglutinin antigen can be of a subtype of hemagglutinin. For example, hemagglutinin in a type A flu virus hemagglutinin antigen can be of 18 or more subtypes. Subtypes of hemagglutinin can be H1, H2, H3, H4, H5, H6, H7, H8, H9, H10, H11, H12, H13, H14, H15, H16, H17, or H18.

Some vaccine compositions can comprise at least 5, at least 10, at least 20, or at least 30 influenza virus hemagglutinin antigen polypeptides. Such a hemagglutinin antigen polypeptide can be represented by a sequence (e.g., an amino acid sequence) that is at least 20% identical, but not more than 95% identical, to the other polypeptides in the vaccine, wherein each polypeptide comprises a target segment that is at least 90% identical among the influenza virus hemagglutinin antigen polypeptides. In some cases, such a hemagglutinin antigen polypeptide can be represented by a sequence (e.g., an amino acid sequence) that is at least 20% identical, but not more than 95% identical, to the other polypeptides in the vaccine. In certain cases, such a hemagglutinin antigen polypeptide can be represented by a sequence (e.g., an amino acid sequence) that is at least 10% identical, but not more than 95% identical, to the other polypeptides in the vaccine. In various cases, such a hemagglutinin antigen polypeptide can be represented by a sequence (e.g., an amino acid sequence) that is at least 20% identical, but not more than 98% identical, to the other polypeptides in the vaccine. In some cases, such a hemagglutinin antigen polypeptide can be represented by a sequence (e.g., an amino acid sequence) that is at least 10% identical, but not more than 98% identical, to the other polypeptides in the vaccine.

Neuraminidase can be a protein found on the surface of a flu virus. Neuraminidase can enable flu virus to be released from a host cell. Neuraminidase can be a member of the glycoside hydrolase family 34. Neuraminidase can be a mushroom shaped protein. Neuraminidase can comprise a head portion, which can comprise four co-planar and roughly spherical subunits and a hydrophobic region. In some cases, the hydrophobic region can be embedded within the interior of a viral membrane. Neuraminidase can comprise a single polypeptide chain. A neuraminidase polypeptide can be a single chain of six conserved polar amino acids, which can be followed by variable amino acids.

A vaccine can comprise a spherical subunit of neuraminidase, a hydrophobic region of neuraminidase, or a combination thereof. In some cases, a vaccine composition can comprise a broadly conserved fragment of neuraminidase, a broadly conserved fragment of a spherical subunit of neuraminidase, a broadly conserved fragment of a hydrophobic region of neuraminidase, or a combination thereof.

In some cases, a neuraminidase antigen can be of a subtype of neuraminidase. A flu virus neuraminidase antigen can be of 11 or more subtypes. Subtypes of neuraminidase can be N1, N2, N3, N4, N5, N6, N7, N8, N9, N10, or N11. In some cases, a neuraminidase antigen can be of a virus that is not a flu virus. In some cases, a neuraminidase can be of a bacterium. For example, neuraminidase can be of *Bacteroides fragilis* or *Pseudomonas aeruginosa* or another bacterium.

Some vaccine compositions can comprise at least 5, at least 10, at least 20, or at least 30 influenza virus neuraminidase antigen polypeptides. Such a neuraminidase antigen polypeptide can be represented by a sequence (e.g., an amino acid sequence) that is at least 20% identical, but not more than 95% identical, to the other polypeptides in the vaccine, wherein each polypeptide comprises a target segment that is at least 90% identical among the influenza virus neuraminidase antigen polypeptides. In some cases, such a neuraminidase antigen polypeptide can be represented by a sequence (e.g., an amino acid sequence) that is at least 20% identical, but not more than 95% identical, to the other polypeptides in the vaccine. In certain cases, such a neuraminidase antigen polypeptide can be represented by a sequence (e.g., an amino acid sequence) that is at least 10% identical, but not more than 95% identical, to the other polypeptides in the vaccine. In various cases, such a neuraminidase antigen polypeptide can be represented by a sequence (e.g., an amino acid sequence) that is at least 20% identical, but not more than 98% identical, to the other polypeptides in the vaccine. In some cases, such a neuraminidase antigen polypeptide can be represented by a sequence (e.g., an amino acid sequence) that is at least 10% identical, but not more than 98% identical, to the other polypeptides in the vaccine.

An antigen can be an HIV antigen. In some cases, an HIV antigen can be the glycoprotein gp160. In some cases, an HIV antigen can be a fragment of, a derivative of, or a modified gp160. In certain cases, a vaccine can comprise a combination of gp160, or fragments, derivatives, or modified versions thereof.

Gp160 can be a protein encoded by the *env* gene in the HIV virus that can form a homotrimer and can be found on the surface of an HIV virus. Gp160 can be cleaved into gp120 and gp41 by a protease such as furin in a subject. Gp120 and gp41 can be transported to the plasma membrane of the host cell of the subject, where gp41 can anchor gp120 to the membrane of the infected cell. In some cases, gp120 can be an antigen. In some cases, gp41 can be an antigen.

Gp160 can be of HIV-1 or HIV-2. Gp160 can comprise three subunits that can have an outward facing glycoprotein portion attached to a membrane-associated portion. The interface between GP160 subunits can be polar.

A vaccine composition can comprise a glycoprotein segment of gp160, a membrane-associated portion of gp160, a broadly conserved fragment of gp160, a broadly conserved fragment of a glycoprotein segment of gp160, a broadly conserved fragment of a membrane-associated portion of gp160, or a combination thereof.

Some vaccine compositions can comprise at least 5, at least 10, at least 20, or at least 30 HIV gp160 antigen polypeptides. Such a gp160 antigen polypeptide can be represented by a sequence (e.g., an amino acid sequence) that is at least 20% identical, but not more than 95% identical, to the other polypeptides in the vaccine, wherein each polypeptide comprises a target segment that is at least 90% identical among the gp160 antigen polypeptides. In certain cases, such a gp160 antigen polypeptide can be represented by a sequence (e.g., an amino acid sequence) that is at least 20% identical, but not more than 95% identical, to the other polypeptides in the vaccine. In various cases, such a gp160 antigen polypeptide can be represented by a sequence (e.g., an amino acid sequence) that is at least 10% identical, but not more than 95% identical, to the other polypeptides in the vaccine. In some cases, such a gp160 antigen polypeptide can be represented by a sequence (e.g., an amino acid sequence) that is at least 20% identical, but not more than 98% identical, to the other polypeptides in the vaccine. In certain cases, such a gp160 antigen polypeptide can be represented by a sequence (e.g., an amino acid sequence) that is at least 10% identical, but not more than 98% identical, to the other polypeptides in the vaccine.

Gp120 can be of HIV-1 or HIV-2. Gp120 can comprise three subunits that can have an outward facing glycoprotein portion attached to a membrane-associated portion. The interface between GP120 subunits can be polar.

A vaccine composition can comprise a glycoprotein segment of gp120, a membrane-associated portion of gp120, a broadly conserved fragment of gp120, a broadly conserved fragment of a glycoprotein segment of gp120, a broadly conserved fragment of a membrane-associated portion of gp120, or a combination thereof.

Some vaccine compositions can comprise at least 5, at least 10, at least 20, or at least 30 HIV gp120 antigen polypeptides. Such a gp120 antigen polypeptide can be represented by a sequence (e.g., an amino acid sequence) that is at least 20% identical, but not more than 95% identical, to the other polypeptides in the vaccine, wherein each polypeptide comprises a target segment that is at least 90% identical among the gp120 antigen polypeptides. In certain cases, such a gp120 antigen polypeptide can be represented by a sequence (e.g., an amino acid sequence) that is at least 20% identical, but not more than 95% identical, to the other polypeptides in the vaccine. In various cases, such a gp120 antigen polypeptide can be represented by a sequence (e.g., an amino acid sequence) that is at least 10% identical, but not more than 95% identical, to the other polypeptides in the vaccine. In some cases, such a gp120 antigen polypeptide can be represented by a sequence (e.g., an amino acid sequence) that is at least 20% identical, but not more than 98% identical, to the other polypeptides in the vaccine. In certain cases, such a gp120 antigen polypeptide can be represented by a sequence (e.g., an amino acid sequence) that is at least 10% identical, but not more than 98% identical, to the other polypeptides in the vaccine.

Gp41 can be of HIV-1 or HIV-2. Gp41 can comprise three subunits that can have an outward facing glycoprotein portion attached to a membrane-associated portion. The interface between GP41 subunits can be polar.

A vaccine composition can comprise a glycoprotein segment of gp41, a membrane-associated portion of gp41, a broadly conserved fragment of gp41, a broadly conserved fragment of a glycoprotein segment of gp41, a broadly conserved fragment of a membrane-associated portion of gp41, or a combination thereof.

Some vaccine compositions can comprise at least 5, at least 10, at least 20, or at least 30 HIV gp41 antigen polypeptides. Such a gp41 antigen polypeptide can be represented by a sequence (e.g., an amino acid sequence) that is at least 20% identical, but not more than 95% identical, to the other polypeptides in the vaccine, wherein each polypeptide comprises a target segment that is at least 90% identical among the gp41 antigen polypeptides. In certain cases, such a gp41 antigen polypeptide can be represented by a sequence (e.g., an amino acid sequence) that is at least 20% identical, but not more than 95% identical, to the other polypeptides in the vaccine. In various cases, such a gp41 antigen polypeptide can be represented by a sequence (e.g., an amino acid sequence) that is at least 10% identical, but not more than 95% identical, to the other polypeptides in the vaccine. In some cases, such a gp41 antigen polypeptide can be represented by a sequence (e.g., an amino acid sequence) that is at least 20% identical, but not more than 98% identical, to the other polypeptides in the vaccine. In certain cases, such a gp41 antigen polypeptide can be represented by a sequence (e.g., an amino acid sequence) that is at least 10% identical, but not more than 98% identical, to the other polypeptides in the vaccine.

An antigen can be broadly neutralizing. A broadly neutralizing antigen can be identified by a broadly neutralizing antibody. A broadly neutralizing antibody can be an antibody which can affect multiple strains of a virus. Some broadly neutralizing antigens can be of at least 80%, 85%, 90%, 95%, 98%, 99%, or 100% of strains.

A broadly neutralizing antigen can comprise surface exposed residues adjacent in tertiary space. That is, a broadly neutralizing antigen can be neutralizing based on its surface properties. In some cases, a broadly neutralizing antigen can comprise neutralizing residues on a non-surface region of the antigen, such as in a pocket, in an active site, or in the interior of an antigen.

An antigen, such as a broadly neutralizing antigen, can be of a flu virus. Such a broadly neutralizing antigen can be located on hemagglutinin. Such a broadly neutralizing antigen can be in a stem of hemagglutinin, in a head of hemagglutinin, or in a portion thereof. In some cases, a broadly neutralizing antigen can be similar to a stem of a hemagglutinin, head of hemagglutinin, or similar to a portion thereof. In some cases, such a portion can be broadly neutralizing. For example, a broadly neutralizing antigen can be at least about 60% similar to, at least about 70% similar to, at least about 80% similar to, at least about 85% similar to, or at least about 90% similar to a head of a hemagglutinin or a stem of a hemagglutinin. In some cases, a broadly neutralizing antigen can be at least about 60% similar to, at least about 70% similar to, at least about 80% similar to, at least about 85% similar to, or at least about 90% similar to a portion of a head of a hemagglutinin or a portion of a stem of a hemagglutinin. In some cases, a broadly neutralizing antigen can be at least about 60% similar to, at least about 70% similar to, at least about 80% similar to, at least about 85% similar to, or at least about 90% similar to a broadly conserved fragment of hemagglutinin, a broadly conserved fragment of a head of a hemagglutinin or a broadly conserved fragment of a stem of a hemagglutinin.

In some cases, a broadly neutralizing antigen can be located on neuraminidase or a portion thereof. In some cases, a broadly neutralizing antigen can be similar to neuraminidase. For example, a broadly neutralizing antigen can be at least about 60% similar to, at least about 70% similar to, at least about 80% similar to, at least about 85% similar to, or at least about 90% similar to neuraminidase or a portion thereof. In some cases, such a portion can be broadly neutralizing. In some cases, a broadly neutralizing antigen can be at least about 60% similar to, at least about 70% similar to, at least about 80% similar to, at least about 85% similar to, or at least about 90% similar to a portion of neuraminidase. In some cases, a broadly neutralizing antigen can be at least about 60% similar to, at least about 70% similar to, at least about 80% similar to, at least about 85% similar to, or at least about 90% similar to a broadly conserved fragment of neuraminidase.

A broadly neutralizing antigen can be of an HIV virus. In some cases, a broadly neutralizing antigen can be located on gp160 or a portion thereof. In some cases, a broadly neutralizing antigen can be similar to gp160. For example, a broadly neutralizing antigen can be at least about 30% similar to, at least about 40% similar to, at least about 50% similar to, at least about 60% similar to, at least about 70% similar to, at least about 80% similar to, at least about 85% similar to, or at least about 90% similar to gp160 or a portion thereof. In certain cases, such a portion can be broadly neutralizing. In various cases, a broadly neutralizing antigen can be at least about 60% similar to, at least about 70% similar to, at least about 80% similar to, at least about 85% similar to, or at least about 90% similar to a portion of gp160. In some cases, a broadly neutralizing antigen can be at least about 60% similar to, at least about 70% similar to, at least about 80% similar to, at least about 85% similar to, or at least about 90% similar to a broadly conserved fragment of gp160.

A broadly neutralizing antigen can be of an HIV virus. In some cases, a broadly neutralizing antigen can be located on gp120 or a portion thereof. In some cases, a broadly neutralizing antigen can be similar to gp120. For example, a broadly neutralizing antigen can be at least about 30% similar to, at least about 40% similar to, at least about 50% similar to, at least about 60% similar to, at least about 70% similar to, at least about 80% similar to, at least about 85% similar to, or at least about 90% similar to gp120 or a portion thereof. In certain cases, such a portion can be broadly neutralizing. In various cases, a broadly neutralizing antigen can be at least about 30% similar to, at least 40% similar to, at least 50% similar to, at least 60% similar to, at least about 70% similar to, at least about 80% similar to, at least about 85% similar to, or at least about 90% similar to a portion of gp120. In some cases, such a portion can be a broadly conserved fragment.

A broadly neutralizing antigen can be of an HIV virus. In some cases, a broadly neutralizing antigen can be located on gp41 or a portion thereof. In some cases, a broadly neutralizing antigen can be similar to gp41. For example, a broadly neutralizing antigen can be at least about 30% similar to, at least about 40% similar to, at least about 50% similar to, at least about 60% similar to, at least about 70% similar to, at least about 80% similar to, at least about 85% similar to, or at least about 90% similar to gp41 or a portion thereof. In certain cases, such a portion can be broadly neutralizing. In various cases, a broadly neutralizing antigen can be at least about 60% similar to, at least about 70% similar to, at least about 80% similar to, at least about 85% similar to, or at least about 90% similar to a portion of gp41. In some cases, such a portion can be a broadly conserved fragment.

In some cases, a vaccine can comprise antigens corresponding to more than one microbe. Some vaccines can comprise antigens against two, three, four, five, six, or more microbes. Antigens can each be present in the vaccine at a lower dose than in currently available vaccines. The combination of diverse antigens can allow for B cells and/or T cells which can broadly identify many strains of the microbe, even strains which are not represented in the vaccine. In some cases, this can be referred to as a swarm effect. The swarm effect can reduce the amount of dose of each antigen as well as reduce the total antigen dose required to elicit an immune response. For example, antigens representative of a particular number of clades of a given order of a clade of a microbe can be included in a vaccine. Including representatives of diverse clades may allow the vaccine to be effective against evolutionarily distinct strains. As another example, antigens representative of a particular amount of operational taxonomic units (OTUs) of a microbe can be included in a vaccine. An OTU can be used to classify groups of closely related entities, such as antigens.

An OTU can be a group of microbes. Microbes in a given OTU can be closely related. An overview of OTUs can be found in Sokal, et al. (Principles of Numerical Taxonomy. W.H. Freeman and Co., San Francisco and London (1963)) and Schloss, et al. (Applied and Environmental Microbiology, Oct. 2006, p. 6773-6779, Vol. 72, No. 10).

An OTU can refer generally to a cluster of antigens grouped by sequence similarity, and can sometimes be a pragmatic proxy for "species" at different taxonomic levels. Administering to a subject a vaccine comprising antigens representing clades of a variety of OTUs can confer immunity across a genetically diverse set of antigens.

In some cases, an OTU can be based on a particular nucleic acid or amino acid sequence of a microbe. For example, an OTU can be based on a sequence of 16S rRNA or a sequence of a gene encoding 16S rRNA. Such an OTU can be referred to as a 16S OUT. In various cases, an OTU can be based on a sequence of 18S rRNA or a sequence of a gene encoding 18S rRNA.

An OTU can be a group of microbes having a threshold similarity. For example, an OUT can be a group of microbes having at least 95%, at least 96%, at least 97%, at least 98%, or at least 99% similarity. In some cases, different microbes can have a similarity higher than the threshold. Such microbes can be in a merged OTU, which can contain multiple species. In certain cases, a single species of a microbe can have paralogs that are less similar than the threshold. Such paralogs can be split across two or more OTUs.

Methods to assign OTUs can include rarefaction curves. Such methods can assess species richness and alpha and beta diversity estimators, implicitly assume that OTUs are observations of organisms with negligible error, and/or the number of observations correlates well with a total number of species or monophyletic groups.

In some cases, a cluster can be spurious, for example, due to artifacts, such as read errors and/or chimeras. In certain cases, an OTU can be based on a genetic distance between sequences of microbes within the OTU. In various cases, an OTU can provide an estimate of richness and/or diversity of a microbial population. In some instances, microbes belonging to a given OTU can have structural similarities. In certain instances, microbes belonging to different OTUs can have different structures.

Microbial sequences can be assigned to an OTU using a software or a tool. Examples of such tools include, but are not limited to, the Distance-Based OTU and Richness (DOTUR) tool, a shared OTUs and similarity (SONS) tool, a LIBSHUFF/∫-LIBSHUFF tool, a TreeClimber tool, a UniFrac tool, or an analysis of molecular variance (AMOVA) tool.

A tool can assign a microbial sequence to an OTU based on an input comprising information of a plurality of microbial sequences. Examples of such inputs can include a distance matrix, a phylogenetic tree, and/or a distance matrix. A DOTUR tool can assign sequences to OTUs by using a furthest, average, or nearest neighbor algorithm for one, several, or each distance level. A DOTUR tool can assign OTUs based on a distance matrix input. A SONS tool can calculate collector's curves for estimates of the fraction and richness of OTUs shared between communities. A SONS algorithm can assign OTUs based on an OTU designation input.

A LIBSHUFF/∫-LIBSHUFF tool can use a Cramer-von Mises statistic to test whether structures of two communities are the same, different, or subsets of one another. A LIBSHUFF/J-LIBSHUFF tool can assign OTUs based on a distance matrix input. A TreeClimber tool can implement a parsimony-based test to determine whether the community structures of two or more communities are significantly different. A TreeClimber tool can assign OTUs based on a phylogenetic tree input.

A UniFrac tool can compare phylogenetic distances between pairs of communities to describe the similarity of their structures. A UniFrac tool can assign OTUs based on a phylogenetic tree input. An AMOVA tool can use an analysis of variance-type formulations to determine whether the genetic diversities of two or more community structures are significantly different. An AMOVA tool can assign OTUs based on a distance matrix input.

An OTU can refer generally to a cluster of antigens grouped by sequence similarity, and can sometimes be a pragmatic proxy for "species" at different taxonomic levels. Including representative clades of a variety of OTUs can result in conferred across a genetically diverse set of antigens. Each OTU can comprise microbial sequences which are similar. In some cases, each OTU can comprise microbial sequences which are at least 90%, at least 91%, at least 92%, at least 93%, or at least 94% homologous of one another. In some cases, each OTU can comprise microbial sequences which are at least 95%, at least 96%, at least 97%, at least 98%, at least 99% homologous of one another.

In an even further example, a set of antigens derived from a library of variants such that the set of antigens broadly represents the library of variants can be included. In some cases, a set of antigens derived from a library of variants such that the set of antigens broadly represents at least 80%, at least 90%, at least 95%, at least 96%, at least 97%, at least 98%, at least 99%, at least 99.5%, or at least 99.9% of the library of variants can be included. In some cases, such a set of antigens can additionally represent one or more variants not included in a library of variants.

An antigen can be a single peptide or protein of interest or a complex of two, three, four, five, six, or more peptides and/or proteins. In some cases, an antigen can refer to an antigen domain, or a region or section of a larger protein or protein complex. In some cases, an antigen can refer to two or more antigen domains or two or more regions or sections of a larger protein or protein complex. In some cases, an antigen may be a whole protein, a fragment of a whole protein, a functional fragment of a whole protein, a peptide, a multimer polypeptide, a polypeptide sequence, or a combination thereof. In some cases, an antigen can be modified (e.g., artificially modified). Such modification can stabilize or improve the antigenicity. Examples of modifications include alterations in a polypeptide sequence (e.g., truncation, lengthening, or mutating). In some cases, an antigen may comprise two or more whole proteins, fragments of whole proteins, functional fragments of whole proteins, peptides, or multimer polypeptides. Sometimes, an antigen can comprise a lipid, a nucleic acid, a polysaccharide, or a combination thereof.

An antigen can be an intact (i.e., an entire or whole) antigen, or a functional portion of an antigen. An antigen can be a peptide functional portion of an antigen. "Intact," as used herein, generally refers to a full length antigen as that antigen polypeptide occurs in nature. An intact antigen may adopt the native protein fold as seen in nature, presenting both primary sequence epitopes as well as tertiary sequence conformational epitopes. This can be in direct contrast to delivery of only a small portion or peptide of the antigen. Delivering an intact antigen to a cell can elicit a broad spectrum immune response, rather than an immune response to just a single or select few epitopes. In some cases, one, several, or all of the epitopes on an antigen can be unknown at the time of developing a vaccine or library.

Alternatively, an antigen can be divided into parts, depending on the size of the initial antigen. Typically, where a whole antigen is a multimer polypeptide, the whole protein can be divided into sub-units and/or domains where each individual subunit or domain of the antigen can be associated with the polymer according to the methods as disclosed herein. In some cases, parts of an antigen can be functional fragments or parts.

A vaccine can comprise a homolog of an antigen (e.g., a hemagglutinin homolog, a neuraminidase homolog, a gp160 homolog, a gp120 homolog, or a gp41 homolog). A homolog can be a molecule having a shared ancestry with an antigen. In some cases, a homolog of an antigen can be an antigen.

In some cases, an antigen can be modified. A modification can comprise a truncation, a tag, an addition of amino acids, glycosylation, methylation, ubiquitination, insertion, deletion, mutation, or other modification. For example, HIV env-Glycoprotein Complex (trimer) can be usually artificially mutated e.g. by disulfide bonds that can keep the antigen stable and/or prevent or delay dissociation of HIV env derived proteins. This can include but is not limited to artificial cleaving sites, removal of entire functional regions (e.g. cutting of a transmembrane region)

In some cases, an antigen can be present at a lower concentration than in currently available vaccines. In some cases, an antigen can be present at a concentration which would be insufficient to induce an immune response if the antigen were administered alone. In some cases, each of the antigens in a vaccine can be present at concentrations which would be insufficient for any of the antigens to induce an immune response if the antigens were administered alone. In such cases, the total antigen concentration, rather than the concentrations of the individual antigens, can be a determining factor for whether or not an immune response is induced. When the total concentration of antigens is a determining factor, the required concentrations of individual antigens can be a function of the total concentration or a function of the number of antigens.

In some cases, one or more antigens can provide immunity in a subject against a broad plurality of variants of a microbe. For example, an antigen can provide immunity to variants of a microbe that are variants based on year of origin, species of origin, specific mutation, or any other suitable variant.

Two antigens in a set of antigens can have an edit distance (e.g., pairwise edit distance). An edit distance can describe the differentness of the two antigens. An edit distance can be a Levenshtein distance. A Levenshtein distance between a first antigen and a second antigen can be the fewest number of edits required to change the first antigen into the second antigen. In the calculation of edit distance, allowed edits can comprise an insertion, deletion, and/or substitution. If the first antigen and the second antigen are identical, the Levenshtein distance can be zero. If the first antigen and the second antigen are the same size, the Levenshtein distance can be at most the size of the first antigen. If the first antigen and the second antigen are different in size, the Levenshtein distance can be at least the difference in size between the first antigen and the second antigen.

Another method of calculating edit distance can be the Longest Common Subsequence method, wherein insertion and deletion can be the only two allowed edits for calculation. Yet another method of calculating edit distance can be the Hamming distance, wherein only antigens of same size may be used.

A smallest edit distance between two or more antigens can be determined. In some cases, the smallest edit distance can be the smallest edit distance between any two antigens of the two or more antigens. The smallest edit distance can be a measure of the similarity of the two or more most similar antigens in a set.

A largest edit distance between two or more antigens can be determined. In some cases, the largest edit distance can be the largest edit distance between any two antigens of the two or more antigens. The largest edit distance can be a measure of the similarity of the two or more most different antigens in a set.

Both the smallest pairwise edit distance between two or more antigens in a set and the largest pairwise edit distance between two or more antigens in a set can be measured or described. The combination of both edit distances can describe the most and least similar antigens in a set, and in some cases, can be a measure of the bounds of variety of antigens in a set.

An edit distance can be measured as a percentage of the size of the antigens. Herein, the size of the antigens can be the average size of the antigens, the maximum size of the antigens, the minimum size of the antigens, or another measure of the size of the antigens. For example, for an two antigens that are each 100 amino acids in size and have a 5 edit difference, the pairwise edit distance can be represented as 5%.

A vaccine can comprise a set of antigens of a microbe wherein the smallest pairwise edit distance between the two antigens is less than a particular threshold. A vaccine can comprise a set of antigens wherein the largest pairwise edit distance between two of the antigens is at least a different particular threshold. In some cases, requiring minimum and maximum differences in included antigens can help ensure immunity will be conferred across a variety of antigens with minimal bias.

A library or vaccine can be described by the difference between the largest edit distance and the smallest edit distance. Two antigens of the set with the greatest edit distance can have an edit distance called "S." Two antigens of the library with the greatest edit distance can have an edit distance called "L." The difference between "S" and "L" can indicate a range of differentness of the antigens present in a vaccine. A larger difference can indicate a greater range of differentness, or greater variety, of antigens. In some cases, "S" can be at least 60% of "L," 70% of "L," 80% of "L," or 90% of "L." In other words, the most different antigen by edit distance in the vaccine composition can be in the top 10%, 20%, 30%, or 40% of different antigens by edit distance in the library.

The average number of nodes between antigens can be calculated for a given vaccine. In some cases, the average nodes of nodes between each antigen in a set of antigens in a vaccine can be at least 1, at least 3, at least 5, at least 10, at least 15, or at least 20. In some cases, the average number of nodes between each of the antigens can be a function of the number of antigens in a set. Sometimes, the average number of nodes between each of the antigens can be lower if the number of antigens in the set is higher.

Antigens in a vaccine can be compared using the number of nodes separating them on a phylogenic tree. For example, closely related antigens can be separated by fewer nodes than less closely related antigens. When describing the number of nodes separating two antigens, antigens separated by fewer nodes can be more closely related or more similar in structure or in sequence, while antigens separated by more nodes can be less closely related or less similar in structure or in sequence. In some cases, for example, two antigens in a vaccine can be separated by at least 1, at least 3, at least 5, at least 10, at least 15, or at least 20 nodes.

The smallest number of nodes between any 2 antigens can be 1 in some vaccines. The largest number of nodes between any two antigens can be at least 5, at least 10, at least 15, or at least 20. In some vaccines, the smallest number of nodes between any 2 antigens can be 1 and the largest number of nodes between any two antigens can be at least 5. In some vaccines, the smallest number of nodes between any 2 antigens can be 1 and the largest number of nodes between any two antigens can be at least 10. In some vaccines, the smallest number of nodes between any 2 antigens can be 1 and the largest number of nodes between any two antigens can be at least 15. In some vaccines, the smallest number of nodes between any 2 antigens can be 1 and the largest number of nodes between any two antigens can be at least 20. In some vaccines, the smallest number of nodes between any 2 antigens can be less than 5 and the largest number of nodes between any two antigens can be at least 5. In some vaccines, the smallest number of nodes between any 2 antigens can be less than 5 and the largest number of nodes between any two antigens can be at least 10. In some vaccines, the smallest number of nodes between any 2 antigens can be less than 5 and the largest number of nodes between any two antigens can be at least 15. In some vaccines, the smallest number of nodes between any 2 antigens can be less than 5 and the largest number of nodes between any two antigens can be at least 20. In some vaccines, the smallest number of nodes between any 2 antigens can be less than 10 and the largest number of nodes between any two antigens can be at least 10. In some vaccines, the smallest number of nodes between any 2 antigens can be less than 10 and the largest number of nodes between any two antigens can be at least 15. In some vaccines, the smallest number of nodes between any 2 antigens can be less than 10 and the largest number of nodes between any two antigens can be at least 20.

The average number of branches between antigens can be calculated for a given vaccine. In some cases, the average number of branches between each antigen in a set of antigens in a vaccine can be at least 1, at least 3, at least 5, at least 10, at least 15, or at least 20. In some cases, the average number of branches between each of the antigens can be a function of the number of antigens in a set. Sometimes, the average number of branches between each of the antigens can be lower if the number of antigens in the set is higher.

Antigens in a vaccine can be compared using the number of branches separating them on a phylogenic tree. For example, closely related antigens can be separated by fewer branches than less closely related antigens. When describing the number of branches separating two antigens, antigens separated by fewer branches can be more closely related or more similar in structure or in sequence, while antigens separated by more branches can be less closely related or less similar in structure or in sequence. In some cases, for example, two antigens in a vaccine can be separated by at least 1, at least 3, at least 5, at least 10, at least 15, or at least 20 branches.

The smallest number of branches between any 2 antigens can be 1 in some vaccines. The largest number of branches between any two antigens can be at least 5, at least 10, at least 15, or at least 20. In some vaccines, the smallest number of branches between any 2 antigens can be 1 and the largest number of branches between any two antigens can be at least 5. In some vaccines, the smallest number of branches between any 2 antigens can be 1 and the largest number of branches between any two antigens can be at least 10. In some vaccines, the smallest number of branches between any 2 antigens can be 1 and the largest number of branches between any two antigens can be at least 15. In some vaccines, the smallest number of branches between any 2 antigens can be 1 and the largest number of branches between any two antigens can be at least 20. In some vaccines, the smallest number of branches between any 2 antigens can be less than 5 and the largest number of branches between any two antigens can be at least 5. In some vaccines, the smallest number of branches between any 2 antigens can be less than 5 and the largest number of branches between any two antigens can be at least 10. In some vaccines, the smallest number of branches between any 2 antigens can be less than 5 and the largest number of branches between any two antigens can be at least 15. In some vaccines, the smallest number of branches between any 2 antigens can be less than 5 and the largest number of branches between any two antigens can be at least 20. In some vaccines, the smallest number of branches between any 2 antigens can be less than 10 and the largest number of branches between any two antigens can be at least 10. In some vaccines, the smallest number of branches between any 2 antigens can be less than 10 and the largest number of branches between any two antigens can be at least 15. In some vaccines, the smallest number of branches between any 2 antigens can be less than 10 and the largest number of branches between any two antigens can be at least 20.

A vaccine can be described by its sequence identity. As used herein, the terms "identical" or percent "identity," in the context of two or more nucleic acids or polypeptide sequences, generally refer to two or more sequences or subsequences that are the same or have a specified percentage of amino acid residues or nucleotides that are the same.

Alternatively, an indication that two nucleic acid sequences or polypeptides are identical is that the polypeptide encoded by the first nucleic acid is immunologically cross reactive with the antibodies raised against the polypeptide encoded by the second nucleic acid, as described below. Thus, a polypeptide is typically identical to a second polypeptide, for example, where the two peptides differ only by conservative substitutions. Another indication that two nucleic acid sequences are substantially identical is that the two molecules or their complements hybridize to each other under stringent conditions, as described herein.

Alternatively, an indication that two nucleic acid sequences or polypeptides are identical is that the polypeptide encoded by the first nucleic acid is immunologically cross reactive with the antibodies raised against the polypeptide encoded by the second nucleic acid, as described below. Thus, a polypeptide is typically identical to a second polypeptide, for example, where the two peptides differ only by conservative substitutions. Another indication that two nucleic acid sequences are substantially identical is that the two molecules or their complements hybridize to each other under stringent conditions, as described herein.

Alternatively, an indication that two nucleic acid sequences or polypeptides are identical is that the polypeptide encoded by the first nucleic acid is immunologically cross reactive with the antibodies raised against the polypeptide encoded by the second nucleic acid, as described below. Thus, a polypeptide is typically identical to a second polypeptide, for example, where the two peptides differ only by conservative substitutions. Another indication that two nucleic acid sequences are substantially identical is that the two molecules or their complements hybridize to each other under stringent conditions, as described herein.
In some cases, each antigen in a set can share at least 90%, at least 95%, or at least 99% sequence identity to at least one antigen in the set. In some cases, sequence identity can be a measure of how similar or different the sequences of the antigens within the vaccine are.

The terms "polypeptide," "oligopeptide," "peptide," and "protein" are used interchangeably herein and generally refer to polymers of amino acids of any length. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, etc.), as well as other suitable modifications. It is understood that, because the polypeptides of this disclosure are based upon an antibody, the polypeptides can occur as single chains or associated chains.

"Polynucleotide" or "nucleic acid," as used interchangeably herein, generally refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase. A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may be further modified after polymerization, such as by conjugation with a labeling component. Other types of modifications include, for example, "caps," substitution of one or more of the naturally-occurring nucleotides with an analog, internucleotide modifications such as, for example, those with uncharged linkages (e.g., methyl phosphonates, phosphotriesters, phosphoamidates, carbamates, etc.) and with charged linkages (e.g., phosphorothioates, phosphorodithioates, etc.), those containing pendant moieties, such as, for example, proteins (e.g., nucleases, toxins, antibodies, signal peptides, ply-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotide(s).

Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid supports. The 5' and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars including, for example, 2'-O-methyl-, 2'-O-allyl, 2'-fluoro- or 2'-azido-ribose, carbocyclic sugar analogs, alpha-anomeric sugars, epimeric sugars such as arabinose, xyloses, or lyxoses, pyranose sugars, furanose sugars, sedoheptuloses, acyclic analogs, and abasic nucleoside analogs such as methyl riboside. One or more phosphodiester linkages may be replaced by alternative linking groups. These alternative linking groups include, but are not limited to, embodiments wherein phosphate is replaced by P(O)S("thioate"), P(S)S ("dithioate"), (O)NR₂ ("amidate"), P(O)R, P(O)OR', CO, or CH₂ ("formacetal"), in which each R or R' is independently H or substituted or unsubstituted alkyl (1-20 C) optionally containing an ether (--O--) linkage, aryl, alkenyl, cycloalkyl, cycloalkenyl, or araldyl. Not all linkages in a polynucleotide need be identical. The preceding description applies to all polynucleotides referred to herein, including RNA and DNA.

"Homology," "identity," or "similarity" can refer to sequence similarity between two peptides or between two nucleic acid molecules. Homology, similarity, and identity can each be determined by comparing a position in each sequence which may be aligned for purposes of comparison. When an equivalent position in the compared sequences is occupied by the same base or amino acid, then the molecules are identical at that position; when the equivalent site occupied by the same or a similar amino acid residue (e.g., similar in steric and/or electronic nature), then the molecules can be referred to as homologous (similar) at that position. Expression as a percentage of homology/similarity or identity refers to a function of the number of identical or similar amino acids at positions shared by the compared sequences. A sequence which is "unrelated" or "non-homologous" may share less than 40% identity or less than 25% identity with a sequence of the present disclosure. In comparing two sequences, the absence of residues (amino acids or nucleic acids) or presence of extra residues also decreases the identity and homology/similarity.

The term "homology" describes a mathematically based comparison of sequence similarities which is used to identify genes or proteins with similar functions or motifs. The nucleic acid (nucleotide or oligonucleotide) and amino acid (protein) sequences of the present disclosure may be used as a "query sequence" to perform a search against public databases to, for example, identify other family members, related sequences or homologs. Such searches can be performed using the NBLAST and XBLAST programs (version 2.0) of Altschul, et al. (1990) J. Mol. Biol. 215:403-10. BLAST nucleotide searches can be performed with the NBLAST program, score=100, wordlength=12 to obtain nucleotide sequences homologous to nucleic acid molecules of the disclosure. BLAST amino acid searches can be performed with the XBLAST program, score=50, wordlength=3 to obtain amino acid sequences homologous to protein molecules of the disclosure. To obtain gapped alignments for comparison purposes, Gapped BLAST can be utilized as described in Altschul et al., (1997) Nucleic Acids Res. 25(17):3389-3402. When utilizing BLAST and Gapped BLAST programs, the default parameters of the respective programs (*e.g*., XBLAST and BLAST) can be used (*see,* www.ncbi.nlm.nih.gov).

As used herein, "identity" can mean the percentage of identical nucleotide or amino acid residues at corresponding positions in two or more sequences when the sequences are aligned to maximize sequence matching, i.e., taking into account gaps and insertions. Identity can be readily calculated by known methods including, but not limited to, those described in Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991; and Carillo, H., and Lipman, D., SIAM J. Applied Math., 48: 1073 (1988). Methods to determine identity are designed to give the largest match between the sequences tested. Moreover, methods to determine identity are codified in publicly available computer programs. Computer program methods to determine identity between two sequences include, but are not limited to, the GCG program package (Devereux, J., et al., Nucleic Acids Research 12(1): 387 (1984)), BLASTP, BLASTN, and FASTA (Altschul, S. F. et al., J. Molec. Biol. 215: 403-410 (1990) and Altschul et al. Nuc. Acids Res. 25: 3389-3402 (1997)). The BLAST X program is publicly available from NCBI and other sources (BLAST Manual, Altschul, S., et al., NCBI NLM NIH Bethesda, Md. 20894; Altschul, S., et al., J. Mol. Biol. 215: 403-410 (1990). The well-known Smith Waterman algorithm may also be used to determine identity.

Ranges of desired degrees of sequence identity are from about 80% to about 100% and integer values therebetween. In general, this disclosure encompasses sequences with about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, or about 99% sequence identity with any sequence provided herein.

The letter "X" as used in amino acid sequences herein is intended to indicate that any of the twenty standard amino acids may be placed at this position unless specifically noted otherwise.

### III. Vaccines

A vaccine can be a composition that can stimulate the immune system. This can involve stimulation of the production of antibodies and can provide immunity against one or more microbes. A vaccine can be prepared from a microbe, which can be a causative agent of a disease, its products, or a synthetic substitute thereof. Examples of microbes are provided above.

A vaccine can be prepared as a pharmaceutical composition. A pharmaceutical composition can be a formulation of a vaccine, including antigens, in a form that can be administered to a subject. In some cases, a pharmaceutical composition can be ready to use. A pharmaceutical composition can be optimized for ease of use, minimization of adverse reactions, ease of storage, a range of storage temperatures, ease of dosing, ease of manufacturing, or any other suitable factor.

A pharmaceutical composition can comprise a vaccine composition as described herein and a pharmaceutically acceptable diluent, adjuvant, excipient, or any combination thereof. In some cases, the pharmaceutical composition can comprise a diluent. A diluent can be a diluting agent. A diluent can serve to make a vaccine less viscous or less dense, to improve its ability to flow, for example, through a needle. In some cases, a diluent can be pre-mixed with the vaccine. In some cases, a diluent can be provided separately from the vaccine and mixed prior to administration.

In some cases, the pharmaceutical composition can comprise an adjuvant. An adjuvant can provide for increased immunogenicity. An adjuvant can provide for slow release of antigen (e.g., the adjuvant can be a liposome) or it can be an adjuvant that is immunogenic in its own right, thereby functioning synergistically with antigens. For example, the adjuvant can be a known adjuvant or other substance that promotes nucleic acid uptake, recruits immune system cells to the site of administration, or facilitates the immune activation of responding lymphoid cells. Adjuvants include, but are not limited to, immunomodulatory molecules (e.g., cytokines), oil and water emulsions, aluminum hydroxide, glucan, dextran sulfate, iron oxide, sodium alginate, Bacto-Adjuvant, synthetic polymers such as poly amino acids and co-polymers of amino acids, saponin, paraffin oil, PS-GAMP and muramyl dipeptide.

In some cases, the adjuvant can be an immunomodulatory molecule. For example, the immunomodulatory molecule can be a recombinant protein cytokine, chemokine, or immunostimulatory agent or nucleic acids encoding cytokines, chemokines, or immunostimulatory agents designed to enhance the immunologic response.

A pharmaceutical composition as provided herein can be packaged in a virus-like particle (VLP). In some cases, a virus-like particle can comprise a vaccine.

In certain cases, the pharmaceutical composition can comprise an excipient. In various cases, an excipient can be an inactive substance that can serve as a vehicle or medium for the vaccine. An excipient can comprise a binder, a coating, a color, a disintegrant, a flavor, a glidant, a lubricant, a preservative, a sorbent, a sweetener, a vehicle, or any combination thereof.

A vaccine composition or pharmaceutical composition herein can be formulated in one of a number of formulations. In some cases, a vaccine can be in the form of an aerosol formulation, an injectable formulation, an oral formulation, or any other suitable formulation.

A vaccine composition as provided herein can be formulated as a unit dose. A unit dose can be the dose of a vaccine which is appropriate for one subject. In some examples, a vaccine can be packaged as a single unit dose. In some other examples, a vaccine can be packaged as multiple unit doses.

An immune response generated in response to a vaccine can depend on the total amount of antigen, rather than the amount of each antigen present. In some cases, each of the antigens can be at a concentration that alone would not provide a significant immune response to the broadly neutralizing antigen in a subject. Such an immune response can comprise recruiting and/or activation if immune cells such as B cells or T cells. In some cases, such an immune response can be a prophylactic immune response. In some cases, the antigens can have a combined concentration that provides an immune response to a broadly neutralizing antigen in the subject.

### IV. Methods of Use

Provided herein are methods for treating or reducing adverse events due to infection and/or exposure to a microbe or toxin. Also provided herein are methods for inhibiting or reducing the likelihood of a disease or condition caused by a microbe or toxin in a subject. The methods can comprise administering to the subject a vaccine composition as described herein. In some cases, methods provided herein can confer immunity to a disease or condition caused by a microbe in a subject.

The terms "treatment" or "treating" are used interchangeably herein. These terms, as used herein, generally refer to an approach for obtaining beneficial or desired results including, but not limited to, a therapeutic benefit and/or a prophylactic benefit. A therapeutic benefit can mean eradication or amelioration of the underlying disorder being treated. Also, a therapeutic benefit can be achieved with the eradication or amelioration of one or more of the physiological symptoms associated with the underlying disorder such that an improvement is observed in the subject, notwithstanding that the subject may still be afflicted with the underlying disorder. A prophylactic effect includes delaying, preventing, or eliminating the appearance of a disease or condition, delaying or eliminating the onset of symptoms of a disease or condition, slowing, halting, or reversing the progression of a disease or condition, or any combination thereof. For prophylactic benefit, a subject at risk of developing a particular disease, or to a subject reporting one or more of the physiological symptoms of a disease may undergo treatment, even though a diagnosis of this disease may not have been made.

A subject administered a vaccine herein can be a mammal, a bird, or any other suitable subject. In some instances, the subject can be a human. In certain instances, the subject can be a pig, or another domesticated animal, for example, a cow, a goat, a sheep, a horse, a chicken, a rooster, a turkey, a parrot, a monkey, a hamster, a guinea pig, a rat, a mouse, a dog, or a cat.

Such a subject can receive the vaccine orally, as an aerosol, as an injection, or a combination thereof. In some cases, more than one dose of the vaccine may be given. In some cases, a subject can receive one, two, three, four, five, six, seven, eight, nine, ten or more doses of a vaccine. Repeated doses can be given on the same day, or they can be spaced by at least 1 day, at least 1 week, at least 1 month, at least 1 year, at least 2 years, at least 3 years, at least 4 years, at least 5 years, or at least 10 years. In some cases, repeated doses can be given as a booster dose, and can be the same dose, a greater dose, or a lesser dose than previous doses. A booster dose can be a primed boost.

Individual doses of a vaccine having repeated doses can comprise the same antigens or different antigens. If individual doses comprise different antigens, they can be different by at least one, two, three, four, five, six, seven, eight, nine, ten or more antigens. In some cases, if individual doses comprise different antigens, the difference can be to maximize exposure to as many different antigens as possible. In some cases, if individual doses comprise different antigens, the additional doses can be "update" doses, which can confer immunity to newly evolved, adapted, or resistant strains.

In certain embodiments, a vaccine which is to be injected can be formulated as a subcutaneous injection. In some cases, a vaccine to be injected can be formulated for intramuscular, intraperitoneal, intravenous, intradermal, or any other suitable type of injection. A vaccine can be administered to a subject who is healthy, a subject who is not healthy, or a subject of unknown health. In some cases, a vaccine can be more effective if the subject is healthy. A vaccine can comprise a recombinant expression vector. In some cases, a vaccine can be made by a recombinant expression vector. In various cases, a vaccine can be stored as a recombinant expression vector.

In some instances, a recombinant expression vector can comprise a nucleic acid molecule encoding a set of antigens that are representative of at least 60% of each of first order clade and second order clades of a microbe; a set of antigens wherein the smallest pairwise edit distance between two of the antigens is no more than 25 and the largest pairwise edit distance between two of the antigens is at least 300; a set of antigens that are representative of at least 60% of all OTUs of the microbe; or a set of antigens that are representative of at least 60% of all OTUs of the microbe.

In certain instances, a recombinant expression vector can comprise a nucleic acid molecule encoding a recombinant expression vector comprising a nucleic acid molecule encoding: a set of antigens that are representative of at least 60% of each of first order clade and second order clades of a microbe; a set of antigens wherein the smallest pairwise edit distance between two of the antigens is no more than 5% of the size of the antigens, and the largest pairwise edit distance is at least 75% of the size of the antigens, a set of antigens that are representative of at least 60% of all OTUs of the microbe; or a set of antigens that are representative of at least 60% of all OTUs of the microbe.

In various instances, a recombinant expression vector can comprise a nucleic acid molecule encoding a set of antigens that are representative of at least 50%, at least 60%, at least 70%, at least 80%, at least 90%, or 100% of each of the first order clades of a microbe. In some cases, a recombinant expression vector can comprise a nucleic acid molecule encoding a set of antigens that are representative of at least 50%, at least 60%, at least 70%, at least 80%, at least 90%, or 100% of each of the second order clades of a microbe. In certain cases, a recombinant expression vector can comprise a nucleic acid molecule encoding a set of antigens that are representative of at least 50%, at least 60%, at least 70%, at least 80%, at least 90%, or 100% of each of the third order clades of a microbe.

In some cases, a recombinant expression vector can comprise a nucleic acid molecule encoding a set of antigens wherein the smallest pairwise edit distance between two of the antigens is no more than 1%, no more than 5%, no more than 10%, no more than 15%, or no more than 20% of the size of the antigens. In some cases, a recombinant expression vector can comprise a nucleic acid molecule encoding a set of antigens wherein the largest pairwise edit distance is at least 60%, at least 70%, at least 80%, at least 90%, at least 95%, or at least 98% of the size of the antigens. In some cases, a recombinant expression vector can comprise a nucleic acid molecule encoding a set of antigens wherein the smallest pairwise edit distance between two of the antigens is no more than 1%, and the largest pairwise edit distance is at least 60%, at least 70%, at least 80%, at least 90%, at least 95%, or at least 98% of the size of the antigens. In certain cases, a recombinant expression vector can comprise a nucleic acid molecule encoding a set of antigens wherein the smallest pairwise edit distance between two of the antigens is no more than 5%, and the largest pairwise edit distance is at least 60%, at least 70%, at least 80%, at least 90%, at least 95%, or at least 98% of the size of the antigens. In various cases, a recombinant expression vector can comprise a nucleic acid molecule encoding a set of antigens wherein the smallest pairwise edit distance between two of the antigens is no more than 10%, and the largest pairwise edit distance is at least 60%, at least 70%, at least 80%, at least 90%, at least 95%, or at least 98% of the size of the antigens. In some cases, a recombinant expression vector can comprise a nucleic acid molecule encoding a set of antigens wherein the smallest pairwise edit distance between two of the antigens is no more than 15%, and the largest pairwise edit distance is at least 60%, at least 70%, at least 80%, at least 90%, at least 95%, or at least 98% of the size of the antigens. In certain cases, a recombinant expression vector can comprise a nucleic acid molecule encoding a set of antigens wherein the smallest pairwise edit distance between two of the antigens is no more than 20%, and the largest pairwise edit distance is at least 60%, at least 70%, at least 80%, at least 90%, at least 95%, or at least 98% of the size of the antigens.

In some cases, a recombinant expression vector can comprise a nucleic acid molecule encoding a set of antigens wherein the smallest pairwise edit distance between two of the antigens is at least 1%, at least 5%, at least 10%, at least 15%, or at least 20% of the size of the antigens. In certain cases, a recombinant expression vector can comprise a nucleic acid molecule encoding a set of antigens wherein the largest pairwise edit distance is no more than 60%, no more than 70%, no more than 80%, no more than 90%, no more than 95%, or no more than 98% of the size of the antigens. In various cases, a recombinant expression vector can comprise a nucleic acid molecule encoding a set of antigens wherein the smallest pairwise edit distance between two of the antigens is at least 1%, and the largest pairwise edit distance is no more than 60%, no more than 70%, no more than 80%, no more than 90%, no more than 95%, or no more than 98% of the size of the antigens. In certain cases, a recombinant expression vector can comprise a nucleic acid molecule encoding a set of antigens wherein the smallest pairwise edit distance between two of the antigens is at least 5%, and the largest pairwise edit distance is no more than 60%, no more than 70%, no more than 80%, no more than 90%, no more than 95%, or no more than 98% of the size of the antigens. In various cases, a recombinant expression vector can comprise a nucleic acid molecule encoding a set of antigens wherein the smallest pairwise edit distance between two of the antigens is at least 10%, and the largest pairwise edit distance is no more than 60%, no more than 70%, no more than 80%, no more than 90%, no more than 95%, or no more than 98% of the size of the antigens. In some cases, a recombinant expression vector can comprise a nucleic acid molecule encoding a set of antigens wherein the smallest pairwise edit distance between two of the antigens is at least 15%, and the largest pairwise edit distance is no more than 60%, no more than 70%, no more than 80%, no more than 90%, no more than 95%, or no more than 98% of the size of the antigens. In certain cases, a recombinant expression vector can comprise a nucleic acid molecule encoding a set of antigens wherein the smallest pairwise edit distance between two of the antigens is at least 20%, and the largest pairwise edit distance is no more than 60%, no more than 70%, no more than 80%, no more than 90%, no more than 95%, or no more than 98% of the size of the antigens.

A method for making a vaccine composition can comprise selecting a set of antigens that are representative of at least 60% of each of the first order clade and second order clades of a microbe, wherein the smallest pairwise edit distance between two of the antigens is no more than 5% of the size of the antigens, and the largest pairwise edit distance is at least 75% of the size of the antigens, representative of at least 60% of all OTUs of the microbe, or representative of at least 90% of all OTUs of the microbe.

In some cases, a method for making a vaccine composition can comprise selecting a set of antigens that are representative of at least 50%, at least 60%, at least 70%, at least 80%, at least 90%, or 100% of each of the first order clades of a microbe. In certain cases, a method for making a vaccine composition can comprise selecting a set of antigens that are representative of at least 50%, at least 60%, at least 70%, at least 80%, at least 90%, or 100% of each of the second order clades of a microbe. In various cases, a method for making a vaccine composition can comprise selecting a set of antigens that are representative of at least 50%, at least 60%, at least 70%, at least 80%, at least 90%, or 100% of each of the third order clades of a microbe.

In some cases, a method for making a vaccine composition can comprise selecting a set of antigens wherein the smallest pairwise edit distance between two of the antigens is no more than 1%, no more than 5%, no more than 10%, no more than 15%, or no more than 20% of the size of the antigens. In certain cases, a method for making a vaccine composition can comprise selecting a set of antigens wherein the largest pairwise edit distance is at least 60%, at least 70%, at least 80%, or at least 90% of the size of the antigens. In various cases, a method for making a vaccine composition can comprise selecting a set of antigens wherein the smallest pairwise edit distance between two of the antigens is no more than 1%, and the largest pairwise edit distance is at least 60%, at least 70%, at least 80%, or at least 90% of the size of the antigens. In some cases, a method for making a vaccine composition can comprise selecting a set of antigens wherein the smallest pairwise edit distance between two of the antigens is no more than 5%, and the largest pairwise edit distance is at least 60%, at least 70%, at least 80%, or at least 90% of the size of the antigens. In certain cases, a method for making a vaccine composition can comprise selecting a set of antigens wherein the smallest pairwise edit distance between two of the antigens is no more than 10%, and the largest pairwise edit distance is at least 60%, at least 70%, at least 80%, or at least 90% of the size of the antigens. In various cases, a method for making a vaccine composition can comprise selecting a set of antigens wherein the smallest pairwise edit distance between two of the antigens is no more than 15%, and the largest pairwise edit distance is at least 60%, at least 70%, at least 80%, or at least 90% of the size of the antigens. In some cases, a method for making a vaccine composition can comprise selecting a set of antigens wherein the smallest pairwise edit distance between two of the antigens is no more than 20%, and the largest pairwise edit distance is at least 60%, at least 70%, at least 80%, or at least 90% of the size of the antigens.

### V. Examples

Vaccines were developed that can, for example, differ from (and/or provide advantages relative to) typical commercial vaccines in at least one of several ways: (1) the inclusion of numerous (e.g., six or more) antigens, for example, 30 antigens, (2) a relatively low amount of each individual antigen, for example, 0.05 µg of each antigen, which can allow for a relatively low amount of total antigen content, and (3) the breadth of immunogenic response elicited by the vaccine against shared sites on the antigens, for example, each of 30 antigens is at 0.05 µg, but shared sites are effectively at a 30× higher dose of 1.5 µg.

For the following examples, flu vaccine compositions were administered to pigs. Several flu vaccine compositions were used. The vehicle comprised squalene and phosphate buffered saline for all compositions. A vehicle control composition comprised squalene and phosphate buffered saline. A bivalent control flu vaccine, designed to mimic commercially available flu vaccines, comprised two flu antigen variants: H1N1_2007 (A/Brisbane/2007) (5 µg) and either H3N2_1997 (A/Sydney/5/1997) or H3N2_2007 (A/Brisbane/2007) (5 µg). A first single antigen flu vaccine composition comprised 50 ng of a first hemagglutinin variant (H1N1_2007; A/Brisbane/2007). A second single antigen flu vaccine composition comprised 50 ng of a second hemagglutinin variant (H3N2_2007; A/Brisbane/2007). A 30-antigen flu vaccine composition comprised 30 flu antigens (30 hemagglutinin variants), representing flu antigens from between 1918 and 2015, selected as described in Example 4. A 27-antigen flu vaccine composition comprised 27 flu antigens (27 hemagglutinin variants), representing flu antigens from between 1918 and 2008. The 30-antigen flu vaccine composition was also employed for general vaccine composition testing. The 27-antigen flu vaccine was employed to test the ability of the vaccine to protect against "future" strains, which might be described as the ability of the vaccine to provide protection against antigen variants which do not exist yet, but may exist in the future.

The following illustrative examples are representative of embodiments of the compositions and methods described herein and are not meant to be limiting in any way.

### Example 1: Quantifying the number of unique antibodies elicited after vaccination

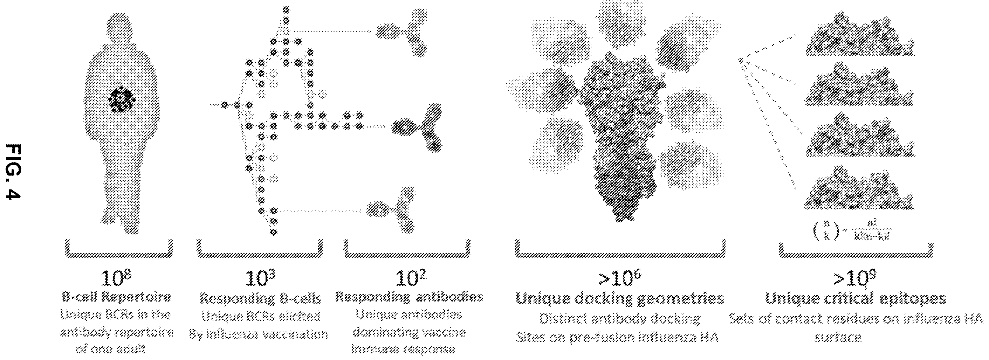

Adult humans can comprise a B cell receptor (BCR) repertoire of about 100 million unique BCRs. After influenza vaccination, about 1,000 unique BCRs can be elicited to begin affinity maturation. Serological analysis indicates that about 10% of these can efficiently convert to productive plasma cells. This can result in a flu shot that can provide serological protection with about 100 unique antibodies. This concept is illustrated in **FIG. 4****.**

### Example 2: Estimating the number of unique B cell epitopes on hemagglutinin

Human antibodies can contact about 20 to 30 residues on the antigen surface they recognize. In some cases, about 12 to 16 such residues can form an epitope that can be referred to as a "potential critical epitope." In some cases, such an epitope can be consequential for maintaining recognition of an antigen by an antibody.

Given roughly 100 unique antibodies from about 1000 B cell lineages elicited by vaccination it was asked: (1) how many unique epitopes existed on the surface of the influenza hemagglutinin protein (HA), (2) given the conservation of those epitopes, what was the probability of an individual antibody to be broadly cross-reactive, and (3) across the roughly 100 unique antibodies, the probability and percentage of a subject's vaccine response to be contain broadly protective antibodies.

In order to generate a database of unique potential critical epitopes on the surface of the influenza hemagglutinin protein (HA), deep computational protein-protein docking of 559 crystallized human antibody structures against a representative HA structure (PDB ID: 3FKU) using Zdock was performed. This resulted in 3,718,346 unique contact residue sets, each containing 25+/-12 HA contact residues. Repeat random subsampling of 14 contact residues from the unique contact residue sets on the HA that could form a potential critical epitope was then performed, resulting in a database of 263,022,674 unique 14-residue potential critical epitopes that could define the binding determinant of a single antibody. While not exhaustive, the database provides the basis for modeling epitope conservation probability of human antibodies binding HAs. This concept is illustrated in **FIG. 4****.**

### Example 3: Quantifying amino acid conservation of all hemagglutinin epitopes

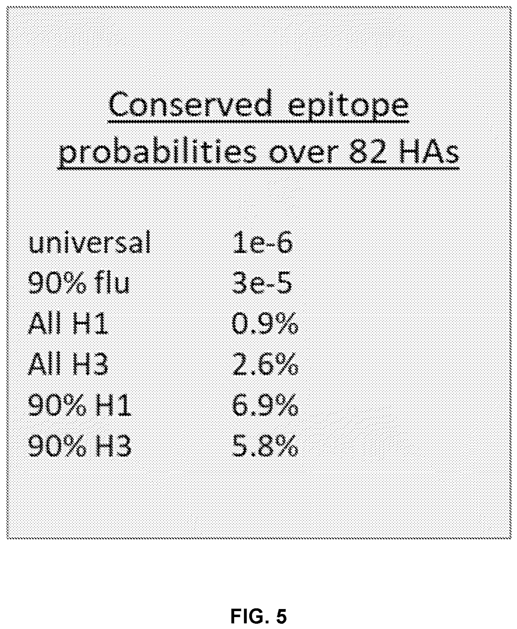

The conservation of the 263 million potential critical epitopes across a panel of 82 HA protein sequences from strains spanning the years 1918-2018 was analyzed. The analysis identified less than one in one million potential critical epitopes that are universally conserved across all HAs. About one in 300,000 potential critical epitopes were observed to be 90% conserved across all strains, while 1% and 3% were observed to be universally conserved within a given HA antigen group, as shown in **FIG. 5****.** Potential critical epitopes that were 90% conserved in H1 or H3 antigen groups were less common (6.9% and 5.8% for H1 and H3, respectively). This can suggest that in some cases, non-broadly conserved epitopes can be immunodominant by virtue of vastly outnumbering broadly conserved epitopes.

The model suggested that truly universally conserved epitope binding antibodies may be too rare to occur in most immune systems, and that broad antibodies recognizing conserved epitopes within a strain can occur, but may represent a minority of responses, and can be unlikely to exist at a concentration sufficient to mediate sufficient protection in following years. For example, given 100 responding antibodies, most subjects are not predicted to elicit antibodies against broadly conserved epitopes. For subjects that can or might be able to elicit antibodies against broadly conserved epitopes given 100 responding antibodies, in most cases that antibody can make up about 1% of their serum response. In such cases, antigen drift between seasons can be enough to render the remaining 99% of the antibody titers of the subject obsolete or nearly obsolete. Combined with natural titer drops that occur over time, such subjects can be eventually left unprotected.

This model was consistent with observations that seasonal variation of about 10%-15% of amino acid positions can be sufficient to disrupt the majority of epitopes against the hemagglutinin, and can lead to the requirement of continual vaccination by current methods.

Further, immunizing with just hemagglutinin stems could provide some benefit, but seasonal variation within stems can continue to render the majority of epitopes obsolete. Variations of the immunogen, including consensus hemagglutinin variants, can provide benefit which can be limited. Enhanced adjuvants would likely not solve the breadth challenge, as they can increase the total number of responding antibodies, and may be able to alter the fundamental critical epitope conservation probability distribution.

While conserved epitopes exist within the stem and head of hemagglutinin, their proportions can be too low to elicit a dominant proportion of an immune response by most subjects, including most humans or most pigs, and there can be a low probability that this can be solved through optimization of the sequence of the immunogen or enhancements of an adjuvant.

### Example 4: Generation of flu vaccine compositions

At present, commercial vaccines generally deliver between about 10-50 µg of total antigen. Such commercial vaccines often include only two antigens (a bivalent vaccine), three antigens (a trivalent vaccine), or four antigens (a quadrivalent vaccine).

To identify an optimally dispersed population of hemagglutinin variants, a method was developed to take as input a large database of homologous variants of a target antigen, and output a stochastically generated subset of optimally dispersed representatives based on amino acid percent identity. Herein, the input was a large database of homologous variants of a hemagglutinin antigen for influenza, and the output was a subset of the antigens which were representative of the input group of antigens as described herein. In this example, two distinct vaccine compositions were designed for administration to subjects.

This method was repeatedly applied for 1000 stochastic iterations to 8600 hemagglutinin variants spanning the time period from 1918 to 2015 to output a set of 30 representative flu antigen variants. These variants comprise the 30 antigen vaccine composition, and are detailed in Table 2. Antigens were purchased from Sino Biological, Inc. (Beijing, China).

**Table 2: 30 antigen vaccine composition**

| **Strain** | **Year** | **Strain Info** |
|---|---|---|
| H1N1 | 1998 | A/swine/Belgium/1998 |
| H1N1 | 1934 | A/Puerto Rico/1934 |
| H1N1 | 2006 | A/Solomon Islands/2006 |
| H1N1 | 1995 | A/Beijing/1995 |
| H1N1 | 1977 | A/USSR/1977 |
| H1N1 | 1976 | A/New Jersey/1976 |
| H1N1 | 2007 | A/Brisbane/2007 |
| H1N1 | 1933 | A/WSN/1933 |
| H1N1 | 2009 | A/California/2009 |
| H2N2 | 1957 | A/Guiyang/1957 |
| H2N2 | 2005 | A/Canada/2005 |
| H3N2 | 2002 | A/Fujian/2002 |
| H3N2 | 1997 | A/Sydney/1997 |
| H3N2 | 2007 | A/Brisbane/2007 |
| H3N2 | 2004 | A/California/2004 |
| H3N2 | 2014 | A/Missouri/2014 |
| H3N2 | 1995 | A/Nanchang/1995 |
| H5N1 | 1997 | A/Hong Kong/1997 |
| H5N1 | 2008 | A/chicken/VietNam/2008 |
| H5N1 | 2010 | A/barnswallow/Hong Kong/2010 |
| H5N1 | 2004 | A/Vietnam/2004 |
| H7N7 | 1975 | A/equine/Kentucky/1975 |
| H7N7 | 2003 | A/Netherlands/2003 |
| H7N9 | 2013 | A/Zhejiang/2013 |
| B | 1988 | B/Yamagata/1988 |
| B | 2012 | B/Utah/2012 |
| B | 2008 | B/Brisbane/2008 |
| H1N1 | 1918 | A/New York/1918 |
| H3N2 | 1968 | A/Hong Kong/1968 |
| H5N1 | 2009 | A/Egypt/2009 |

In some examples, a 27 antigen vaccine composition, designed to be representative of antigen variants spanning the time period from 1918 to 2008, was used. Most of the antigens of this 27 antigen vaccine composition are also included in the 30 antigen vaccine composition. This vaccine composition can be used to determine efficacy of such a composition having antigens representative of an epitope of a microbe against "future antigens," and includes the antigens in Table 3. Antigens were purchased from Sino Biological, Inc. (Beijing, China).

**Table 3: 27 antigen vaccine composition**

| **Strain** | **Year** | **Strain Info** |
|---|---|---|
| H1N1 | 1998 | A/swine/Belgium/1998 |
| H1N1 | 1934 | A/Puerto Rico/1934 |
| H1N1 | 2006 | A/Solomon Islands/2006 |
| H1N1 | 1995 | A/Beijing/1995 |
| H1N1 | 1977 | A/USSR/1977 |
| H1N1 | 1976 | A/New Jersey/1976 |
| H1N1 | 2007 | A/Brisbane/2007 |
| H1N1 | 1933 | A/WSN/1933 |
| H2N2 | 1957 | A/Guiyang/1957 |
| H2N2 | 2005 | A/Canada/2005 |
| H3N2 | 2002 | A/Fujian/2002 |
| H3N2 | 1997 | A/Sydney/1997 |
| H3N2 | 2007 | A/Brisbane/2007 |
| H3N2 | 2004 | A/California/2004 |
| H3N2 | 1995 | A/Nanchang/1995 |
| H5N1 | 1997 | A/Hong Kong/1997 |
| H5N1 | 2008 | A/chicken/VietNam/2008 |
| H5N1 | 2004 | A/Vietnam/2004 |
| H7N7 | 1975 | A/equine/Kentucky/1975 |
| H7N7 | 2003 | A/Netherlands/2003 |
| H1N1 | 1918 | A/New York/1918 |
| H3N2 | 1968 | A/Hong Kong/1968 |
| H1N1 | 1991 | A/Texas/36/1991 |
| H1N1 | 1986 | A/Tawain/01/1986 |
| H1N3 | 1976 | A/duck/NZL/1976 |
| H3N2 | 2003 | A/Wyoming/03/2003 |
| H6N2 | 2000 | A/duck/Shantou/2000 |

In some examples, a vaccine composition comprising 73 flu hemagglutinin antigens from human, swine, and unknown hosts from 1918 through 2018 was used. Antigens in this composition are provided in Table 4. Antigens were purchased from Sino Biological, Inc. (Beijing, China). The amino acid sequences for these antigens are SEQ ID NOs: 1-87, as listed in Table 6.

**Table 4: Flu hemagglutinin antigen vaccine composition**

| **Subtype** | **Host** | **Year** | **Strain Info** |
|---|---|---|---|
| H1N1 | human | 1918 | A/New.York/1/1918 |
| H1N1 | human | 1933 | A/WSN/1933 |
| H1N1 | human | 1934 | A/Puerto.Rico/8/1934 |
| H1N1 | swine | 1935 | A/swine/Ohio/23/1935 |
| H1N1 | swine | 1939 | A/swine/Cambridae/1939.1939 |
| H1N1 | human | 1945 | A/Huston/1945 |
| H1N1 | swine | 1946 | A/H1N1-1946-ACV49556-/Melbourne/1/1946 |
| H1N1 | human | 1957 | A/Denver/1957 |
| H1N1 | human | 1976 | A/New.Jersey/8/1976 |
| H1N1 | human | 1977 | A/USSR/90/1977 |
| H1N1 | human | 1986 | A/Tawain/01/1986 |
| H1N1 | human | 1991 | A/Texas/36/1991 |
| H1N1 | swine | 1995 | A/swine/OMS/2112/1995.1995 |
| H1N1 | human | 1995 | A/Beijing/262/1995 |
| H1N1 | swine | 1996 | A/swine/Eire/89/1996.1996 |
| H1N1 | human | 1999 | A/Taiwan/4845/1999.1999 |
| H1N1 | swine | 2000 | H1N1-2000-swine-AAL87868-/Swine/Minnesota/55551/2000 |
| H1N1 | swine | 2004 | A/swine/Saitama/21/2004.2004 |
| H1N1 | swine | 2004 | A/swine/Kansas/00246/2004 |
| H1N1 | swine | 2004 | A/swine/Denmark/12813-1/2004 |
| H1N1 | swine | 2014 | A/swine/Saskatchewan/SD0056/2014 |
| H1N1 | human | 2005 | A/Iowa/CEID23/2005.2005 |
| H1N1 | human | 2005 | A/Thailand/271/2005.2005/07 |
| H1N1 | swine | 2005 | A/swine/Tainan/46-4/2005 |
| H1N1 | swine | 2005 | A/swine/England/383/2005 |
| H1N1 | swine | 2006 | A/swine/Canada/01093/2006.2006 |
| H1N1 | human | 2006 | A/Solomon.Islands/3/2006 |
| H1N1 | swine | 2006 | A/swine/North Carolina/01169/2006 |
| H1N1 | swine | 2006 | A/swine/Miyazaki/1/2006 |
| H1N1 | human | 2007 | A/Brisbane/59/2007 |
| H1N1 | swine | 2007 | A/swine/England/267/2007 |
| H1N1 | swine | 2009 | A/swine/Korea/VDS3/2009.2009/09 |
| H1N1 | human | 2009 | A/California/07/2009 |
| H1N1 | swine | 2010 | A01049060/2010.2010/11 |
| H1N1 | human | 2011 | A/Wisconsin/28/2011.2011/12 |
| H1N1 | swine | 2011 | A/swine/Hong.Kong/4083/2011.2011/10 |
| H1N1 | human | 2012 | A/Minnesota/14/2012.2012/12 |
| H1N1 | swine | 2012 | A/swine/Belgium/Oostkamp-26/2012.2012/01 |
| H1N1 | swine | 2012 | A/swine/England/7856/2012.2012/02 |
| H1N1 | swine | 2012 | A/swine/England/038712/2012.2012/12 |
| H1N1 | swine | 2013 | AVX47/2013.2013/01 |
| H1N1 | human | 2016 | A/Pavia/65/2016.2016/10 |
| H1N1 | human | 2017 | A/Florida/04/2017 |
| H3N2 | human | 1968 | A/Hong.Kong/1/1968, |
| H3N2 | human | 1985 | A/Guildford/V728/1985 |
| H3N2 | swine | 1995 | A/swine/England/704563/1995.1995/08 |
| H3N2 | human | 1995 | A/Nanchang/933/1995 |
| H3N2 | human | 1997 | A/Sydney/5/1997 |
| H3N2 | swine | 1997 | A/swine/England/90591/1997 |
| H3N2 | swine | 2001 | A/swine/Spain/33601/2001 |
| H3N2 | human | 2002 | A/Fujian/411/2002 |
| H3N2 | human | 2003 | A/Wyoming/03/2003 |
| H3N2 | human | 2004 | A/California/7/2004 |
| H3N2 | swine | 2004 | A/swine/Taiwan/0408/2004 |
| H3N2 | human | 2005 | A/Mexico/InDRE2250/2005 |
| H3N2 | human | 2007 | A/Brisbane/10/2007 |
| H3N2 | human | 2008 | A/Ohio/14/2008 |
| H3N2 | swine | 2010 | A/swine/Denmark/101501-1/2010.2010/08 |
| H3N2 | human | 2011 | A/Delaware/05/2011 |
| H3N2 | swine | 2012 | A01203748/2012.2012/08 |
| H3N2 | swine | 2012 | A/swine/Thailand/CU-P53/2012.2012/01 |
| H3N2 | swine | 2012 | AVX13/2012.2012/01 |
| H3N2 | swine | 2013 | A/swine/Miyazaki/2/2013.2013 |
| H3N2 | swine | 2017 | A01812268/2017.2017/08 |
| H3N2 | human | 2018 | A/Indiana/11/2018 |
| H5N1 | unknown | 1997 | A/Hong.Kong/483/1997 |
| H5N1 | unknown | 2001 | A/Hong.Kong/378.1/2001 |
| H5N1 | unknown | 2003 | A/Beijing/01/2003 |
| H5N1 | unknown | 2004 | A/Vietnam/1194/2004 |
| H5N1 | unknown | 2006 | A/China/2006 |
| H5N1 | unknown | 2007 | A/Egypt/1394-NAMRU3/2007 |
| H5N1 | unknown | 2008 | A/chicken/VietNam/NCVD-016/2008 |
| H5N1 | unknown | 2008 | A/Bangladesh/207095/2008 |
| H5N1 | unknown | 2013 | A/Cambodia/X0123311/2013 |
| H5N1 | unknown | 2015 | A/Egypt/N0001/2015 |
| HAB | human | 1940 | B/Lee/40 |
| HAB | human | 1959 | B/Maryland/1959 |
| HAB | human | 1973 | B/Hong Kong/8/1973 |
| HAB | human | 1986 | B/Ann Arbor/1/1986 |
| HAB | human | 1992 | B/Oita/15/1992 |
| HAB | human | 2001 | B/Hong Kong/22/2001 |
| HAB | human | 2002 | B/Sydney/3/2002 |
| HAB | human | 2004 | B/Hong Kong/CUHK50947/2004 |
| HAB | human | 2006 | B/Kol/583/2006 |
| HAB | human | 2007 | B/Kol/1234/2007 |
| HAB | human | 2007 | B/Kol/1013/2007 |
| HAB | human | 2008 | B/Kol/1373/2008 |

In some examples, a vaccine composition comprising 28 flu neuraminidase antigens from human, swine, and unknown hosts from 1930 through 2019 was used. Antigens in this composition are provided in Table 5. Antigens were purchased from Sino Biological, Inc. (Beijing, China). The amino acid sequences for these antigens are SEQ ID NOs:88-127, as listed in Table 7.

**Table 5: Flu neuraminidase antigen vaccine composition**

| **Strain** | **Host** | **Year** | **Strain Info** |
|---|---|---|---|
| H1N1 | swine | 1930 | A/swine/Iowa/15/1930(H1N1) |
| H1N1 | human | 1936 | A/Henry/1936(H1N1) |
| H1N1 | human | 1943 | A/Iowa/1943(H1N1) |
| H1N1 | human | 1982 | A/Baylor/11515/1982(H1N1) |
| H1N1 | swine | 1993 | A/swine/Denmark/19216B/1993(H1N1) |
| H1N1 | human | 1995 | A/quail/Nanchang/12-340/2000(H1N1) |
| H1N1 | human | 1996 | A/Auckland/6/1996(H1N1) |
| H1N1 | human | 2001 | A/Auckland/606/2001(H1N1) |
| H1N1 | swine | 2006 | A/swine/Canada/01093/2006(H1N1) |
| H1N1 | human | 2008 | A/Arizona/13/2008(H1N1) |
| H1N1 | swine | 2012 | A/swine/Guangxi/NS2176/2012(H1N1) |
| H1N1 | swine | 2016 | A/swine/Alberta/SD0154/2016(H1N1) |
| H1N1 | human | 2017 | A/Idaho/01/2017(H1N1) |
| H1N1 | swine | 2018 | A/Sw/Bulnes/VN1401-P6SP/2018 |
| H3N2 | human | 1968 | A/Albany/18/1968(H3N2) |
| H3N2 | human | 1969 | A/Bilthoven/17938/1969(H3N2) |
| H3N2 | human | 1971 | A/Hong Kong/107/1971(H3N2) |
| H3N2 | swine | 1985 | A/swine/Italy/526/1985(H3N2) |
| H3N2 | human | 1992 | A/Amsterdam/4112/1992(H3N2) |
| H3N2 | human | 1998 | A/Hong Kong/CUHK19579/1998(H3N2) |
| H3N2 | swine | 2000 | A/Swine/Indiana/P12439/00 (H1N2) |
| H3N2 | human | 2005 | A/Australia/NHRC0005/2005(H3N2) |
| H3N2 | swine | 2007 | A/Swine/Spain/SF 12091/2007(H1N2) |
| H3N2 | swine | 2009 | A/swine/Italy/191985/2009(H1N2) |
| H3N2 | swine | 2010 | A/swine/Papenburg/IDT12653/2010(H1N2) |
| H3N2 | swine | 2016 | A/swine/North Carolina/A01668056/2016(H1N2) |
| H3N2 | swine | 2017 | A/swine/Alberta/SD0217/2017 |
| H3N2 | human | 2019 | A/swine/China/JG20/2019 |
| HAB-NA | human | 1936 | HAB-NA-AHZ37009-/B/Nicaragua/AGB2-36 |
| HAB-NA | human | 1940 | HAB-NA-AAA43749-/B/Lee/1940 |
| HAB-NA | human | 1959 | HAB-NA-AAA43735-/B/Maryland/59 |
| HAB-NA | human | 1973 | HAB-NA-BAA03412-/B/Kanagawa/73 |
| HAB-NA | human | 1994 | HAB-NA-AAU94767-/B/Nanchang/560/94 |
| HAB-NA | human | 1998 | HAB-NA-ABQ53764-/B/Siriraj/09/98 |
| HAB-NA | human | 2001 | HAB-NA-AAN39781-/B/Brazil/952/2001 |
| HAB-NA | human | 2005 | HAB-NA-AGZ60070-/B/Christchurch/38/2005 |
| HAB-NA | human | 2006 | HAB-NA-AEG20998-/B/Kol/515/2006 |
| HAB-NA | human | 2006 | HAB-NA-AEA51357-/B/Kol/583/2006 |
| HAB-NA | human | 2006 | HAB-NA-AEA51355-/B/Kol/542/2006 |
| HAB-NA | human | 2007 | HAB-NA-ABX71686-/B/Guangzhou/01/2007 |

Additional amino acid sequences for antigens that can be included in a vaccine are provided as SEQ ID NOs:128-171, as listed in Table 8.

### Example 5: Hemagglutinin inhibition assay

A hemagglutinin inhibition assay can be used to measure an immune response conferred by a vaccine. A sample protocol for a hemagglutinin inhibition assay can begin with the preparation of a serial dilution of a virus, and can include the preparation of at least one negative control or positive control sample, or both a negative control sample and a positive control sample. Antibodies which might be against a virus, for example, in a serum sample, can be incubated with the virus. Red blood cells can then be added to each sample and incubated, and hemagglutinin can be observed. In some cases, the absence of a button can be considered a positive response. In some cases, a titer can be calculated and reported.

### Example 6: Influenza neutralization assay

A sample protocol for an influenza neutralization assay can begin with the preparation of a serial dilution of an antibody or a solution comprising an antibody, for example, serum. Provided next is a sample method for producing such a serial dilution. The antibody dilutions can be prepared using a virus diluent. A virus diluent can be added to wells of a microtiter plate, such as a 96-well plate. In some cases, 50 µL of virus diluent can be added in each well. Then, 50 µL of the antibody or solution comprising an antibody can be added to the first well. In some cases, this can be a 1 mg/mL stock solution. In some cases, this can be serum. In some cases, this can be diluted serum. 2-fold serial dilutions can be accomplished by transferring 50 µL from the first well to each successive well to accomplish dilutions ranging from 1: 1 to 1:128 (antibody solution: diluent). In addition, negative control wells comprising virus diluent but no antibody or solution comprising an antibody can be prepared. The last 50 µL may be discarded. In some cases, this can be done in replicates, for example, of 1, 2, 3, 4, 5, 6, 7, 8, 9, or 10.

The plate can be covered and placed in an incubator. In some cases, the conditions can be about 37 °C with about 5% CO₂. In some cases, the conditions can be exactly 37 °C with about 5% CO₂. In some cases, the conditions can be about 37 °C with exactly 5% CO₂. In some cases, the conditions can be exactly 37 °C with exactly 5% CO₂. The plate can be held in the incubator during preparation of the diluted virus. In some cases, pH should be maintained to avoid deleterious pH effects on the virus when it is added.

The virus can be diluted in virus diluent to a working dilution, which can be 100 TCID₅₀ / 50 µL. TCID₅₀ can be the 50% tissue culture infective dose, and 100 TCID₅₀ can be equal to 100 times the value of TCID₅₀.

50 µL of diluted virus can be added to wells containing antibodies and control wells having no antibodies. In some cases, additional control wells having antibody or solution containing antibody, but no virus, can be prepared. In some cases, a row of wells can be reserved for virus back titration. The plate can be mixed gently, and virus diluent can be added to all wells to bring each well to an equal volume. A back titration can be performed using a standard protocol.

The plate can be covered and again placed in the incubator as described above for 1 hour. After 1 hour, 100 µL of diluted MDCK cells (which should be 70-95% monolayer and low passage <30) can be added to each well of microtiter plates. Each well can contain 1.5 × 10⁴ cells.

The plate can be covered and again placed in the incubator as described above for 18-20 hours. After incubation, the liquid can be removed from wells, and wells can be washed, for example, using 200 µL phosphate buffered saline (PBS). 100 µL of cold 80% acetone can be added to each well, and the plate can be incubated at room temperature for 10-12 minutes for fixation. Acetone can be then removed from the wells, and the plate can be dried, for example, by air drying for about 10 minutes or until dry.

An influenza enzyme linked immunosorbent assay (ELISA) can then be performed using a standard ELISA protocol to determine the amount of virus which has been neutralized. In some cases, an influenza neutralization assay can be performed with the serum of a subject which has been inoculated with a vaccine composition developed according to this disclosure. In some cases, such an influenza neutralization assay can be performed using pig serum, human serum, or the serum of any mammal. In some cases, the serum can comprise antibodies which are head specific antibodies, stem specific antibodies, broadly neutralizing antibodies, universal antibodies, 90% universal antibodies, broadly strain specific antibodies, and/or 90% strain specific antibodies

In an influenza neutralization assay, control samples from a subject vaccinated with a control vaccine can be included. In such cases, the control vaccine can be a monovalent vaccine, a bivalent vaccine, a trivalent vaccine, or a quadrivalent vaccine. When the control vaccine is a trivalent vaccine, it can comprise antigens from the H1N1 strain, the H3N2 strain, and the HAB strain. In some cases, the minimal neutralizing dilution can be at least 2-fold greater using the developed vaccine than using the control vaccine. In some cases, the minimal neutralizing dilution can be at least 2-fold greater using the developed vaccine than using the control vaccine comprising H1N1, H3N2, and HAB antigens when tested using a hemagglutinin inhibition assay or influenza neutralization. In some cases, the minimal neutralizing dilution can be at least 2-fold greater using the developed vaccine than using the control quadrivalent vaccine when tested using a hemagglutinin inhibition assay or influenza neutralization. In some cases, the minimal neutralizing dilution can be at least 10-fold greater using the developed vaccine than using the control vaccine. In some cases, the minimal neutralizing dilution can be at least 10-fold greater using the developed vaccine than using the control vaccine comprising H1N1, H3N2, and HAB antigens when tested using a hemagglutinin inhibition assay or influenza neutralization. In some cases, the minimal neutralizing dilution can be at least 10-fold greater using the developed vaccine than using the control quadrivalent vaccine when tested using a hemagglutinin inhibition assay or influenza neutralization. In some cases, the minimal neutralizing dilution can be at least 100-fold greater using the developed vaccine than using the control vaccine. In some cases, the minimal neutralizing dilution can be at least 100-fold greater using the developed vaccine than using the control vaccine comprising H1N1, H3N2, and HAB antigens when tested using a hemagglutinin inhibition assay or influenza neutralization. In some cases, the minimal neutralizing dilution can be at least 100-fold greater using the developed vaccine than using the control quadrivalent vaccine when tested using a hemagglutinin inhibition assay or influenza neutralization. In certain cases, the minimal neutralizing dilution can be at least 1,000-fold greater using the developed vaccine than using the control vaccine. In various cases, the minimal neutralizing dilution can be at least 1,000-fold greater using the developed vaccine than using the control vaccine comprising H1N1, H3N2, and HAB antigens when tested using a hemagglutinin inhibition assay or influenza neutralization. In some cases, the minimal neutralizing dilution can be at least 1,000-fold greater using the developed vaccine than using the control quadrivalent vaccine when tested using a hemagglutinin inhibition assay or influenza neutralization. In certain cases, the minimal neutralizing dilution can be at least 10,000-fold greater using the developed vaccine than using the control vaccine. In various cases, the minimal neutralizing dilution can be at least 10,000-fold greater using the developed vaccine than using the control vaccine comprising H1N1, H3N2, and HAB antigens when tested using a hemagglutinin inhibition assay or influenza neutralization. In some cases, the minimal neutralizing dilution can be at least 10,000-fold greater using the developed vaccine than using the control quadrivalent vaccine when tested using a hemagglutinin inhibition assay or influenza neutralization. In certain cases, the improvement in minimal neutralizing dilution can be for one antigen, for more than one antigen, or for all antigens tested. In various cases, the improvement in minimal neutralizing dilution can be for one antigen, for more than one antigen, or for all antigens tested.

In some cases, the developed vaccine can neutralize more strains than the control vaccine. For example, the developed vaccine can neutralize at least one more strain than the control vaccine. In certain cases, the developed vaccine can neutralize at least 2, at least 5, at least 10, at least 25, at least 50, or at least 100 more strains than the control vaccine. In various cases, the developed vaccine can neutralize at least 2 times, at least 3 times, at least 4 times, at least 5 times, at least 10 times, at least 50 times, at least 100 times, at least 500 times, at least 1,000 times, at least 5,000 times, or at least 10,000 times more strains than the control vaccine.

### Example 7: ELISA assay

An ELISA can be used to detect antibodies present in serum directed against an antigen such as a flu antigen. In some embodiments, multiple different antigens (e.g., hemagglutinin variants from various strains) are investigated. Briefly, a hemagglutinin antigen variant can be immobilized in a well of a 96-well plate. In some cases, a dilution series can be prepared. Negative control wells comprising no antigen can be prepared.

A solution comprising serum suspected of containing hemagglutinin can be incubated in the wells comprising a hemagglutinin antigen variant or control wells. After an incubation period, the solution comprising serum can be washed away. In some cases, if an antibody or antibodies are present in the serum which can react with the hemagglutinin, then antibody will remain bound to the plate.

A secondary antibody raised against antibodies of the animal from which the serum originated can be applied to the wells. After an incubation period, the secondary antibody can be washed away. Should there be an interaction between the hemagglutinin and an antibody in the serum, secondary antibody can label the hemagglutinin-antibody complex.

The secondary antibody can be labeled by any acceptable means, and signal can be detected in wells where secondary antibody is present. In some cases, signal can correspond to the amount of secondary antibody present. In some cases, signal can correspond to the amount of antibody of serum-origin present. In some cases, signal can correspond to the amount of hemagglutinin-antibody interaction in a well.

### Example 8: Increasing average epitope breadth through Conservation-Concentration Coupling

It was tested whether a vaccination strategy that shifts the probability distribution of epitope conservation *in-vivo* to enrich vaccine-elicited antibodies towards conserved epitopes can be designed.

Without being bound by any one particular theory, the hypothesis was that it can be possible to selectively reward B cells that can recognize, and can affinity mature towards broadly conserved epitopes by coupling the conservation of these epitopes to their concentration in the vaccine formulation, known as Conservation-Concentration Coupling or C3. By administering a mixture of 30 diverse variants of HA, each individually at a dose which is not sufficient to elicit an effective strain-specific immune response, the dominant population of single variant epitopes can be effectively removed from the distribution. Thus, this would selectively favor B cells that recognize shared epitopes across components, with a broadly reactive B cell receiving up to a 30-fold higher dose than a strain specific B cell.

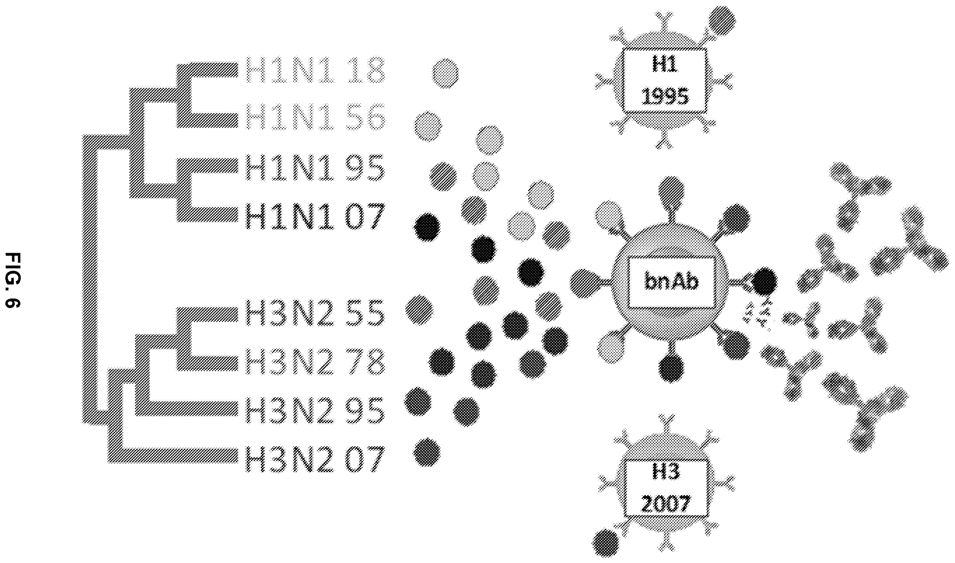

**FIG. 6** schematically illustrates how C3 works. B cells that carry a receptor that targets a conserved epitope receive a higher relative load of antigen, and thus necessary stimulus to differentiate into plasma cells that can produce antibodies. Other B cells with a receptor that can recognize a strain specific epitope can receive a lower relative load of antigens, which can be below the threshold necessary to initiate an immune response.

### Example 9: Study design - Conserved Epitope Focusing with Conservation-Concentration Coupling (C3)

Three *in-vivo* experiments were designed using domesticated outbred pigs (*Sus scrofa*), a model organism and a veterinary target organism for influenza A infection and vaccination. For the first *in-vivo* study, 35 pigs were split into 5 cohorts with 7 pigs per cohort. Each vaccine (C3) formulation comprised a diverse set of 30 HA variants (H1, H2, H3, H5, and H7 spanning 1918-2014). Cohorts of pigs were inoculated with either a bivalent formulation (BIV, 2 HA antigens H1N1 2007 and H3N2 1997 at 5,000 ng/antigen), C3-500 (30 HA antigens at 500 ng/antigen), C3-100 (30 HA antigens at 100 ng/antigen), C3-50 (30 HA antigens at 50 ng/antigen), or a vehicle control (PBS + squalene). The animals were inoculated 3 times, 3 weeks apart, with sera collection 3-4 weeks after each vaccination (Days 29, 50, and 71). RNA was isolated from PBMCs collected 7 days after the third vaccination (Day 50).

In the second and third *in-vivo* studies, all HA representatives after 2008 were removed in order to create a "pre-2009" C3 formulation for examining serological response to *de facto* future viral strains and HAs including pandemic H1N1 2009 through H3N2 2018. A 2008 seasonal bivalent control was similarly formulated, using H1N1 2007 and H3N2 2007.

For the second *in-vivo* study, 20 pigs were separated into 5 cohorts with 4 pigs per cohort. Cohorts were each inoculated with a formulation comprising either 2 HA antigens at 5,000 ng/antigen (BIV), 27 HA antigens at 50 ng/antigen (C3-50), H1N1 2007 at 50 ng (single low-dose H1), H3N2 2007 at 50 ng (single low-dose H3), or PBS + squalene (vehicle control). Cohorts for single HAs administered at low dose (50 ng) were included to validate a working Lₜₕᵣₑₛₕ (stochiometric limit below which a given antigen does not induce an immune response) in pigs. The animals were each inoculated six times, 21 days apart. Additionally, 3 cohorts (BIV, C3-50, and vehicle control) received a seventh inoculation 9 weeks after sixth inoculation. For C3-50, the seventh inoculation comprised a high-dose boost (27 HA at 500 ng/antigen). Sera were collected immediately prior to each inoculation on the same day. Additional sera were collected 4 weeks after the sixth inoculation (Day 135) and 4 weeks after the seventh inoculation (Day 195). RNA was isolated from peripheral blood mononuclear cells collected 7 days after the sixth inoculation (Day 112).

For the third *in-vivo* study, 25 pigs were separated into five cohorts with five pigs per cohort. Cohorts were each inoculated with a formulation comprising either 5,000 ng/antigen + squalene (BIV), C3-50 + squalene, C3-50 + alum, C3-50 + TLR, or a vehicle control (PBS + squalene + alum + MPLA + imiquimod. The animals received three inoculations each 21 days apart, with sera collection immediately prior to each inoculation on the same day. Additional sera were collected four weeks after the third inoculation (Day 70). All 3 C3-50 cohorts received 28 HA antigens at 50 ng/antigen for the first two inoculations, and 500 ng/antigen for the third inoculation.

### Example 10: Swarm effect

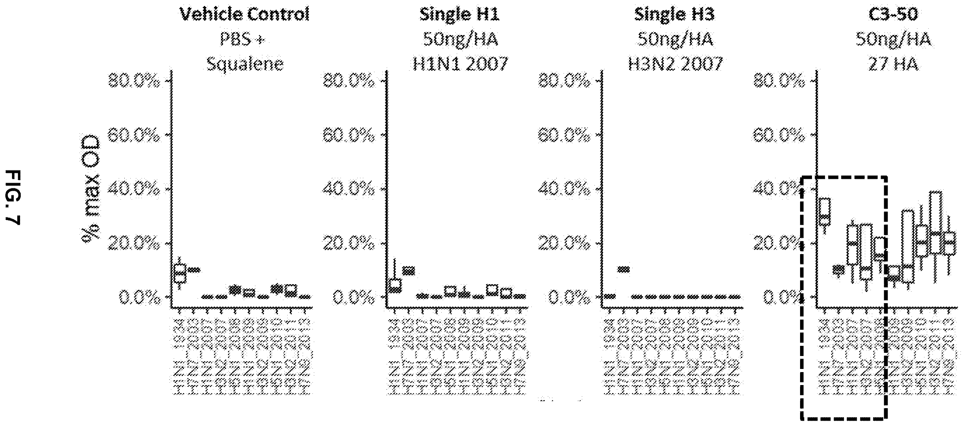

To support the theory of concentration conservation coupling, Lₜₕᵣₑₛₕ was determined *in-vivo.* First, it was determined that a 50 ng dose of HA is under the threshold of immune activation by administering a single antigen, either H1N1 2007 or H3N2 2007, at a dose of 50 ng. Six total doses were administered to pigs 21 days apart (corresponding to the second study in the previous example). In both cases, a sera response was not detected by ELISA to either antigen 28 days after the sixth inoculation, as illustrated in **FIG. 7****.**

However, when the two antigens are administered in combination with 25 other HAs spanning 1918-2008, also each at 50 ng (C3-50), a sera response to both antigens was detected. Additionally, a C3-50 inoculation resulted in sera responses to other HAs in C3 and a panel of "future" post-2008 strains not included in the formulation, including H1N1, H3N2, H5N1, and H7N9, spanning 2009-2013. Thus, a low-dose ensemble can elicit an immune response even though individual components can be below their threshold dose.

In this case, in a pool of 27 antigens at individually sub-physiological doses, shared epitopes can be at up to a 27-fold higher dose, and therefore can be successfully targeted and can result in preferentially broad responses that can cross to heterologous strains.

**FIG. 7** illustrates ELISA signals (% max OD₄₅₀ₙₘ) of individual pig sera to 10 HA antigens (H1, H3, H5, and H7 spanning 1934-2013) across immunization cohorts (each *n* = 4) of C3-50 (27 HAs at 50 ng/HA), single low-dose (50 ng) H1N1 2007 antigen, single low-dose (50 ng) H3N2 2007 antigen, and vehicle control (PBS + squalene). Box and whiskers plots show median and IQR; whiskers show 1.5× IQR of the lower and upper quartiles. Points represent outliers. Antigens shown in the dashed line box indicate HAs present in the 1918-2008 C3 vaccine formulation.

### Example 11: Conservation-Concentration Coupling responses are primarily subtype-specific and require a minimum total subtype antigen dose

The analysis of C3-elicted binding response was separated by HA subtype. The C3-50 formulation the first study described in Example 9 contained 10 H1s, 7 H3s, 5 H5s, 3 H7s, and 2 H2s, each individually at 50 ng. Serum responses were correlated to total subtype dose, with H1 and H3 providing the strongest responses, H5 providing a weaker response, and H2 and H7 appearing poorly responded to by C3-50. These results support predictions that responses in one subtype do not necessarily elevate responses to other subtypes. Thus, in some cases broad intra-subtype responses can be much more common than fully universal antibodies. The data suggests a potential minimum clade dose of 7-10 members (i.e., 350-500 ng of the most conserved epitopes) for best effect.

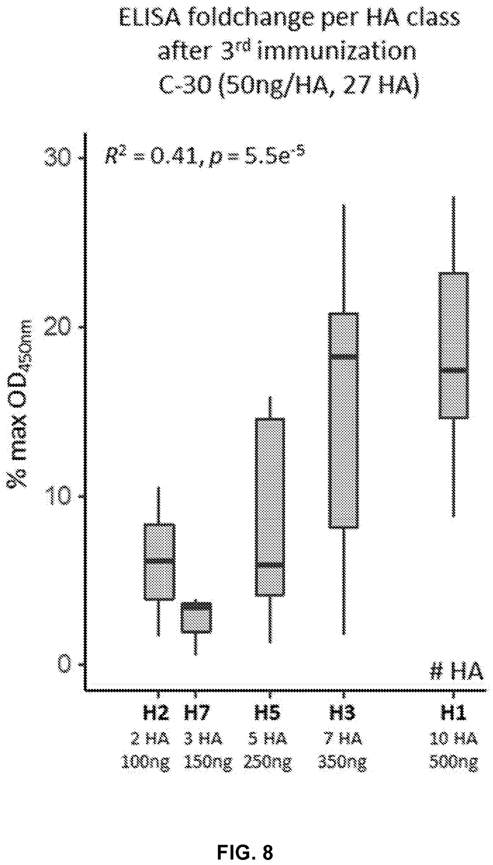

Data illustrating this experiment is illustrated in **FIG. 8****.** ELISA signals (% max OD₄₅₀ₙₘ) from pigs of the C3-50 cohort (study #1) are shown separated into the different HA antigen classes present in the C3-50 formulation (H1, H2, H3, H5, and H7). The X-axis shows the number of HAs per class. Sera were collected 28 days after the third vaccination (Day 71). Pearson's correlation coefficient for number of HA antigens versus ELISA signal is shown as R² with corresponding p-value. Box and whiskers plots show median and IQR; whiskers show 1.5× IQR of the lower and upper quartiles. P values are calculated using Kruskal-Wallis rank sum test with Dunn's correction for multiple comparisons. Selected P values are reported as adjusted and indicated as * P < 0.05, ** P < 0.01, *** P < 0.001, and **** P< 0.0001.

### Example 12: Conservation-Concentration Coupling broad reactivity with an inverse dose response

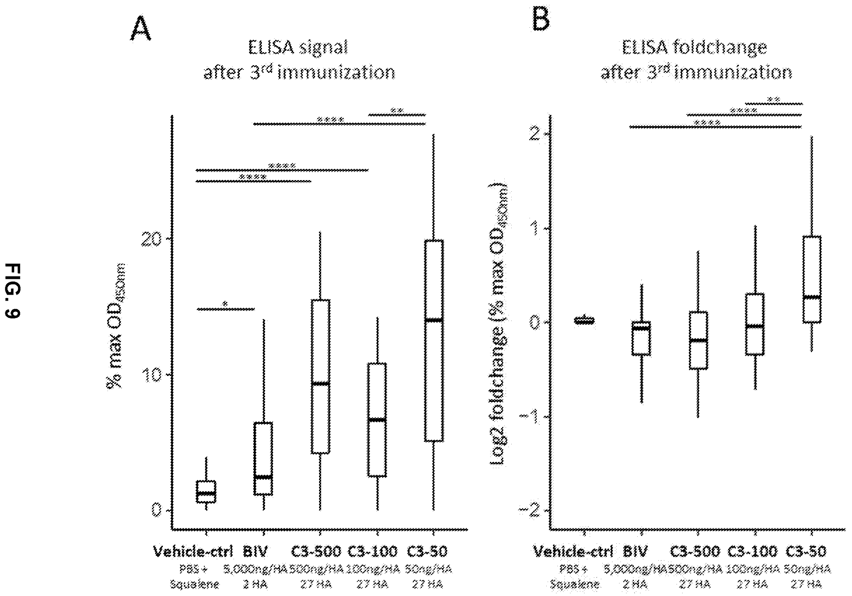

In some cases, lowering the dose for each individual component may be able to increase the pressure to target only conserved epitopes and thus provide greater breadth of serological response. To evaluate the relationship between dose and response of C3, three dosage cohorts of the C3 were tested alongside BIV and vehicle control groups. Sera responses from each of 7 pigs per cohort from the first study described in Example 9 to a panel of 36 antigens indicate that the lowest dose of C3 (50 ng/HA) provided the highest sera response to the most antigens, spanning H1N1, H1N3, H1N3, H2N2, H3N2, H5N1, H6N2, and H17N10 from 1918-2014. This inverse dose-response is in accordance with model predictions, and a unique property of this vaccination strategy. All three C3 formulations produced an improved median serum response compared to our bivalent formulation as measured by ELISA, as shown in panel B of **FIG. 9****.** Additionally, an inverse dose response was observed where the lowest dose of C3 yielded the highest median serum response as shown in panel A of **FIG. 9****,** and highest fold-change in sera titer over time, from 7 days to 28 days post-third inoculation shown in panel B of **FIG. 9****,** consistent with the model's predictions of conservation-concentration coupling leading to a shift towards breadth by diluting out competing epitopes.

**FIG. 9****,** panel A illustrates average ELISA signals (% max OD450nm) per pig across all tested strains (n = 36, spanning H1, H2, H3, H5, H7, and H17 ranging from 1918-2014) per pig (study #1). Sera were collected 28 days after the third vaccination (Day 71). **FIG. 9****,** panel B illustrates fold-change of average ELISA signal (% max OD450nm) per pig from C) between 7 days and 28 days post-third vaccination. Box and whiskers plots show median and IQR; whiskers show 1.5× IQR of the lower and upper quartiles. P values are calculated using Kruskal-Wallis rank sum test with Dunn's correction for multiple comparisons. Selected P values are reported as adjusted and indicated as * P < 0.05, ** P < 0.01, *** P < 0.001, and **** P< 0.0001.

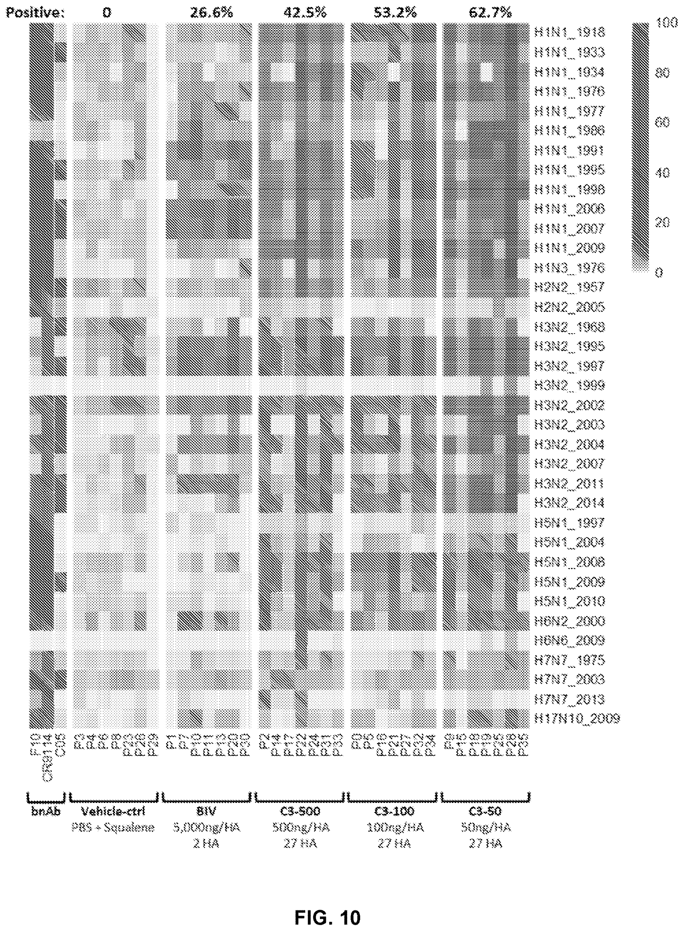

When analyzing responses on a per-cohort, per-animal basis, more animals were observed responding more broadly as a function of inverse dose response, with the lowest dose (C3-50 ng/antigen) exhibiting greatest breadth, as shown in the heatmap of **FIG. 10****.**

**FIG. 10** provides a heatmap of ELISA signals (% max OD450nm) from individual pig sera of 5 immunization cohorts binding against 36 HAs (H1, H2, H3, H5, H6, H7, and H17 spanning 1918-2014) as well as broadly neutralizing human antibodies (C05, F10, and CR9114). Immunization cohorts were three different C3 formulations of 30 HAs at 500 ng/HA, 100 ng/HA, and 50 ng/HA, a BIV cohort (H1N1 2007 and H3N2 1997 each at 5,000 ng) and vehicle control cohort (PBS + squalene). Sera were collected 28 days after the third inoculation (Day 71, study #1). Heatmap shading was adapted using quantile breaks to adjust for the ELISA data distribution. Positive strain coverage was determined as percent values above average of the vehicle control + 3× SD for each strain.

Collectively, data show that breadth of the response is coupled to the concentration of conserved epitopes and that the C3 formulation at the lowest dose of 50 ng/antigen provided the broadest mean strains bound per pig serum in the most pigs.

### Example 13: Conservation-Concentration Coupling induces uniform B cell expansion

Next, IgM and IgG B cell receptor (BCR) repertoires of pig peripheral blood mononuclear cells (PBMCs) were analyzed of pigs described in the second study (as described in Example 9), 7 days after the sixth inoculation. In contrast with the high variability in clonal expansion observed among pigs receiving either bivalent or vehicle immunization schedules, significantly more uniform BCR repertoire clonotype expansion were observed in the pigs receiving the C3-50 formulation. Thus, C3 appeared to generate a more uniform, and therefore potentially more predictable, immune response.

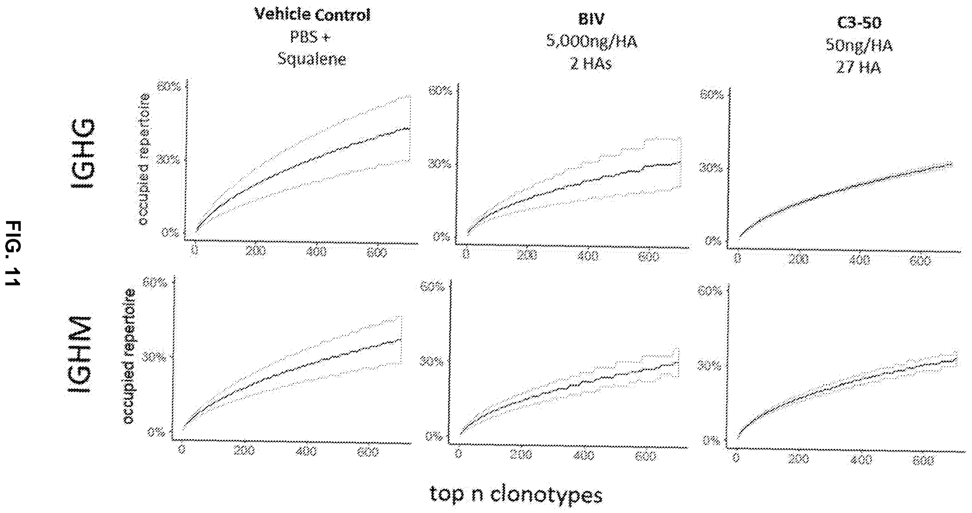

Data illustrating these observations are provided in **FIG. 11****,** as peripheral BCR repertoire occupied by top clonotypes separated by isotype. The black line shows the average repertoire occupied as percent (%) of total sequences for top n clonotypes across pigs per immunization group. The gray area indicates 95% confidence interval. Immunization cohorts were from the second *in-vivo* study described in Example 9; a C3 formulation of 27 HAs at 50 ng/HA (n = 4), a bivalent cohort (H1N1 2007 and H3N2 2007 each at 5,000 ng, n = 3) and vehicle control (PBS + squalene, n = 4). Lymphocytes were collected 7 days after sixth inoculation (Day 112). Isotypes were determined by C region specific priming during library preparation. Clonotypes are defined as sequences with identical CDRH3 amino acid sequence.

### Example 14: Conservation-Concentration Coupling elicits broadly neutralizing antibodies

To isolate monoclonal pig anti-HA antibodies produced in C3 vaccinated animals, libraries were generated of m13 phage displayed pig antibodies from C3 animals of the first *in-vivo* study described in Example 9. From those libraries, we isolated pig anti-HA antibodies, including the bnAb 9C5, from a pig which received low-dose C3 (50 ng/antigen).

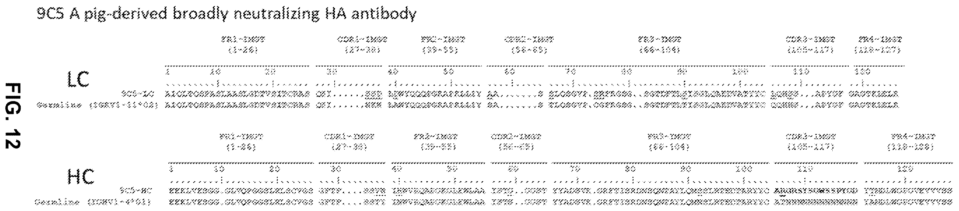

Data representing the results of this experiment are provided in **FIG. 12****.** Sequence alignment of broadly neutralizing porcine monoclonal antibody 9C5 isolated through phage display. Heavy and Light chain nucleotide sequences were aligned using IMGT V Quest against pig (*Sus scrofa*) germline genes. Mutations compared to detected germline genes are underlined and the CDRH3 is marked in bold. CDRH3 was reported by IMGT with 7 added amino acid positions 111.1-111.3 and 112.4-112.1 in the IMGT numbering scheme. Phage libraries were constructed from B cell pools per immunization cohort from lymphocytes 7 days post third vaccination (Day 50, study #1) and panned 4 rounds against H3N2 2007 and H1N1 2007. Monoclonal 9C5 was derived from one pig (C3-50 cohort, 30 HAs at 50 ng/HA).

The data show that C3 antigen formulations can induce broadly neutralizing antibodies, which show improved performance when compared to known human bnAbs. This suggests that the approach provided herein is effectively overcoming the stochastic hurdles of these exceedingly rare epitopes and that antibodies targeting these can be able to broadly neutralize a series of influenza strains, even if they were not part of the original antigen formulation.

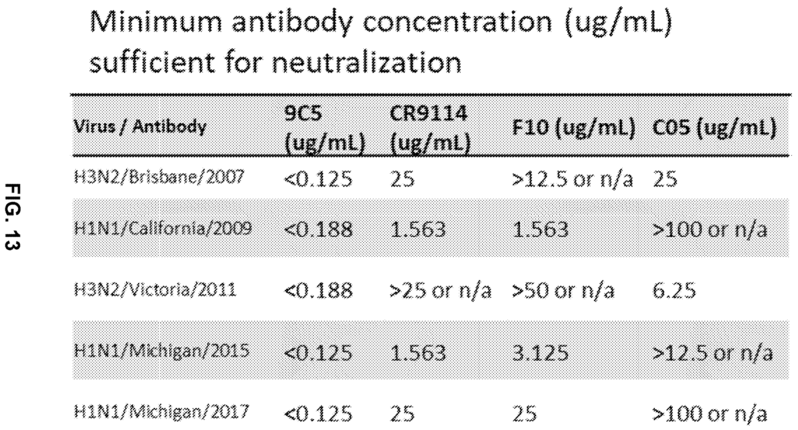

Further, an experiment was performed to determine minimum antibody concentration (ug/mL) sufficient for *in vitro* neutralization of a panel of 5 Influenza A strains (H1N1/California/2009, H1N1/Michigan/2015, H1N1/Michigan/2017, H3N2/Brisbane/2007, and H3N2/Victoria/2011). Results are provided in **FIG. 13****.** Pig monoclonal 9C5, isolated from one pig (C3-50, in-vivo study #1) using phage display library, was tested as scFv-huFc fusion chimera construct in 2-Ml HEK293 supernatant, quantitated by Protein A standard curve on Octet QK at 32 ug/Ml. Shown in comparison to control bnAbs CR9114, F10, and C05 tested for neutralizing capability as purified human IgG1.

### Example 15: Neutralization of "future" influenza strains

Here it was determined whether C3 formulations elicited neutralizing antibodies through microneutralization assays on sera from pigs inoculated with C3. For sera from the second *in-vivo* study described in Example 9, similar neutralization capacities were observed from sera of both the C3-50 vaccination cohort and the sera of pigs vaccinated with the bivalent control for strains that were part of the bivalent control cohort (H1N1 and H3N2 from 2007). In addition, for tested respective future strains, C3-50 pigs were able to neutralize 5/5 (H1N1 2009, H3N2 2009, H3N2 2011, H3N2 2014, and H1N1 2015) whereas the bivalent control group only showed neutralizing titers against 2/5 (H3N2 2009 and 2011).

For sera from the third *in-vivo* study described in Example 9, pigs from the bivalent control group neutralized 2/6 strains tested with one "future" virus, H3N2 2009, not part of the formulations. The C3-50 cohorts alum and squalene exhibited improved neutralization, with neutralization against 4/6 and 5/6 strains tested. Both neutralizing 3 and 4 future viruses not part of the C3-50 formulation. Pigs that received C3-50 + TLR agonists as adjuvants were unable to neutralize any "future" strains and only neutralized H3N2 2007. Collectively, the data show that C3 formulations administered at 50 ng produce sera with broadly neutralizing capacities at a similar level to that of a bivalent formulation while also neutralizing a broader range of "future" viruses not part of the antigen formulation. Thus, a conservation-concentration coupling approach can focus the immune response towards highly conserved epitopes on the HA coat protein. Moreover, these epitopes can elicit a neutralizing serum response at a similar level to a normal bivalent formulation, and additionally covering viral strains emerging almost a decade of viral evolution later.

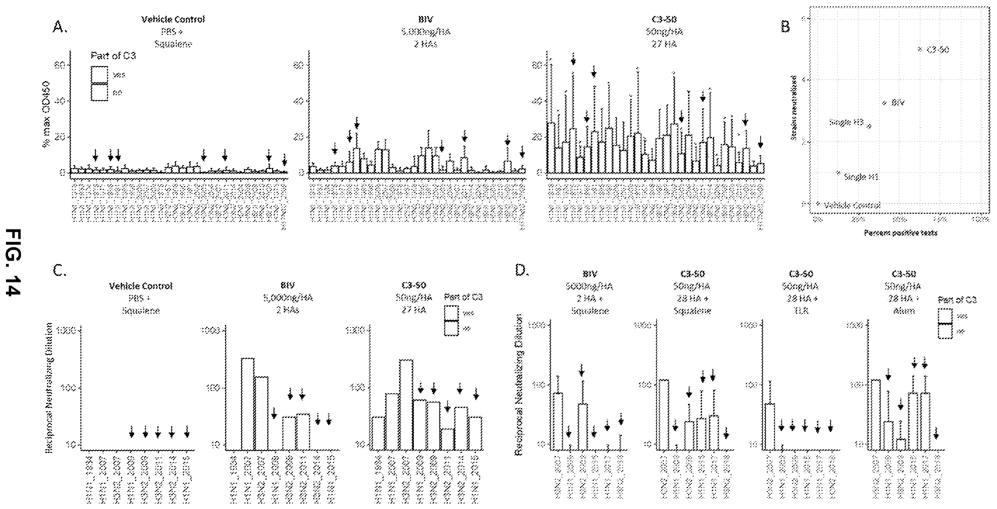

Data illustrating these results are provided in **FIG. 14****.** Panel A in **FIG. 14** provides ELISA signals (% max OD450nm) of pig sera from the first *in-vivo* study to 30 HA antigens (H1, H2, H3, H5, H6, H7, and H17 spanning 1918-2014) across immunization cohorts (each n = 7 pigs) of C3-50 (30 HAs at 50 ng/HA), BIV cohort (H1N1 2007 and H3N2 1997 each at 5,000 ng) and vehicle control (PBS + squalene). Sera were collected 28 days after the third vaccination (Day 71). Bar represent mean across all pigs of a cohort, error bars indicate positive standard deviation. Arrows indicate HAs not present in the C3 vaccine formulation (in-vivo study #1). Asterisks indicate statistically significant differences between C3-50 and bivalent formulation with adjusted p values < 0.05 or lower. P values were determined using strain-wise Wilcoxon Rank Sum test with alternative hypothesis of BIV signals being less than C3-50 signals. Benjamini Hochberg correction was applied to adjust for multiple testing.

Panel B in **FIG. 14** provides neutralization breadth shown as average number of strains neutralizing per pig by cohort and as number of positive tests per cohort are shown as scatterplot. Sera were collected 28 days after the seventh vaccination (Day 195, study #2). Viruses tested include H1N1/PuertoRico/1934, H1N1/Brisbane/2007, H1N1/California/2009, H1N1/Michigan/2015, H3N2/Brisbane/2007, H3N2/Perth/2009, H3N2/Victoria/2011, and H3N2/HongKong/2014. Sera were not pooled. Cohorts were vehicle control (PBS + squalene), seasonal BIV (H1N1 2007 and H3N2 2007 each at 5,000n g), single low-dose H1 antigen (H1N1 2007, 50 ng) single low-dose H3 antigen (H3N2 2007, 50 ng), and C3-50 (27 HAs at 50 ng/HA), *in-vivo* study #2. Error bars indicate standard deviation.

Panel C in **FIG. 14** provides neutralization strength for study #2 reported as reciprocal neutralizing dilution per strain across cohorts using bar charts. Bars indicate the mean across all pigs (n = 4) of a cohort and error bars indicate standard deviation. "Future" strains tested that are not part of the 2008 C3 formulation are indicated by arrows. Sera were collected 28 days after the 7^{th} vaccination (Day 195).

Panel D in **FIG. 14** provides neutralization strength for study #3 reported as reciprocal neutralizing dilution per strain across cohorts using bar charts. Bars indicate the mean across all pigs (n = 5) of a cohort and error bars indicate standard deviation. Points show the individual value per pig. "Future" strains tested that are not part of the 2008 C3 formulation are indicated by arrows. Sera were collected 28 days after the third vaccination (Day 70). Cohorts were vehicle control (PBS + squalene + TLR-agonist + alum), seasonal BIV (H1N1 2007 and H3N2 2007 each at 5,000 ng + squalene), and 3 cohorts of C3-50 (28 HAs at 50 ng/HA), with squalene, TLR-agonist, and alum adjuvants, respectively.

### Example 16: Selection of 32 most diverse HIV gp160 sequences for HIV clade A, B, and C respectively.

HIV sequences were downloaded from www.hiv.lanl.gov/ and separated into clades. Sequences were filtered by length to exclude sequences shorter than 800 amino acids and sequences from Clade A, B or C (of HIV-1 Group M) selected for further processing.

Sequences were aligned using hmmalign (HMMER v. 3.0, hmmer.org/) with a custom made HMM profile for HIV gp160 proteins. Next, a pairwise hamming distance matrix was generated using a custom-made python (v. 3.7.2) script. The sequences were subsequently clustered based on the hamming distance matrix using UPGMA clustering (UPGMA: unweighted pair group method with arithmetic mean) implemented in the hclust function of the R (v. 3.6.1) base stats package. The clustered sequences were divided into 32 subclusters using the cutree function of the R (v 3.6.1) base stats package.

Representative sequences were extracted for each cluster as their medoids using the medoids function of GDAtools (v.1.4). The output for this analysis was 96 amino acid sequences, 32 for each HIV Clade A, B and C. These sequences are SEQ ID NOs:172-267, as listed in Table 9.

### VI. Compositions

A composition can be a vaccine or a library of antigens from which antigens of a vaccine are selected. A library of antigens can comprise a plurality of antigens. Such a composition can comprise a minimum number of variants of a microbe. In some cases, such a library can comprise at least 1×10¹, at least 1×10², at least 1×10³, at least 1×10⁴, at least 1×10⁵, at least 1×10⁶, at least 1×10⁷, at least 1×10⁸, at least 1×10⁹, or at least 1×10¹⁰ different variants of a microbe.

A library of antigens can comprise a subset of all known sequences of a microbe. A library comprising more sequences can be considered more complete, and in some embodiments, can be considered more desirable. In some cases, a library can comprise at least 80%, 85%, 90%, or 95% of all known sequences of a microbe.

Antigens in a vaccine can comprise a specified number of amino acids. In some cases, an antigen in a vaccine can comprise no more than 10, no more than 15, no more than 20, no more than 25, no more than 30, no more than 35, no more than 40, no more than 45, no more than 50, no more than 55, no more than 60, no more than 65, no more than 70, no more than 75, no more than 80, no more than 85, no more than 90, no more than 95, no more than 100, no more than 110, or no more than 120 amino acids. In some cases, an antigen in a vaccine can comprise at least 10, at least 15, at least 20, at least 25, at least 30, at least 35, at least 40, at least 45, at least 50, at least 55, at least 60, at least 65, at least 70, at least 75, at least 80, at least 85, at least 90, at least 95, at least 100, at least 110, or at least 120 amino acids. In some cases, an antigen in a vaccine can comprise from 10 to 120 amino acids, from 10 to 100 amino acids, from 10 to 90 amino acids, from 10 to 80 amino acids, from 10 to 70 amino acids, from 10 to 60 amino acids, from 10 to 50 amino acids, from 10 to 40 amino acids, from 10 to 30 amino acids, from 20 to 120 amino acids, from 20 to 110 amino acids, from 20 to 100 amino acids, from 20 to 90 amino acids, from 20 to 80 amino acids, from 20 to 70 amino acids, from 20 to 60 amino acids, from 20 to 50 amino acids, from 20 to 40 amino acids, from 20 to 30 amino acids, from 30 to 120 amino acids, from 30 to 110 amino acids, from 30 to 100 amino acids, from 30 to 90 amino acids, from 30 to 80 amino acids, from 30 to 70 amino acids, from 30 to 60 amino acids, from 30 to 50 amino acids, from 30 to 40 amino acids, from 40 to 120 amino acids, from 40 to 110 amino acids, from 40 to 100 amino acids, from 40 to 90 amino acids, from 40 to 80 amino acids, from 40 to 70 amino acids, from 40 to 60 amino acids, from 40 to 50 amino acids, from 50 to 120 amino acids, from 60 to 110 amino acids, from 50 to 100 amino acids, from 50 to 90 amino acids, from 50 to 80 amino acids, from 50 to 70 amino acids, from 50 to 60 amino acids, from 60 to 120 amino acids, from 60 to 110 amino acids, from 60 to 100 amino acids, from 60 to 90 amino acids, from 60 to 80 amino acids, from 60 to 70 amino acids, from 70 to 120 amino acids, from 70 to 110 amino acids, from 70 to 100 amino acids, from 70 to 90 amino acids, from 70 to 80 amino acids, from 80 to 120 amino acids, from 80 to 110 amino acids, from 80 to 100 amino acids, from 80 to 90 amino acids, from 90 to 120 amino acids, from 90 to 110 amino acids, from 90 to 100 amino acids, from 100 to 120 amino acids, from 100 to 110 amino acids, or from 110 to 120 amino acids.

In some cases, an antigen in a vaccine can comprise at least 200, at least 300, at least 400, at least 500, at least 600, at least 700, at least 800, at least 900, or at least 1000 amino acids. In some cases, an antigen in a vaccine can comprise no more than 200, no more than 300, no more than 400, no more than 500, no more than 600, no more than 700, or no more than 800 amino acids. In some cases, an antigen in a vaccine can comprise from 10 to 1000 amino acids, from 10 to 900 amino acids, from 10 to 800 amino acids, from 10 to 700 amino acids, from 10 to 600 amino acids, from 10 to 500 amino acids, from 10 to 400 amino acids, from 10 to 300 amino acids, from 10 to 200 amino acids, from 100 to 1000 amino acids, from 100 to 800 amino acids, from 100 to 700 amino acids, from 100 to 600 amino acids, from 100 to 500 amino acids, from 100 to 400 amino acids, from 100 to 300 amino acids, from 100 to 200 amino acids, from 200 to 1000 amino acids, from 200 to 900 amino acids, from 200 to 800 amino acids, from 200 to 700 amino acids, from 200 to 600 amino acids, from 200 to 500 amino acids, from 200 to 400 amino acids, from 200 to 300 amino acids, from 300 to 1000 amino acids, from 300 to 900 amino acids, from 300 to 800 amino acids, from 300 to 700 amino acids, from 300 to 600 amino acids, from 300 to 500 amino acids, from 300 to 400 amino acids, from 400 to 1000 amino acids, from 400 to 900 amino acids, from 400 to 800 amino acids, from 400 to 700 amino acids, from 400 to 600 amino acids, from 400 to 500 amino acids, from 500 to 1000 amino acids, from 500 to 900 amino acids, from 500 to 800 amino acids, from 500 to 700 amino acids, from 500 to 600 amino acids, from 600 to 1000 amino acids, from 600 to 900 amino acids, from 600 to 800 amino acids, from 600 to 700 amino acids, from 700 to 1000 amino acids, from 700 to 900 amino acids, from 700 to 800 amino acids, from 800 to 1000 amino acids, from 800 to 900 amino acids, or from 900 to 1000 amino acids.

A vaccine can comprise a specified number of antigens. The antigens in a vaccine can be selected for inclusion based on the total number of antigens available, similarity to pathogenic strains, similarity to other included antigens, or differences from other included antigens. Vaccines herein can comprise a set of antigens which can comprise at least 5, at least 6, at least 7, at least 8, at least 9, at least 10, at least 15, at least 20, at least 30, at least 40, at least 50, at least 60, at least 70, at least 80, at least 90, at least 100, at least 200, at least 300, at least 400, at least 500, at least 600, at least 700, at least 800, at least 900, or at least 1000 different antigens. In selected cases, a vaccine can comprise at least 30 antigens. In some cases, a vaccine can comprise a set of antigens which can comprise no more than 5, no more than 6, no more than 7, no more than 8, no more than 9, no more than 10, no more than 15, no more than 20, no more than 30, no more than 40, no more than 50, no more than 60, no more than 70, no more than 80, no more than 90, no more than 100, no more than 200, no more than 300, no more than 400, no more than 500, no more than 600, no more than 700, no more than 800, no more than 900, or no more than 1000 different antigens. In selected cases, a vaccine can comprise no more than 30 antigens. In some cases, a vaccine can comprise from 5 to 1000, from 5 to 900, from 5 to 800, from 5 to 700, from 5 to 600, from 5 to 500, from 5 to 400, from 5 to 300, from 5 to 200, from 5 to 100, from 5 to 90, from 5 to 80, from 5 to 70, from 5 to 60, from 5 to 50, from 5 to 40, from 5 to 30, from 5 to 20, from 5 to 10, from 10 to 1000, from 10 to 900, from 10 to 800, from 10 to 700, from 10 to 600, from 10 to 500, from 10 to 400, from 10 to 300, from 10 to 200, from 10 to 100, from 10 to 90, from 10 to 80, from 10 to 70, from 10 to 60, from 10 to 50, from 10 to 40, from 10 to 30, from 10 to 20, from 20 to 1000, from 20 to 900, from 20 to 800, from 20 to 700, from 20 to 600, from 20 to 500, from 20 to 400, from 20 to 300, from 20 to 200, from 20 to 100, from 20 to 90, from 20 to 80, from 20 to 70, from 20 to 60, from 20 to 50, from 20 to 40, from 20 to 30, from 30 to 1000, from 30 to 900, from 30 to 800, from 30 to 700, from 30 to 600, from 30 to 500, from 30 to 400, from 30 to 300, from 30 to 200, from 30 to 100, from 30 to 90, from 30 to 80, from 30 to 70, from 30 to 60, from 30 to 50, from 30 to 40, from 40 to 1000, from 40 to 900, from 40 to 800, from 40 to 700, from 40 to 600, from 40 to 500, from 40 to 400, from 40 to 300, from 40 to 200, from 40 to 100, from 40 to 90, from 40 to 80, from 40 to 70, from 40 to 60, from 40 to 50, from 50 to 1000, from 50 to 900, from 50 to 800, from 50 to 700, from 50 to 600, from 50 to 500, from 50 to 400, from 50 to 300, from 50 to 200, from 50 to 100, from 50 to 90, from 50 to 80, from 50 to 70, from 50 to 60, from 100 to 1000, from 100 to 900, from 100 to 800, from 100 to 700, from 100 to 600, from 100 to 500, from 100 to 400, from 100 to 300, from 100 to 200, from 200 to 1000, from 200 to 900, from 200 to 800, from 200 to 700, from 200 to 600, from 200 to 500, from 200 to 400, from 200 to 300, from 300 to 1000, from 300 to 900, from 300 to 800, from 300 to 700, from 300 to 600, from 300 to 500, from 300 to 400, from 400 to 1000, from 400 to 900, from 400 to 800, from 400 to 700, from 400 to 600, from 400 to 500, from 500 to 1000, from 500 to 900, from 500 to 800, from 500 to 700, from 500 to 600, from 600 to 1000, from 600 to 900, from 600 to 800, from 600 to 700, from 700 to 1000, from 700 to 900, from 700 to 800, from 800 to 1000, from 800 to 900, or from 800 to 1000 different antigens.

In some cases, an intact antigen can be divided into functional fragments, or parts, of the whole antigen, for example, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 30, at least 35, at least 40, at least 45, at least 50, at least 55, at least 60, at least 65, at least 70, at least 75, at least 80, at least 85, at least 90, at least 95 or at least 100 portions (e.g., pieces or fragments), inclusive, and where each individual functional fragment of the antigen can be associated with the polymer according to the methods as disclosed herein. In some cases the antigen may be divided into at most 2, at most 3, at most 4, at most 5, at most 6, at most 7, at most 8, at most 9, at most 10, at most 11, at most 12, at most 13, at most 14, at most 15, at most 16, at most 17, at most 18, at most 19, at most 20, at most 21, at most 22, at most 23, at most 24, at most 25, at most 30, at most 35, at most 40, at most 45, at most 50, at most 55, at most 60, at most 65, at most 70, at most 75, at most 80, at most 85, at most 90, at most 95, or at most 100 portions (e.g., pieces or fragments), inclusive.

In some cases, a broadly conserved sequence can be at least 40% conserved across a set of strains of a microbe (e.g., at least 10% of strains, at least 25% of strains, at least 50% of strains, at least 75% of strains, at least 90% of strains, at least 95% of strains, or all strains of a microbe). In some cases, a broadly conserved sequence can be a sequence that has limited variation across species or strains of a microbe. A sequence that is conserved across a plurality of microbes. can be the same or substantially the same across a plurality of microbes. For example, a sequence that is 40%, 50%, 60%, 70%, 80%, 90%, or 100% conserved can have structural or sequence identity of 40%, 50%, 60%, 70%, 80%, 90%, or 100% across a set of microbes.

In certain cases, a broadly conserved sequence can be at least 50% conserved across a set of strains of a microbe (e.g., at least 10% of strains, at least 25% of strains, at least 50% of strains, at least 75% of strains, at least 90% of strains, at least 95% of strains, or all strains of a microbe). In various cases, a broadly conserved sequence can be at least 60% conserved across a set of strains of a microbe (e.g., at least 10% of strains, at least 25% of strains, at least 50% of strains, at least 75% of strains, at least 90% of strains, at least 95% of strains, or all strains of a microbe). In some cases, a broadly conserved sequence can be at least 70% conserved across a set of strains of a microbe (e.g., at least 10% of strains, at least 25% of strains, at least 50% of strains, at least 75% of strains, at least 90% of strains, at least 95% of strains, or all strains of a microbe). In certain cases, a broadly conserved sequence can be at least 80% conserved across a set of strains of a microbe (e.g., at least 10% of strains, at least 25% of strains, at least 50% of strains, at least 75% of strains, at least 90% of strains, at least 95% of strains, or all strains of a microbe). In various cases, a broadly conserved sequence can be at least 90% conserved across a set of strains of a microbe (e.g., at least 10% of strains, at least 25% of strains, at least 50% of strains, at least 75% of strains, at least 90% of strains, at least 95% of strains, or all strains of a microbe).

An antigen can be representative of a clade if its sequence can be up to 10% of the average size of antigens of the represented clade different than other members of the represented clade. In some cases, an antigen can be representative of a clade if its sequence is up to 15%, up to 20%, up to 25%, or up to 30% of the average size of antigens of the represented clade different than other members of the represented clade.

An antigen can be representative of a clade if its sequence is no more than 75% of the average size of antigens of the represented clade different than other members of the represented clade. In some clade, an antigen can be representative of a clade if its sequence is no more than 50%, no more than 55%, no more than 60%, no more than 65%, no more than 70%, no more than 75%, no more than 80%, no more than 85%, no more than 90%, or no more than 95% different than other members of the represented clade.

An antigen can be representative of a clade if its sequence is up to 5, up to 10, up to 15, up to 20, up to 25, up to 30, up to 35, up to 40, up to 45, up to 50, up to 55, up to 60, up to 70, up to 80, up to 90, up to 100, up to 150, up to 200, up to 250, or up to 300 amino acids different from a subset of strains of the represented clade. In some cases, an antigen can be representative of a clade if its sequence is up to 5, up to 10, up to 15, up to 20, up to 25, up to 30, up to 35, up to 40, up to 45, up to 50, up to 55, up to 60, up to 70, up to 80, up to 90, up to 100, up to 150, up to 200, up to 250, or up to 300 amino acids different from at least 50% of strains of the represented clade. In some cases, an antigen can be representative of a clade if its sequence is up to 5, up to 10, up to 15, up to 20, up to 25, up to 30, up to 35, up to 40, up to 45, up to 50, up to 55, up to 60, up to 70, up to 80, up to 90, up to 100, up to 150, up to 200, up to 250, or up to 300 amino acids different from at least 60% of strains of the represented clade. In some cases, an antigen can be representative of a clade if its sequence is up to 5, up to 10, up to 15, up to 20, up to 25, up to 30, up to 35, up to 40, up to 45, up to 50, up to 55, up to 60, up to 70, up to 80, up to 90, up to 100, up to 150, up to 200, up to 250, or up to 300 amino acids different from at least 70% of strains of the represented clade. In some cases, an antigen can be representative of a clade if its sequence is up to 5, up to 10, up to 15, up to 20, up to 25, up to 30, up to 35, up to 40, up to 45, up to 50, up to 55, up to 60, up to 70, up to 80, up to 90, up to 100, up to 150, up to 200, up to 250, or up to 300 amino acids different from at least 80% of strains of the represented clade. In some cases, an antigen can be representative of a clade if its sequence is up to 5, up to 10, up to 15, up to 20, up to 25, up to 30, up to 35, up to 40, up to 45, up to 50, up to 55, up to 60, up to 70, up to 80, up to 90, up to 100, up to 150, up to 200, up to 250, or up to 300 amino acids different from at least 90% of strains of the represented clade. In some cases, an antigen can be representative of a clade if its sequence is up to 5, up to 10, up to 15, up to 20, up to 25, up to 30, up to 35, up to 40, up to 45, up to 50, up to 55, up to 60, up to 70, up to 80, up to 90, up to 100, up to 150, up to 200, up to 250, or up to 300 amino acids different from all strains of the represented clade.

An antigen can be representative of a clade if its edit distance is up to 5% of the average size of antigens of the represented clade. In some cases, an antigen can be representative of a clade if its edit distance is up to 10%, up to 15%, or up to 20% of the average size of antigens of the represented clade. An antigen can be representative of a clade if its sequence is up to 10% of the average size of antigens of the represented clade different than at least 95%, at least 96%, at least 97%, at least 98%, or at least 99% of all strains of the represented clade. In some cases, an antigen can be representative of a clade if its sequence is up to 15% of the average size of antigens of the represented clade different than at least 95%, at least 96%, at least 97%, at least 98%, or at least 99% of all strains of the represented clade. In some cases, an antigen can be representative of a clade if its sequence is up to 20% of the average size of antigens of the represented clade different than at least 95%, at least 96%, at least 97%, at least 98%, or at least 99% of all strains of the represented clade.

An antigen can be representative of a clade if its edit distance is no more than 75% of the average size of antigens of the represented clade. In some cases, an antigen can be representative of a clade if its edit distance is no more than 70%, no more than 80%, no more than 90%, no more than 95%, or no more than 99% of the average size of antigens of the represented clade.

An antigen can be representative of a clade if its sequence is no more than 90% of the average size of antigens of the represented clade different than at least 95%, at least 96%, at least 97%, at least 98%, or at least 99% of all strains of the represented clade. In some cases, an antigen can be representative of a clade if its sequence is no more than 80% of the average size of antigens of the represented clade different than at least 95%, at least 96%, at least 97%, at least 98%, or at least 99% of all strains of the represented clade. In some cases, an antigen can be representative of a clade if its sequence is no more than 70% of the average size of antigens of the represented clade different than at least 95%, at least 96%, at least 97%, at least 98%, or at least 99% of all strains of the represented clade.

An antigen can be representative of a clade if its edit distance is up to 5% of the average size of at least 95%, at least 96%, at least 97%, at least 98%, or at least 99% of all strains of the represented clade. In some cases, an antigen can be representative of a clade if its edit distance is up to 10% of the average size of at least 95%, at least 96%, at least 97%, at least 98%, or at least 99% of all strains of the represented clade. In some cases, an antigen can be representative of a clade if its edit distance is up to 15% of the average size of at least 95%, at least 96%, at least 97%, at least 98%, or at least 99% of all strains of the represented clade.

An antigen can be representative of a clade if its edit distance is up to no more than 90% of the average size of at least 95%, at least 96%, at least 97%, at least 98%, or at least 99% of all strains of the represented clade. In some cases, an antigen can be representative of a clade if its edit distance is no more than 80% of the average size of at least 95%, at least 96%, at least 97%, at least 98%, or at least 99% of all strains of the represented clade. In some cases, an antigen can be representative of a clade if its edit distance is no more than 70% of the average size of at least 95%, at least 96%, at least 97%, at least 98%, or at least 99% of all strains of the represented clade.

Some vaccines can comprise antigens having an average edit distance from each of the other antigens which is at least 5% of the average size of antigens in the clade. Some vaccines can comprise antigens having an average edit distance from at least one of the other antigens which is at least 1%, at least 2%, at least 3%, at least 4%, at least 5%, at least 6%, at least 7%, at least 8%, at least 9%, at least 10%, at least 15%, at least 20%, or at least 25% of the average size of antigens in the clade. In such vaccines, some vaccines can comprise antigens having an average edit distance from each of the other antigens which is at least 1%, at least 2%, at least 3%, at least 4%, at least 5%, at least 6%, at least 7%, at least 8%, at least 9%, at least 10%, at least 15%, at least 20%, or at least 25% of the average size of antigens in the clade. In such vaccines, the actual edit distances can be varied, or can be similar. In some cases, no antigens will have an actual edit distance which is exactly at least 5% of the average size of antigens in the clade.

In some cases, the smallest pairwise edit distance between two or more of the antigens in the set may be no more than 1. In some cases, the smallest pairwise edit distance between two or more of the antigens in the set may be no more than 2, no more than 3, no more than 4, no more than 5, no more than 6, no more than 7, no more than 8, no more than 9, no more than 10, no more than 20, no more than 30, no more than 40, or no more than 50. In some cases, the smallest pairwise edit distance between two or more of the antigens in the set can be at least 1. In some cases, the smallest pairwise edit distance between two or more of the antigens in the set can be at least 2, at least 3, at least 4, at least 5, at least 6, at least 7, at least 8, at least 9, at least 10, at least 20, at least 30, at least 40, or at least 50.

In some cases, the smallest pairwise edit distance can be no more than about 1%, no more than about 2%, no more than about 3%, no more than about 4%, no more than about 5%, no more than about 6%, no more than about 7%, no more than about 8%, no more than about 9%, no more than about 10%, no more than about 15%, no more than about 20%, or no more than about 25% of the size of the antigens. In some cases, the smallest pairwise edit distance can be at least about 5% of the size of the antigens. In some cases, the smallest pairwise edit distance can be at least about 1%, at least about 2%, at least about 3%, at least about 4%, at least about 5%, at least about 6%, at least about 7%, at least about 8%, at least about 9%, at least about 10%, at least about 15%, at least about 20%, or at least about 25% of the size of the antigens.

In some cases, the largest pairwise edit distance can be at least 5, at least 10, at least 20, at least 30, at least 40, at least 50, at least 60, at least 70, at least 80, at least 90, at least 100, at least 150, at least 200, at least 250, at least 300, at least 350, at least 400, at least at least 450, at least 500, at least 550, at least 600, at least 700, or at least 800. In some cases, the largest pairwise edit distance can be no more than 5, no more than 10, no more than 20, no more than 30, no more than 40, no more than 50, no more than 60, no more than 70, no more than 80, no more than 90, no more than 100, no more than 150, no more than 200, no more than 250, no more than 300, no more than 350, no more than 400, no more than 450, no more than 500, no more than 550, no more than 600, no more than 700, or no more than 800.

In some cases, the largest pairwise edit distance can be no more than about 75% of the size of the antigens. In some cases, the largest pairwise edit distance can be no more than about 50%, no more than about 55%, no more than about 60%, no more than about 65%, no more than about 70%, no more than about 75%, no more than about 80%, no more than about 85%, no more than about 90%, or no more than about 95% of the size of the antigens. In some cases, the largest pairwise edit distance can be at least about 75% of the size of the antigens. In some cases, the largest pairwise edit distance can be at least about 50%, at least about 55%, at least about 60%, at least about 65%, at least about 70%, at least about 75%, at least about 80%, at least about 85%, at least about 90% or at least about 95% of the size of the antigens.

In some cases, the smallest pairwise edit distance between two or more of the antigens in the set can be no more than 1, and the largest pairwise edit distance can be at least 5, at least 10, at least 20, at least 30, at least 40, at least 50, at least 60, at least 70, at least 80, at least 90, at least 100, at least 200, at least 300, at least 400, at least 500, at least 600, at least 700, or at least 800. In some cases, the smallest pairwise edit distance between two or more of the antigens in the set can be at least 1, and the largest pairwise edit distance can be at least 5, at least 10, at least 20, at least 30, at least 40, at least 50, at least 60, at least 70, at least 80, at least 90, at least 100, at least 200, at least 300, at least 400, at least 500, at least 600, at least 700, or at least 800. In some cases, the smallest pairwise edit distance between two or more of the antigens in the set can be at least 1, and the largest pairwise edit distance can be no more than 5, no more than 10, no more than 20, no more than 30, no more than 40, no more than 50, no more than 60, no more than 70, no more than 80, no more than 90, no more than 100, no more than 200, no more than 300, no more than 400, no more than 500, no more than 600, no more than 700, or no more than 800. In some cases, the smallest pairwise edit distance between two or more of the antigens in the set can be no more than 1, and the largest pairwise edit distance can be no more than 5, no more than 10, no more than 20, no more than 30, no more than 40, no more than 50, no more than 60, no more than 70, no more than 80, no more than 90, no more than 100, no more than 200, no more than 300, no more than 400, no more than 500, no more than 600, no more than 700, or no more than 800.

In some cases, the smallest pairwise edit distance between two or more of the antigens in the set can be no more than about 1%, and the largest pairwise edit distance can be at least about 75%. In some cases, the smallest pairwise edit distance between two or more of the antigens in the set can be at least about 1%, and the largest pairwise edit distance can be at least about 75%. In some cases, the smallest pairwise edit distance between two or more of the antigens in the set can be at least about 1%, and the largest pairwise edit distance can be no more than 75%. In some cases, the smallest pairwise edit distance between two or more of the antigens in the set can be no more than about 1%, and the largest pairwise edit distance can be no more than about 75%.

In some cases, the smallest pairwise edit distance between two or more of the antigens in the set can be no more than about 5%, and the largest pairwise edit distance can be at least about 75%. In some cases, the smallest pairwise edit distance between two or more of the antigens in the set can be at least about 5%, and the largest pairwise edit distance can be at least about 75%. In some cases, the smallest pairwise edit distance between two or more of the antigens in the set can be at least about 5%, and the largest pairwise edit distance can be no more than 75%. In some cases, the smallest pairwise edit distance between two or more of the antigens in the set can be no more than about 5%, and the largest pairwise edit distance can be no more than about 75%.

In some cases, the smallest pairwise edit distance between two or more of the antigens in the set can be no more than about 20%, and the largest pairwise edit distance can be at least about 75%. In some cases, the smallest pairwise edit distance between two or more of the antigens in the set can be at least about 20%, and the largest pairwise edit distance can be at least about 75%. In some cases, the smallest pairwise edit distance between two or more of the antigens in the set can be at least about 20%, and the largest pairwise edit distance can be no more than 75%. In some cases, the smallest pairwise edit distance between two or more of the antigens in the set can be no more than about 20%, and the largest pairwise edit distance can be no more than about 75%.

In some cases, the smallest pairwise edit distance between two or more of the antigens in the set can be no more than about 1%, and the largest pairwise edit distance can be at least about 90%. In some cases, the smallest pairwise edit distance between two or more of the antigens in the set can be at least about 1%, and the largest pairwise edit distance can be at least about 90%. In some cases, the smallest pairwise edit distance between two or more of the antigens in the set can be at least about 1%, and the largest pairwise edit distance can be no more than 90%. In some cases, the smallest pairwise edit distance between two or more of the antigens in the set can be no more than about 1%, and the largest pairwise edit distance can be no more than about 90%.

In some cases, the smallest pairwise edit distance between two or more of the antigens in the set can be no more than about 5%, and the largest pairwise edit distance can be at least about 90%. In some cases, the smallest pairwise edit distance between two or more of the antigens in the set can be at least about 5%, and the largest pairwise edit distance can be at least about 90%. In some cases, the smallest pairwise edit distance between two or more of the antigens in the set can be at least about 5%, and the largest pairwise edit distance can be no more than 90%. In some cases, the smallest pairwise edit distance between two or more of the antigens in the set can be no more than about 5%, and the largest pairwise edit distance can be no more than about 90%.

In some cases, the smallest pairwise edit distance between two or more of the antigens in the set can be no more than about 20%, and the largest pairwise edit distance can be at least about 90%. In some cases, the smallest pairwise edit distance between two or more of the antigens in the set can be at least about 20%, and the largest pairwise edit distance can be at least about 90%. In some cases, the smallest pairwise edit distance between two or more of the antigens in the set can be at least about 20%, and the largest pairwise edit distance can be no more than 90%. In some cases, the smallest pairwise edit distance between two or more of the antigens in the set can be no more than about 20%, and the largest pairwise edit distance can be no more than about 90%.

A vaccine can comprise a set of antigens wherein the smallest pairwise edit distance between the two of the antigens can be no more than 1%, no more than 2%, no more than 3%, no more than 4%, no more than 5%, no more than 6%, no more than 7%, no more than 8%, no more than 9%, no more than 10%, no more than 11%, no more than 12%, no more than 13%, no more than 14%, no more than 15%, no more than 16%, no more than 17%, no more than 18%, no more than 19%, or no more than 10% inclusive. A vaccine can comprise a set of antigens wherein the smallest pairwise edit distance between the two of the antigens can be at least 1%, at least 2%, at least 3%, at least 4%, at least 5%, at least 6%, at least 7%, at least 8%, at least 9%, at least 10%, at least 11%, at least 12%, at least 13%, at least 14%, at least 15%, at least 16%, at least 17%, at least 18%, at least 19%, or at least 10% inclusive. A vaccine can comprise a set of antigens wherein the largest pairwise edit distance between two of the antigens can be at least 50%, at least 55%, at least 60%, at least 65%, at least 70%, at least 75%, at least 80%, at least 85%, at least 90%, or at least 95%, inclusive. A vaccine can comprise a set of antigens wherein the largest pairwise edit distance between two of the antigens can be no more than 50%, no more than 55%, no more than 60%, no more than 65%, no more than 70%, no more than 75%, no more than 80%, no more than 85%, no more than 90%, or no more than 95%, inclusive.

In some cases, each antigen in a set can share at least 90%, at least 95%, or at least 99% sequence identity to at least one antigen in the set over a length of at least 100 amino acids. In some cases, each antigen in a set can share at least 90%, at least 95%, or at least 99% sequence identity to at least one antigen in the set over a length of at least 300 amino acids. In some cases, each antigen in a set can share at least 90%, at least 95%, or at least 99% sequence identity to at least one antigen in the set over a length of at least 500 amino acids.

A vaccine can comprise antigens in a set which differ from other antigens in the set by at least 1%, at least 5%, or at least 10% sequence identity. In some cases, a vaccine can comprise antigens in a set which differ from all other antigens in the set by at least 1%, at least 5%, or at least 10% sequence identity.

A vaccine can comprise antigens in a set which differ from at least one other antigen in the set by no more than 75% sequence identity. In some cases, a vaccine can comprise antigens in a set which differ from all other antigens in the set by no more than 75% sequence identity.

A vaccine can comprise a set of antigens that are representative of at least 40%, at least 45%, at least 50%, at least 55%, at least 60%, at least 65%, at least 70%, at least 75%, at least 80%, at least 85%, or at least 90% of fifth order clades of a microbe. In some cases, vaccine can comprise a set of antigens that are representative of at least 40%, at least 45%, at least 50%, at least 55%, at least 60%, at least 65%, at least 70%, at least 75%, at least 80%, at least 85%, or at least 90% of sixth order clades of a microbe. In further cases, vaccine can comprise a set of antigens that are representative of at least 40%, at least 45%, at least 50%, at least 55%, at least 60%, at least 65%, at least 70%, at least 75%, at least 80%, at least 85%, or at least 90% of seventh order clades of a microbe. In some cases, a vaccine can comprise a set of antigens that are representative of at least 40%, at least 45%, at least 50%, at least 55%, at least 60%, at least 65%, at least 70%, at least 75%, at least 80%, at least 85%, or at least 90% of eighth order clades of a microbe. In some cases, a vaccine can comprise a set of antigens that are representative of at least 40%, at least 45%, at least 50%, at least 55%, at least 60%, at least 65%, at least 70%, at least 75%, at least 80%, at least 85%, or at least 90% of ninth order clades of a microbe. In some cases, a vaccine can comprise a set of antigens that are representative of at least 40%, at least 45%, at least 50%, at least 55%, at least 60%, at least 65%, at least 70%, at least 75%, at least 80%, at least 85%, or at least 90% of tenth order clades of a microbe. In some cases, a vaccine can comprise a set of antigens that are representative of at least 40%, at least 45%, at least 50%, at least 55%, at least 60%, at least 65%, at least 70%, at least 75%, at least 80%, at least 85%, or at least 90% of eleventh order clades of a microbe. In some cases, a vaccine can comprise a set of antigens that are representative of at least 40%, at least 45%, at least 50%, at least 55%, at least 60%, at least 65%, at least 70%, at least 75%, at least 80%, at least 85%, or at least 90% of twelfth order clades a microbe. In some cases, a vaccine can comprise a set of antigens that are representative of at least 40%, at least 45%, at least 50%, at least 55%, at least 60%, at least 65%, at least 70%, at least 75%, at least 80%, at least 85%, or at least 90% of thirteenth order clades of a microbe. In some cases, a vaccine can comprise a set of antigens that are representative of at least 40%, at least 45%, at least 50%, at least 55%, at least 60%, at least 65%, at least 70%, at least 75%, at least 80%, at least 85%, or at least 90% of fourteenth order clades of a microbe. In some cases, a vaccine can comprise a set of antigens that are representative of at least 40%, at least 45%, at least 50%, at least 55%, at least 60%, at least 65%, at least 70%, at least 75%, at least 80%, at least 85%, or at least 90% of fifteenth order clades of a microbe. In some cases, a vaccine can comprise a set of antigens that are representative of at least 40%, at least 45%, at least 50%, at least 55%, at least 60%, at least 65%, at least 70%, at least 75%, at least 80%, at least 85%, or at least 90% of twentieth order clades of a microbe In some cases, a vaccine can comprise a set of antigens that are representative of at least 40%, at least 45%, at least 50%, at least 55%, at least 60%, at least 65%, at least 70%, at least 75%, at least 80%, at least 85%, or at least 90% of twenty-fifth order clades of a microbe. In some cases, a vaccine can comprise a set of antigens that are representative of at least 40%, at least 45%, at least 50%, at least 55%, at least 60%, at least 65%, at least 70%, at least 75%, at least 80%, at least 85%, or at least 90% of thirtieth order clades of a microbe.

A vaccine can comprise antigens that are representative of at least 60% of each of the third, fourth, fifth, sixth, seventh, eighth, ninth, tenth, eleventh, twelfth, thirteenth, fourteenth, fifteenth, or other suitable order clades of a microbe. In some cases, a vaccine can comprise antigens that are representative of at least 70% of each of the third, fourth, fifth, sixth, seventh, eighth, ninth, tenth, eleventh, twelfth, thirteenth, fourteenth, fifteenth, or other suitable order clades of a microbe. In further cases, a vaccine can comprise antigens that are representative of at least 80% of each of the third, fourth, fifth, sixth, seventh, eighth, ninth, tenth, eleventh, twelfth, thirteenth, fourteenth, fifteenth, or other suitable order clades of a microbe. In yet further cases, a vaccine can comprise antigens that are representative of at least 90% of each of the third, fourth, fifth, sixth, seventh, eighth, ninth, tenth, eleventh, twelfth, thirteenth, fourteenth, fifteenth, or other suitable order clades of a microbe.

The smallest number of branches between any 2 antigens can be 1 in some vaccines. The largest number of branches between any two antigens can be at least 5, at least 10, at least 15, or at least 20. In some vaccines, the smallest number of branches between any 2 antigens can be 1 and the largest number of branches between any two antigens can be at least 5. In some vaccines, the smallest number of branches between any 2 antigens can be 1 and the largest number of branches between any two antigens can be at least 10. In some vaccines, the smallest number of branches between any 2 antigens can be 1 and the largest number of branches between any two antigens can be at least 15. In some vaccines, the smallest number of branches between any 2 antigens can be 1 and the largest number of branches between any two antigens can be at least 20. In some vaccines, the smallest number of branches between any 2 antigens can be less than 5 and the largest number of branches between any two antigens can be at least 5. In some vaccines, the smallest number of branches between any 2 antigens can be less than 5 and the largest number of branches between any two antigens can be at least 10. In some vaccines, the smallest number of branches between any 2 antigens can be less than 5 and the largest number of branches between any two antigens can be at least 15. In some vaccines, the smallest number of branches between any 2 antigens can be less than 5 and the largest number of branches between any two antigens can be at least 20. In some vaccines, the smallest number of branches between any 2 antigens can be less than 10 and the largest number of branches between any two antigens can be at least 10. In some vaccines, the smallest number of branches between any 2 antigens can be less than 10 and the largest number of branches between any two antigens can be at least 15. In some vaccines, the smallest number of branches between any 2 antigens can be less than 10 and the largest number of branches between any two antigens can be at least 20.

A vaccine which is a flu vaccine can comprise antigens selected from Table 2, or a fragment or homologue thereof. If a vaccine comprises a fragment or homologue of an antigen, the fragment or homologue can be of at least a certain size. In some cases, a fragment or homologue can be at least 10, at least 15, at least 20, at least 25, at least 30, at least 35, at least 40, at least 45, at least 50, at least 55, at least 60, at least 65, at least 70, at least 75, at least 80, at least 85, at least 90, at least 95, at least 100, at least 110, at least 120, at least 150, at least 200, at least 300, at least 400, at least 500, at least 600, at least 700, at least 800, at least 900, or at least 1000 amino acids. In some cases, a fragment or homologue can be at most 10, at most 15, at most 20, at most 25, at most 30, at most 35, at most 40, at most 45, at most 50, at most 55, at most 60, at most 65, at most 70, at most 75, at most 80, at most 85, at most 90, at most 95, at most 100, at most 110, or at most 120 amino acids, at most 150 amino acids, at most 200 amino acids, at most 300 amino acids, at most 400 amino acids, at most 500 amino acids, at most 600 amino acids, at most 700 amino acids, at most 800 amino acids, at most 900 amino acids, or at most 1000 amino acids.

In some cases, an antigen in a vaccine can be selected from SEQ ID NOs: 1-87. In some cases, an antigen in a vaccine can be at least about 40%, at least about 45%, at least about 50%, at least about 55%, at least about 60%, at least about 65%, at least about 70%, at least about 75%, at least about 80%, at least about 90%, or at least about 95% identical to a sequence selected from SEQ ID NOs: 1-87.

In some cases, an antigen in a vaccine can be selected from SEQ ID NOs:88-127. In some cases, an antigen in a vaccine can be at least about 40%, at least about 45%, at least about 50%, at least about 55%, at least about 60%, at least about 65%, at least about 70%, at least about 75%, at least about 80%, at least about 90%, or at least about 95% identical to a sequence selected from SEQ ID NOs:88-127.

In some cases, an antigen in a vaccine can be selected from SEQ ID NOs: 128-171. In some cases, an antigen in a vaccine can be at least about 40%, at least about 45%, at least about 50%, at least about 55%, at least about 60%, at least about 65%, at least about 70%, at least about 75%, at least about 80%, at least about 90%, or at least about 95% identical to a sequence selected from SEQ ID NOs: 128-171.

In some cases, an antigen in a vaccine can be selected from SEQ ID NOs:172-267. In some cases, an antigen in a vaccine can be at least about 40%, at least about 45%, at least about 50%, at least about 55%, at least about 60%, at least about 65%, at least about 70%, at least about 75%, at least about 80%, at least about 90%, or at least about 95% identical to a sequence selected from SEQ ID NOs: 172-267.

**Table 6: Amino acid sequences of hemagglutinin antigens that can be included in a flu vaccine**

| **SEQ ID** | **Sequence** |
|---|---|
| 1 | EDTICIGYHANNSTDTVDTVLEKNVTVTHSVNLLEDSHNGKLCKLKGIAPLQLGKCNIAGWLLGNPECDLLLTASSWSYIVETS NSENGTCYPGDFIDYEELREQLSSVSSFEKFEIFPKTSSWPNHETTKGVTAACSYAGASSFYRNLLWLTKKGSSYPKLSKSYVN NKGKEVLVLWGVHHPPTGTDQQSLYQNADAYVSVGSSKYNRRFTPEIAARPKVRDQAGRMNYYWTLLEPGDTITFEATGNLI APWYAFALNRGSGSGIITSDAPVHDCNTKCQTPHGAINSSLPFQNIHPVTIGECPKYVRSTKLRMATGLRNIPSIQSRGLFGAIAG FIEGGWTGMIDGWYGYHHQNEQGSGYAADRKSTQNAIDGITNKVNSVIEKMNTQFTSVGKEFNHLEKRIENLNRKVDDGFLD VWTYNAELLVLLENERTLDYHDSNVKNLYEKVRSQLKNNAKEIGNGCFEFYHKCDDSCMESVKNGTYDYPKYSEESKLNRE EIDGVKLES |
| 2 | ADTICIGYHANNSTDTVDTIFEKNVAVTHSVNLLEDRHNGKLCKLKGIAPLQLGKCNIIGWLLGNPECDSLLPARSWSYIVETP NSENGACYPGDFIDYEELREQLSSVSSLERFEIFPKESSWPNHTFNGVTASCSHRGKSSFYRNLLWLTKKGDSYPKLTNSYVNN KGKEVLVLWGVHHPSSSDEQQSLYSNGNAYVSVASSNYNRRFTPEIAARPKVKDQHGRMNYYWTLLEPGDTIIFEATGNLIAP WYAFALSRGFESGIITSNASMHECNTKCQTPQGSINSNLPFQNIHPVTIGECPKYVRSTKLRMVTGLRNIPSIQSRGLFGAIAGFIE GGWTGMIDGWYGYHHQNEQGSGYAADQKSTQNAINGITNKVNSIIEKMNTQFTAVGKEFNNLEKRMENLNKKVDDGFLDIW TYNAELLVLLENGRTLDFHDLNVKNLYEKVKSQLKNNAKEIGNGCFEFYHKCDNECMESVRNGTYDYPKYSEESKLNREKID GVKLES |
| 3 | ADTICIGYHANNSTDTVDTVLEKNVTVTHSVNLLEDSHNGKLCRLKGIAPLQLGKCNIAGWLLGNPECDPLLPVRSWSYIVETP NSENGICYPGDFIDYEELREQLSSVSSFERFEIFPKESSWPNHNTNGVTAACSHEGKSSFYRNLLWLTEKEGSYPKLKNSYVNKK GKEVLVLWGIHHPPNSKEQQNLYQNENAYVSVVTSNYNRRFTPEIAERPKVRDQAGRMNYYWTLLKPGDTIIFEANGNLIAP MYAFALSRGFGSGIITSNASMHECNTKCQTPLGAINSSLPYQNIHPVTIGECPKYVRSAKLRMVTGLRNIPSIQSRGLFGAIAGFIE GGWTGMIDGWYGYHHQNEQGSGYAADQKSTQNAINGITNKVNTVIEKMNIQFTAVGKEFNKLEKRMENLNKKVDDGFLDI WTYNAELLVLLENERTLDFHDSNVKNLYEKVKSQLKNNAKEIGNGCFEFYHKCDNECMESVRNGTYDYPKYSEESKLNREK VDGVKLES |
| 4 | ADTLCIGYHANNSTDTVDTVLEKNVTVTHSVNLLEDSHNGKLCRLGGIAPLQLGKCNIAGWLLGNPECDLLLTVSSWSYIVET SNSDNGTCYPGDFIDYEELREQLSSVSSFERFEIFPKTSSWPNHETTNGVTAACPYAGANSFYRNLLWLVKKGDSYPKLSKSYV NNKGKEVLVLWGVHHPPTSTEQQSLYQNADAYVSVGSSKYNRRFTPEIAARPKVRGQAGRMNYYWTLLEPGDTITFEATGNL VAPRYAFALNKGSGSGVITSDAPVHDCDTKCQTPHGAINSSLPFQNIHPVTIGECPKYVKSTKLRMATGLRNIPSIQSRGLFGAIA GFIEGGWTGMIDGWYGYHQQNGQGSGYAADQKSTQNAIDGITNKVNSVIEKMNTQFTAVGKEFNNLERRIENLNKKVDDGF LDVWTYNAELLVLLENERTLDFHDSNVKNLYEKVRSQLRNNAKEIGNGCFEFYHKCDNACMESVRNGTYDYPKYSEESKLN REEIDGVKLES |
| *5* | ADTICIGYQANNSTDTVDTLLEKNVTVTHSVNLLEDSHNGKLCKLKGIAPLQLGKCNIAGWLLGNPECDSLLPARSWSYIVETP NSETGACYPGDFIDYEELREQLSSVSSLERFEIFPKESSWPNHNTTGVTKSCSHRGESSFYRNLLWLTKKGNSYPELNNSYVNN KGKEVLVLWGVHHPSNSNNQQTLYHNANAYVSVVSSNYNRRFTPEIAARPKVRDQTGRMNYYWTLLEPGDTIIFEATGNLIA PWYAFALSRGFGSGIITSNASMHECNTKCQTPQGAINSSLPFQNIHPVTIGECPKYVRSTKLRMVTGLRNIPSIQSRGLFGAIAGFI EGGWTGMIDGWYGYHHQNEQGSGYAADQKSTQNAINRITNKVNSVIEKMNTQFTAVGKEFNNLEKRMENLNKEVDDGFLDI WTYNAELLVLLENEMTLDFHDSNVKNLYEKVKSQLKNNAKEIGNGCFEFYHKCDNECMESVRNGTYDYPKYSEDSKLNREK IDGVKLEP |
| 6 | ADTICIGYHANNSTDTVDTVLEKNVTVTHSVNLLEDSHNGKLCRLKGIAPLQLRKCNIAGWILGNPECESLLSERSWSYIVETP NSENGTCYPGDFTNYEELREQLSSVSSFERFEIFPKESSWPKHNTTRGVTAACSHAGKSSFYRNLLWLTEKDGSYPNLNNSYVN KKGKEVLVLWGVHHPSNIKDQQTLYQKENAYVSVVSSNYNRRFTPEIAERPKVRGQAGRMNYYWTLLKPGDTIMFEANGNLI APWYAFALSRGFGSGIITSNASMHECDTKCQTPQGAINSSLPFQNIHPVTIGECPKYVRSTKLRMVTGLRNIPSIQSRGLFGAIAG FIEGGWTGMIDGWYGYHHQNEQGSGYAADQKSTQNAINGITNKVNSVIEKMNTQFTAVGKEFNNLEKRMENLNKKVDDGFL DIWTYNAELLILLENERTLDFHDSNVKNLYEKVKSQLRNNAKEIGNGCFEFYHKCNNECMESVKNGTYDYPKYSEESKLNRE KIDGVKLES |
| 7 | ADTICIGYHANNSTDTVDTVLEKNVTVTHSVNLLEDSHNGKLCRLKGIAPLQLGKCNIAGWILGNPECDSLLPASSWSYIVETP NSKNGICYPGDFIDYEELREQLSSVSSFERFEIFPKESSWPKHSTTKGVTAACSHAGKSSFYRNLLWLTKKEDSYPKLSNSYVNK KGKEVLVLWGVHHPSSSKEQQTLYQNENAYVSVVSSNYNRRFIPEIAERPEVKDQAGRINYYWTLLEPGDTIIFEANGNLVAP WYAFALSRGFGSGIITSNASMHECNTKCQTPQGAINSSLPFQNIHPVTIGECPKYVKSAKLRMVTGLRNIPSIQSRGLFGAIAGFI EGGWTGMIDGWYGYHHQNEQGSGYAADQKSTQNAINGITNKVNSVIEKMNTQFTAVGKEFNNLEKRMENLNKKVDDGFLD IWTYNAELLVLLENERTLDFHDSNVKNLYEKVKIQLKNNAKEIGNGCFEFYHKCDNECMESVRNGTYDYPKYSKESKLNREKI DGVKLES |
| 8 | ADTICIGYHANNSTDTVDTVLEKNVTVTHSVNLLEDSHNGKLCRLKGKAPLQLGNCNIAGWVLGNPECESLLSNRSWSYIAET PNSENGTCYPGDFADYEELREQLSSVSSFERFEIFPKESSWPNHTTRGVTAACPHARKSSFYKNLVWLTEANGSYPNLSRSYVN NQEKEVLVLWGVHHPSNIEEQRALYRKDNAYVSVVSSNYNRRFTPEIAKRPKVRDQSGRMNYYWTLLEPGDTIIFEATGNLIA PWYAFALSRGPGSGIITSNAPLDECDTKCQTPQGAINSSLPFQNIHPVTIGECPKYVRSTKLRMVTGLRNIPSIQSRGLFGAIAGFI EGGWTGMMDGWYGYHHQNEQGSGYAADQKSTQNAINGITNKVNSVIEKMNTQFTAVGKEFNKLEKRMENLNKKVDDGFM DIWTYNAELLVLLENERTLDFHDSNVKNLYEKVKNQLRNNAKELGNGCFEFYHKCDNECMESVKNGTYDYPKYSEESKLNR EKIDGVKLES |
| 9 | ADTLCIGYHANNSTDTVDTVLEKNVTVTHSVNLLEDRHNGKLCKLGGIAPLHLGKCNIAGWLLGNPECELLLTVSSWSYIVET SNSDNGTCYPGDFINYEELREQLSSVSSFERFEIFPKTSSWPNHETNRGVTAACPYAGANSFYRNLIWLVEKGNSYPKLSKSYV NNKGKEVLVLWGIHHPPTSTDQQSLYQNADAYVFVGSSKYNRKFKPEIAARPKVRGQAGRMNYYWTLIEPGDTITFEATGNL VVPRYAFAMNRGSGSGIIISDAPVHDCNTKCQTPKGAINTSLPFQNIHPVTIGECPKYVKSTKLRMATGLRNIPSIQSRGLFGAIA GFIEGGWTGMIDGWYGYHHQNEQGSGYAADQRSTQNAIDGITNKVNSVIEKMNTQFTAVGKEFNHLEKRIENLNKKVDDGFL DIWTYNAELLVLLENERTLDFHDSNVKNLYEKVRSQLRNNAKEIGNGCFEFYHKCDDTCMESVKNGTYDYPKYSEESKLNRE EIDGVKLES |
| 10 | ADTICIGYHANNSTDTVDTVLEKNVTVTHSVNLLEDSHNGKLCRLKGIAPLQLGKCNIAGWILGNPECESLFSKKSWSYIAETP NSENGTCYPGYFADYEELREQLSSVSSFERFEIFPKESSWPKHNVTRGVTASCSHKGKSSFYRNLLWLTEKNGSYPNLSKSYVN NKEKEVLVLWGVHHPSNIEDQKTIYRKENAYVSVVSSNYNRRFTPEIAERPKVRGQAGRINYYWTLLEPGDTIIFEANGNLIAP WHAFALNRGFGSGIITSNASMDECDTKCQTPQGAINSSLPFQNIHPVTIGECPKYVRSTKLRMVTGLRNIPSIQSRGLFGAIAGFI EGGWTGMIDGWYGYHHQNEQGSGYAADQKSTQNAINGITNKVNSVIEKMNTQFTAVGKEFNKLEKRMENLNKKVDDGFLD IWTYNAELLVLLENERTLDFHDSNVKNLYEKVKSQLKNNAKEIGNGCFEFYHKCNNECMESVKNGTYDYPKYSEESKLNREK IDGVKLES |
| 11 | ADTICIGYHANNSTDTVDTVLEKNVTVTHSVNLLEDSHNGKLCRLKGIAPLQLGNCSIAGWILGNPECESLFSKKSWSYIAETP NSENGTCYPGYFADYEELREQLSSVSSFERFEIFPKESSWPNHTVTKGVTASCSHKGKSSFYRNLLWLTEKNGSYPTLSKSYVN NKEKEVLVLWGVHHPSNIGDQRAIYHTENAYVSVVSSHYNRRFTPEIAKRPKVRGQEGRINYYWTLLEPGDTIIFEANGNLIAP WYAFALSRGFGSGIITSNASMDECDAKCQTPQGAINSSLPFQNVHPVTIGECPKYVRSTKLRMVTGLRNIPSIQSRGLFGAIAGFI EGGWTGMIDGWYGYHHQNEQGSGYAADQKSTQNAINGITNKVNSVIEKMNTQFTAVGKEFNKLERRMENLNKKVDDGFLDI WTYNAELLVLLENERTLDFHDSNVKNLYEKVKSQLKNNAKEIGNGCFEFYHKCNNECMESVKNGTYDYPKYSEESKLNREKI DGVKLES |
| 12 | ADTICIGYHANNSTDTVDTVLEKNVTVTHSVNLLEDSHNGKLCRLKGIAPLQLGNCSVAGWILGNPKCESLFSKESWSYIAETP NPENGTCYPGYFADYEELREQLSSVSSFERFEIFPKESSWPNHTVTKGVTTSCSHNGKSSFYRNLLWLTEKNGLYPNLSKSYVN NKEKEVLVLWGVHHPSNIRDQRAIYHTENAYVSVVSSHYSRRFTPEIAKRPKVRGQEGRINYYWTLLEPGDTIIFEANGNLIAP WYAFALSRGFGSGIITSNASMDECDAKCQTPQGAINSSLPFQNVHPVTIGECPKYVRSTKLRMVTGLRNIPSIQSRGLFGAIAGFI EGGWTGMIDGWYGYHHQNEQGSGYAADQKSTQNAINGITNKVNSVIEKMNTQFTAVGKEFNKLERRMENLNKKVDDGFLDI WTYNAELLVLLENGRTLDFHDSNVKNLYEKVKSQLKNNAKEIGNGCFEFYHKCNNECMESVKNGTYDYPKYSEESKLNRGKI DGVKLES |
| 13 | ADTICVGYHANNSTDTVDTILEKNVTVTHSVNLLENNHNGKLCSLNGIAPLQLGDCNVAGWILGNPECDSLLTANSWSYIIETS NSENGTCYPGEFADYEELRELLSSVSSFERFEIFPKATSWPNHETTKGVTAACSHSGARSFYRNLLWIVKKGNSYPKLSKSYTN NRRKEVLVIWGVHHPPTNNDQQSLYQNADAYVSVGSSKYYRRFTPEIADRPKVRGQAGRMNYYWTMLDQGDTITFEATGNL IAPWYAFALNKGLSSGIIMSDAPVHNCTTRCQTPYGALNSNLPFQNVHPITIGKCPKYVKSTQLRMATGLRNVPSIQSRGLFGAI AGFIEGGWTGMVDGWYGYHHQNEQGSGYAADQKSTQNAIDGISNKVNSIIEKMNIQFTSVGKEFNDLEKRVENLNKKVDDG FLDVWTYNAELLVLLENERTLDFHDFNVRNLYEKVKSQLRNNAKEVGNGCFEFYHKCDDECMESVRNGTYNYPKYSEESKL NREKIDGVKLES |
| 14 | ADTICIGYHANNSTDTVDTVLEKNVTVTHSVNLLEDSHNGKLCLLKGIAPLQLGNCSVAGWILGNPECESLISKESWSYIVETP NPENGTCYPGYFADYEELREQLSSVSSFERFEIFPKESSWPNHTVTGVTASCSHNGKSSFYRNLLWLTEKNGLYPNLSNSYVNN KEKEVLVLWGVHHPSNIRDQRAIYHTENAYVSVVSSHYSRRFTPEIAKRPKVRGQEGRINYYWTLLEPGDTIIFEANGNLIAPW YAFALSRGFGSGIITSNAPMNECDAKCQTPQGAINSSLPFQNVHPVTIGECPKYVRSTKLRMVTGLRNIPSIQSRGLFGAIAGFIE GGWTGMMDGWYGYHHQNEQGSGYAADQKSTQNAINGITNKVNSVIEKMNTQFTAVGKEFNKLERRMENLNKKVDDGFLDI WTYNAELLVLLENERTLDFHDSNVKNLYEKVKSQLKNNAKEIGNGCFEFYHKCNNECMESVKNGTYDYPKYSEESKLNREKI DGVKLES |
| *15* | ADTICIGYHANNSTDTVDTVLEKNVTVTHSVNLLENNHNGKLCNLNGIAPLQLGKCNVAGWLLGNPECGLLLNANAWSYIIET SDSKNGTCYPGDFIDYEELREQLGSVSSFEKFEIFPKASSWPDHETTKGTTAACPYSGVESFYRNLLWIIKKGNSYPKISKSYTNN RGKEVLVLWGVHHPPTTSDQQTLYQNIDAYVSVGSSKYNRRFTPEIATRPKVRGLAGRMNYYWTLLDQGDTIMFEATGNLIA PWYAFALNKGSDSGIITSDTPVHDCDTKCQTPYGALNSSLPFQNVHPITIGECPKYVKSTKLRMATGLRNVPSIQSKGLFGAIAG FIEGGWTGMIDGWYGYHHQNEQGSGYSADQKSTQNAIDGITNKVNSVIEKMNTQFTAVGKEFNNLERRIENLNKKVDDGFLD VWTYNAELLVLLENERTLDFHDSNVRDLYERVKSQLRNNAKEIGNGCFEFYHKCDDECMENVKNGTYDYPKYSEEAKLNRE KIDGVKIES |
| 16 | ADTICIGYHANNSTDTVDTIFEKNVAVTHSVNLLEDSHNGKLCLLKGIAPLQLGNCSVAGWILGNPECELLISKESWSYIVETPN PENGTCYPGYFADYEELREQLSSVSSFERFEIFPKESSWPNHTVTGVSASCSHNGKSSFYRNLLWLTGKNGLYPNLSKSYANNK EKEVLVLWGVHHPPNIGDQQGLYSNGNAYVSVASSNYNRRFTPEIAARPKVKDQHGRMNYYWTLLEPGDTIIFEATGNLIAP WYAFALSRGFESGIITSNASMHECNTKCQTPQGSINSNLPFQNIHPVTIGECPKYVRSAKLRMVTGLRNIPSIQSRGLFGAIAGFIE GGWTGMVDGWYGYHHQNEQGSGYAADQKSTQNAINGITNKVNSVIEKMNTQFTAVGKEFNRLERRMENLNKKVDDGFLDI WTYNAELLVLLENERTLDFHDSNVKNLYEKVKSQLKNNAKEIGNGCFEFYHKCNNECMESVKNGTYDYPKYSEESKLNREKI DGVKLES |
| 17 | ADTLCIGYHANNSTDTVDTVLEKNVTVTHSVNLLENKHNGKLCKLRGVAPLHLGKCNIAGWLLGNPECESLFEASSWSYIVET PNSDNGTCYPGDFINYEELREHLSSVSSFERFEIFPKANSWPNHDTDSGVTASCPYAGAKSFYRNLIWLVKKGSSYPKLSKSYIN DKKKEVLVIWGIHHPPNSTDQQTLYQNADAYVFVGSSKYSKRFKPEIAARPKVRDQAGRMNYYWTLIEPGDTITFEATGNLVV PRYAFAMTRGSGSGIIISDTPVHDCNTTCQTPKGAINTSLPFQNIHPVTIGECPKYVKSTKLKMATGLRNIPSIQSRGLFGAIAGFI EGGWTGMIDGWYGYHHQNEQGSGYAADQKSTQNAIDGITNKVNSVIEKMNTQFTAVGKEFNQLEKRIESLNNKVDDGFLDI WTYNAELLVLLENERTLDYHDSNVKNLYEKVRSQLKNNAKEIGNGCFEFYHKCDDTCMESVKNGTYDYPKYSEEAKLNREEI DGVKLES |
| 18 | ADTLCIGYHANNSTDTIDTVLEKNVTVTHSVNLLENKHNGKLCKLGGMTPLHLGKCNIAGWLLGNPECELLLSTVSSWSYIVET PYSDNGTCYPGDFTNYEELREQLSSVSSFERFEIFPKASSWPNHETNRGVTSACPYAGENDFYRNLLWLVKKGNSYPKLSKSYV NNKGKEVLILWGIHHPPTSADQQSLYQNADAYVFVGTSTYNRRFTPEVAARPKVRGQAGRMDYYWTLIGPGDTITFEATGNL VVPRYAFAMNRGSGSGIIISDIPFHNCTTKCQTPKGAINTSLPFQNVHPVTIGECPKYVKSTKLRMVTGLRNIPSIQSRGLFGAIA GFIEGGWTGMIDGWYGYHHQNEQGSGYAADQKSTQNAIDKITNKVNSVIEKMNTQFTAVGKEFNHLERRIENLNRKVDDGFL DVWTYNAELLILLENERTLDLHDSNVKNLYEKVKSQLRNNAKEIGNGCFEFYHKCDDACMESVRNGTYDYPKYSEESRLNRE EIDGVKLES |
| 19 | ADTLCIGYHANNSTDTVDTILEKNVTVTHSVNLLEDRHNGKLCKLRGVAPLHLGKCNIAGWLLGNPECESLFTARSWSYIVET SNSDNGTCYPGDFINYEELREQLSSVSSFERFEIFPNTSSWPNHDTNRGVTAACPHAGTKSFYRNLIWLVKKGDSYPKISKSYIN NKEKEVLVLWGIHHPSTSADQQSLYQNADAYVFVGSSKYSKKFKPEIATRPKVRDQEGRMDYYWTIVEPGDKITFEATGNLV VPRYAFELKRNSGSGIIISDTSVHDCNTTCQTPKGAINTSLPFQNIHPVTIGECPKYVKSTKLRMATGLRNIPSIQSRGLFGAIAGFI EGGWTGMIDGWYGYHHQNEQGSGYAADLKSTQNAIDGITNKVNSIIEKMNTQFTAVGKEFSHLEKRIENLNKKVDDGFLDIW TYNAELLVLLENERTLDYHDSNVKNLYEKVKSQLKNNAKEIGNGCFEFYHKCDDMCMESVKNGTYDYPKYSEEAKLNREEI DGVKLES |
| 20 | ADTICVGYHANNSTDTVDTILEKNVTVTHSVNLLESNHNGKLCSLNGKAPLQLGNCNVAGWILGNPECDLLLTANSWSYIIET SNSKNGACYPGEFADYEELRELLSTVSSFERFEIFPKATSWPHHETTKGTTVACPHSGVNSFYRNLLWIVKKGNSYPKLSKSYT NSKGKEVLVIWGVHHPPTDSDQQTLYQNNHTYVSVGTSKYYRRFTPEIVARPKIREQAGRMNYYWTLLDQGDTITLEATGNLI VPWHAFALNKGSNSGIMISDADFHNCTTKCQTPHGALKSNLPFQNVHPITIGECPKYVKSTQLRMATGLRNVPSIQSRGLFGAI AGFIEGGWTGMIDGWYGYHHQNEQGSGYAADQKSTQIAIDGISNKVNSVIEKMNIQFASVGKEFNDLEKRIENLNKKVDDGF VDVWTYNAELLILLENERTLDFHDFNVKNLYEKVKSQLRNNAKEIGNGCFEFYHKCDNECMESVKNGTYNYPKYSEESKLNR EEIDGVKLES |
| 21 | ADTLCIGYHANNSTDTVDTILEKNVTVTHSVNLLEDKHNGRLCKLGGIAPLNLGRCNIAGWLLGNPECESLFTVSSWSYIVETS NSYNGTCYPGDFINYEELREQLSSVSSFERFEIFPMASSWPNHETTKGVTAACSHAGENSFYRNLIWLVKKENTYPKISKSYINN KGKEVLVLWGIHHPPTNNDQQSLYQNADTYVFVGTSKYNKKFKPEIATRPKVRDQAGRMNYYWTLVEPGDTITFEATGNLV APRYAFAMNKGSGSGIIVSDIPAHDCNTNCQTPKGAINTSLPFQNVHPVTIGECPKYVKSTRLRMATGLRNIPSIQSRGLFGAIAG FIEGGWTGMIDGWYGYHHQNGQGSGYAADQKSTQNAIDGITNKVNSVIEKMNTQFTAVGKEFNHLEKRIENLNKKVDDGFL DVWTYNAELLILLENERTLDFHDSNVKNLYEKARRQLKNNAKEIGNGCFEFYHKCDNACMESVKNGTYDYPKYSEESRLNRE EIDGVKLDS |
| 22 | ADTLCIGYHANNSTDTVDTVLEKNVTVTHSVNLLENKHDGKLCKLRGIAPLHLGKCNIAGWILGNPECESLFTTSSWSYIVETP NSDNGTCYPGDFINYEELREHLSSVSSFERFEIFPKANSWPYHDTNKGVTAACPYAGANSFYRNLIWLVKKGNSYPKLSKSYIN NKKKEVLVIWGIHHPPTSTDQQTLYQNADAYVFVGSSKYSKRFKPEIAARPKVRDQAGRMDYYWTLIEPGDTITFEATGNLVV PRYAFTMKRGSGSGIIVSDAPVHDCNTTCQTPKGAINTSLPFQNIHPVTIGECPKYVKSTKLRMATGLRNIPSIQSRGLFGAIAGFI EGGWTGMIDGWYGYHHQNEQGSGYAADQKSTQNAIDGITNKVNSIIEKMNTQFTAVGKEFSQLEKRIESLNNKVDDGFLDIW TYNAELLVLLENERTLDYHDSNVKNLYEKVRSQLKNNAKEIGNGCFEFYHKCDDTCMESVKNGTYDYPKYSEEAKLNRQEID GVKLES |
| 23 | ADTLCIGYHANNSTDTVDTILEKNVTVTHSVNLLEDRHNGKLCNLRGEAPLHLGKCNIAGWLLGNPECELLFAVNSWSYIVET SNSDNGTCYPGDFTSYEELREQLSSVSSFERFEIFPKASSWPNHETNRGVTAACPYAGTNSFYRNLIWLVKKGNSYPKLSKSYV NNKKKEVLVLWGIHHPPTNADQQSLYQNADAYVFVGSSKYNKKFKPEIAKRPKVRGQAGRMNYYWTLVEPGDTITFEATGN LVAPRYAFAMNRDPGSGIITSDAPIHDCNATCQTPKGAINTSLPFQNIHPITIGECPKYVKSTRLRMATGLRNIPSIQSRGLFGAIA GFIEGGWTGMIDGWYGYHHQNGQGSGYAADQKSTQNAIDRITNKVNSVIEKMNIQFTAVGKEFNHLERRIENLNKKVDDGFL DVWTYNAELLVLLENERTLDFHDSNVKTLYEKVKTQLRNNAKEIGNGCFEFYHKCDDTCMESIKNGTYDYPKYSKESKLNRE EIDGVQLES |
| 24 | ADTLCVGYHANNSTDTVDTVLEKNVTVTHSVNLLEDNHNGKLCKLRGIAPLHLGKCNIAGWLLGNPECELLFTAKSWSYIVE TSNPDNGICYPGDFSNYEELREQLSSVSSFERFEIFPNASSWPNHETNRGITAACPYAGANSFYRNLIWLVKKGNSYPKINKSYIN NKGKEVLVLWGIHHPSTSNDQQSLYQNADAYVFVGSSKYNRRFKPEIATRPKVRDQAGRMNYYWTLVEPGDTITFEATGNLI APRYAFAMTRGSGSGIMISDTPVYDCNTICQTPKGAINTSLPFQNIHPVTIGKCPKYVKSTKLRLATGLRNIPSIQYRGLFGAIAG FIEGGWTGMVDGWYGYHHQNEQGSGFAADRKSTQNAIDGITNKVNSVIEKVNTQFTAVGKEFNHLEKRIENLNKKVDDGFL DVWTYNAELLVLLENERTLDFHDSNVKNLYEKVRSQLRNNAKEIGNGCFEFYHKCDNTCMESVKNGTYDYPRYSEESKLNRE EIDGVKLES |
| 25 | ADTICVGYHANNSTDTVDTILEKNVTVTHSVNILETTHNGKLCSLSGIAPLQLGNCNIAGWILGNPECDLLLTVNSWSYIIETSN SVNGTCYPGEFADYEELREQLSSVASFERFEIFPKTTSWPNHETTRGTTAACSHSGTRSFYRNLLWIVKKGNSYPKISKSYTNKK GKEVLVIWGVHHPPTNNDQQSLYQNAYTYVSVESSKYYRRFTPEIASRPKVRGQEGRMNYYWTLLDQGDTITFEATGNLIAP WYAFALNKGSDSGIIISDAQVHNCTTKCQTPHGALNSTLPFQNVHPITIGECPKYVKSTHLRMATGLRNVPSIQSRGLFGAIAGFI EGGWTGMIDGWYGYHHQNEQGSGYAADKKSTQIAIDGISNKVNSVIEKMNIQFTSVGKEFNNLEKRIENLNKKVDDGFLDIW TYNAELFVLLENERTLDFHDFNVKNLYERVKSQLRNNAKEVGNGCFEFYHKCDNECMESVKNGTYNYPKYSEESKLNREEID GVKLES |
| 26 | ADTLCIGYHANNSTDTVDTVLEKNITVTHSVNLLEDKHNGKLCKLRGVAPLHLGKCNIAGWLLGNPECESLFNVSSWSYIVET SNSDNGTCYPGDFTNYEELKEQLSSVSSFERFEIFPKEGSWPNHETNRGVTAACPHAGANSFYKNLIWLVKRGNSYPKLNKSY VNNKKKEVLVLWGIHHPPTSTDQQSLYQNADAYVFVGSSKYSKKFKPEIATRPKVRDQAGRMNYYWTLVEPEDTITFEATGN LVVPRYAFAMNRGSGSGIITSDTPVHDCNTTCQTPKGAINTSLPFQNVHPITIGECPKYVKSTKLRMATGLRNIPSIQSRGLFGAI AGFIEGGWTGMIDGWYGYHHQNGQGSGYAADQKSTQNAIDGVTNKVNSIIEKMNTQFAAVGKEFNHLEKRIENLNKKVDDG FLDVWTYNAELLVLLENERTLDFHDSNVRNLYDKVRSQLRNNAKEIGNGCFEFYHKCDDTCMESVKNGTYDYPKYSEESKLN REEIDGVRLDS |
| 27 | ADTICIGYHANNSTDTVDTVLEKNVTVTHSVNLLEDSHNGKLCLLKGIAPLQLGNCSVAGWILGNPECELLISRESWSYIVEKP NPENGTCYPGHFADYEELREQLSSVSSFERFEIFPKESSWPNHTTTGVSASCSHNGESSFYKNLLWLTGKNGLYPNLSKSYANN KEKEVLVLWGVHHPPNIGDQRALYHKENAYVSVVSSHYSRKFTPEIAKRPKVRDQEGRINYYWTLLEPGDTIIFEANGNLIAPR YAFALSRGFGSGIINSNAPMDECDAKCQTPQGAINSSLPFQNVHPVTIGECPKYVRSAKLRMVTGLRNIPSIQSRGLFGAIAGFIE GGWTGMVDGWYGYHHQNEQGSGYAADQKSTQNAINGITNKVNSVIEKMNTQFTAVGKEFNKLERRMENLNKKVDDGFIDI WTYNAELLVLLENERTLDFHDSNVKNLYEKVKSQLKNNAKEIGNGCFEFYHKCNDECMESVKNGTYDYPKYSEESKLNREKI DGVKLES |
| 28 | ADTLCIGYHANNSTETVDTVLEKNVTVTHSINLLEDKHNGKLCRLRGVTPLHLGKCNIAGWLLGNPECESLSTASSWSYIVETS NSDNGTCYPGDFINYEELREQLSSVSSFERFEIFPNASSWPDHETNKGVTAACPHAGAKSFYRNLLWLVKKKNSYPKLSKSYIN NKGKEVLVLWGIHHPSNIQDQETLYQNADTYVFVGSSKYSKKFKPEIATRPKVRDQTGRMNYYWTLVEPGDTITFEATGNLVI PKYAFAMKRGSGSGIIISDATVHDCNTTCQTPKGAINTSLPFQNIHPVTIGECPKYVKSTKLRMATGLRNVPSIQSRGLFGAIAGF IEGGWTGMIDGWYGYHHQNEQGSGYAADRKSTQNAIDGITNKVNSVIEKMNTQFSAVGKEFNHLEKRIENLNKKVDDGFLD VWTYNAELLVLLENERTLDYHDSNVKNLYEKVRSQLKNNAKEIGNGCFEFYHKCDDACMESVKNGTYDYPKYSEEAKLNRE KIDGVKLES |
| 29 | ADTICIGYHANNSTDTVDTVLEKNVTVTHSVNLLEDTHNGKLCKLGGMAPLHLGRCNIAGWLLGNPECELISTVSSWSYIVET SNSDNGTCYPGDFTNYEELREQLSSVSSFEKFEMFPKISSWPNHETNRGVTAACPYAGANSFYRNLIWLVKKGNSYPKLSKSY VNNKGKEVLVLWGIHHPPTSADQQSLYQNVDTYVFVGSSTYNRKFKPEIAVRPKVRDQTGRMNYYWTLIKPGDTITFEATGN LVAPRYAFAMSRGSGSGIITSDMPVHNCTTKCQTPKGALNTSLPFQNVHPVTIGECPKYVKSTKLRMATGLRNIPSIQSRGLFGA IAGFIEGGWTGMIDGWYGYHHQNEQGSGYAADQKSTQNAIDGITNKVNSVIEKMNTQFTAVGKEFNHLERRMENLNKKVDD GFLDVWTYNAELLILLENERTLDLHDSNVKNLYEKVRAQLRNNAKEIGNGCFEFYHKCDDACMESVKNGTYDYPKYSEESRL NREEIDGVKLES |
| 30 | ADTICIGYHANNSTDTVDTVLEKNVTVTHSVNLLENSHNGKLCLLKGIAPLQLGNCSVAGWILGNPECELLISKESWSYIVEKP NPENGTCYPGHFADYEELREQLSSVSSFERFEIFPKESSWPNHTVTGVSASCSHNGESSFYRNLLWLTGKNGLYPNLSKSYANN KEKEVLVLWGVHHPPNIGDQKALYHTENAYVSVVSSHYSRKFTPEIAKRPKVRDQEGRINYYWTLLEPGDTIIFEANGNLIAPR YAFALSRGFGSGIINSNAPMDKCDAKCQTPQGAINSSLPFQNVHPVTIGECPKYVRSAKLRMVTGLRNIPSIQSRGLFGAIAGFIE GGWTGMVDGWYGYHHQNEQGSGYAADQKSTQNAINGITNKVNSVIEKMNTQFTAVGKEFNKLERRMENLNKKVDDGFIDI WTYNAELLVLLENERTLDFHDSNVKNLYEKVKSQLKNNAKEIGNGCFEFYHKCNDECMESVKNGTYDYPKYSEESKLNREKI DGVKLES |
| 31 | ADTICVGYHANNSTDTIDTILEKNVTVTHSVNLLENSHNGKLCSLNGIAPLQLGSCNVAGWILGNPECDLLLTVNSWSYIVETS NSENGTCYPGEFADYEELREQLSSVSSFERFEIFPKTTSWPNHDTTKGTTAACSHSGTNSFYRNLLWIVKKGNSYPKLNSSYTN NKGKEVLIIWGVHHPPTNNDQQSLYQNADTYVSVGSSKYYQRFTPEIAARPKVRGQAGRMNYYWTLLDQGDTITFEATGNLI APWYAFALNKNSGSGIMISDAQVHNCTTKCQTPHGAMNSNLPFQNVHPVTIGECPKYVKSTQLRMATGLRNVPSIQSRGLFGA IAGFIEGGWTGMIDGWYGYHHQNGQGSGYAADQKSTQLAIDGISNKVNSVIEKMNIQFTSVGKEFNNLEKRIENLNKKVDDGF LDVWTYNAEILILLENERTLDFHDFNVKNLYEKVKSQLRNNAKEVGNGCFEFYHKCDNECMESVRNGTYNYSKYSEESKLNR EEIDGVKLES |
| 32 | ADTLCVGYHANNSTDTVDTILEKNVTVTHSVNLLEDRHNGKLCKLGGIAPLHLGKCNIAGWLLGNPECDSLLTVRSWSYIVET SNSDNGTCYPGEFINYEELKEQLSSVSSFERFEIFPKASSWPNHETNKGVTAACPYAGSNSFYRNLIWLVKKGNSYPKLNKSYIN NKGKEVLVLWGIHHPPTNNDQQSLYQNEDAYVFIGSSKYNKKFKPEIATRPKVRGQAGRMDYYWLLMEPGETITFEATGNLV VPRYAFAMNRDARSGIIISDVSVHDCNTTCQTPKGALNTSLPFQNIHPVTIGECPKYVKSTKLRMATGLRNIPSIQSRGLFGAIAG FIEGGWTGMIDGWYGYHHQNEQGSGYAADQKSTQNAIDGITNKINSVIEKMNTQFAAVGKEFNHLEKRLENLNKKVDDGFL DVWTYNAELLVLLENERTLDFHDSNVKNLYEKVRSQLRNNAKEIGNGCFEFYHRCDDACMESVKNGTYDYPVYSEESRLNR EEIDGVKLDS |
| 33 | ADTLCIGYHANNSTDTVDTVLEKNVTVTHSVNLLEDKHNGKLCKLRGVAPLHLGKCNIAGWILGNPECESLSTASSWSYIVET PSSDNGTCYPGDFIDYEELREQLSSVSSFERFEIFPKTSSWPNHDSNKGVTAACPHAGAKSFYKNLIWLVKKGNSYPKLSKSYIN DKGKEVLVLWGIHHPSTSADQQSLYQNADAYVFVGSSRYSKKFKPEIAIRPKVRDREGRMNYYWTLVEPGDKITFEATGNLV VPRYAFAMERNAGSGIIISDTPVHDCNTTCQTPKGAINTSLPFQNIHPITIGKCPKYVKSTKLRLATGLRNIPSIQSRGLFGAIAGFI EGGWTGMVDGWYGYHHQNEQGSGYAADLKSTQNAIDEITNKVNSVIEKMNTQFTAVGKEFNHLEKRIENLNKKVDDGFLDI WTYNAELLVLLENERTLDYHDSNVKNLYEKVRSQLKNNAKEIGNGCFEFYHKCDNTCMESVKNGTYDYPKYSEEAKLNREEI DGVKLES |
| 34 | ADTICIGYHANNSTDTVDTVLEKNVTVTHSVNLLEDNHNGKLCSLKGIVPLQLGNCSVAGWILGNPECELLISKKSWSYIVETP NPENGACYPGDFADYEELREQLSSVSSFERFEIFPKESSWPNHNVNGVSAACSHNGERSFYRNLLWLTGKNGLYPNLTNSYVN SKEKEVLILWGVHHPSNIRDQRTLYQTENAYVSVMSSHYSRRFTPEITKRPKVRDQEGRINYYWTLLEPGDTIIFEASGNLIAPW YGFELSRGFGSGIITSSAPTDECDAKCQTPQGAINSSLPFQNVHPVTIGECPKYVRSAKLRMVTGLRNIPSIQSRGLFGAIAGFIEG GWTGMVDGWYGYHHQNEQGSGYAADQESTQNAINGITNKVNSVIEKMNTQFTAVGKEFNKLERRMENLNKKVDDGFLDIW TYNAELLVLLENERTLDFHDSNVRSLYEKVRVQLKNNAKEIGNGCFEFYHKCNNECMESVRNGTYDYPKYSEESKLNREKID GVKLDS |
| 35 | ADTLCIGYHANNSTDTVDTVLEKNVTVTHSVNLLENRHNGKLCKLRGVAPLHLGKCNIAGWLLGNPECESLSTASSWSYIVET SNSDNGTCYPGDFINYEELREQLSSVSSFERFEIFPKTSSWPNHDTNRGVTAACPHDGTNSFYRNLIWLVKKGNSYPKINKSYIN NKEKEILVLWAIHHPSTSADQQSLYQNADAYVFVGSSRYSRKFEPEVATRPKVRDQAGRMNYYWTLVEPGDKITFEATGNLV VPRYAFALKRNSGSGIIISDTSVHDCDTNCQTPNGAINTSLPFQNIHPVTIGECPKYVKSTKLRMATGLRNIPSIQSRGLFGAIAGF IEGGWTGMIDGWYGYHHQNEQGSGYAADLKSTQNAIDGITNKVNSVIEKMNTQFTAVGKEFSHLERRIENLNKKVDDGFLDI WTYNAELLVLLENERTLDYHDSNVKNLYEKVRSQLKNNAKEIGNGCFEFYHKCDDMCMESVKNGTYDYPKYSEEAKLNREE IDGVKLES |
| 36 | ADTLCVGYHANNSTDTVDTVLEKNVTVTHSVNLLEDRHNGKLCKLRGVPPLHLGKCNIAGWLLGNPECEPLLTTSSWSYIVE TSISENGTCYPGDFIDYEELREQLSSVSSFERFEIFPKTSSWPNHDSNSGVTAACPHAGAKSFYRNLIWLVKKGNSYPKLSKSYIN NKGKEVLVLWGIHHPPTSADQQSLYQNVDTYVFVGSSRYSKKFKPEIATRPKVRDQAGRMNYYWTLVEPGDKITFEATGNLI VPRYAFAMRRNSGSGIIISDTPVHDCNATCQTPNGAINTSLPFQNIHPVTIGECPKYIKSTKLRMATGLRNVPSIQSRGLFGAIAGF IEGGWTGMIDGWYGYHHRNEQGSGYAADLKSTQNAIDGITNKVNSVIEKMNTQFTAVGKEFNHLEKRIENLNKKVDDGFLDI WTYNAELLVLLENERTLDYHDSNVKNLYEKVKSQLKNNAKEIGNGCFEFYHKCDNTCMESVKNGTYDYPKYSEEAKLNREEI DGVRLES |
| 37 | ADTICIGYHANNSTDTVDTVLEKNVTVTHSVNLLEDSHNGKLCLLKGIAPLQLGNCSVAGWILGNPECESLISKKSWSYIVETP NPENGACYPGEFADYEELREQLSSVSSFERFEIFPKESSWPNHTATGESASCSHNGKKSFYRNLIWLTVKNGLYPNLSKSYEND KEKEVLILWGVHHPPNIENQRTLYHTENAYVSVVSSHYSGRFTPEITKRPKVRDQEGRINYYWTLLEPGDTIIFEANGNLIAPWY AFALSRGLGSGIITSNAPMDECDSKCQTPQGAINSSLPFQNVHPVTIGECPKYVRSAKLRMVTGLRNIPSIQSRGLFGAIAGFIEG GWTGMVDGWYGYHHQNEQGSGYAADQESTQNAINGITNKVNSVIEKMNTQFTAVGKEFNKLERRMENLNKKVDDGFLDIW TYNAELLVLLENERTLDFHDSNVKSLYEKVKSQLKNNAKEIGNGCFEFYHKCNNECMESVKNGTYDYPKYYEESKLNREKID GVKLDS |
| 38 | ADTICIGYHANNSTDTVDTVLEKNVTVTHSINLLEDSHNGKLCKLKGIAPLQLGDCSIAGWILGNPNCESLFSKKSWSYIAEIPN PENGICYPGYFSDYEELREQLSSISSFERFEIFPKESSWPKHSVNKGVTASCSHKGKSSFYRNLLWLTEKNGSYPNLSKSYVNSRE KEVLVLWGVHHPSNIEDQRAIYRKENAYISVVSSLYNRRFTPEIARRPKVRDQEGRINYYWTILEPRDTIIFEATGNLIAPWYAFS LSRGFKSGIIVSNSSIDNCDTKCQTPQGAINSSLPFQNVHPVTIGECPKYVRSTKLRMATGLRNIQSIQSRGLFGAIAGFIEGGWTG MIDGWYGYHHQNEQGSGYAADLKSTQNAIDGITNKVNSVIDKMNTQFASVGKEFNKLEKRMENLNKKVDDGFLDIWTYNAE LLVLLENERTLDFHDSNVKSLYNKVKGQLKSNAKEIGNGCFEFYHKCDNECMESVKNGTYDYKEYSEESKLNREKIDGVELK S |
| 39 | ADTICIGYHANNSTDTVDTVLEKNVTVTHSVNLLEDSHNGKLCRLNGISPLQLGTCSIAGWVLGNPKCESLFSRRSWSYIAEVP NPENGICYPGHFSDYEELREQLSSVSSFERFEIFPKESSWPGHNLTKGVTASCSHNGKSSFYRNLLWLTGEKGLYPKVNKSYVN NKKKEVLVLWGVHHPSNIEDQRAIYHEENAYVSVVSSHYSRRFTPEIARRPKIRNQEGRINYYWTLLEPDDKIIFEANGNLIAPR YAFALSRGFGSGIIVSNTSIDECNTKCQTPQGAINSSLPFQNVHPVTIGECPKYVRSTKLRMVTGLRNIPSIQSRGLFGAIAGFIEG GWTGMIDGWYGYHHRNEQGSGYAADQESTQNAINGITNKVNSVIEKMNTQFTAVGKEFNKLEKRMENLNKKVDDGFLDIW TYNAELLVLLENERTLDFHDSNVKNLYEKVKSQLKNNAKEIGNGCFEFYHKCDDDCMESVKNGTYDYPRYSEESSLNRGKID GVKLES |
| 40 | ADTICIGYHANNSTDTVDTILEKNVTVTHSVNLLESSHNGKLCRMKGMAPLQLGKCSIAGWILGNPECESSFSRKSWSYIAEAP NPENGICYPGYFSDYEELREQLSSVSSVERFEIFPKESSWPKHNVTRGKTVSCPHNGKSSFYKNLLWLTEKDGSYPNVSKPYVN NKEKEVLVIWGVHNPSNIEDQRAIYRKETAYVSVVSSHYNRRFTPEIEKRPKIKNQEGRINYYWTLLEPGETIIFEANGNFIAPW YAFALSRGFRSGIIVSNASMDECDTECQTPQGAINNSLPFQNVHPVAIGECPKYIKSTRLRMVTGLRNIPSIQSRGLFGAIAGFIEG GWTGMIDGWYGYHHRNEQGSGFAADQESTQNAINGITNKVNSIIGKMNTQFTAAGKEFNRLEKRMENLNKKVDDGLLDIWT YNAELLVLLENERTLDFHDSNVKNLYEKVKSQLKNNAKEIGNGCFEFYHKCNNECMESVKNGTYDYPKYSEESSLNRRKIDG VKLES |
| 41 | ADTICVGYHANNSTDTVDTILEKNVTVTHSINLLESSHNGKLCSLNGIAPLQLGNCNIAGWILGNPECDLSFTANSWSYIIETSNS ENGACYPGKFTDYEELREKLSSVSSFERFEIFPKATSWPNHETNKGTTAACSHSGVKSFYRNLLWIVKKGNSYTKVNKSYTNN KGKEVLVIWGVHHPPTNNVQQTLYQNASTYVSVGTSKYYRRFTPEIVARPKVRDQAGRINYYWTLLDQGDTITFEATGNLIAP WYAFALNKGSVSGIIISDANVHNCTTKCQTPHGALKSRLPFQNVHPITIGECPKYVKSTQLRMATGLRNIPSIQSRGLFGAIAGFI EGGWTGMIDGWYGYHHQNEQGSGYAADQKSTQSAIDGISNKVNSIIEKMNVQFTSVGKEFNNLEKRLENLNKKVDDGFLDV WTYNAELLILLENERTLDFHDFNVKNLYEKVKSQLRDNAKEVGNGCFEFYHKCDNECMESIKNGTYDYPKYSEESKLNREEIY GVKLES |
| 42 | ADTICVGYHANNSTDTVDTILEKNVTVTHSVNLLENSHNGKLCSLNGKAPLQLENCNVAGWILGNPECDFLLTAYSWSYIIETS NSKNGTCYPGEFNNYEELREQLSTVSSLERFEIFPKATSWPNHDTTKGTTVACSHSGANSFYRNLLWIVKKENSYPKLSKSYTN NKGKEVLVIWGVHHPPTDSDQQTLYQNNNTYVSVESSKYYQRFTPEIVARPKVRKQAGRMNYYWILLDQGDTITFEATGNLI APWYAFALDKGSSSGIMMSDAHVQNCTTKCQTPHGALKSNLPFQNVHPITIGECPKYVKSTQLRMATGLRNIPSIQSRGLFGAI AGFIEGGWTGMIDGWYGYHHQNEQGSGYAADKKSTQIAIDGISNKVNSIIEKMNIQFTSVGKEFNNLEKRIENLNKKVDDGFL DVWTYNAELLILLENERTLDFHDFNVRNLYEKVKSQLRNNAREIGNGCFEFYHKCDNECMESIRNGTYNYPRYSEESKLNREK IDGVKLES |
| 43 | ADTLCIGYHANNSTDTVDTVLEKNVTVTHSVNLLEDKHNGKLCKLRGVAPLHLGKCNIAGWILGNPECESLSTASSWSYIVET SNSDNGTCYPGDFINYEELREQLSSVSSFERFEIFPKTSSWPNHDSNKGVTAACPHAGAKSFYKNLIWLVKKGNSYPKLNQSYI NDKGKEVLVLWGIHHPSTTADQQSLYQNADAYVFVGTSRYSKKFKPEIATRPKVRDQEGRMNYYWTLVEPGDKITFEATGNL VVPRYAFTMERNAGSGIIISDTPVHDCNTTCQTPEGAINTSLPFQNIHPITIGKCPKYVKSTKLRLATGLRNVPSIQSRGLFGAIAG FIEGGWTGMVDGWYGYHHQNEQGSGYAADLKSTQNAIDKITNKVNSVIEKMNTQFTAVGKEFNHLEKRIENLNKKVDDGFL DIWTYNAELLVLLENERTLDYHDSNVKNLYEKVRNQLKNNAKEIGNGCFEFYHKCDNTCMESVKNGTYDYPKYSEEAKLNR EKIDGVKLES |
| 44 | TATLCLGHHAVPNGTLVKTITDDQIEVTNATELVQSSSTGKICNNPHRILDGIDCTLIDALLGDPHCDVFQNETWDLFVERSKAF SNCYPYDVPDYASLRSLVASSGTLEFITEGTWTGVTQNGGSNACKRGPGSGFFSRLNWLTKSGSTYPVLNVTMPNNDNFDKLY IWGVHHPSTNQEQTSLYVQASGRVTVSTRRSQQTIIPNIGSRPWVRGLSSRISIYWTIVKPGDVLVINSNGNLIAPRGYFKMRTG KSSIMRSDAPIDTCISECITPNGSIPNDKPFQNVNKITYGACPKYVKQNTLKLATGMRNVPEKQTRGLFGAIAGFIENGWEGMID GWYGFRHQNSEGTGQAADLKSTQAAIDQINGKLNRVIEKTNEKFHQIEKEFSEVEGRIQDLEKYVEDTKIDLWSYNAELLVAL ENQHTIDLTDSEMNKLFEKTRRQLRENAEDMGNGCFKIYHKCDNACIESIRNGTYDHDVYRDEALNNRFQIKGVELKS |
| 45 | TATLCLGHHAVPNGTLVKTITNDQIEVTNATELVQSSSTGRICDSPHRILDGKNCTLIDALLGDPHCDGFQNEKWDLFVERSKA FSNCYPYDVPDYASLRSLVASSGTLEFINEGNWTGVTQSGGSYACKRGSVNSFFSRLNWLYESEYKYPALNVTMPNNGKFDK LYIWGVHHPSTDKEQTNLYVRASGRVTVSTKRSQQTVIPNIGSRPWVRGLSSRISIYWTIVKPGDILLINSTGNLIAPRGYFKIRT GKSSIMRSDAPIGTCSSECITPNGSIPNDKPFQNVNKITYGACPRYVKQNTLKLATGMRNVPEKQTRGIFGAIAGFIENGWEGMV DGWYGFRHQNSEGTGQAADLKSTQAAIDQINGKLNRLIEKTNEKFHQIEKEFSEVEGRIQDLEKYVEDTKIDLWSYNAELLVA LENQHTIDLTDSEMNKLFEKTRKQLRENAEDMGNGCFKIYHKCDNACIGSIRNGTYDHDVYRDEALNNRFQIKGVELKS |
| 46 | IATLCLGHHAVPNGTLVKTITSDQIEVTNATELVQSFSTGEICNNPHRILDGMDCTLVDALLGDPHCDGFQNGTWDLFVERSKA FSNCYPYDVPDYASLRSLIASSGTLEFTNEGFNWAGVTQNGGSSACRRGSDSSFFSRLNWLYKLGNTYPVLNVTMPNNDNFDK LYIWGIHHPSTDQEQTRLYARESGRVTVSTKRSQQTVTSNVGPRPWVRGLSSRISIYWTIVKPGDILLINSNGNLIAPRGYFKMR AGKSSIMRSDAPISTCISECITPNGNVPNDKPFQNVNKITYGACPKYIRQKTLKLATGMRNVPEKQTRGIFGAKAGFIENGWEG MVDGWYGFRHQNSEGTGQAADLKSTQTAIDQINGKLNRVIEKTNEKFHQIEKEFSEVEGRIQDLEKYVEDTKIDLWSYNAELL VALENQHTIDLTDSEMNKLFEKTRRQLRENAEDIGNGCFKIYHRCDNACIGSIRNGTYNHNVYRDEALNNRFQIRGVELKS |
| 47 | TATLCLGHHAVPNGTLVKTITNDQIEVTNATELVQSSSTGRICDSPHRILDGKNCTLIDALLGDPHCDGFQNKEWDLFVERSKA YSNCYPYDVPDYASLRSLVASSGTLEFTNEGNWTGVAQDGTSYACKRGSVKSFFSRLNWLHKLEYKYPALNVTMPNNDKFD KLYIWGVHHPSTDSVQTSLYVQASGRVTVSTKRSQQTVIPNIGSRPWVRGISSRISIYWTIVKPGDILLINSTGNLIAPRGYFKIRS GKSSIMRSDAPIGNCNSECITPNGSIPNDKPFQNVNRITYGACPRYVKQNTLKLATGMRNVPEKQTRGIFGAIAGFIENGWEGM VDGWYGFRHQNSEGTGQAADLKSTQAAINQINGKLNRLIEKTNEKFHQIEKEFSEVEGRIQDLEKYVEDTKIDLWSYNAELLV ALENQHTIDLTDSEMNKLFERTRKQLRENAEDMGNGCFKIYHKCDNACIGSIRNGTYDHDVYRDEALNNRFQIKGVELKS |
| 48 | TATLCLGHHAVPNGTLVKTITNDQIEVTNATELVQSSSTGRICDSPHRILDGENCTLIDALLGDPHCDGFQNKEWDLFVERSKA YSNCYPYDVPDYASLRSLVASSGTLEFNNESNWTGVAQNGTSYACKRSSIKSFFSRLNWLHQLKYKYPALNVTMPNNDKFDK LYIWGVHHPSTDSDQTSIYAQASGRVTVSTKRSQQTVIPNIGSRPWVRGISSRISIHWTIVKPGDILLINSTGNLIAPRGYFKIRSG KSSIMRSDAPIGKCNSECITPNGSIPNDKPFQNVNRITYGACPRYVKQNTLKLATGMRNVPEKQTRGIFGAIAGFIENGWEGMV DGWYGFRHQNSEGTGQAADLKSTQAAINQINGKLNRLIEKTNEKFHQIEKEFSEVEGRIQDLEKYVEDTKIDLWSYNAELLVA LENQHTIDLTDSEMNKLFERTRKQLRENAEDMGNGCFKIYHKCDNACIGSIRNGTYDHDVYRDEALNNRFQIKGVELKS |
| 49 | IAMLCLGHHAVPNGTLVKTITNDQVEVTNATELVQSSSTGKICNHPHRILDGMDCTLIDSLLGDPHCDSFQNETWDLFVERSRA FSNCYPYDVPDYASLRSLVASSGTLEFINEDFNWAEVTQNGGSSACKRGSDSSFFSRLNWLYKSGNTYPTLNVTIPNNGNFDKL YIWGIHHPSTTKEQTRLYVQESGKVTVSTKRSQQTVIPNVGPRPWVRGLSSRISIYWTIVKPGDLLLISSTGNLIAPRGYFRLRAG KSSIIRSEAPIGTCISECITPNGSIPNDKPFQNVNKITYGACPKYVKQNTLKLATGMRNVPEKQTRGIFGAKAGFIENGWEGMIDG WYGFRHQNSEGTGQAADLKSTQAVIDQINGKLNRIIEKTNEKFHQIEKEFSEVEGRVQDLEKYVEDTKIDLWSYNAELLIALEN QHTIDLADSEMNKLFEKTRRLLRENAEDIGNGCLKIYHRCDNACIGSIRNGTYNHDVYRDEALNNRFQIKGIELKS |
| 50 | TATLCLGHHAVPNGTLVKTITDDQIEVTNATELVQNFSMGKICNNPHRILDGANCSLIDALLGDPHCDDLQNEKWDLFIERSKA FSNCYPYDVPEYTSLRSLIASSGTLEFTNEGFNWTGVTQNGGSSACKRGPNNSFFSRLNWLYKSGNTYPILNVTMQNSDDFDKL YIWGVHHPSTDREQTNLYVQASGKVTVSTKRSQQTIIPNIGSRPWIRGLSSRISIYWTIVKPGDILMINSNGNLIAPRGYFKVYTG RSSIMKSDAPLDTCNSECITPNGSIPNDKPFQNVNKITYGACPKYIKQNTLKLATGMRNIPEKQTRGIFGAIAGFIENGWEGMVN GWYGFRHQNSEGTGQAADLKSTQAAINQINGKLNRIIEKTNEKFHQIEKEFSEVEGRIQDLEKYVEDTKIDLWSYNAELLVALE NQHTIDLTDSEMNKLFEKTRKQLRENAEDMGNGCFKIYHKCDNSCMESIRNGTYDHNEYRNEALSNRFQIKSVELKS |
| 51 | TATLCLGHHAVPNGTIVKTITNDQIEVTNATELVQSSSTGGICDSPHQILDGENCTLIDALLGDPQCDGFQNKKWDLFVERSKA YSNCYPYDVPDYASLRSLVASSGTLEFNNESNWTGVTQNGTSSACKRRSNKSFFSRLNWLTHLKYKYPALNVTMPNNEKFDK LYIWGVLHPGTDSDQIRLYAQASGRITVSTKRSQQTVIPNIGSRPRVRDVSSRISIYWTIVKPGDILLINSTGNLIAPRGYFKIRSGK SSIMRSDAPIGKCNSECITPNGSIPNDKPFQNVNRITYGACPRYIKQNTLKLATGMRNVPEKQTRGIFGAIAGFIENGWEGMVDG WYGFRHQNSEGTGQAADLKSTQAAINQINGKLNRLIGKTNEKFHQIEKEFSEVEGRIQDLEKYVEDTKIDLWSYNAELLVALE NQYTIDLTDSEMNKLFERTKKQLRENAEDMGNGCFKIYHKCDNACIGSIRNGTYDHDVYRDEALNNRFQIKGVELKS |
| 52 | TATLCLGHHAVPNGTIVKTITNDQIEVTNATELVQSSSTGGICDSPHQILDGENCTLIDALLGDPQCDGFQNKKWDLFVERSKA YSNCYPYDVPDYASLRSLVASSGTLEFNNESNWAGVTQNGTSSACKRRSNKSFFSRLNWLTHLKYKYPALNVTMPNNEKFDK LYIWGVHHPGTDSDQISLYAQASGRITVSTKRSQQTVIPNIGSRPRVRDVSSRISIYWTIVKPGDILLINSTGNLIAPRGYFKIRSGK SSIMRSDAPIGKCNSECITPNGSIPNDKPFQNVNRITYGACPRYVKQNTLKLATGMRNVPEKQTRGIFGAIAGFIENGWEGMVD GWYGFRHQNSEGTGQAADLKSTQAAINQINGKLNRLIGKTNEKFHQIEKEFSEVEGRIQDLEKYVEDTKIDLWSYNAELLVAL ENQHTIDLTDSEMNKLFERTKKQLRENAEDMGNGCFKIYHKCDNACIESIRNGTYDHDVYRDEALNNRFQIKGVELKS |
| 53 | TATLCLGHHAVPNGTIVKTITNDQIEVTNATELVQSSSTGGICDSPHQILDGENCTLIDALLGDPQCDGFQNKKWDLFVERSKA YSNCYPYDVPDYASLRSLVASSGTLEFNNESNWTGVTQNGTSSSCKRRSNNSFFSRLNWLTHLKFKYPALNVTMPNNEKFDKL YIWGVHHPGTNNDQISLYTQASGRITVSTKRSQQTVIPNIGSRPRVRDIPSRISIYWTIVKPGDILLINSTGNLIAPRGYFKIRSGKS SIMRSDAPIGKCNSECITPNGSIPNDKPFQNVNRITYGACPRYVKQNTLKLATGMRNVPEKQTRGIFGAIAGFIENGWEGMVDG WYGFRHQNSEGIGQAADLKSTQAAINQINGKLNRLIGKTNEKFHQIEKEFSEVEGRIQDLEKYVEDTKIDLWSYNAELLVALEN QHTIDLTDSEMNKLFERTKKQLRENAEDMGNGCFKIYHKCDNACIGSIRNGTYDHDVYRDEALNNRFQIKGVELKS |
| 54 | TATLCLGHHAVPNGTLVQTITDDQVEVTNATELVQSSSVGRICNSPHRILDGGNCTLIDALLGDPHCDAFQNEKWDLFVERSK AFSNCYPYDMQDYVSLRSLVASSGTLEFTNEGFNWTGVTQSGGSYACKRGSGNSFFSRLNWLYQSENKYPALNVTMPNNGNF DKLYIWGVHHPSTDREQTNLYVRGSGRVTVSTKRSQQTVIPNIGPGPWVRGLSSRMSIYWTIVKPGDTLLITSNGNLIAPRGYF KIHAGKSSIMRSDAPIGTCSSECITPNGSIPNDKPFQNVNKITYGACPKYVKQNTLKLATGMRNIPEKQNRGIFGAIAGFIENGWE GMVDGWYGFRHQNSEGTGQAADLKSTQAAIDQINGKLNRVIEKTNEKFHQIEKEFSEVEGRIQDLEKYVEDTKIDLWSYNAEL LVALENQHTVDLTDSEMNKLFEKTRRQLRENAEDMGNGCFKIYHKCDNTCIGSIRNGTYDHDIYRDEALNNRFQIKDVVLKS |
| 55 | TATLCLGHHAVPNGTIVKTITNDQIEVTNATELVQSSSTGEICDSPHQILDGKNCTLIDALLGDPQCDGFQNKKWDLFVERSKA YSNCYPYDVPDYASLRSLVASSGTLEFNNESFNWTGVTQNGTSSACIRRSKNSFFSRLNWLTHLNFKYPALNVTMPNNEQFDK LYIWGVHHPGTDKDQIFLYAQASGRITVSTKRSQQTAIPNIGYRPRVRNIPSRISIYWTIVKPGXILLINSTGNLIAPRGYFKIRSGK SSIMRSDAPIGKCNSECITPNGSIPNDKPFQNVNRITYGACPRYVKQNTLKLATGMRNVPEKQTRGIFGAIAGFIENGWEGMVD GWYGFRHQNSEGRGQAADLKSTQAAIDQINGKLNRLIGKTNEKFHQIEKEFSEVEGRIQDLEKYVEDTKIDLWSYNAELLVAL ENQHTIDLTDSEMNKLFEKTKKQLRENAEDMGNGCFKIYHKCDNACIGSIRNGTYDHDVYRDEALNNRFQIKGVELKS |
| 56 | TATLCLGHHAVPNGTIVKTITNDQIEVTNATELVQSSSTGEICDSPHQILDGENCTLIDALLGDPQCDGFQNKKWDLFVERSKA YSNCYPYDVPDYASLRSLVASSGTLEFNNESNWTGVTQNGTSSACIRRSNNSFFSRLNWLTHLKFKYPALNVTMPNNEKFDKL YIWGVHHPGTDNDQIFPYAQASGRITVSTKRSQQTVIPNIGSRPRVRNIPSRISIYWTIVKPGDILLINSTGNLIAPRGYFKIRSGKS SIMRSDAPIGKCNSECITPNGSIPNDKPFQNVNRITYGACPRYVKQNTLKLATGMRNVPEKQTRGIFGAIAGFIENGWEGMVDG WYGFRHQNSEGIGQAADLKSTQAAIDQINGKLNRLIGKTNEKFHQIEKEFSEVEGRIQDLEKYVEDTKIDLWSYNAELLVALEN QHTIDLTDSEMNKLFEKTKKQLRENAEDMGNGCFKIYHKCDNACIGSIRNGTYDHDVYRDEALNNRFQIKGVELKS |
| 57 | TATLCLGHHAVPNGTIVKTITNDQIEVTNATELVQSSSTGEICDSPHQILDGENCTLIDALLGDPQCDGFQNKKWDLFVERSKA YSNCYPYDVPDYASLRSLVASSGTLEFNNESFNWTGVTQNGTSSACIRRSNNSFFSRLNWLTHFKFKYPALNVTMPNNEQFDK LYIWGVHHPGTDNDQIFLYAQASGRITVSTKRSQQTVIPNIGSRPRVRIPSRISIYWTIVKPGDILLINSTGNLIAPRGYFKIRSGKS SIMKSDAPIGKCNSECITPNGSIPNDKPFQNVNRITYGACPRYVKQNTLKLATGMRNVPEKQTRGIFGAIAGFIENGWEGMVDG WYGFRHQNSEGRGQAADLKSTQAAIDQINGKLNRLIGKTNEKFHQIEKEFSEVEGRIQDLEKYVEDTKIDLWSYNAELLVALE NQHTIDLTDSEMNKLFEKTKKQLRENAEDMGNGCFKIYHKCDNACIGSIRNGTYDHDVYRDEALNNRFQIKGVELKS |
| 58 | TATLCLGHHAVPNGTLVKTITGEQIEVTNATELVQSLSMGKICKNPHRILDGANCTLMDALLGDPHCDGFQNEKWDLFIERSK AFSNCYPYDVPEYTSLRSIIASSGTLEFFSENFNWTGVTQNGGSNSCKRGPNSSFFSRLNWLYKSGNNYPILNVTMPNSEGFDKL YIWGVHHPSTENEQTNLYAQAPGKITVSTRSSQQTVIPNIGPRPWIRGLSSRISIYWTIVKPGDILMINSNGNLIAPRGHFKVHTG RSSIMRSDAPIDTCSSECITPNGSIPNDKPFQNVNKITYGVCPKYVKQSTLKLATGMRNIPERQTRGIFGAIAGFIENGWEGMVD GWYGFRHQNSEGTGQAADLKSTQAAINQINGKLNRVIEKTNEKFHQIEKEFSEVEGRIQDLEKYVEDTKIDLWSYNAELLVAL ENQHTIDLTDSEMNKLFEKTRKQLRENAEDMGNGCLKIYHKCDNSCMRSIRNGTYDHNEYRDEALNNRFQIKSVELKL |
| 59 | TATLCLGHHAVPNGTIVKTITNDQIEVTNATELVQSSSTGEICNSPHQILDGENCTLIDALLGDPQCDGFQNKKWDLFVERSKA HSNCYPYDVPDYASLRSLVASSGTLEFNNESNWTGVTQNGTSSACIRRSNNSFFSRLNWLTHLNFKYPALNVTMPNNEQFDKL YIWGVHHPGTDKDQIFLYAQASGRITVSTKRSQQAVIPNIGSRPRVRDIPSRVSIYWTIVKPGDILLINSTGNLIAPRGYFKIRSGK SSIMRSDAPIGKCNSSCITPNGSIPNDKPFQNVNRITYGACPRYVKQNTLKLATGMRNVPEKQTRGIFGAIAGFIENGWEGMVD GWYGFRHQNSEGRGQAADLKSTQAAIDQINGKLNRLIGKTNEKFHQIEKEFSEVEGRIQDLEKYVEDTKIDLWSYNAELLVAL ENQHTIDLTDSEMNKLFEKTKKQLRENAEDMGNGCFKIYHKCDNACIGSIRNGTYDHDVYRDEALNNRFQIKGVELKS |
| 60 | TATLCLGHHAVPNGTLVKTITNDQIEVTNATELVQSSSTGKICNSPHQILDGKNCTLIDALLGDPHCEEFQNKEWDLFVERSTA YSNCYPYYVPDYASLRSLVASSGTLKFTKEDFNWTGVSQDGSSFSCKRKSVNSFFSRLNWLHKSTNKYPALNVTMPNIDKFDK LYIWGVHHPSTDSDQISLYAQASGRVMVSTKRSQQAVIPNIGSRPWVRGVSSIISIHWTIVKPGDILLINSTGNLIAPRGYFKIRGG KSSIMRSDEPIDNCNFDCITPNGSIPNDKPFQNVNRITYGACPRYVKQNTLKLATGMRNVPEKQTRGIFGAIAGFIENGWEGMV NGWYGFRHQNSEGTGQAADLKSTQAAISQINGKLNRLIEKTNEKFHQIEKEFSEVEGRVQDLEKYVEDTKIDLWSYNAELLVA LENQHTIDLTDSEMNKLFERTRKQLRENAEDMGNGCFKIYHKCDNACIGSIRNGTYDHDVYRDEALNNRFQIKGVELKS |
| 61 | TATLCLGHHAVPNGTLVKTITDDQVEVTNATELVQNSSAGRICDNPHRILDGKNCTLIDALLGDPHCDSFKNKEWDLFVERSE AYSDCYPYDVPDYDYIRSIVASSGTLEFTNESFNWTGVAQDGTSNSCKRGSVKSFFSRLNWLHKLEYKYPALNVTMPNNDRFD KLYIWGVHHPSTDSDQTNLYVQASGKVVVSTKRSQQTVIPNIGFRPWIRGISSRISIYWTIVKPGDILLINSTGNLIAPRGYFKIRS GKSSIMRSDTPIGNCNSECITPNGSIPNDKPFQNVNRITYGACPRYIKRNTLKLATGMRNVPEKQTRGIFGAIAGFIENGWEGMID GWYGFRHQNSEGTGQAADLKSTQAAINQINGKLNRLVEKTNEKFHQIEKEFSEVEGRIQDLEKYIEDTKIDLWSYNAELLIALE NQHTIDLTDSEMNKLFEKTKKQLRENAEDMGNGCFKIYHKCDNACIGSIRNGTYDHNVYRDEALNNRFQIKGVKLKS |
| 62 | TATLCLGHHAVPNGTLVKTVTDDQIEVTNATELVQSLSTGKICNSPHQILDGGNCTLIDALLGDPHCDRFQNRKWDLFVERST AYSNCYPYDVPDYASLRSLIASSGTLEFTKEDFNWTGVAQDGASYSCKRGSVKSFFSRLNWLHKLEYKYPALNVTMPNNDRF NKLYIWGVHHPSTDSDQTRLYVQASGKVTVSTKRSQQTVIPNIGPIPWIRGVSSRISIYWTIVNPGDILLINSTGNLIAPRGYFKLR NGRSSVMRSDAPIDNCNSECITPNGSIPNDKPFQNVNRITYGTCPRYVKQNTLKLATGMRNVPEKQTRGIFGAIAGFIENGWEG MVDGWYGFRHQNSEGTGQAADLKSTQTAINQINGKLNRLIEKTNEKFHQIEKEFSEVEGRIQDLEKYVEDTKIDLWSYNAELL VALENQHTVDLTDSEMNKLFEKTRRQLRENAEDMGNGCFKIYHKCDNACIGSIINGTYDHDVYRDEALNNRFQIKGVELKS |
| 63 | MAMLCLGHHAVPNGTLVKTITNDHIEVTNATELVQSSSMGRICNSPHQILDGENCTLIDALLGDPHCDAFQNKEWDLFVERST AYSNCYPYDVPDYASLRALVASSGTLEFNNENFNWTGVAQNGASTACKRRSSNSFFSRLNWLYQLRYKYPALNVTMPNNEK FDKLYIWGVHHPSTDNDQISLYAQASGRVIVSTKRSQQTVIPNIGSRPWVRGVSSTISIYWTIVKAGDILLINSTGNLIAPRGYFKI RNGKSSIMRSNAPIGRCNAGCITPNGSISNDKPFQNVNRITYGACPRYVKQKTLRLATGMRNVPEKQTRGIFGAIAGFIENGWE GMVDGWYGFRHQNSEGTGQAADLKSTQAAISQISGKLNRLIGKTNEKFHQIEKEFSEVEGRIQDIEKYVEDTKIDLWSYNAELL VALENQHTIDLTDSEMNKLFERTRKQLRENAEDMGNGCFKIYHKCDNACIESIRNGTYDHDVYRDEALNNRFQIKGIELKS |
| 64 | TATLCLGHHAVPNGTLVKTITNDQIEVTNATELVQSSSTGRICDSPHRILDGKNCTLIDALLGDPHCDGFQNKEWDLFIERSKAY SNCYPYDVPDYSSLRSLVASSGTLEFTNEDFNWTGVAQDGGSYSCKRGSVKSFFSRLNWLHKLEYKYPALNVTMPNNDKFDK LYIWGVHHPATDKDQISLYAQAAGRIIVSTKRSQQAVIPNIGSRPRVRDIPSRISIYWTIVRPGDILLINSPGNLIAPRGYFKIRSGK SSIMRSDAPIGKCNSACITPNGSIPNDKPFQKVNRITYGACPRYVKQNAVKLATGMRNRPEKQTRGIFGAIAGFIENGWEGMVH GWDGFTHQPSGGTGQAADLKSTQAAIDQINGKLNRLIGKTNEKFHQIEKEFSEVEGRIQDLEKYVEDTKIDLWSYNAELLVAL ENQHTIDLTDSEMNKLFEKTKKQLRENAEDMGNGCFKIYHKCDNACIGSIRNGTYDHDVYRDEALNNRFQIKGVELKS |
| 65 | TATLCLGHHAVPNGTIVKTITNDRIEVTNATELVQNSSIGEICDSPHQILDGENCTLIDALLGDPQCDGFQNKKWDLFVERSKAY SNCYPYDVPDYASLRSLVASSGTLEFNNESNWTGVKQNGTSSACIRKSSSSFFSRLNWLTHLNYTYPALNVTMPNNEQFDKLYI WGVHHPGTDKDQIFLYAQSSGRITVSTKRSQQAVIPNIGSRPRIRDIPSRISIYWTIVKPGDILLINSTGNLIAPRGYFKIQSGKSSI MRSDAPIGKCKSECITPNGSIPNDKPFQNVNRITYGACPRYVKHSTLKLATGMRNVPEKQTRGIFGAIAGFIENGWEGMVDGW YGFRHQNSEGRGQAADLKSTQAAIDQINGKLNRLIGKTNEKFHQIEKEFSEVEGRIQDLEKYVEDTKIDLWSYNAELLVALENQ HTIDLTDSEMNKLFEKTKKQLRENAEDMGNGCFKIYHKCDNACIGSIRNGTYDHNVYRDEALNNRFQIKGVELKS |
| 66 | SDQICIGYHANNSTEQVDTIMEKNVTVTHAQDILERTHNGKLCDLNGVKPLILRDCSVAGWLLGNPMCDEFINVPEWSYIVEK ASPANDLCYPGNFNDYEELKHLLSRISHFEKIQIIPKSSWSNHDASSGVSSACPYLGKSSFFRNVVWLIKKNSTYPTIKRSYNNTN QEDLLVLWGIHHPNDAAEQTKLYQNPTTYISVGTSTLNQRLVPEIATRPKVNGQSGRIEFFWTILKPNDAINFESNGNFIAPEYA YKIVKKGDSTIMKSELEYGNCNTKCQTPMGAINSSMPFHNIHPLTIGECPKYVKSNRLVLATGLRNAPQRERRRKKRGLFGAIA GFIEGGWQGMVDGWYGYHHSNEQGSGYAADQESTQKAIDGVTNKVNSIINKMNTQFEAVGREFNNLERRIENLNKKMEDGF LDVWTYNAELLVLMENERTLDFHDSNVKNLYDKVRLQLRDNAKELGNGCFEFYHKCDNECMESVKNGTYDYPQYSEEARL NREEISGVKLES |
| 67 | SDQICIGYHANNSTELVDTIMEKNVTVTHAQDILEKTHNGKLCDLDGVKPLILRDCSVAGWLLGNPMCDEFINVPEWSYIVEK ANPANDLCYPGDFNDYEELKHLLSRINHFEKIQIIPKSSWPNHEASSGVSSACPYQGKSSFFRNVVWLIKKNSAYPTIKRSYNNT NQEDLLVLWGIHHPNDAAEQTKLYQNPTTYISVGTSTLNQRLVPKIATRSKVNGQSGRMEFFWTILKPNDAINFESNGNFIAPE YAYKIVKKGDSAIMKSELEYGNCNTKCQTPLGAINSSMPFHNIHPLTIGECPKYVKSNRLVLATGLRNTPQRERRRKKRGLFGA IAGFIEGGWQGMVDGWYGYHHSNEQGSGYAADKESTQKAIDGVTNKVNSIIDKMNTQFEAVGREFNNLERRIENLNKKMED GFLDVWTYNAELLVLMENERTLDFHDSNVKNLYDKVRLQLRDNAKELGNGCFEFYHKCDNECMESVKNGTYDYPQYSEEA RLNREEISGVKLES |
| 68 | SDQICIGSHANNSTEQVDTIMEKNVTVTHAQDILEKTHNGKLCDLDGVKPLILRDCSVAGWLLGNPMCDEFINVPEWSYIVEK ASPANDLCYPGDFNDYEELKHLLSRINHFEKIQIIPKSSWSNHEASSGVSSACPYLGRPSFFRNVVWLIKKNNTYPTIKRSYNNT NQEDLLVLWGIHHPNDEAEQIKLYQNPTTYISVGTSTLNQRLVPKIATRSKVNGQSGRMEFFWAILKPNDAINFESNGNFIAPEY AYKIVKKGDSAIMKSELEYGNCNTKCQTPMGAINSSMPFHNIHPLTIGECPKYVKSNRLVLATGLRNAPQRERRRKKRGLFGAI AGFIEGGWQGMVDGWYGYHHSNEQGSGYAADKESTQKAIDGVTNKVNSIIDKMNTQFEAIGREFNNLERRIENLNKKMEDG FLDVWTYNAELLVLMENERTLDFHDSNVKNLYEKVRLQLRDNAKGLGNGCFEFYHKCDNECMESVKNGTYDYPQYSEEATL NREEISGVKLES |
| 69 | SDQICIGYHANNSTEQVDTIMEKNVTVTHAQDILEKTHNGKLCDLDGVKPLILRDCSVAGWLLGNPMCDEFINVPEWSYIVEK ANPVNDLCYPGDFNDYEELKHLLSRINHFEKIQIIPKSSWSSHEASLGVSSACPYQGKSSFFRNVVWLIKKNSTYPTIKRSYNNT NQEDLLVLWGIHHPNDAAEQTKLYQNPTTYISVGTSTLNQRLVPRIATRSKVNGQSGRMEFFWTILKPNDAINFESNGNFIAPE YAYKIVKKGDSTIMKSELEYGNCNTKCQTPMGAINSSMPFHNIHPLTIGECPKYVKSNRLVLATGLRNSPQRERRRKKRGLFGA IAGFIEGGWQGMVDGWYGYHHSNEQGSGYAADKESTQKAIDGVTNKVNSIIDKMNTQFEAVGREFNNLERRIENLNKKMED GFLDVWTYNAELLVLMENERTLDFHDSNVKNLYDKVRLQLRDNAKELGNGCFEFYHKCDNECMESVRNGTYDYPQYSEEA RLKREEISGVKLES |
| 70 | SDQICIGYHANNSTEQVDTIMEKNVTVTHAQDILEKTHNGKLCDLDGVKPLILRDCSVAGWLLGNPMCDEFINVPEWSYIVEK ANPANDLCYPGNFNDYEELKHLLSRINHFEKIQIIPKSSWSDHEASSGVSSACPYQGTPSFFRNVIWLIKKNNTYPTIKRSYNNTN QEDLLILWGIHHSNDAAEQTKLYQNPTTYISVGTSTLNQRLVPKIATRSKVNGQSGRMDFFWTILKPNDAINFESNGNFIAPEYA YKIVKKGDSAILKSEVEYGNCNTKCQTPIGAINSSMPFHNIHPLTIGECPKYVKSNKLVLATGLRNSPLRERRRKKRGLFGAIAG FIEGGWQGMVDGWYGYHHSNEQGSGYAADKESTQKAIDGVTNKVNSIIDKMNTQFEAVGREFNNLERRIENLNKKMEDGFL DVWTYNAELLVLMENERTLDFHDSNVKNLYDKVRLQLKDNAKELGNGCFEFYHKCDNECMESVRNGTYDYPQYSEEARLK REEISGVKLES |
| 71 | SDQICIGYHANNSTEQVDTIMEKNVTVTHAQDILEKTHNGKLCNLDGVKPLILRDCSVAGWLLGNPMCDEFLNVPEWSYIVEK INPANDLCYPGNFNDYEELKHLLSRINHFEKIQIIPKNSWSDHEASGVSSACPYQGRSSFFRNVVWLTKRDNAYPTIKRSYNNTN QEDLLVLWGIHHPNDAAEQTRLYQNPTTYISVGTSTLNQRLVPKIATRSKVNGQSGRMEFFWTILKSNDAINFESNGNFIAPEN AYKIVKKGDSTIMKSELEYGNCNTKCQTPIGAINSSMPFHNIHPLTIGECPKYVKSNRLVLATGLRNSPQRERRRKKRGLFGAIA GFIEGGWQGMVDGWYGYHHSNEQGSGYAADKESTQKAIDGVTNKVNSIIDKMNTQFEAVGREFNNLERRIENLNKKMEDGF LDVWTYNAELLVLMENERTLDFHDSNVKNLYDKVRLQLRDNAKELGNGCFEFYHRCDNECMESVRNGTYDYPQYSEEARL KREEISGVKLES |
| 72 | SDQICIGYHANNSTEQVDTIMEKNVTVTHAQDILEKTHNGKLCNLDGVKPLILKDCSVAGWLLGNPMCDEFLNVSEWSYIVEK ASPANGLCYPGDFNDYEELKHLLSRINHLKKIKIIPKSYWSNHEASSGVSAACSYLGEPSFFRNVVWLIKKNNTYPPIKVNYTNT NQEDLLVLWGIHHPNDEKEQIRIYQNPNTSISVGTSTLNQRLVPKIATRPKVNGQSGRMEFFWTILKPNDSINFDSNGNFIAPEY AYKIAKKGDSVIMKSELEYGNCNTKCQTPMGAINSSMPFHNIHPLTIGECPKYVKSNRLVLATGLRNAPQRERRRKKRGLFGAI AGFIEGGWQGMVDGWYGYHHSNEQGSGYAADKESTQKAIDGITNKVNSIIDKMNTQFEIVGREFNNLERRIENLNKKMEDGF LDVWTYNAELLVLMENERTLDFHDSNVKNLYEKVRLQLRDNAKELGNGCFEFYHKCDNECMESVRNGTYDYPQYSEEARLS REEISGVKMES |
| 73 | SDQICIGYHANNSTEQVDTIMEKNVTVTHAQDILEKTHNGKLCDLDGVKPLILRDCSVAGWLLGNPMCDEFLNVPEWSYIVEK INPANDLCYPGNFNDYEELKHLLSRINHFEKIQIIPKSSWSDHEASSGVSSACPYQGRSSFFRNVVWLIKKNDAYPTIKISYNNTN QEDLLVLWGIHHPNDAAEQTRLYQNPTTYISVGTSTLNLRLVPKIATRSKVNGQSGRMEFFWTILKPNDAINFESNGNFIAPEN AYKIVKKGDSTIMKSELEYGNCNTKCQTPVGAINSSMPFHNIHPLTIGECPKYVKSNRLVLATGLRNSPQRERRRKKRGLFGAI AGFIEGGWQGMVDGWYGYHHSNEQGSGYAADKESTQKAIDGVTNKVNSIIDKMNTQFEAVGREFNNLERRIENLNKKMEDG FLDVWTYNAELLVLMENERTLDFHDSNVKNLYDKVRLQLRDNAKELGNGCFEFYHRCDNECMESVRNGTYDYPQYSEESRL KREEISGVKLES |
| 74 | SDQICIGYHANNSTEQVDTIMEKNVTVTHAQDILEKTHNGKLCDLDGVRPLILRDCSVAGWLLGNPMCDEFINVPEWSYIVEK ANPVNDLCYPGVFNDYEELKHLLSRINHFEKIQIIPKSSWPSHEASLGVSAACPYQGQSSFFRNVVWLIKKNNTYPTIKRSYNNT NQEDLLVMWGIHHPNDAAEQTKLYQNPTTYISVGTSTLNQRLTPRIATRSKVNGQSGRMEFFWTILKPNDAINFESNGNFIAPE YAYKIVKKGDSTIMRSELEYGNCNTKCQTPMGAINSSMPFHNIHPLTIGECPKYVKSTRLVLATGLRNSPQRERRRKKRGLFGA IAGFIEGGWQGMVDGWYGYHHSNEQGSGYAADKESTQKAIDGVTNKVNSIIDKMNTQFEAVGREFNNLERRIENLNKKMED GFLDVWTYNAELLVLMENERTLDFHDSNVKNLYDKVRLQLRDNAKELGNGCFEFYHKCDNECMESVRNGTYDYPQYSEEA RLKREEISGVKLES |
| 75 | SDQICIGYHANNSTEQVDTIMEKNVTVTHAQDILEKTHNGKLCNLDGVKPLILRDCSVAGWLLGNPMCDEFLNVPEWSYIVEK INPANDLCYPGNFNDYEELKHLLSRINHFEKIQIIPKDSWSDHEASGVSSACPYQGRSSFFRNVVWLTKKNDAYPTIKKSYNNT NQEDLLVLWGIHHPNDAAEQTRLYQNPTTYISVGTSTLNQRLVPKIATRSKVNGQSGRMEFFWTILKSNDAINFESNGNFIAPE NAYKIVKKGDSTIMKSELEYSNCNTKCQTPIGAINSSMPFHNIHPLTIGECPKYVKSNRLVLATGLRNSPQRERRRKKRGLFGAI AGFIEGGWQGMVDGWYGYHHSNEQGSGYAADKESTQRAIDGVTNKVNSIIDKMNTQFEAVGREFNNLERRIENLNKKMEDG FLDVWTYNAELLVLMENERTLDFHDSNVKNLYDKVRLQLRDNAKELGNGCFEFYHRCDNECMESVRNGTYDYPQYSEEARL KREEISGVKLES |
| 76 | DRICTGITSSNSPHVVKTATQGEVNVTGVIPLTTTPTKSHFANLKGTQTRGKLCPNCFNCTDLDVALGRPKCMGNTPSAKVSIL HEVKPATSGCFPIMHDRTKIRQLPNLLRGYENIRLSTSNVINTETAPRGSYKVGTSGSCPNVANGNGFFNTMAWVIPKDNNKTA INPVTVEVPYICSEGEDQITVWGFHSDDKTQMERLYGDSNPQKFTSSANGVTTHYVSQIGGFPNQTEDEGLKQSGRIVVDYMV QKPGKTGTIVYQRGILLPQKVWCASGRSKVIKGSLPLIGEADCLHEKYGGLNKSKPYYTGEHAKAIGNCPIWVKTPLKLANGT KYRPPAKLLKERGFFGAIAGFLEGGWEGMIAGWHGYTSHGAHGVAVAADLKSTQEAINKITKNLNYLSELEVKNLQRLSGAM NELHDEILELDEKVDDLRADTISSQIELAVLLSNEGIINSEDEHLLALERKLKKMLGPSAVEIGNGCFETKHKCNQTCLDRIAAG TFNAGDFSLPTFDSLNITAASLNDDGLDNHTILLYY |
| 77 | DRICTGITSSNSPHVVKTATQGEVNVTGVIPLTTTPTKSHFANLKGTQTRGKLCPNCLNCTDMDVALGRPKCMGTIPSAKVSIL HEVKPVTSGCFPIMHDRTKIRQLPNLLRGYENIRLSTRNVINAETAPGGPYTVGTSGSCPNVTNGKGFFETMAWAVPKNKNKT ATNPLTVEVPYICTKGEDQITVWGFHSDDETLMVILYGDSKPQKFTSSANGVTTHYVSQIGGFPNQTEDEGLKQSGRIVVDYIV QKPGKTGTIVYQRGVLLPQKVWCASGRSKVIKGSLPLIGEADCLHEKYGGLNKSKPYYTGEHAKAIGNCPIWVKTPLKLANGT KYRPPAKLLKERGFFGAIAGFLEGGWEGMIAGWHGYTSHGAHGVAVAADLKSTQEAINKITKNLNSLSELEVKNLQRLSGAM DELHNEILELDEKVDDLRADTISSQIELAVLLSNEGIINSEDEHLLALERKLKKMLGPSAVEIGNGCFETKHKCNQTCLDRIAAG TFNAGEFSLPTFDSLNITAASLNDDGLDNHTILLYY |
| 78 | DRICTGITSSNSPHVVKTATQGEVNVTGVIPLTTTPTKSHFANLKGTQTRGKLCPNCLNCTDLDVALGRPKCMGNIPSAKASILH EVKPGTSGCFPIMHDRTKIRQLPNLLRGYENIRLSARNVTNAETAPGGPYIVGTSGSCPNVTNGNGFFATMAWAVPKNKTATN PLTVEVPYICTKGEDQITVWGFHSDDETQMVKLYGDSKPQKFTSSANGVTTHYVSQIGGFPNQAEDEGLPQSGRIVVDYMVQK PGKTGTIAYQRGVLLPQKVWCASGRRKVIEGSLPLIGEADCLHEKYGGLNKSKPYYTGEHAKAIGNCPIWVKTPLKLANGTKY RPPAKLLKERGFFGAIAGFLEGGWEGMIAGWHGYTSHGAHGVAVAADLKSTQEAINKITKNLFSLSELEVKNLHRLSGAMDE LHNEILELDEKVDDLRADTISSQIELAVLLSNEGIINSEDEHLLALERKLKKMLGPSAVEIGNGCFETKHKCNQTCLDRIAAGTF NAGEFSLPTFDSLNITAASLNDDGLDNHTILLYY |
| 79 | DRICTGITSSNSPHVVKTATQGEVNVTGVIPLTTTPTKSHFANLKGTKTRGKLCPKCLNCTDLDVALGRPKCMGTIPSAKASILH EVKPVTSGCFPIMHDRTKIRQLPNLLRGYENIRLSTHNVINAEAAPGGPYIVGTSGSCPNVTNGNGFFATMAWAVPKNNNNKT ATNPLTVEVPFICTEGEDQITVWGFHSDNEIQMVKLYGDSKPQKFTSSANGVTTHYVSQIGGFPKQAEDGGLPQSGRIVVDYM VQKSGKTGTITYQRGILLPQKVWCASGRSKVIKGSLPLIGEADCLHEKYGGLNKSKPYYTGEHAKAIGNCPIWVKTPLKLANG TKYRPPAKLLKERGFFGAIAGFLEGGWEGMIAGWHGYTSHGAHGVAVAADLKSTQEAINKITKNLNSLSELEVKNLQRLSGA MDELHNEILELDEKVDDLRADTISSQIELAVLLSNEGIINSEDEHLLALERKLKKMLGPSAVDIGNGCFETKHKCNQTCLDRIAA GTFNAGEFSLPTFDSLNITAASLNDDGLDNHTILLYY |
| 80 | DRICTGITSSNSPHVVKTATQGEVNVTGVIPLTTTPTKSHFANLKGTKTRGKLCPNCLNCTDLDVALGRPMCIGNIPSAKASILH EVRPVTSGCFPIMHDRTKIRQLPNLLRGYEHIRLSTHNVINAERAPGGPYRLGTSGSCPNVTSRSGFFATMAWAVPRDNKTATN PLTVEVPYICTKGEDQITVWGFHSDNETQMKNLYGDSNPQKFTSSANGITTHYVSQIGGFPNQTEDGGLPQSGRIVVDYMVQK PGKTGTIVYQRGVLLPQKVWCASGRSKVIKGSLPLIGEADCLHEKYGGLNKSKPYYTGEHAKAIGNCPIWVKTPLKLANGTKY RPPAKLLKERGFFGAIAGFLEGGWEGMIAGWHGYTSHGAHGVAVAADLKSTQEAINKITKNLNSLSELEVKNLQRLSGAMDE LHNEILELDEKVDDLRADTISSQIELAVLLSNEGIINSEDEHLLALERKLKKMLGPSAVDIGNGCFETKHKCNQTCLDRIAAGTF NAGEFSLPTFDSLNITAASLNDDGLDNHTILLYY |
| 81 | DRICTGITSSNSPHVVKTATQGEVNVTGVIPLTTTLTKSHFANLKGTKTRGKLCPKCLNCTDLDVALGRPKCTGNIPSAKVSILH EVRPVTSGCFPIMHDRTKIRQLPNLLRGYERIRLSNHNVINAEKAPGGPYKIGTSGSCPNVTNGNGFFATMAWAVPKNENNKT ATNSLTIEVPYICTEGEDQITVWGFHSDNEIQMVKLYGDSKPQKFTSSANGVTTHYVSQIGGFPNQIEDGGLPQSGRIVVDYMV QKSGKTGTITYQRGILLPQKVWCASGRSKIIKGSLPLIGEADCLHEKYGGLNKSKPYYTGEHAKAIGNCPIWVKTPLKLANGTK YRPPAKLLKERGFFGAIAGFLEGGWEGMIAGWHGYTSHGAHGVAVAADLKSTQEAINKITKNLNSLSELEVKNLQRLSGAMD ELHNEILELDEKVDDLRADTISSQIELAVLLSNEGIINSEDEHLLALERKLKKMLGPSAVEIGNGCFETKHKCNQTCLDRIAAGTF NAGEFSLPTFDSLNITAASLNDDGLDNHTILLYY |
| 82 | DRICTGITSSNSPHVVKTATQGEVNVTGAIPLTTTPAKSHFANLKGTKTRGKLCPTCLNCTDLDVALGGPMCVGITPSAKASILH EVRPVTSGCFPIMHDRTKIRQLPNLLRGYEKIRLSAQNVINAEKAPGGPYKLGTSGSCPNATSKSGFFATMAWAVPRDDNKTA TNPLTVEVPHICANEEDQITVWGFHSDNKTQMKNLYGDSNPQKFTSSANGITTHYVSQIGGFPDQTEDGGLPQSGRIVVDYMV QKPGKTGTIVYQRGILLPQKVWCASGRSKVIKGSLPLIGEADCLHEKYGGLNKSKPYYTGEHAKAIGNCPIWVKTPLKLANGT KYRPPAKLLKERGFFGAIAGFLEGGWEGMIAGWHGYTSHGAHGVAVAADLKSTQEAINKITKNLNSLSELEVKNLQRLSGAM DELHNEILELDEKVDDLRADTISSQIELAVLLSNEGIINSEDEHLLALERKLKKMLGPSAVDIGNGCFETKHKCNQTCLDRIAAG TFNAGEFSLPTFDSLNITAASLNDDGLDNHTILLYY |
| 83 | DRICTGITSSNSPHVVKTATQGEVNVTGVIPLTTTPTKSYFANLKGTRTRGKLCPDCLNCTDLDVALGRPMCVGTTPSAKASIL HEVRPVTSGCFPIMHDRTKIRQLPNLLRGYENIRLSTQNVIDAENAPGGPYRLGTSGSCPNATSKSGFFATMAWAVPKDNNKN ATNPLTVEVPYVCKEGEDQITVWGFHSDNKTQMKNLYGDSNPQKFTSSANGVTTHYVSQIGGFPAQTEDGGLPQSGRIVVDY MVQKPRKTGTIVYQRGVLLPQRVWCASGRSKVIKGSLPLIGEADCLHEKYGGLNKSKPYYTGEHAKAIGNCPIWVKTPLKLA NGTKYRPPAKLLKERGFFGAIAGFLEGGWEGMIAGWHGYTSHGAHGVAVAADLKSTQEAINKITKNLNSLSELEVKNLQRLS GAMDELHNEILELDEKVDDLRADTISSQIELAVLLSNEGIINSEDEHLLALERKLKKMLGPSAVDIGNGCFETKHKCNQTCLDRI AAGTFNAGEFSLPTFDSLNITAASLNDDGLDNHTILLYY |
| 84 | DRICTGITSSNSPHVVKTATQGEVNVTGVIPLTTTPTKSYFANLKGTRTRGKLCPDCLNCTDLDVALGRPMCVGTTPSAKASIL HEVRPVTSGCFPIMHDRTKIRQLPNLLRGYENIRLSTQNVIDAEKAPGGPYRLGTSGSCPNATSKSGFFATMAWAVPKDNYKN ATNPLTVEVPYICTEGEGQITVWGFHSDNKPQMKNLYGDSNPQKFTSSASGVTTHYVSQIGDFPDQTEDGGLPQSGRIVVDYM MQKPGKTGTIVYQRGVLLPQKVWCASGRSKVIKGSLPLIGEADCLHEKYGGLNKSKPYYTGEHAKAIGNCPIWVITPQKLANG TTFSPPAKLLKERGFFGAIAGFLEGGWEGMIAGWHGYTSHGAHGVAVAADLKSTQEAINKITKNLNSLSELEVKNLQRLSGAM DELHNEILELDEKVDDLRADTISSQIELAVLLSNEGIINSEDEHLLALERKLKKMLGPSAVDIGNGCFETKHKCNQTCLDRIAAG TFNAGEFSLPTFDSLNITAASLNDDGLDNHTILLYY |
| 85 | DRICTGITSSISPLVDKTATQGEVNVTGVIPLTTTPTKSYFANVKGTRTRGKLCPDCLNCTDLDVALGRPMCVGTTPSAKASILH EVRPVTSGCFPIMHDRTKIRQLPNLLRGYENIRLSTQNVIDAQKAPGGPYRLGTSGSCPNATSKIGFFATMACAVPKDNYKNAT NPLTVEVPYIRTEGEDQITVWGFHSDNKTQMKNLYGDSNPPKFTSSANGVTTHYVSQTGDFPDQTEDGGLPPSGRIVVDYMM QKPGKTGTIAYQRGVLLPQKVWCASGRSKVIKGSLPLIGEADCLHEKYGGLNKSKPPHTGEHTKAIGNCPIWVKTPLKLANGT KYRPSAKLLKERGFFGAIAGFLEGGWEGMIAGWHGYTSHGAHGVAVAADLKSTQEAINKITKNLNSLSELEVKNLQRLSGAM DELHNEILELDEKVDDLRADTISSQIELAVLLSNEGIINSEDEHLLALERKLKKMLGPSAVDIGNGCFETKHKCNQTCLDRIAAG TFNAGEFFLPTFDSLNITAASLNDDGLDNHTILLYY |
| 86 | DRICTEITSSNSPLVVKTATQGEVNVTGVIPLTTTPTKSYFANLKGTRTRGKLCPDCLNCTDLDVALGRPMCVGTTPSAKASILH EVRPVTSGCFPIMHDRTKIRQLPNLLRGYENIRLSTQNVIDAEKAPGGPYRLGTSGSCPNATSKIGFFATMAWAVPKDNYKNAT NPLTVEVPYICTEGEDQITVWGFHSDNKTQMKNLYGDSNPQKFTSSANGVTTHYVSQIGDFPDQTEDGGLPQSGRIVVDYMM QKPGKTGPIVYQRGVLLPQKVWCASGRSKVIKGSLPLIGEADCLHEKYGGLNKSKYSHTGDHAKTLGNCPIWVKTPLKLANG TKYRPPAKLLKERGFFGAIAGFLEGGWEGMIAGWHGYTSHGAHGVAVAADLKSTQEAINKITKNLNSLSELEVKNLQRLSGA MDELHNEILELDEKVDDLRADTISSQIELAVLLSNEGIINSEDEHLLALERKLKKMLGPSAVDIGNGCFETKHKCNQTCLDRIAA GTFNAGEFSLPTFDSLNITAASLNDDGLDNHTILLYY |
| 87 | DRICTGITSSNSPHVVKTATQGEVNVTGVIPLTTTPTKSHFANLKGTETRGKLCPKCLNCTDLDVALGRPKCTGTTPSARASILH EVRPVTSGCFPILHDRTKIRQLPNLLRGYEHIRLSTHNVINAENAPGGPYKIGTSGSCPNITNGNGFFATMAWAVPKNDKNKTA TNPLSVEVPYICTEGEDQITVWGFHSDNETQMAKLYGDSKPQKFTSSANGVATHYVSQIGGFPNQTEDGGLPQSGRIVVDYMV QKSGKTGTITYQRGILLPQRGWCASGRSKVIKGSLPLFGEADCLHEKYGGLNKSKPYYTGEHAKAIGNCPIWVKTPLKLANGT KYRPPAKLFKERGFFGAIAGFLEGGWEGMIAGWHGYTSHGAHGVPVAADLKSTQEAINKITKNLNSLSELEVKNLQRLSGAM DELHNEILELDEKVDDLRADTISSQIELAVLLSNEGIINSEDEHLLALERKLKKMLGPSAVEIGNGCFETKHKCNQTCLDRIAAG TFDAGEFSLPTFDSLNITAASLNDDGLDNHTILLYY |

**Table 7: Amino acid sequences of neuraminidase antigens that can be included in a flu vaccine**

| **SEQ ID** | **Sequence** |
|---|---|
| 88 | HSIQTRDQNHPETCNQSIITYENNTWVNQTYVNISNANIVAGQDATSMILAGNSSLCPISRWAIYSKDNSIRIGSKGDIFVIREPFI SCSHLECRTFFLTQGALLNDKHSNGTVKDRSPYRTLMSCPIGEAPSPYNSRFESVAWSASACHDGMSWLTIGISGPDNGAVAVL KYNGIITDTIKSWRNKILRTQESECACVNGSCFTIMTDGPSNGQASYKIFKIEKGRIIKSIELNAPNYHYEECSCYPDTSKVMCVC RDNWHGSNRPWVSFDQNLDYQIGYICSGVFGDNPRSNDGTGSCGPVSSNGANGVKGFSFRYGNGVWIGRTKSISSRSGFEMIW DPNGWTETDSSFSVKQDIVAITDWSGYSGSFVQHPELTGLDCIRPCFWVELIRGQPKENTIWTSGSSISFCGVNSDTVGWSWPD GAELPFTIDK |
| 89 | HSIQTGSQNHTGICNQNIITYKNSTWVNQTYVNISNTNVVAGQDTTSVILTGNSSLCPIRGWAIYSKDNSIRIGSKGDVFVIREPFI SCSHLECRTFFLTQGALLNDRHSNGTVKDRSPYRALMSCPVGEAPSPYNSRFESVAWSASACHDGMGWLTIGISGPDNGAVAV LKYNGIITETIKSWRKKILRTQESECACVNGSCFTIMTDGPSNGLASYKIFKIEKGKVTKSIELNAPNSHYEECSCYPDTGKVMC VCRDNWHGSNRPWVSFDQNLDYQIGYICSGVFGDNPRPKDGTGSCGPVYVDGANGVKGFSYRYGNGVWIGRTKSNSSRHGF EMIWDPNGWTETDSKFSVRQDVVAMTDWSGYSGSFVQHPELTGLDCMRPCFWVELIRGRPKENTIWTSGSSISFCGVNSDTV DWSWPDGAELPFTIDK |
| 90 | HSIQTGSQNHTGTCNQSIITYKNSTWVNQTYVNISNTNVVAGKDTTSVILAGNSSLCPIRGWAIYSKDNGVRIGSKGDVFVIREP FISCSHLECRTFFLTQGALLNDKHSNGTVKDRSPYRALMSCPVGEAPSPYNSRFESVAWSASACHDGMGWLTIGISGPDDGAV AVLKYNGIITETIKSWRKEILRTQESECACVNGSCFTIMTDGPSGGPASYKIFKIEKGKVTKSIELDAPNSHYEECSCYPDTGKVM CVCRDNWHGSNRPWVSFDQNLDYQMGYICSGVFGDNPRPKDGKGNCGPVYVDGANGVKGFSYRYGNGVWIGRTKSNSSRQ GFEMIWDPNGWTETDSNFFVKQDVVAVTDWSGYSGSFVQHPELTGLDCMRPCFWVELIRGRPKEKTIWTSGSSISFCGVNSDT VDWSWPDGAELPFTIDK |
| 91 | HSIQTGSQNHTGICNQRIITYENSTWVNQTYVNINNTNVVAGKDTTSVTLAGNSSLCPVRGWAIYSKDNSIRIGSKGDVFVIREP FISCSHLECRTFFLTQGALLNDKHSNGTVKDRSPYRALMSCPIGEAPSPYNSRFESVAWSASACHDGMGWLTIGISGPDDGAVA VLKYNGIITETIKSWRKRILRTQESECVCINGSCFTIMTDGPSNGPASYRIFKIEKGKITKSIELDAPNSHYEECSCYPDTGTVMCV CRDNWHGSNRPWVSFNQNLDYQIGYICSGVFGDNPRPKDGKGSCGPVTVDGADGVKGFSYRYGNGVWIGRTKSNSSRKGFE MIWDPNGWTDTDSNFLVKQDVVAMTDWSGYSGSFVQHPELTGLDCMRPCFWVELIRGRPREKTTIWTSGSSISFCGVNSDTV NWSWPDGAELPFTIDK |
| 92 | HSIQIGTQNQPETCNQSVITYENNTWVNQTYVNISNTNFVAEQTVVSVKLAGNSSLCPVSGWAIYSKDNSVRIGSKGDVFVIRE PFISCSHLECRTFFLTQGALLNDKHSNGTIKDRSPYRTLMSCPVGEVPSPYNSRFESVAWSASACHDGTSWLTIGISGPDNGAVA VLKYNGIITDTIKSWRNNILRTQESECACVNGSCFTVMTDGPSNGQASYKIFKIERGKVVKSVELNAPNYHYEECSCYPDSGEIT CVCRDNWHGSNRPWVSFNQNLEYKIGYICSGIFGDNPRPNDRTGSCGPVFSNGANGVKGFSFKYGNGVWIGRTKSTSSRSGFE MIWDPNGWTGTDNNFSVKQDIVGITDWSGYSGSFVQHPELTGLDCMRPCFWVELIRGRPKENTIWTSGSSISFCGVTSDTVGW SWPDGAELPFTIDK |
| 93 | HSIQTGSQNHTGICNQRIITYENSTWVNHTYVNINNTNVVAGKDKTSVTLAGNSSLCSISGWAIYTKDNSIRIGSKGDVFVIREPF ISCSHLECRTFFLTQGALLNDKHSNGTVKDRSPYRALMSCPLGEAPSPYNSKFESVAWSASACHDGMGWLTIGISGPDNGAVA VLKYNGIITETIKSWKKRILRTQESECVCVNGSCFTIMTDGPSNGAASYKIFKIEKGKVTKSIELNAPNSHYEECSCYPDTGTVM CVCRDNWHGSNRPWVSFNQNLDYQIGYICSGVFGDNPRPKDGEGSCNPVTVDGADGVKGFSYRYGNGVWIGRTKSNRLRKG FEMIWDPNGWTDTDSDFSVKQDVVAMTDWSGYSGSFVQHPELTGLDCIRPCFWVELVRGRPRENTTIWTSGSSISFCGVNSDT ANWSWPDGAELPFTIDK |
| 94 | HSIQTGSQNHTGICNQRIITYENSTWVNQTYVNINNTNVVAGKDKTSMTLAGNSSLCPIRGWAIYTKDNSIRIGSKGDVFVIREP FISCSHLECRTFFLTQGALLNDKHSNGTVKNRSPYRALMSCPLGEAPSPYNSRFESVAWSASACHDGLGWLTIGISGPDNGAVA VLKYNGIITETIKSWKKRILRTQESECVCMNGSCFTIMTDGPSDGAASYRIFKIEKGRVIKSIELDAPNYHYEECSCYPDTGTVM CVCRDNWHGSNRPWVSFNQNLDYQIGYICSGVLGDNPRPKDGEGSCNPVTADGADGVKGFSYRYGNGVWIGRTKSNRLRKG FEMIWDPNGWTDTDSDFSMKQDIVAMTDWSGYSGSFVQHPELTGLDCMRPCFWVELVRGLPRENTTIWTSGSSISFCGVNSD TANWSWPDGAELPFTIDK |
| 95 | HSIQTGSQNHTGVCNQRIITYENSTWVNHTYVNINNTNVIVGKDKTSVTLAGNSSLCSISGWAIYTKDNSIRIGSKGDVFVIREPF ISCSHLECRTFFLTQGALLNDKHSNGTVKDRSPYRALMSCPLGEAPSPYNSKFESVAWSASACHDGMGWLTIGISGPDNGAVA VLKYNGIITETIKSWKKQILRTQESECVCVNGSCFTIMTDGPSNGAASYKIFKIEKGKITKSIELNAPNFHYEECSCYPDTGTVMC VCRDNWHGSNRPWVSFNQNLDYQIGYICSGVFGDNPRPKDGEGSCNPVTVDGANGVKGFSYKYGNGVWIGRTKSNRLRKGF EMVWDPNGWTDTDSDFSVKQDVVAITDWSGYSGSFVQHPELTGLNCIRPCFWVELVRGLPKENTTIWTSGSSISFCGVNSDTA NWSWPDGAELPFTIDK |
| 96 | HSIQTGWKNHTETCIQNVITYENNTWVNQTYVNISNTKTVTGQGVASTMVAGNSSLCPINGWAVYSKDNSIRIGSKGDIFVIRE PFISCSNLECRTFFLTQGALLNDRHSNGTVKDRSPYRTLMSCPIGEAPSPYNSRFESVAWSASACHDGVGWLTIGISGPDNGAV AVLKYNGIITDTIKSWRNKILRTQESECVCINGSCFTIMTDGPSNEQASYKVFKIEKGKIIKTIELDAPNYHYEECSCYPDTSKVM CVCRDNWHASNRPWVSFDQNLNYQIGYICSGVFGDNPRSNDGKGSCGPVPSSGANGVKGFSFRYGSGVWIGRTKSISSRSGFE MIWDPNGWTETDSSFSMKQDIIALTDWSGYSGSFVQHPELTGMDCIRPCFWVELIRGQPKESTIWTSGSSISFCGVNSETASWS WPDGADLPFTIDK |
| 97 | HSIQTGSQNSTGICNQRIITYENSTWVNHTYVNINNTNVVAGEDKTSVTLVGNSSLCSISGWAIYTKDNSIRIGSKGDVFVIREPFI SCSHLECRTFFLTQGALLNDKHSNGTVKDRSPYRALMSCPLGEAPSPYNSKFESVAWSASACHDGMGWLTIGISGPDNGAVAV LKYNGIITGTIKSWKKQILRTQESECVCMNGSCFTIMTDGPSNKAASYKIFKIEKGKVTKSIELNAPNFYYEECSCYPDTGIVMC VCRDNWHGSNRPWVSFNQNLDYQIGYICSGVFGDNPRPEDGEGSCNPVTVDGANGVKGFSYKYGNGVWIGRTKSNRLRKGF EMIWDPNGWTNTDSDFSVKQDVVAITDWSGYSGSFVQHPELTGLDCIRPCFWVELVRGLPRENTTIWTSGSSISFCGVNSDTA NWSWPDGAELPFTIDK |
| 98 | HSIQTESQNKPEICNQNVITYENNTWVNQTYVTVSNTNFVAEQAVASVKLVGNSSLCPVSGWAIYSKDNSVRIGSKGDVFVIRE PFISCSHLECRTFFLTQGALLNDKHSNGTIKDRSPYRTLMSCPIGEVPSPYNSRFESVAWSASACHDGTSWLTIGISGPDNGAVA VLKYNGIITDTIKSWRKNILRTQESECACVNGSCFTIMTDGPSNGQASYKVFKIERGKVVKSVELNAPNYHYEECSCYPESSEIT CVCRDNWHGSNRPWVSFNQNLEYQMGYICSGIFGDNPRPNDKTGSCGPVFSNGANGVKGFSFRYGNGVWIGRTKSTSSRMGF EMIWDPDGWIRTDDKFSVKQDIIGITDWSGYSGSFVQHPELTGLDCMRPCFWVELIRGRPKENTIWTSGSSISFCGVNSDTVGW SWPDGAELPFTIDK |
| 99 | HSIQTGGKNYSEMCNQNVITYVNNTWVNRTYVNISNTNVATVQGVNSIILSGNSSLCPVSGWAIYSKDNGIRIGSKGDIFVIREP FISCSHLECRTFFLTQGALLNDKHSNGTVKDRSPYRTLMSCPIGEAPSPYNSRFESVAWSASACHDGMGWLTIGISGPDNGAVA VLKYNGIITDTIKSWRNKILRTQESECVCMNGSCFTVLTDGPSDGQASYKILKVEKGKIIKSIELNAPNYHYEECSCYPDAGKVM CVCRDNWHASNRPWVSFDQNLDYQIGYICSGVFGDNPRSNDGKGKCGPVFPNGANGVKGFSFKYGNGIWIGRTKSINSRRGF EMIWDPNGWTETDSSFSMKQDVIALTDWSGYSGSFVQHPELTGMNCIRPCFWVELIRGQPKEGTIWTSGSSISFCGVNSETTSW SWPDGADLPFTIDK |
| 100 | HSIQIGSQSQIETCNQSVITYENNTWVNQTYVNISNTNFAAGQSVVSVKLAGNSSLCPVSGWAIYSKDNSVRIGSKGDVFVIREP FISCSPFECRTFFLTQGALLNDKHSNGTIKDRSPYRTLMSCPIGEVPSPYNSRFESVAWSASACHDGINWLTIGISGPDSGAVAVL KYNGIITDTIKSWRNNILRTQESECACVNGSCFTIMTDGPSDGQASYKIFRIEKGKIIKSVEMKAPNYHYEECSCYPDSSEITCVC RDNWHGSNRPWVSFNQNLEYQMGYICSGVFGDNPRPNDKTGSCGPVSSNGANGVKGFSFKYGNGVWIGRTKSISSRKGFEMI WDPNGWTGTDNKFSIKQDIVGINEWSGYSGSFVQHPELTGLDCIRPCFWVELIRGRPEENTIWTSGSSISFCGVNSDTAGWSWP DGAELPFTIDK |
| 101 | HSIQLGSQNYTKTCTQSVITYENNTWVNQTYVNISNTNLAVGQSVVSAKLAGNSSLCPVSGWAIYSKDNSIRIGSKGDVFVIRE PFISCSPLECRTFFLTQGALLNDQHSNGTIKDRSPYRTLMSCPIGEVPSPYNSRFESVAWSASACHDGINWLTIGISGPDNGAVAV LKYNGIITDTIKSWRNNILRTQESECVCVNGSCFTVMTDGPSNGQASYKIFRIEKGKIVKSVEMNAPNYHYEECSCYPDSSEITC VCRDNWHGSNRPWVSFNQNLEYQIGYICSGIFGDNPRPNDKTGSCGPVSSNGANGVKGFSFKYGNGVWIGRTKSISSRKGFEM IWDPNGWTGTDNNFSIKQDIVGINEWSGYSGSFVQHPELTGLDCIRPCFWVELIRGRPKENTVWTSGSSISFCGVNSDTVGWSW PDGAELPFTIDK |
| 102 | HFKQYECDSPASNQVMPCEPIIIERNITEIVYLNNTTIEKEICPKVVEYRNWSKPQCQITGFAPFSKDNSIRLSAGGDIWVTREPYV SCDHGKCYQFALGQGTTLDNKHSNDTIHDRIPHRTLLMNELGVPFHLGTRQVCIAWSSSSCHDGKAWLHVCITGDDKNATAS FIYDGRLVDSIGSWSQNILRTQESECVCINGTCTVVMTDGSASGRADTRILFIEEGKIVHISPLSGSAQHVEECSCYPRYPGVRCIC RDNWKGSNRPVVDINMEDYSIDSSYVCSGLVGDTPRNDDRSSNSNCKNPNNERGNQGVKGWAFDNGDDVWMGRTISKDLRS GYETFKVIGGWSTPNSKSQINRQVIVDSDNRSGYSGIFSVEGKSCINRCFYVELIRGRKQETRVWWTSNSIVVFCGTSGTYGTGS WPDGANINFMPI |
| 103 | HFKQHECDSPSSNQVMLCEPIIIERNITEIVYLNNTTIEKETCPKLVEYRNWSKPQCKITGFAPFSKDNSIRLSAGGDIWVTREPYV SCDPGKCYQFALGQGTTLDNKHSNDTIHDRIPHRTLLMNELGVPFHLGTRQVCIAWSSSSCHDGKAWLHVCVTGDDKNATAS FIYDGRLVDSIGSWSQNILRTQESECVCINGTCTVVMTDGSASGRADTRILFIEEGKIVHISPLSGSAQHVEECSCYPRYPDVRCIC RDNWKGSNRPIVDINMKDYSIDSSYVCSGLVGDTPRNDDRSSKSNCRNPNNERGNHGVKGWAFDNGNDVWMGRTISKDLRS GYETFKVIGGWSTPNSKSQINRQVIVDSDNRSGYSGIFSVEGKSCINRCFYVELIRGREQETRVWWTSNSIVVFCGTSGTYGTGS WPDGANINFMPI |
| 104 | HFKQYECDSPANNQVMPCEPIIIERNITEIVYLTNTTIEKEICPKLVEYRNWSKPQCKITGFAPFSKDNSIRLSAGGDIWVTREPYV SCDPGKCYQFALGQGTTLDNKHSNDTIHDRIPHRTLLMNELGVPFHLGTRQVCIAWSSSSCHDGKAWLHVCVTGDDENATAS FIYDGRLVDSIGSWSQNILRTQESECVCINGTCTVVMTDGSASGRADTRILFIEEGKIVHISPLSGSAQHVEECSCYPRYPGVRCIC RDNWKGSNRPVVDINVKDYSIDSRYVCSGLVGDTPRNNDKSSNSNCRNPNNDKGNHGVKGWAFDDGNDVWMGRTISKDSR SGYETFKVIGGWSTPNSKSQINRQVIVDSDNRSGYSGIFSVEGKSCINRCFYVELIRGREQETKVWWTSNSIVVFCGTSGTYGTG SWPDGADINLMSI |
| 105 | HFKQYECDSPANNQVMPCEPIIIERNITKIMYLTNTTIEKEICPKSVEYRNWSKPQCKITGFAPFSKDNSIRLSAGGAIWVTREPY VSCDPGKCYQFALGQGTTLDNKHSNDTIHDRTPHRTLLMNELGVPFHLGTRQVCIAWSSSSCHDGKAWLHVCVTGYDKNAT ASFIYDGRLVDSIGSWSQNILRTQESECVCINGTCTVVMTDGSASGKADTKILFIEEGKIVHVSPLSGSAQHVEECSCYPRYPGV RCICRDNWKGSNRPIVDINVKDYSIDSSYVCSGLVGDTPRNNDRSSNSYCRNPNNERGNHGVKGWAFDDGDDVWMGRTISKD SRLGYETFKVIGGWSTPNSKLQINKQVIVDSDNRSGYSGIFSVEGKSCINRCFYVELIRGRRQETRVWWTSNSIVVFCGTSGTYG TGSWPDGADINLMPI |
| 106 | HFKQYECNSPPNNQVMLCEPTIIERNITEIVYLTNTTIEKEKCPKLVEYRNWSKPQCKITGFAPFSKDNSIRLSAGGDIWVTREPY VSCDPGKCYQFALGQGTTLNNRHSNDTVHDRTPYRTLLMNELGVPFHLGTKQVCIAWSSSSCHDGKAWLHVCVTGHDENAT ASFIYDGRLVDSIGSWSKNILRTQESECVCINGTCTVVMTDGSASERADTKILFIEEGKIVHISPLSGSAQHVEECSCYPRYPGVR CVCRDNWKGSNRPIVDINVKDYSIVSSYVCSGLVGDTPRKNDSSSSSYCRNPNNEKGSHGVKGWAFDDGNDVWMGRTISEEL RSGYETFKVIGGWSTPNSKLQINRQVIVDRGNRSGYSGIFSVEGKSCINRCFYVELIRGRKQENKVWWTSNSIVVFCGTSGTYG TGSWPDGADINLMPI |
| 107 | HFKQYECNSPPNNQVMLCEPTIIERNITEIVYLTNTTIEKEICPKLAEYRNWSKPQCNITGFAPFSKDNSIRLSAGGDIWVTREPY VSCDPDKCYQFALGQGTTLNNGHSNDTVHDRTPYRTLLMNELGVPFHLGTKQVCIAWSSSSCHDGKAWLHVCVTGDDENAT ASFIYNGRLVDSIGSWSKKILRTQESECVCINGTCTVVMTDGSASGKADTKILFIEEGKIVHTSPLSGSAQHVEECSCYPRYPGVR CVCRDNWKGSNRPIVDINVKDYSIVSSYVCSGLVGDTPRKNDSSSSSHCLNPNNEEGGHGVKGWAFDDGNDVWMGRTISEKL RSGYETFKVIEGWSKPNSKLQINRQVIVDRGNRSGYSGIFSVEGKSCINRCFYVELIRGRKQETEVLWTSNSIVVFCGTSGTYGT GSWPDGADINLMPI |
| 108 | HFKQYECNSPPNNQVILCEPTIIERNITEIVYLTNTTIEKEICPKLAEYRNWSKPQCKITGFAPFSKDNSIRLSAGGDIWVTREPYV SCDPDKCYQFALGQGTTLNNRHSNDTVHDRTPYRTLLMNELGVPFHLGTKQVCIAWSSSSCHDGKAWLHVCVTGHDENATA SFIYNGRLVDSIGSWSKKILRTQESECVCVNGTCTVVMTDGSASGRADTKILFIEEGQIVHISPLLGSAQHVEECSCYPRYPGVR CVCRDNWKGSNRPIVDINVKDYSIVSSYVCSGLVGDTPRKNDRSSSSNCLNPNNEEGGHGVKGWAFDDGNDVWMGRTISEKL RSGYETFKVIEGWSKPNSKLQINRQVIVDRDDRSGYSGIFSVEGKSCINRCFYVELIRGRKQETEVWWTSNSIVVFCGTPGTYGT GSWPDGADINLMPI |
| 109 | HFKQYEFNSPPNNQVMLCEPTIIERNITEIVYLTNTTIEKEICPKLAEYRNWSKPQCDITGFAPFSKDNSIRLSAGGDIWVTREPYV SCDPDRCYQFALGQGTTLNNVHSNDTVHDRTPYRTLLMNELGVPFHLGTKQVCIAWSSSSCHDGKAWLHVCVTGDDKNATA SFIYNGRLVDSIVSWSKKILRTQESECVCINGTCTVVMTDGSASGKADTKILFIEEGKIVHTSTLSGSAQHVEECSCYPRYPGVRC VCRDNWKGSNRPIVDINIKDYSIVSSYVCSGLVGDTPRKNDSSSSSHCLDPNNEEGGHGVKGWAFDDGNDVWMGRTISEKLRS GYETFKVIEGWSNPNSKLQINRQVIVDRGNRSGYSGIFSVEGKSCINRCFYVELIRGRKEETEVLWTSNSIVVFCGTSGTYGTGS WPDGADINLMPI |
| 110 | HFKQYECDPPANNQVMTCKPIIIERNTTEIVYLTNVTIEKETYPKIVEYRNWSMPQCKITGFAPFSKDNSIRLSAGGDIWVTREPY VSCEPGKCYQFALGQGTTLDNKHSNDTIHDRTPHRTLLMNELGVPFYLGTRQVCIAWSSSSCYDGKAWLHVCITGHDKNATA SFIYDGRLVDSIGSWSMNILRTQESECVCINGTCTVVMTDGSASGKADTRILFIEEGKIIHISPLAGSAQHVEECSCYPRYPGVRCI CRDNWKGSNRPIVDINVKDYSIDSSYVCSGLVGDTPRNNDKSSSSYCRNPNNEKGGHGVKGWAFDDGNDLWMGRTISEDIRS GYETFKVIGGWSIPNSKLQVNRQVIVDSNNRSGYSGIFSVEGKSCINRCFYVELIRGRRTEARVGWTSNSIVVFCGTSGTYGTGT WPDGADINLMPI |
| 111 | HFKQYECSSPSTNQVRLCEPTIIERNITEIVYLTNTTIEKEICPKLAEYRDWSKPQCKIKGFAPFSKDNSIRLSAGGDIWVTREPYV SCDPDKCYQFALGQGTTLNNRHSNDTVHDRTPYRTLLMNELGVPFHLGTKQVCIAWSSSSCHDGKAWLHVCVTGHDENATA SFIYNGRLVDSIGSWSKKILRTQESECVCINGTCTVVMTDGSASGRADTKILFIEEGKIVHISQLSGSAQHVEECSCYPRYPGVRC VCRDNWKGSNRPIVDINVKDRSIASSYVCSGLVGDTPRKNDSSSSSHCLTPNNEEGGHGVKGWAFDDGNDVWMGRTISEKLR LGYETFKVIEGWSKPNSKLQINRQVIVDKGNRSGYSGIFSVEGKSCINRCFYVELIRGRKQETEVWWTSNSIVVFCGTSGTYGT GSWPDGADINLMPI |
| 112 | HFRQCECNSSAINQTMPCEPTKIEKNITEIVYLTNTTIKTEVCPKLVKYRNWAKPQCKITGFAPFSKDNSIRLSAGGAIWVTREPY VSCDLSKCYQFALGQGTTLDNRHSNDTIHDRTPYRTLLMNELGVPFHLGTRQVCIAWSSSSCHDGKAWLHVCVTGYDKNATA SLIYDGRLVDSIGSWSQNILRTQESECVCINGTCTVVMTDGSASGKADTRILFVEEGKIIHISPLTGSAQHVEECSCYPRYPGVRC VCRDNWKGSNRPVVDINVKDYKINSSYVCSGLVGNTPRNNDRSSNSNCQNPNNQRGNHGVKGWAFDDGNDIWMGRTISNDS RLGYETFKVIGGWSKPNSKVQTNRQVIVDSDNRSGYSGVFSVEGKSCINRCFYVELIRGRRQETRVWWTSNSIVVFCGTSGTY GSGSWPDGADINLMPI |
| 113 | HFNQCDCHYPPNNQVTLCEPTITERNTTKIVYLTNTTIEKENCPKLAEYRDWSKPQCKITGFAPFSKDNSIRLSAGGDIWVTREP YVSCDPDKCYQFALGQGTTLNNRHSNDTFHDRTPYRTLLMNELGIPFHLGTKQVCIAWSSSSCHDGKAWLHVCITGHDENAT ASIIYNGRLVDSIGSWSKKILRTQESECVCINGTCTVVMTDGSASGRADTKILFIEEGKIIHISPLIGSAQHVEECSCYPRYPGVRCI CRDNWKGSNRPVVDINVKDYSIVSSYVCSGLVGDTPRKDDKSSSSNCLNPNNEKGEHGVKGWAFDDGKDVWMGRTINETLR SGYETFKVIEGWSKSNSKLQINRQVIVEKSDRSGYSGIFSVEGKSCINRCFYVELIRGRKQETAVWWTSNSIVVFCGTSGTYGTG SWPDGADINLMPV |
| 114 | HFEQYDCNPFPNNQVMLCKPTIIERNTTEFVYLTNTTIEKEICPKLAEYRNWAKPQCTITGFAPFSRDNSIRLSAGGDIWVTREPY VSCDPDKCYQFALGQGTTLNNGHSNDTVHDRTPYRTLLMNELGVPFHLGTRQVCIAWSSSSCHDGKAWLHVCITGDDKNAT ASFIYGGKLVDSIGSWSKNILRTQESECVCINGTCTVVMTDGSASGKADTKILFIKEGKIVHISTLSGSAQHVEECSCYPRYPGVR CVCRDNWKGSNRPVVDINVKDYSIASSYVCSGLVGDTPRKNDSFSNSHCLNPNNEEGGHGVKGWAFDDGSDVWMGRTISEK FRLGYETFKVIEGWSRPNSKLQTNRQVIVERGNRSGYSGIFSVEGKSCINRCFYVELIRGRKEETKVWWTSNSIVVFCGTSGTYG TGSWPDGADINIMPI |
| *115* | HLRQYEFNSPPKNNQVMLCEPTIIERNITEIVYLTNTTIEKEICPKLAEYRNWSKPQCDITGFAPFSKDNSIRLSAGGDIWVTREPY VSCGPDKCFQFALGQGTTLNNVHSNDTVHDRTPYRTLLMSELGVPFHLGTKQVCIAWSSSSCHDGKAWLHVCVTGDDKNAT ASIIYNGRLVDSIVSWSKKILRTQESECVCINGICTVVMTDGSASGKADTKILFIEEGKIVHTSTLSGSAQHVEECSCYPRYPGVR CVCRDNWKGSNRPIVDINIRDYSIVSNYVCSGLVGDTPRKPDSSSSSNCLDPNDEEGGHGVKGWAFDDGNDVWMGRTISEKSR LGYETFKVIGGWFNPNSKSQINRQVIVDRGNRSGYSGIFSVEGKNCINRCFYVELIRGRKDEIEVLWTSNSIVVFCGTSGTYGTG SWPDGADLKLMSV |
| 116 | LYSDILLKFSPTEITAPTMPLGCANASNVQAVNRSATKGVTLLLPEPEWTYPRLSCPGSTFQKALLISPHRFGETKGNSAPLIIREP FIACGPNECKHFALTHYAAQPGGYYNGTRGDRNKLRHLISVKLGKIPTVENSIFHMAAWSGSACHDGKEWTYIGVDGPDNNA LLKVKYGEAYTDTYHSYANKILRTQESACNCIGGNCYLMITDGSASGVSECRFLKIREGRIIKEIFPTGRVKHTEECTCGFASNK TIECACRDNRYTAKRPFVKLNVEADTAEIRLMCTDTYLDTPRPNDGSITGPCESDGDKGSGGIKGGFVHQRMKSKIGRWYSRT MSKTERMGMGLYVKYDGDPWADSDALAFSGVMVSMKEPGWYSFGFEIKDKKCDVPCIGIEMVHDGGKETWHSAATAIYCL MGSGQLLWDTVTGVDMAL |
| 117 | LYSDVLLKFSSTKTTAPTMSLECTNASNAQTVNHSATKEMTFPPPEPEWTYPRLSCQGSTFQKALLISPHRFGEIKGNSAPLIIRE PFVACGPKECRHFALTHYAAQPGGYYNGTRKDRNKLRHLVSVKLGKIPTVENSIFHMAAWSGSACHDGREWTYIGVDGPDN DALVKIKYGEAYTDTYHSYAHNILRTQESACNCIGGDCYLMITDGSASGISKCRFLKIREGRIIKEILPTGRVEHTEECTCGFASN KTIECACRDNSYTAKRPFVKLNVETDTAEIRLMCTKTYLDTPRPDDGSIAGPCESNGDKWLGGIKGGFVHQRMASKIGRWYSR TMSKTNRMGMELYVKYDGDPWTDSDALTLSGVMVSIEEPGWYSFGFEIKDKKCDVPCIGIEMVHDGGKDTWHSAATAIYCL MGSGQLLWDTVTGVDMAL |
| 118 | LYSDILLKFSPTKRTAPTMSLECVNVSNAQAVNHSATKEMTFLLPEPEWTYPRLSCQGSTFQKALLISPHRFGETRGNSAPLIIRE PFVACGPKECRHFALTHYAAQPGGYYNGTRKDRNKLRHLISVKLGKIPTVENSIFHMAAWSGSACHDGREWTYIGVDGPDSD ALIKIKYGEAYTDTYHSYAHNILRTQESACNCIGGDCYLMITDGSASGISKCRFLKIREGRIIKEIFPAGRVEHTEECTCGFASNKT IECACRDNSYTAKRPFVKLNVETDTAEIRLMCTETYLDTPRPDDGSITGPCESNGDKGLGGIKGGFVHQRMASKIGRWYSRTM SKTERMGMELYVKYDGDPWTDSDALAPSGVMVSIKEPGWYSFGFEIKDKKCDVPCIGIEMVHDGGKETWHSAATAIYCLMG SGQLLWDTVTGVDMAL |
| 119 | LYSDILLKFSPTKITAPTMSLDCANVSNVQAVNRSVTKEMTFLLPEPEWTYPRLSCQGSTFQKALLISPHRFGETRGNSAPLIIRE PFVACGPKECRHFALTHYAAQPGGYYNGTRNDRNKLRHLISVKLGKIPTVENSIFHMAAWSGSACHDGREWTYIGVDGPDSN ALIKIKYGEAYTDTYHSYANNILRTQESACNCIGGDCYPMITDGSASGISKCRFLKIREGRIIKEIFPTGRVDHTEECTCGFASNKT IECACRDNSYTAKRPFVKLNVETDTAEIRLMCTETYLDTPRPDDGSITGPCESNGDKGLGGIKGGFVHQRMASKTGRWYSRTM SKTERMGMELYVRYDGDPWTDSDALAPSGVMVSMKEPGWYSFGFEIKDKKCDVPCIGIEVVQVGGKETWHSAATAIYCLMG SGQLLWDIVTGVAMAL |
| 120 | LYSDILLKFSPTEKSAPTMSLDCANASNVQAVNRSATKGMTLLLPEPEWTYPRLSCQGSTFQKALLISPHRFGETRGNSAPLIIRE PFIACGPKECKHFALTHYAAQPGGYYNGTREDRNKLRHLISVKLGKIPTVENSIFHMAAWSGSACHDGREWTYIGVDGPDSNA LIKIKYGEAYTDTYHSYANNILRTQESACNCIGGDCYLMITDGSASGISKCRFLKIREGRIIKEIFPTGRVDHTEECTCGFASNKTI ECACRDNSYTAKRPFVKLNVETDTAEIRLMCTETYLDTPRPDDGSKTGPCESNGDKGSGGIKGGFVHQRMASKIGRWYSRTM SKTERMGMELYVKYDGDPWTDSDALAPSGVMVSMEEPGWYSFGFEIKDKKCDVPCIGIEMVHDGGKNTWHSAATAIYCLM GSGQLLWDTVTGVDMAL |
| 121 | LYSDILLKFSPTKVIAPTVSLDCANASNVQAVNHSATKEMTFLLPEPEWTYPRLSCQGSTFQKALLISPHRFGEAKGNSAPLIIRE PFIACGPKECKHFALTHYAAKPGGYYNGTREDRNKLRHLISVNLGKIPTVENSIFHMAAWSGSACHDGREWTYIGVDGPDSNA LIKIKYGEAYTDTYHSYANNILRTQESACNCIGGDCYLMITDGSASGISRCRFLKIREGRIIKEIFPTGRVEHTEECTCGFASNKTI ECACRDNSYTAKRPFVKLNVETDTAEIRLMCTETYLDTPRPDDGSITGPCESNGDKGNGGIKGGFVHQRMASRIGRWYSRTMS KTKRMGMELYVKYDGDPWTDSDALTPSGVMISMEEPGWYSFGFEIKDKKCDVPCIGIEMVHDGGKKTWHSAATAIYCLMGS GQLLWDTVTGVDMAL |
| 122 | LYSDILLKFSSTEITAPTMPLNCANASNVQAVNHSATKGVTLPLPEPEWTYPRLSCPGSTFQKALLISPHRFGETKGNSAPLIIREP FIACGPKECKHFALTHYAAQPGGYYNGTREDRNKLRHLISVKLGKIPTVENSIFHMAAWSGSACHDGREWTYIGVDGPDSNAL LKIKYGEAYTDTYHSYANNILRTQESACNCIGGDCYLMITDGSASGVSECRFLKIREGRIIKEIFPTGRVEHTEECTCGFASNKTI ECACRDNSYTAKRPFVKLNVETDTAEIRLMCTETYLDTPRPDDGSITGPCESNGDKGSGGIKGGFVHQRMASKIGRWYSRTMS KNKRMGMGLYVKYDGDPWIDSDALTLSGVMVSMEEPGWYSFGFEIKDKKCDVPCIGIEMVHDGGKETWHSAATAIYCLMGS GQLLWDTVTGVNMAL |
| 123 | LYSDILLKFSTTEITAPTMPLDCANASNVQAVNHSAAKGVTLLLPEPEWTYPRLSCPGSTFQKALLISPHRFGETKGNSAPLIIRE PFIACGPKECKHFALTHYAAQPGGYYNGTREDRNKLRHLISVKLGKIPTVENSIFHMAAWSGSACHDGKEWTYIGVDGPDSNA LLKIKYGEAYTDTYHSYAHNILRTQESACNCIGGNCYLMITDGSASGISECRFLKIREGRIIKEIFPTGRVKHTEECTCGFANNKTI ECACRDNSYTAKRPFVKLNVETDTAEIRLMCTETYLDTPRPDDGSITGPCESNGNKGSGGIKGGFVHQRMASKIGRWYSRTMS KTKRMGMGLYVKYDGDPWTDSDALVFSGVMISVEEPGWYSFGFEIKDKKCDVPCIGIEMVHDGGKETWHSAATAIYCLMGS GQLLWDTVTGVNMTL |
| 124 | LYSDILLKFSRTEITAPILRLACANASNVQAVNRSATKGVTLLLPEPEWTYPRLSCPGSTFQKALLISPHRFGETKGNSAPLIIREP FIACGPKECKHFALTHYAAQPGGYYNGTREDRNKLRHLISVKLGKIPTVENSIFHMAAWSGSACHDGREWTYIGVDGPDSNAL LKIKYGGAFTDTYHSYAKNILSPQESACNCIGGDCYLMITDGPASGISECRFLKIREGRIIKEIFPTGRVKHTEECTCGFASNKTIE CACRDNSYTAKRPFVKLNVETDTAEIRLMCTETYLDTPRPNDGSITGPCESNGDKGSGGIKGGFVHQRMASKIGRWYSRTMSK TKRMGMGLYVKYDGDPWTDSEALALSGVMVSMEEPGWYSFGFEIKDKKCDVPCIGIEMVHDGGKTTWHSAATAIYCLMGS GQLLWDTVTGVNMTL |
| 125 | LYSDILLKFSRTEITAPIMPLDCANASNVQAVNRSATKGVTLLLPEPEWTYPRLSCPGSTFQKALLISPHRFGETKGNSAPLIIREP FIACGPKECKHFALTHYAAQPGGYYNGTREDRNKLRHLISVKLGKIPTVENSIFHMAAWSGSACHDGREWTYIGVDGPDSNAL LKIKFGEAYTDTYHSYAKNILGTQESACNCIGGDCNLMITDGPASGISESRFLKIGEGRIIKEIFPTGRVKHTEECTCGFASNKTIE CACRDNSYTAKRPFVKLNVETDTAEIRLMCTETYLDTPRPNDGSITGPCESNGDKGSGGIKGGFVHQRMASKIGRWYSRTMSK TERMGMGLYVKYDGDPWADSDALAFSGVMVSMKEPGWYSFGFEIKDKKCDVPCIGIEMVHDGGKETWHSAATAIYCLMGS GQLLWDTVTGVNMAL |
| 126 | LYSDILLKFSPTEITAPTMPLDCANTSNVQAVNRSATKGVTLLLPEPEWTYPRLSCPGSTFQKALLISPHRFGETKGNSAPLIIREP FIACGPNECKHFALTHYAAQPGGYYNGTRGDRNKLRHLISVKLGKIPTVENSIFHMAAWSGSACHDGKEWTYIGVDGPDNNA LLKITYGEAYPGTYHSYAIKNPRTQESAPNCFGGHSFPIITHAPPSGVWEPRFLKIREGRIINEIFPTGRVKPPEECTCGFASNNTIE CACRDNNCTAKRPFVKFHVESDPAEIRAMCTDTYLDSPRPDDGSITGPCDSNGDKGSGGIKGGFVHQRMESKIGRWYSRTMSK TERMGMGLYVKYDGDPWADSDALANSGVMVSMNEPGSSSFGFEIKDKKCDVPCIGIEMVHDGGKETWHSAATAIYCLMGSG QLLWDTVTGVDMAL |
| 127 | LYSDILLKFSPTEITAPTMPLDCANASNVQAVNRSATKGVTLFLPEPEWTYPRLSCPGSTFQKALLISPHRFGETKGNSAPLIIREP FIACGPTECKHFALTHYAAQPGGYYNGTREDRNKLRHLISVKLGKIPTVENSIFHMAAWSGSACHDGKEWTYIGVDGPDSNAL LKIKYGEAYTDTYHFYAKNILRTQESACSCIGGDCYLMITDGPASGVSECRFLKIREGRIIKEIFPTGRVKHTEECTCGFASNKTIE CACRDNSYTAKRPFVKLNVETDTAEIRLMCTETYLDTPRPNDGSITGPCESDGDKGSGGIKGGFVHQRMASKIGRWYSRTMSK TKRMGMGLYVKYDGDPWTDSEALALSGVMVSMEEPGWYSFGFEIKDKKCDVPCIGIEMVHDGGKTTWHSAATAIYCLMGS GQLLWDTVTGVDMAL |

**Table 8: Amino Acid sequences of hemagglutinin antigens from Sino Biologicals**

| **SEQ ID** | **Catalog #** | **Sequence** |
|---|---|---|
| 128 | 40016-V08B | MKAIIVLLMVVTSNADRICTGITSSNSPHVVKTATQGEVNVTGVIPLTTTPTKSHFANLKGTETRGKLCPKCL NCTDLDVALGRPKCTGKIPSARVSILHEVRPVTSGCFPIMHDRTKIRQLPNLLRGYEHIRLSTHNVINAENAPG GPYKIGTSGSCPNITNGNGFFATMAWAVPKNDKNKTATNPLTIEVPYICTEGEDQITVWGFHSDNETQMAKL YGDSKPQKFTSSANGVTTHYVSQIGGFPNQTEDGGLPQSGRIVVDYMVQKSGKTGTITYQRGILLPQKVWCA SGRSKVIKGSLPLIGEADCLHEKYGGLNKSKPYYTGEHAKAIGNCPIWVKTPLKLANGTKYRPPAKLLKERG FFGAIAGFLEGGWEGMIAGWHGYTSHGAHGVAVAADLKSTQEAINKITKNLNSLSELEVKNLQRLSGAMDE LHNEILELDEKVDDLRADTISSQIELAVLLSNEGIINSEDEHLLALERKLKKMLGPSAVEIGNGCFETKHKCNQ TCLDRIAAGTFDAGEFSLPTFDSLNITAASLNDDGLDNHTILLYYSTAASSLAVTLMIAIFVVYMVSRDNVSCS ICL |
| 129 | 11716-V08B | MKAIIVLLMVVTSNADRICTGITSSNSPHVVKTATQGEVNVTGVIPLTTTPTKSHFANLKGTETRGKLCPKCL NCTDLDVALGRPKCTGNIPSARVSILHEVRPVTSGCFPIMHDRTKIRQLPNLLRGYEHIRLSTHNVINAENAPG GPYKIGTSGSCPNVTNGNGFFATMAWAVPKNDNNKTATNSLTIEVPYICTEGEDQITVWGFHSDNEIQMAKL YGDSKPQKFTSSANGVTTHYVSQIGGFPNQTEDGGLPQSGRIVVDYMVQKSGKTGTITYQRGILLPQKVWCA SGRSKVIKGSLPLIGEADCLHEKYGGLNKSKPYYTGEHAKAIGNCPIWVKTPLKLANGTKYRPPAKLLKERG FFGAIAGFLEGGWEGMIAGWHGYTSHGAHGVAVAADLKSTQEAINKITKNLNSLSELEVKNLQRLSGAMDE LHNEILELDEKVDDLRADTISSQIELAVLLSNEGIINSEDEHLLALERKLKKMLGPSAVEIGNGCFETKHKCNQ TCLDRIAAGTFDAGEFSLPTFDSLNITAASLNDDGLDNHTILLYYSTAASSLAVTLMIAIFVVYMVSRDNVSCS ICL |
| 130 | 40157-V08B | MKAIIVLLMVVTSNADRICTGITSSNSPHVVKTATQGEVNVTGVIPLTTTPTKSHFANLKGTETRGKLCPKCL NCTDLDVALGRPKCTGKIPSARVSILHEVRPVTSGCFPIMHDRTKIRQLPNLLRGYEHIRLSTHNVINAENAPG GPYKIGTSGSCPNITNGNGFFATMAWAVPKNDKNKTATNPLTIEVPYICTEGEDQITVWGFHSDNETQMAKL YGDSKPQKFTSSANGVTTHYVSQIGGFPNQTEDGGLPQSGRIVVDYMVQKSGKTGTITYQRGILLPQKVWCA SGRSKVIKGSLPLIGEADCLHEKYGGLNKSKPYYTGEHAKAIGNCPIWVKTPLKLANGTKYRPPAKLLKERG FFGAIAGFLEGGWEGMIAGWHGYTSHGAHGVAVAADLKSTQEAINKITKNLNSLSELEVKNLQRLSGAMDE LHNEILELDEKVDDLRADTISSQIELAVLLSNEGIINSEDEHLLALERKLKKMLGPSAVEIGNGCFETKHKCNQ TCLDRIAAGTFDAGEFSLPTFDSLNITAASLNDDGLDNHTILLYYSTAASSLAVTLMIAIFVVYMVSRDNVSCS ICL MKAIIVLLMVVTSNADRICTGITSSNSPHVVKTATQGEVNVTGVIPLTTTPTKSHFANLKGTETRGKLCPKCL NCTDLDVALGRPKCTGNIPSARVSILHEVRPVTSGCFPIMHDRTKIRQLPNLLRGYEHIRLSTHNVINAENAPG GPYKIGTSGSCPNVTNGNGFFATMAWAVPKNDNNKTATNSLTIEVPYICTEGEDQITVWGFHSDNEIQMAKL YGDSKPQKFTSSANGVTTHYVSQIGGFPNQTEDGGLPQSGRIVVDYMVQKSGKTGTITYQRGILLPQKVWCA SGRSKVIKGSLPLIGEADCLHEKYGGLNKSKPYYTGEHAKAIGNCPIWVKTPLKLANGTKYRPPAKLLKERG FFGAIAGFLEGGWEGMIAGWHGYTSHGAHGVAVAADLKSTQEAINKITKNLNSLSELEVKNLQRLSGAMDE LHNEILELDEKVDDLRADTISSQIELAVLLSNEGIINSEDEHLLALERKLKKMLGPSAVEIGNGCFETKHKCNQ TCLDRIAAGTFDAGEFSLPTFDSLNITAASLNDDGLDNHTILLYYSTAASSLAVTLMIAIFVVYMVSRDNVSCS ICL MKAIIVLLMVVTSNADRICTGITSSNSPHVVKTATQGEVNVTGVIPLTTTPTKSHFANLKGTKTRGKLCPNCL NCTDLDVALGRPMCMGTIPSAKASILHEVRPVTSGCFPIMHDRTKIRQLPNLLRGYENIRLSTHNVINAERAP GGPYRLGTSGSCPNVTSRNGFFATMAWAVPRDNKTATNPLTVEVPYICTKGEDQITVWGFHSDDKTQMKNL YGDSNPQKFTSSANGVTTHYVSQIGDFPNQTEDGGLPQSGRIVVDYMVQKPGKTGTIVYQRGVLLPQKVWC ASGRSKVIKGSLPLIGEADCLHEKYGGLNKSKPYYTGEHAKAIGNCPIWVKTPLKLANGTKYRPPAKLLKER GFFGAIAGFLEGGWEGMIAGWHGYTSHGAHGVAVAADLKSTQEAINKITKNLNSLSELEVKNLQRLSGAMD ELHNEILELDEKVDDLRADTISSQIELAVLLSNEGIINSEDEHLLALERKLKKMLGPSAVDIGNGCFETKHKCN QTCLDRIAAGTFNAGEFSLPTFDSLNITAASLNDDGLDNHTILLYYSTAASSLAVTLMIAIFIVYMVSRDNVSC SIC MKAIIILLMVVTSNADRICTGITSSNSPHVVKTATQGEVNVTGVIPLTTTPTKSYFANLKGTKTRGKLCPDCLN CTDLDVALGRPMCVGTTPSAKASILHEVRPVTSGCFPIMHDRTKIRQLANLLRGYENIRLSTQNVIDAEKAPG GPYRLGTSGSCPNATSKSGFFATMAWAVPKDNNKNATNPLTVEVPYICAEGEDQITVWGFHSDNKTQMKNL YGDSNPQKFTSSANGVTTHYVSQIGGFPDQTEDGGLPQSGRIVVDYMMQKPGKTGTIVYQRGVLLPQKVWC ASGRSKVIKGSLPLIGEADCLHEKYGGLNKSKPYYTGEHAKAIGNCPIWVKTPLKLANGTKYRPPAKLLKER GFFGAIAGFLEGGWEGMIAGWHGYTSHGAHGVAVAADLKSTQEAINKITKNLNSLSELEVKNLQRLSGAMD ELHNEILELDEKVDDLRADTISSQIELAVLLSNEGIINSEDEHLLALERKLKKMLGPSAVDIGNGCFETKHKCN QTCLDRIAAGTFNAGEFSLPTFDSLNITAASLNDDGLDNHTILLYYSTAASSLAVTLMLAIFIVYMVSRDNVSC SICL MKAIIVLLMVVTSNADRICTGITSSNSPHVVKTATQGEVNVTGVIPLTTTPTKSYFANLKGTRTRGKLCPDCL NCTDLDVALGRPMCVGTTPSAKASILHEVRPVTSGCFPIMHDRTKIRQLPNLLRGYEKIRLSTQNVIDAEKAP GGPYRLGTSGSCPNATSKIGFFATMAWAVPKDNYKNATNPLTVEVPYICTEGEDQITVWGFHSDNKTQMKS LYGDSNPQKFTSSANGVTTHYVSQIGDFPDQTEDGGLPQSGRIVVDYMMQKPGKTGTIVYQRGVLLPQKVW CASGRSKVIKGSLPLIGEADCLHEEYGGLNKSKPYYTGKHAKAIGNCPIWVKTPLKLANGTKYRPPAKLLKE RGFFGAIAGFLEGGWEGMIAGWHGYTSHGAHGVAVAADLKSTQEAINKITKNLNSLSELEVKNLQRLSGAM DELHNEILELDEKVDDLRADTISSQIELAVLLSNEGIINSEDEHLLALERKLKKMLGPSAVDIGNGCFETKHKC NQTCLDRIAAGTFNAGEFSLPTFDSLNITAASLNDDGLDNHTILLYYSTAASSLAVTLMLAIFIVYMVSRDNVS CSICL |
| 131 | 40498-V08B | MKAIIILLMVVTSNADRICTGITSSNSPHVVKTATQGEVNVTGVIPLTTTPTKSYFANLKGTKTRGKLCPDCLN CTDLDVALGRPMCVGTTPSAKASILHEVRPVTSGCFPIMHDRTKIRQLANLLRGYENIRLSTQNVIDAEKAPG GPYRLGTSGSCPNATSKSGFFATMAWAVPKDNNKNATNPLTVEVPYICAEGEDQITVWGFHSDNKTQMKNL YGDSNPQKFTSSANGVTTHYVSQIGGFPDQTEDGGLPQSGRIVVDYMMQKPGKTGTIVYQRGVLLPQKVWC ASGRSKVIKGSLPLIGEADCLHEKYGGLNKSKPYYTGEHAKAIGNCPIWVKTPLKLANGTKYRPPAKLLKER GFFGAIAGFLEGGWEGMIAGWHGYTSHGAHGVAVAADLKSTQEAINKITKNLNSLSELEVKNLQRLSGAMD ELHNEILELDEKVDDLRADTISSQIELAVLLSNEGIINSEDEHLLALERKLKKMLGPSAVDIGNGCFETKHKCN QTCLDRIAAGTFNAGEFSLPTFDSLNITAASLNDDGLDNHTILLYYSTAASSLAVTLMLAIFIVYMVSRDNVSC SICL |
| 132 | 40463-V08B | MKAIIVLLMVVTSNADRICTGITSSNSPHVVKTATQGEVNVTGVIPLTTTPTKSYFANLKGTRTRGKLCPDCL NCTDLDVALGRPMCVGTTPSAKASILHEVRPVTSGCFPIMHDRTKIRQLPNLLRGYEKIRLSTQNVIDAEKAP GGPYRLGTSGSCPNATSKIGFFATMAWAVPKDNYKNATNPLTVEVPYICTEGEDQITVWGFHSDNKTQMKS LYGDSNPQKFTSSANGVTTHYVSQIGDFPDQTEDGGLPQSGRIVVDYMMQKPGKTGTIVYQRGVLLPQKVW CASGRSKVIKGSLPLIGEADCLHEEYGGLNKSKPYYTGKHAKAIGNCPIWVKTPLKLANGTKYRPPAKLLKE RGFFGAIAGFLEGGWEGMIAGWHGYTSHGAHGVAVAADLKSTQEAINKITKNLNSLSELEVKNLQRLSGAM DELHNEILELDEKVDDLRADTISSQIELAVLLSNEGIINSEDEHLLALERKLKKMLGPSAVDIGNGCFETKHKC NQTCLDRIAAGTFNAGEFSLPTFDSLNITAASLNDDGLDNHTILLYYSTAASSLAVTLMLAIFIVYMVSRDNVS CSICL |
| 133 | 40323-V08B | MELIVLLILLNPYTFVLGDRICIGYQANQNNQTVNTLLEQNVPVTGAQEILETNHNGKLCSLNGVPPLDLQSC TLAGWLLGNPNCDSLLEAEEWSYIKINESAPDDLCFPGNFENLQDLLLEMSGVQNFTKVKLFNPQSMTGVTT NNVDQTCPFEGKPSFYRNLNWIQGNSGLPFNIEIKNPTSNPLLLLWGIHNTKDAAQQRNLYGNDYSYTIFNFG EKSEEFRPEIGQRDEVKAHQDRIDYYWGSLPAQSTLRIESTGNLIAPEYGFYYKRKEGKGGLMKSKLPISDCS TKCQTPLGALNSTLPFQNVHQQTIGNCPKYVKATSLMLATGLRNNPQMEGRGLFGAIAGFIEGGWQGMIDG WYGYHHENQEGSGYAADKEATQKAVDAITNKVNSIIDKMNSQFESNIKEFNRLELRIQHLSDRVDDALLDIW SYNTELLVLLENERTLDFHDANVKNLFEKVKAQLKDNAIDEGNGCFLLLHKCNNSCMDDIKNGTYKYMDY REESHIEKQKIDGVKLTDYSRYYIMTLYSTIASSVVLGSLIIAAFLWGCQKGSIQCKICI |
| 134 | 40324-V08B | MITILILVLPIVVGDQICIGYHSNNSTQTVNTLLESNVPVTSSHSILEKEHNGLLCKLKGKAPLDLIDCSLPAWL MGNPKCDELLTASEWAYIKEDPEPENGICFPGDFDSLEDLILLVSNTDHFRKEKIIDMTRFSDVTTNNVDSACP YDTNGASFYRNLNWVQQNKGKQLIFHYQNSENNPLLIIWGVHQTSNAAEQNTYYGSQTGSTTITIGEETNTY PLVISESSILNGHSDRINYFWGVVNPNQNFSIVSTGNFIWPEYGYFFQKTTNISGIIKSSEKISDCDTICQTKIGAI NSTLPFQNIHQNAIGDCPKYVKAQELVLATGLRNNPIKETRGLFGAIAGFIEGGWQGLIDGWYGYHHQNSEG SGYAADKEATQKAVDAITTKVNNIIDKMNTQFESTAKEFNKIEMRIKHLSDRVDDGFLDVWSYNAELLVLLE NERTLDFHDANVNNLYQKVKVQLKDNAIDMGNGCFKILHKCNNTCMDDIKNGTYNYYEYRKESHLEKQKI DGVKLSENSSYKIMIIYSTVASSVVLGLIILAAIEWGCFKGNLQCRICI |
| 135 | 11085-V08B | MKAILVVLLYTFATANADTLCIGYHANNSTDTVDTVLEKNVTVTHSVNLLEDKHNGKLCKLRGVAPLHLGK CNIAGWILGNPECESLSTASSWSYIVETPSSDNGTCYPGDFIDYEELREQLSSVSSFERFEIFPKTSSWPNHDSN KGVTAACPHAGAKSFYKNLIWLVKKGNSYPKLSKSYINDKGKEVLVLWGIHHPSTSADQQSLYQNADAYVF VGSSRYSKKFKPEIAIRPKVRDREGRMNYYWTLVEPGDKITFEATGNLVVPRYAFAMERNAGSGIIISDTPVH DCNTTCQTPKGAINTSLPFQNIHPITIGKCPKYVKSTKLRLATGLRNIPSIQSRGLFGAIAGFIEGGWTGMVDG WYGYHHQNEQGSGYAADLKSTQNAIDEITNKVNSVIEKMNTQFTAVGKEFNHLEKRIENLNKKVDDGFLDI WTYNAELLVLLENERTLDYHDSNVKNLYEKVRSQLKNNAKEIGNGCFEFYHKCDNTCMESVKNGTYDYPK YSEEAKLNREEIDGVKLESTRIYQILAIYSTVASSLVLVVSLGAISFWMCSNGSLQCRICI |
| 136 | 40132-V08B | MKAKLLVLLCAFTATYADTICIGYHANNSTDTVDTVLEKNVTVTHSVNLLEDSHNGKLCRLKGIAPLQLGNC SVAGWILGNPKCESLFSKESWSYIAETPNPENGTCYPGYFADYEELREQLSSVSSFERFEIFPKESSWPNHTVT KGVTTSCSHNGKSSFYRNLLWLTEKNGLYPNLSKSYVNNKEKEVLVLWGVHHPSNIRDQRAIYHTENAYVS VVSSHYSRRFTPEIAKRPKVRGQEGRINYYWTLLEPGDTIIFEANGNLIAPWYAFALSRGFGSGIITSNASMDE CDAKCQTPQGAINSSLPFQNVHPVTIGECPKYVRSTKLRMVTGLRNIPSIQSRGLFGAIAGFIEGGWTGMIDG WYGYHHQNEQGSGYAADQKSTQNAINGITNKVNSVIEKMNTQFTAVGKEFNKLERRMENLNKKVDDGFLD IWTYNAELLVLLENGRTLDFHDSNVKNLYEKVKSQLKNNAKEIGNGCFEFYHKCNNECMESVKNGTYDYP KYSEESKLNRGKIDGVKLESMGVYQILAIYSTVASSLVLLVSLGAISFWMCSNGSLQCRICI |
| 137 | 40392-V08B | MKAILLVLLCTFAATNADTLCIGYHANNSTDTVDTVLEKNVTVTHSVNLLEDRHNGKLCKLGGIAPLHLGK CNIAGWLLGNPECELLLTVSSWSYIVETSNSDNGTCYPGDFINYEELREQLSSVSSFERFEIFPKTSSWPNHETN RGVTAACPYAGANSFYRNLIWLVEKGNSYPKLSKSYVNNKGKEVLVLWGIHHPPTSTDQQSLYQNADAYV FVGSSKYNRKFKPEIAARPKVRGQAGRMNYYWTLIEPGDTITFEATGNLVVPRYAFAMNRGSGSGIIISDAPV HDCNTKCQTPKGAINTSLPFQNIHPVTIGECPKYVKSTKLRMATGLRNIPSIQSRGLFGAIAGFIEGGWTGMID GWYGYHHQNEQGSGYAADQRSTQNAIDGITNKVNSVIEKMNTQFTAVGKEFNHLEKRIENLNKKVDDGFL DIWTYNAELLVLLENERTLDFHDSNVKNLYEKVRSQLRNNAKEIGNGCFEFYHKCDDTCMESVKNGTYDYP KYSEESKLNREEIDGVKLESTRIYQILAIYSTVASSLVLLVSLGAISFWMCSNGSLQCRICI |
| 138 | 40090-V08B | MKATILVVLCIFTASNEDTICIGYHANNSTDTVDTVLEKNVTVTHSVNLLEDSHNGKLCKLKGIAPLQLGKCN IAGWLLGNPECDLLLTASSWSYIVETSNSENGTCYPGDFIDYEELREQLSSVSSFEKFEIFPKTSSWPNHETTKG VTAACSYAGASSFYRNLLWLTKKGSSYPKLSKSYVNNKGKEVLVLWGVHHPPTGTDQQSLYQNADAYVSV GSSKYNRRFTPEIAARPKVRDQAGRMNYYWTLLEPGDTITFEATGNLIAPWYAFALNRGSGSGIITSDAPVHD CNTKCQTPHGAINSSLPFQNIHPVTIGECPKYVRSTKLRMATGLRNIPSIQSRGLFGAIAGFIEGGWTGMIDGW YGYHHQNEQGSGYAADRKSTQNAIDGITNKVNSVIEKMNTQFTSVGKEFNHLEKRIENLNRKVDDGFLDVW TYNAELLVLLENERTLDYHDSNVKNLYEKVRSQLKNNAKEIGNGCFEFYHKCDDSCMESVKNGTYDYPKYS EESKLNREEIDGV |
| 139 | 11684-V08B | MKANLLVLLCALAAADADTICIGYHANNSTDTVDTVLEKNVTVTHSVNLLEDSHNGKLCRLKGIAPLQLGK CNIAGWLLGNPECDPLLPVRSWSYIVETPNSENGICYPGDFIDYEELREQLSSVSSFERFEIFPKESSWPNHNTN GVTAACSHEGKSSFYRNLLWLTEKEGSYPKLKNSYVNKKGKEVLVLWGIHHPPNSKEQQNLYQNENAYVS VVTSNYNRRFTPEIAERPKVRDQAGRMNYYWTLLKPGDTIIFEANGNLIAPMYAFALSRGFGSGIITSNASMH ECNTKCQTPLGAINSSLPYQNIHPVTIGECPKYVRSAKLRMVTGLRNIPSIQSRGLFGAIAGFIEGGWTGMIDG WYGYHHQNEQGSGYAADQKSTQNAINGITNKVNTVIEKMNIQFTAVGKEFNKLEKRMENLNKKVDDGFLD IWTYNAELLVLLENERTLDFHDSNVKNLYEKVKSQLKNNAKEIGNGCFEFYHKCDNECMESVRNGTYDYPK YSEESKLNREKVDGVKLESMGIYQILAIYSTVASSLVLLVSLGAISFWMCSNGSLQCRICI |
| 140 | 40133-V08B | MKAKLLVLLCTFTATYADTICIGYHANNSTDTVDTVLEKNVTVTHSVNLLEDSHNGKLCLLKGIAPLQLGNC SVAGWILGNPECESLISKESWSYIVETPNPENGTCYPGYFADYEELREQLSSVSSFERFEIFPKESSWPNHTVTG VTASCSHNGKSSFYRNLLWLTEKNGLYPNLSNSYVNNKEKEVLVLWGVHHPSNIRDQRAIYHTENAYVSVV SSHYSRRFTPEIAKRPKVRGQEGRINYYWTLLEPGDTIIFEANGNLIAPWYAFALSRGFGSGIITSNAPMNECD AKCQTPQGAINSSLPFQNVHPVTIGECPKYVRSTKLRMVTGLRNIPSIQSRGLFGAIAGFIEGGWTGMMDGW YGYHHQNEQGSGYAADQKSTQNAINGITNKVNSVIEKMNTQFTAVGKEFNKLERRMENLNKKVDDGFLDI WTYNAELLVLLENERTLDFHDSNVKNLYEKVKSQLKNNAKEIGNGCFEFYHKCNNECMESVKNGTYDYPK YSEESKLNREKIDGVKLESMGVYQILAIYSTVASSLVLLVSLGAISFWMCSNGSLQCRICI |
| 141 | 11708-V08B | MKVKLLVLLCTFTATYADTICIGYHANNSTDTVDTVLEKNVTVTHSVNLLEDSHNGKLCLLKGIAPLQLGNC SVAGWILGNPECELLISRESWSYIVEKPNPENGTCYPGHFADYEELREQLSSVSSFERFEIFPKESSWPNHTTTG VSASCSHNGESSFYKNLLWLTGKNGLYPNLSKSYANNKEKEVLVLWGVHHPPNIGDQRALYHKENAYVSV VSSHYSRKFTPEIAKRPKVRDQEGRINYYWTLLEPGDTIIFEANGNLIAPRYAFALSRGFGSGIINSNAPMDEC DAKCQTPQGAINSSLPFQNVHPVTIGECPKYVRSAKLRMVTGLRNIPSIQSRGLFGAIAGFIEGGWTGMVDG WYGYHHQNEQGSGYAADQKSTQNAINGITNKVNSVIEKMNTQFTAVGKEFNKLERRMENLNKKVDDGFIDI WTYNAELLVLLENERTLDFHDSNVKNLYEKVKSQLKNNAKEIGNGCFEFYHKCNDECMESVKNGTYDYPK YSEESKLNREKIDGVKLESMGVYQILAIYSTVASSLVLLVSLGAISFWMCSNGSLQCRICI |
| 142 | 11692-V08H | MKAKLLVLLYAFVATDADTICIGYHANNSTDTVDTIFEKNVAVTHSVNLLEDRHNGKLCKLKGIAPLQLGKC NIIGWLLGNPECDSLLPARSWSYIVETPNSENGACYPGDFIDYEELREQLSSVSSLERFEIFPKESSWPNHTFNG VTASCSHRGKSSFYRNLLWLTKKGDSYPKLTNSYVNNKGKEVLVLWGVHHPSSSDEQQSLYSNGNAYVSV ASSNYNRRFTPEIAARPKVKDQHGRMNYYWTLLEPGDTIIFEATGNLIAPWYAFALSRGFESGIITSNASMHE CNTKCQTPQGSINSNLPFQNIHPVTIGECPKYVRSTKLRMVTGLRNIPSIQYRGLFGAIAGFIEGGWTGMIDGW YGYHHQNEQGSGYAADQKSTQNAINGITNKVNSIIEKMNTQFTAVGKEFNNLEKRMENLNKKVDDGFLDIW TYNAELLVLLENGRTLDFHDLNVKNLYEKVKSQLKNNAKEIGNGCFEFYHKCDNECMESVRNGTYDYPKY SEESKLNREKIDGVKLESMGVYQILAIYSTVASSLVLLVSLGAISFWMCSNGSLQCRICI |
| 143 | 11052-V08H | MKVKLLVLLCTFTATYADTICIGYHANNSTDTVDTVLEKNVTVTHSVNLLENSHNGKLCLLKGIAPLQLGNC SVAGWILGNPECELLISKESWSYIVEKPNPENGTCYPGHFADYEELREQLSSVSSFERFEIFPKESSWPNHTVT GVSASCSHNGESSFYRNLLWLTGKNGLYPNLSKSYANNKEKEVLVLWGVHHPPNIGDQKALYHTENAYVS VVSSHYSRKFTPEIAKRPKVRDQEGRINYYWTLLEPGDTIIFEANGNLIAPRYAFALSRGFGSGIINSNAPMDK CDAKCQTPQGAINSSLPFQNVHPVTIGECPKYVRSAKLRMVTGLRNIPSIQSRGLFGAIAGFIEGGWTGMVDG WYGYHHQNEQGSGYAADQKSTQNAINGITNKVNSVIEKMNTQFTAVGKEFNKLERRMENLNKKVDDGFIDI WTYNAELLVLLENERTLDFHDSNVKNLYEKVKSQLKNNAKEIGNGCFEFYHKCNDECMESVKNGTYDYPK YSEESKLNREKIDGVKLESMGVYQILAIYSTVASSLVLLVSLGAISFWMCSNGSLQCRICI |
| 144 | 40393-V08B | MEAKLFVLFCVFNALKADTICVGYHANNSTDTVDTILEKNVTVTHSVNLLENSHNGKLCSLNGKAPLQLGN CNVAGWILGNPECDLLLTANSWSYIIETSNSKNGKCYPGEFADYEELREQLSTVSSFERFEIFPKATSWPNHET TKGTTTACSHSGASSFYRNLLWIVKKGNSYPKLSKSYTNNKGKEVLVIWGVHHPPTNSDQQTLYQNAYTYV SVESSKYYRRFTPEIAARPKVRGQAGRMNYYWTLLDQGDTITFEATGNLIAPWYAFALNKGSNSGIMMSDA HVHNCTTKCQTPHGALKSNLPFQNVHPITIGECPKYVKSTQLRMATGLRNVPSIQSRGLFGAIAGFIEGGWTG MIDGWYGYHHQNEQGSGYAADQKSTQIAIDGISNKVNSVIEKMNIQFTSVGKEFNNLEKRIENLNKKVDDGF LDIWTYNAELLILLENERTLDFHDFNVKNLYEKVKSQLRNNAKEVGNGCFEFYHKCDNECMESVKNGTYNY PKYSEESKLNREEIDGVKLESMGVHQILAIYSTVASSLVLLVSLGAISFWMCSNGSLQCRICI |
| 145 | 40134-V08B | MKAKLLVLLCALSATDADTICIGYHANNSTDTVDTVLEKNVTVTHSVNLLEDSHNGKLCRLKGIAPLQLGKC NIAGWILGNPECESLFSKKSWSYIAETPNSENGTCYPGYFADYEELREQLSSVSSFERFEIFPKERSWPKHNVT RGVTASCSHKGKSSFYRNLLWLTEKNGSYPNLSKSYVNNKEKEVLVLWGVHHPSNIEDQKTIYRKENAYVS VVSSNYNRRFTPEIAERPKVRGQAGRINYYWTLLEPGDTIIFEANGNLIAPWHAFALNRGFGSGIITSNASMDE CDTKCQTPQGAINSSLPFQNIHPVTIGECPKYVRSTKLRMVTGLRNIPSIQSRGLFGAIAGFIEGGWTGMIDGW YGYHHQNEQGSGYAADQKSTQNAINGITNKVNSVIEKMNTQFTAVGKEFNKLEKRMENLNKKVDDGFLDI WTYNAELLVLLENERTLDFHDSNVKNLYEKVKSQLKNNAKEIGNGCFEFYHKCNNECMESVKNGTYDYPK YSEESKLNREKIDGVKLESMGVYQILAIYSTVASSLVLLVSLGAISFWMCSNGSLQCRICI |
| 146 | 40131-V08B | MKAKLLVLLCAFTATDADTICIGYHANNSTDTVDTVLEKNVTVTHSVNLLEDSHNGKLCRLKGIAPLQLGNC SIAGWILGNPECESLFSKKSWSYIAETPNSENGTCYPGYFADYEELREQLSSVSSFERFEIFPKESSWPNHTVTK GVTASCSHKGKSSFYRNLLWLTEKNGSYPTLSKSYVNNKEKEVLVLWGVHHPSNIGDQRAIYHTENAYVSV VSSHYNRRFTPEIAKRPKVRGQEGRINYYWTLLEPGDTIIFEANGNLIAPWYAFALSRGFGSGIITSNASMDEC DAKCQTPQGAINSSLPFQNVHPVTIGECPKYVRSTKLRMVTGLRNIPSIQSRGLFGAIAGFIEGGWTGMIDGW YGYHHQNEQGSGYAADQKSTQNAINGITNKVNSVIEKMNTQFTAVGKEFNKLERRMENLNKKVDDGFLDI WTYNAELLVLLENERTLDFHDSNVKNLYEKVKSQLKNNAKEIGNGCFEFYHKCNNECMESVKNGTYDYPK YSEESKLNREKIDGVKLESMGVYQILAIYSTVASSLVLLVSLGAISFWMCSNGSLQCRICI |
| 147 | 11685-V08H | MEARLFVLFCTFTVLKADIICVGYHANNSTDTVDTVLEKNVTVTHSVNLLESSHNGKLCSLNGIPPLQLGKCN VAGWLLGNPECDQLLTANSWSYIIETSNSENGTCYPGEFIDYEELREQLSSVSSFEKFEIFPKASSWPNHETTK GVTAACPYLGNSNFYRNLLWITKKGNSYPELSKSYTNNKGKEVLILWGVHHPPSTNEQQNLYQNANAYVSV GSSKYNRRFTPEIAARPKVRGQAGRMNYYWTLLDQGDTITFEATGNLIAPWYAFALNKNSDSGIMMSDAPV HNCDTKCQTPHGALNSSLPFQNVHPITIGECPKYIKSTKLRMATGLRNVPSIQSRGLFGAIAGFIEGGWTGMID GWYGYHHQNEQGSGYAADQKSTQNAIDGITNKVNSVIEKMNTQFTAVGKEFNNLEKRIENLNKKVDDGFL DVWTYNAELLVLLENERTLDFHDSNVRNLYEKVKSQLRNNAKEIGNGCFEFYHKCDDECMESVKNGTYDY PKYSEESKLNREEIDGVKLESMGVYQILAIYSTVASSLVLLVSLGAISFWMCSNGSLQCRVCI |
| 148 | 11688-V08H | MAIIYLILLFTAVRGDQICIGYHANNSTEKVDTILERNVTVTHAKDILEKTHNGKLCKLNGIPPLELGDCSIAG WLLGNPECDRLLSVPEWSYIMEKENPRDGLCYPGSFNDYEELKHLLSSVKHFEKVKILPKDRWTQHTTTGGS RACAVSGNPSFFRNMVWLTKKGSNYPVAQGSYNNTSGEQMLIIWGVHHPNDETEQRTLYQNVGTYVSVGT STLNKRSTPEIATRPKVNGQGGRMEFSWTLLDMWDTINFESTGNLIAPEYGFKISKRGSSGIMKTEGTLENCE TKCQTPLGAINTTLPFHNVHPLTIGECPKYVKSEKLVLATGLRNVPQIESRGLFGAIAGFIEGGWQGMVDGW YGYHHSNDQGSGYAADKESTQKAFDGITNKVNSVIEKMNTQFEAVGKEFSNLERRLENLNKKMEDGFLDV WTYNAELLVLMENERTLDFHDSNVKNLYDKVRMQLRDNVKELGNGCFEFYHKCDDECMNSVKNGTYDYP KYEEESKLNRNEIKGVKLSSMGVYQILAIYATVAGSLSLAIMMAGISFWMCSNGSLQCRICI |
| 149 | 40119-V08B | MKTIIALSYILCLVFAQKLPGNDNSTATLCLGHHAVPNGTIVKTITNDQIEVTNATELVQSSSTGGICDSPHQIL DGENCTLIDALLGDPQCDGFQNKKWDLFVERSKAYSNCYPYDVPDYASLRSLVASSGTLEFNNESFNWAGV TQNGTSSACKRRSNKSFFSRLNWLTHLKYKYPALNVTMPNNEKFDKLYIWGVHHPGTDSDQISLYAQASGRI TVSTKRSQQTVIPNIGSRPRVRDVSSRISIYWTIVKPGDILLINSTGNLIAPRGYFKIRSGKSSIMRSDAPIGKCNS ECITPNGSIPNDKPFQNVNRITYGACPRYVKQNTLKLATGMRNVPEKQTRGIFGAIAGFIENGWEGMVDGWY GFRHQNSEGTGQAADLKSTQAAINQINGKLNRLIGKTNEKFHQIEKEFSEVEGRIQDLEKYVEDTKIDLWSYN AELLVALENQHTIDLTDSEMNKLFERTKKQLRENAEDMGNGCFKIYHKCDNACIESIRNGTYDHDVYRDEA LNNRFQIKGVELKSGYKDWILWISFAISCFLLCVALLGFIMWACQKGNIRCNICI |
| 150 | 11715-V08B | MKTIIALSYILCLVFAQKLPGNDNSTATLCLGHHAVPNGTIVKTITNDQIEVTNATELVQSSSTGGICDSPHQIL DGENCTLIDALLGDPQCDGFQNKKWDLFVERSKAYSNCYPYDVPDYASLRSLVASSGTLEFNNESFNWAGV TQNGTSSACKRRSNKSFFSRLNWLTHLKYKYPALNVTMPNNEKFDKLYIWGVHHPGTDSDQISLYAQASGRI TVSTKRSQQTVIPNIGSRPRVRDVSSRISIYWTIVKPGDILLINSTGNLIAPRGYFKIRSGKSSIMRSDAPIGKCNS ECITPNGSIPNDKPFQNVNRITYGACPRYVKQNTLKLATGMRNVPEKQTRGIFGAIAGFIENGWEGMVDGWY GFRHQNSEGTGQAADLKSTQAAINQINGKLNRLIGKTNEKFHQIEKEFSEVEGRIQDLEKYVEDTKIDLWSYN AELLVALENQHTIDLTDSEMNKLFERTKKQLRENAEDMGNGCFKIYHKCDNACIESIRNGTYDHDVYRDEA LNNRFQIKGVELKSGYKDWILWISFAISCFLLCVALLGFIMWACQKGNIRCNICI |
| 151 | 40120-V08B | MKTIIALSYILCLVFAQKLPGNDNSTATLCLGHHAVPNGTIVKTITNDQIEVTNATELVQSSSTGGICDSPHQIL DGENCTLIDALLGDPQCDGFQNKKWDLFVERSKAYSNCYPYDVPDYASLRSLVASSGTLEFNNESFNWTGV TQNGTSSACKRRSNKSFFSRLNWLTHLKYKYPALNVTMPNNEKFDKLYIWGVLHPGTDSDQIRLYAQASGRI TVSTKRSQQTVIPNIGSRPRVRDVSSRISIYWTIVKPGDILLINSTGNLIAPRGYFKIRSGKSSIMRSDAPIGKCNS ECITPNGSIPNDKPFQNVNRITYGACPRYIKQNTLKLATGMRNVPEKQTRGIFGAIAGFIENGWEGMVDGWY GFRHQNSEGTGQAADLKSTQAAINQINGKLNRLIGKTNEKFHQIEKEFSEVEGRIQDLEKYVEDTKIDLWSYN AELLVALENQYTIDLTDSEMNKLFERTKKQLRENAEDMGNGCFKIYHK |
| 152 | 40145-V08B | MKTIIALSHILCLVFAQKLPGNDNSTATLCLGHHAVPNGTIVKTITNDQIEVTNATELVQNSSIGEICDSPHQIL DGENCTLIDALLGDPQCDGFQNKKWDLFVERSKAYSNCYPYDVPDYASLRSLVASSGTLEFNNESFNWTGV TQNGTSSACIRRSNNSFFSRLNWLTQLNFKYPALNVTMPNNEQFDKLYIWGVHHPVTDKDQIFLYAQSSGRI TVSTKRSQQAVIPNIGYRPRIRNIPSRISIYWTIVKPGDILLINSTGNLIAPRGYFKIRSGKSSIMRSDAPIGKCNSE CITPNGSIPNDKPFQNVNRITYGACPRYVKQSTLKLATGMRNVPEKQTRGIFGAIAGFIENGWEGMVDGWYG FRHQNSEGRGQAADLKSTQAAIDQINGKLNRLIGKTNEKFHQIEKEFSEVEGRIQDLEKYVEDTKIDLWSYNA ELLVALENQHTIDLTDSEMNKLFEKTKKQLRENAEDMGNGCFKIYHKCDNACIGSIRNGTYDHDVYRDEAL NNRFQIKGVELKSGYKDWILWISFAISCFLLCVALLGFIMWACQKGNIRCNICI |
| 153 | 40494-V08B-*50* | MKTIIALSYILCLVFAQKLPENDNSTATLCLGHHAVPNGTIVKTITSDRIEVTNATELVQNSSIGEICDSPHQILD GKNCTLIDALLGDPQCDGFQNKKWDLFVERSKAYSNCYPYDVPDYASLRSLVASSGTLEFNDESFNWAGVT QNGTSSACIRGSNSSFFSRLNWLTHSNFKYPALNVTMPNNEQFDKLYIWGVHHPGTDKDQIFLYAQSSGRITV STKRSQQAVIPNIGSRPRIRNIPSRISIYWTIVKPGDILLINSTGNLIAPRGYFKIRSGKSSIMRSDAPIGKCKSECI TPNGSIPNDKPFQNVNRITYGACPRYVKQSTLKLATGMRNVPEKQTRGIFGAIAGFIENGWEGMKDGWYGF RHQNSEGRGQAADLKSTQAAIDQINGKLNRLIGKTNEKFHQIEKEFSEVEGRIQDLEKYVEDTKIDLWSYNAE LLVALENQHTIDLTDSEMNKLFEKTKKQLRENAEDMGNGCFKIYHKCDNACIGSIRNGTYDHDVYRDEALN NRFQIKGVELKSGYKDWILWISFAISCFLLCVALLGFIMWACQKGNIRCNICI |
| 154 | 40118-V08B | MKTIIALSYILCLVFAQKLPGNDNSTATLCLGHHAVPNGTIVKTITNDQIEVTNATELVQSSSTGGICDSPHQIL DGENCTLIDALLGDPQCDGFQNKKWDLFVERSKAYSNCYPYDVPDYASLRSLVASSGTLEFNNESFNWTGV TQNGTSSSCKRRSNNSFFSRLNWLTHLKFKYPALNVTMPNNEKFDKLYIWGVHHPGTNNDQISLYTQASGRI TVSTKRSQQTVIPNIGSRPRVRDIPSRISIYWTIVKPGDILLINSTGNLIAPRGYFKIRSGKSSIMRSDAPIGKCNS ECITPNGSIPNDKPFQNVNRITYGACPRYVKQNTLKLATGMRNVPEKQTRGIFGAIAGFIENGWEGMVDGWY GFRHQNSEGIGQAADLKSTQAAINQINGKLNRLIGKTNEKFHQIEKEFSEVEGRIQDLEKYVEDTKIDLWSYN AELLVALENQHTIDLTDSEMNKLFERTKKQLRENAEDMGNGCFKIYHKCDNACIGSIRNGTYDHDVYRDEA LNNRFQIKGVELKSGYKDWILWISFAISCFLLCVALLGFIMWACQKGNIRCNICI |
| 155 | 40149-V08B | MKTIIALSYILCLVFAQKIPGNDNSTATLCLGHHAVPNGTLVKTITNDQIEVTNATELVQSSSTGRICDSPHRIL DGENCTLIDALLGDPHCDGFQNKEWDLFVERSKAYSNCYPYDVPDYASLRSLVASSGTLEFNNESFNWTGV AQNGTSYACKRSSIKSFFSRLNWLHQLKYKYPALNVTMPNNDKFDKLYIWGVHHPSTDSDQTSIYAQASGR VTVSTKRSQQTVIPNIGSRPWVRGISSRISIHWTIVKPGDILLINSTGNLIAPRGYFKIRSGKSSIMRSDAPIGKCN SECITPNGSIPNDKPFQNVNRITYGACPRYVKQNTLKLATGMRNVPEKQTRGIFGAIAGFIENGWEGMVDGW YGFRHQNSEGTGQAADLKSTQAAINQINGKLNRLIEKTNEKFHQIEKEFSEVEGRIQDLEKYVEDTKIDLWSY NAELLVALENQHTIDLTDSEMNKLFERTRKQLRENAEDMGNGCFKIYHKCDNACIGSIRNGTYDHDVYRDE ALNNRFQIKGVELKSGYKDWILWISFAISCFLLCVVLLGFIMWACQKGNIRCNICI |
| 156 | 40485-V08B-50 | MKTIIALSYILCLVFAQKLPGNDNSTATLCLGHHAVPNGTLVKTITNDQIEVTNATELVQSSSTGRICDSPHRIL DGKNCTLIDALLGDPHCDGFQNKEWDLFVERSKAYSNCYPYDVPDYASLRSLVASSGTLEFTNEGFNWTGV AQDGTSYACKRGSVKSFFSRLNWLHKLEYKYPALNVTMPNNDKFDKLYIWGVHHPSTDSVQTSLYVQASG RVTVSTKRSQQTVIPNIGSRPWVRGISSRISIYWTIVKPGDILLINSTGNLIAPRGYFKIRSGKSSIMRSDAPIGNC NSECITPNGSIPNDKPFQNVNRITYGACPRYVKQNTLKLATGMRNVPEKQTRGIFGAIAGFIENGWEGMVDG WYGFRHQNSEGTGQAADLKSTQAAINQINGKLNRLIEKTNEKFHQIEKEFSEVEGRIQDLEKYVEDTKIDLWS YNAELLVALENQHTIDLTDSEMNKLFERTRKQLRENAEDMGNGCFKIYHKCDNACIGSIRNGTYDHDVYRD EALNNRFQIKGVELKSGYKDWILWISFAISCFLLCVVLLGFIMWACQKGNIRCNICI |
| 157 | 40146-V08B-50 | MKTIIALSYILCLVFAQKLPGNDNSTATLCLGHHAVPNGTIVKTITNDQIEVTNATELVQNSSIGEICDSPHQIL DGENCTLIDALLGDPQCDGFQNKKWDLFVERSKAYSNCYPYDVPDYASLRSLVASSGTLEFNNESFNWTGV TQNGTSSACIRRSNNSFFSRLNWLTHLNFKYPALNVTMPNNEQFDKLYIWGVHHPGTDKDQIFLYAQSSGRI TVSTKRSQQAVIPNIGSRPRIRNIPSRISIYWTIVKPGDILLINSTGNLIAPRGYFKIRSGKSSIMRSDAPISKCKSE CITPNGSIPNDKPFQNVNRITYGACPRYVKQSTLKLATGMRNVPEKQTRGIFGAIAGFIENGWEGMVDGWYG FRHQNSEGRGQAADLKSTQAAIDQINGKLNRLIGKTNEKFHQIEKEFSEVEGRIQDLEKYVEDTKIDLWSYNA ELLVALENQHTIDLTDSEMNKLFEKTKKQLRENAEDMGNGCFKIYHKCDNACIGSIRNGTYDHDVYRDEAL NNRFQIKGVELKSGYKDWILWISFAISCFLLCVALLGFIMWACQKGNIRCNICI |
| 158 | 40043-V08H | MKTIIALSYILCLVFAQKLPGNDNSTATLCLGHHAVPNGTIVKTITNDQIEVTNATELVQSSSTGEICDSPHQIL DGKNCTLIDALLGDPQCDGFQNKKWDLFVERSKAYSNCYPYDVPDYASLRSLVASSGTLEFNNESFNWTGV TQNGTSSACIRRSKNSFFSRLNWLTHLNFKYPALNVTMPNNEQFDKLYIWGVHHPGTDKDQIFLYAQASGRI TVSTKRSQQTVSPNIGSRPRVRNIPSRISIYWTIVKPGDILLINSTGNLIAPRGYFKIRSGKSSIMRSDAPIGKCNS ECITPNGSIPNDKPFQNVNRITYGACPRYVKQNTLKLATGMRNVPEKQTRGIFGAIAGFIENGWEGMVDGWY GFRHQNSEGRGQAADLKSTQAAIDQINGKLNRLIGKTNEKFHQIEKEFSEVEGRIQDLEKYVEDTKIDLWSYN AELLVALENQHTIDLTDSEMNKLFEKTKKQLRENAEDMGNGCFKIYHKCDNACIGSIRNGTYDHDVYRDEA LNNRFQIKGVELKSGYKDWILWISFAISCFLLCVALLGFIMWACQKGNIRCNICI |
| 159 | 11056-V08H | MKTIIALSYILCLVFTQKLPGNDNSTATLCLGHHAVPNGTIVKTITNDQIEVTNATELVQSSSTGEICDSPHQIL DGENCTLIDALLGDPQCDGFQNKKWDLFVERSKAYSNCYPYDVPDYASLRSLVASSGTLEFNNESFNWTGV TQNGTSSACIRRSNNSFFSRLNWLTHLKFKYPALNVTMPNNEKFDKLYIWGVHHPGTDNDQIFPYAQASGRI TVSTKRSQQTVIPNIGSRPRVRNIPSRISIYWTIVKPGDILLINSTGNLIAPRGYFKIRSGKSSIMRSDAPIGKCNS ECITPNGSIPNDKPFQNVNRITYGACPRYVKQNTLKLATGMRNVPEKQTRGIFGAIAGFIENGWEGMVDGWY GFRHQNSEGIGQAADLKSTQAAIDQINGKLNRLIGKTNEKFHQIEKEFSEVEGRIQDLEKYVEDTKIDLWSYN AELLVALENQHTIDLTDSEMNKLFEKTKKQLRENAEDMGNGCFKIYHKCDNACIGSIRNGTYDHDVYRDEA LNNRFQIKGVELKSGYKDWILWISFAISCFLLCVALLGFIMWACQKGNIRCNICI |
| 160 | 40116-V08B | MKTIIALSYIFCLALGQDLPGNDNSTATLCLGHHAVPNGTLVKTITDDQIEVTNATELVQSSSTGKICNNPHRI LDGIDCTLIDALLGDPHCDVFQNETWDLFVERSKAFSNCYPYDVPDYASLRSLVASSGTLEFITEGFTWTGVT QNGGSNACKRGPGSGFFSRLNWLTKSGSTYPVLNVTMPNNDNFDKLYIWGVHHPSTNQEQTSLYVQASGR VTVSTRRSQQTIIPNIGSRPWVRGLSSRISIYWTIVKPGDVLVINSNGNLIAPRGYFKMRTGKSSIMRSDAPIDT CISECITPNGSIPNDKPFQNVNKITYGACPKYVKQNTLKLATGMRNVPEKQTRGLFGAIAGFIENGWEGMIDG WYGFRHQNSEGTGQAADLKSTQAAIDQINGKLNRVIEKTNEKFHQIEKEFSEVEGRIQDLEKYVEDTKIDLW SYNAELLVALENQHTIDLTDSEMNKLFEKTRRQLRENAEDMGNGCFKIYHKCDNACIESIRNGTYDHDVYR DEALNNRFQIKGVELKSGYKDWILWISFAISCFLLCVVLLGFIMWACQRGNIRCNICI |
| 161 | 40154-V08B | MKTIIALSYILCLVFAQKLPGNDNSTATLCLGHHAVPNGTLVKTITNDQIEVTNATELVQSSSTGRICDSPHQIL DGENCTLIDALLGDPHCDGFQNKEWDLFVERSKAYSNCYPYDVPDYASLRSLVASSGTLEFNNESFNWTGV AQNGTSSACKRRSIKSFFSRLNWLHQLENRYPALNVTMPNNDKFDKLYIWGVHHPSTDSVQTSVYVQASGR VTVSTKRSQQTVIPNIGSRPWVRGVSSRISIYWTIVKPGDILLINSTGNLIAPRGYFKIRSGKSSIMRSDAPIGKC NSECITPNGSIPNDKPFQNVNRITYGACPRYVKQNTLKLATGMRNVPEKQTRGIFGAIAGFIENGWEGMMDG WYGFRHQNSEGTGQAADLKSTQAAINQINGKLNRLIEKTNEKFHQIEKEFSEVEGRIQDLEKYVEDTKIDLWS YNAELLVALENQHTIDLTDSEMNKLFERTRKQLRENAEDMGNGCFKIYHKCDNACIGSIRNGTYDHDVYRD EALNNRFQIKGVELKSGYKDWILWISFAISCFLLCVVLLGFIMWACQKGNIRCNICI |
| 162 | 11702-V08H | MEKIVLLLAIVSLVKSDQICIGYHANNSTEQVDTIMERNVTVTHAQDILEKTHNGKLCNLDGVKPLILRDCSV AGWLLGNPMCDEFLNVPEWSYIVEKINPANDLCYPGNFNDYEELKHLLSRINHFEKIQIIPKNSWSDHEASGV SSACPYQGRSSFFRNVVWLTQKDNAYPTIKRSYNNTNQEDLLVLWGIHHPNDAAEQTRLYQNPTTYISVGTS TLNQRLIPKIATRSKVNGQSGRMEFFWTILKSNDAINFESNGNFIAPENAYKIVKKGDSTIMKSELEYGNCNTK CQTPIGAINSSMPFHNIHPLTIGECPKYVKSNRLVLATGLRNSPQGERRRKKRGLFGAIAGFIEGGWQGMVDG WYGYHHSNEQGSGYAADKESTQKAIDGVTNKVNSIIDKMNTQFEAVGREFNNLERRIENLNKKMEDGFLDV WTYNAELLVLMENERTLDFHDSNVKNLYDKVRLQLRDNAKELGNGCFEFYHRCDNECMESVRNGTYDYP QYSEEARLKREEISGVKLESIGTYQILSIYSTVASSLALAIMVAGLFLWMCSNGSLQCRICI |
| 163 | 40158-V08B | MEKIVLLLAMIGLVKSDQICIGYHANNSTEQVDTIMEKNVTVTHAQDILEKTHNGKLCNLDGVKPLILKDCS VAGWLLGNPMCDEFLNVSEWSYIVEKASPANGLCYPGDFNDYEELKHLLSRINHLKKIKIIPKSYWSNHEASS GVSAACSYLGEPSFFRNVVWLIKKNNTYPPIKVNYTNTNQEDLLVLWGIHHPNDEKEQIRIYQNPNTSISVGT STLNQRLVPKIATRPKVNGQSGRMEFFWTILKPNDSINFDSNGNFIAPEYAYKIAKKGDSVIMKSELEYGNCN TKCQTPMGAINSSMPFHNIHPLTIGECPKYVKSNRLVLATGLRNAPQREGGRRRKRGLFGAIAGFIEGGWQG MVDGWYGYHHSNEQGSGYAADKESTQKAIDGITNKVNSIIDKMNTQFEIVGREFNNLERRIENLNKKMEDG FLDVWTYNAELLVLMENERTLDFHDSNVKNLYEKVRLQLRDNAKELGNGCFEFYHKCDNECMESVRNGTY DYPQYSEEARLSREEISGVKMESMVTYQILSIYSTVASSLALAIMVAGLSLWMCSNGSLQCRICI |
| 164 | 11062-V08H1 | MEKIVLLFAIVSLVKSDQICIGYHANNSTEQVDTIMEKNVTVTHAQDILEKTHNGKLCDLDGVKPLILRDCSV AGWLLGNPMCDEFINVPEWSYIVEKANPVNDLCYPGDFNDYEELKHLLSRINHFEKIQIIPKSSWSSHEASLG VSSACPYQGKSSFFRNVVWLIKKNSTYPTIKRSYNNTNQEDLLVLWGIHHPNDAAEQTKLYQNPTTYISVGT STLNQRLVPRIATRSKVNGQSGRMEFFWTILKPNDAINFESNGNFIAPEYAYKIVKKGDSTIMKSELEYGNCN TKCQTPMGAINSSMPFHNIHPLTIGECPKYVKSNRLVLATGLRNSPQRERRRKKRGLFGAIAGFIEGGWQGM VDGWYGYHHSNEQGSGYAADKESTQKAIDGVTNKVNSIIDKMNTQFEAVGREFNNLERRIENLNKKMEDG FLDVWTYNAELLVLMENERTLDFHDSNVKNLYDKVRLQLRDNAKELGNGCFEFYHKCDNECMESVRNGT YDYPQYSEEARLKREEISGVKLESIGIYQILSIYSTVASSLALAIMVAGLSLWMCSNGSLQCR |
| 165 | 40160-V08B | MEKIVLLFAIISLVKSDHICIGYHANNSTEQVDTIMEKNVTVTHAQDILEKTHNGKLCDLNGVKPLILKDCSV AGWLLGNPMCDEFIDVPEWSYIVEKANPANDLCYPGNFNDYEELKHLLSRINHFEKIRIIPKDSWPDHEASLG VSAACSYQGNSSFFRNVVWLLKKDNAYPIIKKSYNNTNKEDLLVLWGIHHPNDEAEQTRLYQNPTTYVSIGT STLNQRLVPRIATRSKVNGQSGRIDFFWTILKPNDAINFESNGNFIAPEYAYKIVKKGDSTIMRSEVEYGNCST RCQTPMGAINSSMPFHNIHPLTIGECPKYVKSNKLVLATGLRNSPQIERRRRKRGLFGAIAGFIEGGWQGMVD GWYGYHHSNEQGSGYAADKESTQKAIDGVTNKVNSIIDKMNTQFEAVGREFNNLERRIENLNKKMEDGFLD VWTYNAELLVLMENERTLDFHDSNVKNLYDKVRLQLRDNAKELGNGCFEFYHKCDNECMESVRNGTYDY PQYSEEARLKREEISGVKLESIGTYQILSIYSTVASSLVLAIMVAGLSLWMCSNGSLQCRICI |
| 166 | 11689-V08H-50 | MEKIVLLLATVSLVKSDQICIGYHANNSTEQVDTIMEKNVTVTHAQDILERTHNGKLCDLNGVKPLILRDCSV AGWLLGNPMCDEFINVPEWSYIVEKASPANDLCYPGNFNDYEELKHLLSRISHFEKIQIIPKSSWSNHDASSG VSSACPYLGKSSFFRNVVWLIKKNSTYPTIKRSYNNTNQEDLLVLWGIHHPNDAAEQTKLYQNPTTYISVGTS TLNQRLVPEIATRPKVNGQSGRIEFFWTILKPNDAINFESNGNFIAPEYAYKIVKKGDSTIMKSELEYGNCNTK CQTPMGAINSSMPFHNIHPLTIGECPKYVKSNRLVLATGLRNAPQRERRRKKRGLFGAIAGFIEGGWQGMVD GWYGYHHSNEQGSGYAADQESTQKAIDGVTNKVNSIINKMNTQFEAVGREFNNLERRIENLNKKMEDGFLD VWTYNAELLVLMENERTLDFHDSNVKNLYDKVRLQLRDNAKELGNGCFEFYHKCDNECMESVKNGTYDY PQYSEEARLNREEISGVKLESMGTYQILSLYSTVASSLALAIMVAGLSLWMCSNGSLQCRICI |
| 167 | 40166-V08B | MIAIIVIAILAAAGKSDKICIGYHANNSTTQVDTILEKNVTVTHSVELLENQKEERFCKILNKAPLDLRGCTIEG WILGNPQCDLLLGDQSWSYIVERPNAQYGICYPGVLNEVEELKALIGAGERVERFEMFPKSTWAGVDTSSGV TRACPYNSGSSFYRNLLWIIKTKSAAYPVIRGTYNNTGKQPILYFWGVHHPPDINEQNTLYGSGDRYVRMGT ENMNFAKSPEIAARPAVNGQRGRIDYYWSVLKPGETLNVESNGNLIAPWYAYKFVSTNNKGAIFKSNLPIEN CDATCQTIAGALRTNKTFQNVSPLWIGECPKYVKSESLRLATGLRNVPQIETRGLFGAIAGFIEGGWTGMIDG WYGYHHENSQGSGYAADRESTQKAIDGITNKVNSIIDKMNTQFEAVDHEFSNLERRIDNLNKRMEDGFLDV WTYNAELLVLLENERTLDLHDANVKNLYEKVKSQLRDNANDLGNGCFEFWHKCDNECIESVKNGTYDYPK YQDESKLNRQEIESVKLENLGVYQILAIYSTVSSSLVLVGLIIAMGLWMCSNGSMQCRICI |
| 168 | 40399-V08H1-50 | MIAFIVIAILVTTGKSDKICIGYHANNSTTKVDTILEKNVTVTHSVELLENQKEERFCKISNKAPLDLRDCTLEG WILGNPRCGILLADQSWSYIVERPNARNGICYPGTLNEVEELKALIGSGERVERFEMFPKSTWAGVDTNSGVS SACPLGNGPSFYRNLLWIIKHTTSGYPVIKGTYTNTGDKSVLYFWGVHHPPDTTEQNVLYGSGNRYVRMGTE SMNFARSPEVAARPAVNGQRGRIDYFWSILKPGETLNVESNGNLIAPWYAYRFVNKDSKGAIFRSNLPIENCD ATCQTTEGVIRTNKTFQNVSPLWIGECPKYVKSKSLRLATGLRNVPQIETRGLFGDIAGFIEGGWTGMIDGWY GYHHENSQGSGYAADRESTQKAIDGITNKVNSIIDKMNTQFEAVGHEFSNLERRIDNLNKRMEDGFLDVWT YNAELLVLLENERTLDLHDANVKNLHERVKSQLRDNANDLGNGCFEFWHKCDNECMESVKNGTYNYPKY QAESRLNKQKIESVKLEEFGVYQILAIYSTVSSSLVLVGLIMAMGLWMCSNGSMQCRICI |
| 169 | 40171-V08B | MNTQILILAISAFLCVRADKICLGHHAVSNGTKVDTLTEKGIEVVNATETVEQKNIPKICSKGKQTIDLGQCGL LGTTIGPPQCDQFLEFSANLIIERREGDDICYPGKFDNEETLRQILRKSGGIKKENMGFTYTGVRTNGETSACR RSRSSFYAEMKWLLSNTDNGVFPQMTKSYKNTKREPALIIWGIHHSGSTAEQTRLYGSGNKLVTVWSSKYQ QSFAPSPGPKPQINGQSGRIDFYWLMLDPNDTVTFSFNGAFIAPDRASFLRGKSLGIQSDAQLDNNCEGECYHI GGTIISNLPFQNINSRAIGKCPRYVKQKSLMLATGMKNVPENSIHKQLTHHMRKKRGLFGAIAGFIENGWEGL IDGWYGYRHQNAQGEGTAADYKSTQSAINQITGKLNRLIEKTNQQFELIDNEFNEIEKQIGNVINWTRDSIIEV WSYNAEFLVAVENQHTIDLTDSEMNKLYEKVRRQLRENAEEDGNGCFEIFHQCDNDCMASIRNNTYDHKK YRKEAIQNRIQIDAVKLSSGYKDIILWFSFGASCFLFLAIAMVLAFICIKNGNMRCTICI |
| 170 | 11082-V08B | SKSRGYKMNTQILVFALVASIPTNADKICLGHHAVSNGTKVNTLTERGVEVVNATETVERTNVPRICSKGKR TVDLGQCGLLGTITGPPQCDQFLEFSADLIIERREGSDVCYPGKFVNEEALRQILRESGGIDKETMGFTYSGIRT NGTTSACRRSGSSFYAEMKWLLSNTDNAAFPQMTKSYKNTRKDPALIIWGIHHSGSTTEQTKLYGSGNKLIT VGSSNYQQSFVPSPGARPQVNGQSGRIDFHWLILNPNDTVTFSFNGAFIAPDRASFLRGKSMGIQSEVQVDAN CEGDCYHSGGTIISNLPFQNINSRAVGKCPRYVKQESLLLATGMKNVPEIPKRRRRGLFGAIAGFIENGWEGLI DGWYGFRHQNAQGEGTAADYKSTQSAIDQITGKLNRLIEKTNQQFELIDNEFTEVERQIGNVINWTRDSMTE VWSYNAELLVAMENQHTIDLADSEMNKLYERVKRQLRENAEEDGTGCFEIFHKCDDDCMASIRNNTYDHS KYREEAIQNRIQIDPVKLSSGYKDVILWFSFGASCFILLAIAMGLVFICVKNGNMRCTICI |
| 171 | 40325-V08H | TQILVFALIAIIPTNADKICLGHHAVSNGTKVNTLTERGVEVVNATETVERTNIPRICSKGKRTVDLGQCGLLG TITGPPQCDQFLEFSADLIIERREGSDVCYPGKFVNEEALRQILRESGGIDKEAMGFTYSGIRTNGATSACRRSG SSFYAEMKWLLSNTDNAAFPQMTKSYKNTRKSPALIVWGIHHSVSTAEQTKLYGSGNKLVTVGSSNYQQSF VPSPGARPPVNGLSGRIDFHWLMLNPNDTVTFSFNGAFIAPDRASFLRGKSMGIQSGVQVDANCEGDCYHSG GTIISNLPFQNIDSRAVGKCPRYVKQRSLLLATGMKNVPEIPKGRGLFGAIAGFIENGWEGLIDGWYGFRHQN AQGEGTAADYKSTQSAIDQITGKLNRLIEKTNQQFELIDNEFNEVEKQIGNVINWTRDSITEVWSYNAELLVA MENQHTIDLADSEMDKLYERVKRQLRENAEEDGTGCFEIFHKCDDDCMASIRNNTYDHSKYREEAMQNRIQ IDPVKLSSGYKDVILWFSFGASCFILLAIVMGLVFICVKN |

**Table 9: Sequences of gp160 antigens that can be included in an HIV vaccine**

| **ASC number** | **Clade** | **SEQ ID** | **Sequence** |
|---|---|---|---|
| A.IN.01.1579 A.DQ083238 | A | 172 | MRVMETQRSYQHLWRWGTMILGMLIIYSAAENLWVTVYYGVPVWKDAETTLFCASDAKAYET EKHNVWATHACVPTDPNPQEIHLENVTEDFNMWKNNMVEQMHTDIISLWDQSLKPCVKLTPLC VTLNCSNVNNTQEVKNCTFNMTTELRDKRQKVSSLFYKLDIVPIEEGQGNSSNSSYEEYRLINCN TSAITQACPKVTFEPIPIHYCTPAGFAILKCKDEEFNGTGPCRNVSTVQCTHGIKPVVSTQLLLNGS LAKNKVIIRSENITNNVKTIIVQLAEPVIINCTRPNNNTRESVRIGPGQTFYTSNIIGDIRQAHCNVS KSAWNKTLHVVGEQLEKYFVNKTIKFDRPTGGDLEITTHSFNCGGEFFYCNTSELFNSTWNSSTQ GTNNTELNDTITLPCRIKQVINMWQRVGQAMYAPPIKGIIKCVSNITGLILTRDGGINSTNGTETFR PGGGDMRDNWRSELYKYKVVQIEPLGVAPTKARRRTVGREKRAIGLGAVFLGFLGAAGSTMG AASITLTVQARQLLSGIVQQQSNLLRAIEAQQQMLRLTVWGIKQLQARVLAVERYLRDQQLLGI WGCSGKLICTTNVPWNSSWSNKTQEEIWGNMTWLQWDKEISNYTEIIYNLIEKSQNQQEKNEKD LLALDKWATLWSWFDITKWLWYIKIFIMIVGGLIGLRIVFAVLSVINRVRQGYSPLSFQTHTPNPR GLDRPGGIEEEGGEQDRTRSIRLVSGFLALAWDDLRSLCLFSYHRLRDFVWIAKRTVELLGHSSL KGLRLGWEGLKYLWNLLVYWGLELKKSAINLFDTIAIVVAGWTDRIIEIGQGIGRGILHIPRRIRQ GLENFLL |
| A.KE.00.00KE | A | 173 | MRVTGIQKNCQSLWRWGTMILGMLMICSVVGNLWVTVYYGVPVWKEADTTLFCASNARAYD TEVHNVWATHACVPTDPNPQEIDLENVTEEFNMWKNNMVEQMHTDIISLWDQFLKPCVKLTPL CVTLDCGYNVTNLTSTSNMKEDITNCSYNMTTEIRDRKQKVYSLFYRLDIVPIDEEKNNNSVTSP YRLINCNTSAITQACPKVSFEPIPIYYCAPAGFAILKCKDAEFNGTGPCKNVSTVQCTHGIRPVIST QLLLNGSLAENGTKIRSENITNNAKTIIVQLNETVQINCTRPNNNTRRSVRIGPGQAFYATGGITG DIRRAYCNVSRQKWEQALKGVVIQLRKHFNNKTITFTNSSGGDVEITTHSFNCGGEFFYCNTSGL FNSTWDNNTDPTNTTSNDTITLQCRIKQFINMWQRTGQAMYAPPIQGVIRCDSNITGLLLTRDGG VGNNTTETFRPGGGDMRDNWRSELYKYKVVKIEPLGVAPTGAKRRVVEREKRAVGIGAVFIGF LGAAGSTMGAASVTLTVQARQLLSGIVQQQSNLLRAIEAQQHLLKLTVWGIKQLQARVLAVER YLRDQQLLGIWGCSGKLICTTNVPWNSSWSNKSQEEIWGNMTWLQWDKEISKYTQTIYDLFEES QNQQEKNEQDLLALDKWANLWTWFDITNWLWYIKIFIMIVGGLIGLRIVFSVLSVINRVRQGYSP LSFQTLTPSPRGLDRLGRIEEEGGERDRDRSIRLVSGFLALAWDDLRNLCLFSYHRLRDFILIAART VELLGHNSLKGLRLGWEGLKYLWNLLIYWGRELKFSAINLIDTIAIAVAGWTDRIIEAGQRLCRA ILNIPRRIRQGAERAWL |
| A.KENYA | A | 174 | MRVMGTQRNCQHLLRWGTMILGMIIICSAAENLWVTVYYGVPVWKNAETTLFCASDAKAYET EKHNVWATHACVPTDPNPQEIHLENVTEEFNMWKNNMVEQMHTDIISLWDQSLKPCVKLTPLC VTLNCSNVNVTATSGTTHGGRGRMKNCSYNITTELRDRRQKVYSLFYRLDVVPINENNSSNNEY RLINCNTSAITQACPKVTFEPIPIHYCAPAGFAILKCKDKKFNGTGPCTNVSTVQCTHGIKPVVSTQ LLLNGSLAEGEVMIRSENITNNAKNIIVQFAEPVKINCTRPNNNTRMSIRIGPGQAFYATGDIIGNI RQAHCNVSRAEWNTTLQKVVTKLREYFGNNKTIKFANSSGGDLEITTHSFNCGGEFFYCNSSGL FNSTWTNMQESNSTESNDTITLPCRIKIINMWQRAGQAMYAPPIQGIIKCVSNITGLILTRDGGDN NSESETFRPGGGDMRDNWRSELYKYKVVKIEPLGVAPTKAKRRVVEREKRAVGIGAVFLGFLG AAGSTMGAASITLTVQARQLLSGIVQQQSNLLRAIEAQQHLLKLTVWGIKQLQARVLAVERYLR DQQLLGIWGCSGKIICTTNVPWNSSWSNKSQSEIWENMTWLQWDKEISNYTDIIYNLLEESQNQ QEKNEQDLLALDKWANLWNWFDISNWLWYIKIFIMIVGGLIGLRIVFTVLSIINRVRQGYSPLSFQ THTPAPRGLDRPERIEEEGGEQDRDRSIRLVTGFLALAWDDLRSLCLFSYHRLRDFILIATETVELL GHSSLRGLRLGWEGLKYLWNLLLYWGQELKISASNLVDTIAIVVAGWTDRVIEIAQGIGRAILHI PRRIRQGFERALL |
| A.RW.SF1703 | A | 175 | MRVMGIQMNCQNLLRWGTMILGMLIICSATSKLWVTVYYGVPVWKDAETTLFCASDAKAYER EVHNVWATHACVPTDPDPQEIYLENVTEGFNMWKNNMVEQMHTDIISLWDQSLKPCVKLTPLC VTLNCSHNITTHNITTNNPNITKYMEGEIKNCSYNMTTELRDKKQKVYSLFYKLDVVPIDKNNG NNNIRTQYRLINCNTSAITQACPKVSFEPIPIHYCAPAGFAILKCNDAEFNGTGPCKNVSTVQCTH GIRPVISTQLLLNGSLAEGRVKIRSENITNNAKTIIVQLNKTVEINCTRPNNNTRKSVRIGPGQAFY ATGDIIGDIRQAYCNVSRADWNKTLQGVANQLKSYFSNKTIIFASSSGGDLEITTHSFNCGGEFFY CDTSGLFNSTWGGNSTDSIQESNSTESNDTIILPCRIKQIINMWQRVGQAMYAPPIQGVIKCISNIT GLLLTRDGGDNNSANETFRPTGGNMRDNWRSELYKYKVVKIEPLGVAPTPAKRRVVQREKRAV GIGAVFIGFLGAAGSTMGAASITLTVQARQLLSGIVQQQSNLLRAIEAQQHLLKLTVWGIKQLQA RVLAVERYLKDQQLLGIWGCSGKLICTTNVPWNSSWSNRTQSEIWNNMTWLQWDKEISNYTDII YNLIEESQIQQEKNEQELLALDKWANLWNWFDISKWLWYIRIFIMIVGGLIGLRIVFAVLSIINRV RQGYSPLSFQIHTPNPRGPDRPERIEEEGGEQGRDRSIRLVSGFLALAWDDLRSLCLFSYHRLRDFI LIAARTVELLKRSSLRGLRLGWEGLKYLWNLLLYWGRELKNSAINLLDTIAIAVAGWTDRVIEIG QAICRAILNIPRRIRQGFERALL |
| A.NG.NG1935 | A | 176 | MRARGMQRNWQHWGRWGILFLLIIYSAAENLWVTVYCGVPVWKDAKTTLFCASDARAYHPE AHNVWATHACVPTDPNPQEIHLENVTEKFNMWKNNMVEQMHEDIISLWDQSLKPCVKLTPLCV TLDCHDLNSSNSSNYTSSNMQGEMTNCSFNMTTELRDKKQKVNAFFYRLDVVQIKENNSTQYR LINCNTSAITQACPKVSFEPIPIHYCAPAGFAILKCNDKKFNGTGPCKNVSTVQCTHGIKPVVSTQL LLNGSLAEEEVVIRSENITNNAKTIIVQLANPVRINCTRPGNNTRKSVRIGPGQTFYATGEIIGDIRQ AHCNVNRTEWDSSLQKVATQLRQYFKNTTKIIFTKHSGGDLEITTHSFNCGGEFFYCNTSGLFNS TWDNNNIASSNHTGSNDTITLQCRIKQIINLWQKVGQAMYAPPIPGIIRCESNITGLLLTRDGGNN NSTNETFRPGGGDMRDNWRSELYKYKVVKIEPLGVAPTKAKRRVVARKKRAVGLGAVFLGFL GAAGSTMGAASIALTVQARQLLSGIVQQQSNLLRAIEAQQHMLKLTVWGIKQLQARVLAIERYL KDQQLLGIWGCSGKLICTTNVPWNSGWSNKSQNEIWDKMTWLEWDKEISNYTQLIYTLIEESQN QQEKNEQELLALDKWASLWSWFDISNWLWYIKIFIMVVGGLIGLRIVFAVLSVVRRVRQGYSPL SLQTLIPSPRGPDRPEGIEERGGEQDKDRSIRLVSGFLALAWDDLRSLCLFSYHRLRDFILIAARIV ETLGHRGWQGLKYLGNLVQYWSQELKNSAISLLDTIAIAVAEWTDRVIEIGQRICRAILNIPRRIR QGLERALL |
| A.GM.00.N98 45.HQ385459 | A | 177 | MKVRGTQRNYAPLWKWGTIFLGLIIICKATENLWVTVYYGVPVWRDAETILFCASDAKAYEKE VHNVRDTHACVPTDPNPQEIELENVTEEFNMGKNNMVEQMHTDIISLWDQSLKPCVKLTPICVT LNCHNITTNDTGTGNVTGTGNVTDNNIGEMTNCSFNITTEIRDKRQNVYSLFYKLDVVPINGNGS GNDSEYRLINCNTSTITQACPKVSFEPIPIHYCAPAGFAILKCRDKNFNGTGPCRNVSSVQCTHGIK PVVSTQLLLNGSLAEGDVVIRSENLTNNAKTIIVQLNESVPINCTRPNNNTRKSVRIGPGQALYAT DIIGNIRQAHCNISKQNWNTALQKIAAQLRQHFYRTINFTKSSGGDLEITTHSFNCRGEFFYCDTS GLFNSIWNSTAISQNVEESNDTITLPCKIRQIVNMWQRVGQAIYARPIQGIINCTSNITGLLLTRDG GDNDNKTETFRPGGGNMKDNWRSELYRYKVVKIEPLGVAPTKAKRRVVEREKRAVGLGAVIF GFLGAAGSTMGAASITLTAQATQLLSGIVQQQSNLLRAIEAQQHLLKLTVWGIKQLQARVLALE RYLRDQQLLGIWGCSGKLICTTTVPWNSSWSNKSQNEIWENMTWQWDKEISNYTDIIYDLIAES QNQQEKNEKDLLALDKWASLWNWFDISKWLWYIKLFIMIVGGLIGLRIVFTVLSIINRVRQGYSP LSFQTHTPSPGGLDRPERIEEEGGDRDRDRSTRLVSGFLALVWDDLRSLCLFSYHRLRDFTLIAAR TVELLGHSSLKGLRLGWEGLKYLWNLLSYWGRELKSSAITLLNATAIAVAEGTDRVIEVGQRAG RALLHIPRRIRQGWKRALL |
| A.UG.07.191845 | A | 178 | MRVMGIQKNWQHLWKWGTLILGMIMICSTAGNLWVTVYYGVPVWKDADTTLFCASDAKAYE TEKHNVWATHACVPTDPNPQETYLENVTEEFNMWKNNMVEQMHTDIISLWDQSLKPCVKLTPL CVTLHCTDVNVTNTNVTNTNGTSTSSSKVTINDREEIKNCSFNVTTELRDKEKKVYSLFYKLDVV KIDKNKSDYILINCNTSTITQACPKVSFEPIPIHYCAPAGFAILKCRDIDFNGTGPCKNVSTVQCTH GIKPVVSTQLLLNGSLAEGGIRIRFENITNNAKTIIVQLNSSVPINCTRPNNNTRQSVRIGPGQSFFA TGEIIGDIRQAHCNVSKSDWHKTLQQVAEQLEKHFKNKTIIFDQPSGGDLEITTHSFNCGGEFFYC NTAGLFNRNWTWSKENGTRMQNKTDSNGTITLQCRIKQIINMWQRTGQAMYAPPIQGVIKCAS NITGLLLTRDGGDNKNNSTNSTNQNNTEIFRPIGGDMRNNWRSELYKYKVVKIEPLGVAPTTAR RRVVGREKRAVLGGLGAVFLGFLGAAGSTMGAASITLTVQARQLLSGIVQQQSNLLRAIEAQQH LLKLTVWGIKQLQARVLAVERYLRDQQLLGIWGCSGKLICPTNVPWNSSWSNKTMSEIWDNMT WLQWDKEISNYTNIIYNLIEESQNQQEKNEQDLLALDKWASLWNWFDISKWLWYIKIFIMIVGG LIGLRIIFAVFSIINRVRQGYSPLSFQTHPPNPGGLDRPGRIEEEGGEQDRGRSIRLVSGFLALAWD DLRSLCIFSYHRLRDFTLIAERTVELLGHNSLKGLRLGWEGLKYLWNLLVYWGQELKTSAISLFD TLAIVVAGWTDRVIEIGQRLGRGFLHIPRRIRQGLERVLL |
| A.GM.09.N05 7856.HQ3854 53 | A | 179 | MRVREIPRNCPLLWRWGTMIFWILLICNAEELWVTVFYGVPVWRDAETTLFCASDAKAYDTEV HNIWATHACVPTDPNPQEIGLENVTEEFNMWKNNMVEQMHTDIISLWDQSLKPCVQLTPLCVTL NCSHNISTTNSTSTGNISTINSTSADMRNCSFNMTTELRDKKQNVYSLFYKLDVVQIGNSSSSEYR LINCNISTITQACPKVSFEPIPIHYCAPAGYAILKCREKNFNGTGPCTNVSTVQCTHGIKPVVSTQL LLNGSLAEGDVMIRSENITDNAKTIIVQFNESVMINCTRPNNNTRRSVRIGPGQTFYATGEIIGDIR RAHCNVSGEQWRKALQNVKVQLRKYFNTTIEFANSSGGDLEITTHSFNCRGEFFYCDTTGLFNN TEKNDTIMLPCKIRQIVRMWQRVGQAMYAPPIPGVINCLSNITGLLLTRDGGDSTNNTEKFRPGG GDIRDNWRSELYKYKVVKIEPLGVAPTKAKRRVVERRKRAGLAAVFLGFLGAAGSTMGAASIT LTVQARQLLSGIVQQQNNLLKAIEAQQQMLRLTVWGIKQLQARVLALERYLRDQQLLGIWGCS GKLICTTNVPWNSSWSNKTYNSIWDNMTWLQWDKEINNYTNTIYTLIEKAQNQQEKNEQDLLA LDKWASLWNWFDITNWLWYIRIFIMIAGGLIGLRIVFAVLSIINRVRKGYSPLSFQTPTPNPGDLD RPERIEEEGGEQGRDRSIRLVSGFLALVWDDLRSLCLFSYHRLRDLVLLTTRAVELLGHNSLKGL RLGWNVLKQLGNLLSYWGQELKNSAINLLDTIAIAVANWTDRVIEIGQRVGRAICNIPRRIRQGL ERLLL |
| A.CD.97.97CD | A | 180 | MKVRGIQRNSQHLWRWGTLIFWTIIICSAAEKLWVTVYYGVPVWKDAETTLFCASHANAYEKE QHIVWATHACVPTDPNPLEMDLDNVTEEFNMWKNNMVEQIHDDIIRLWDRSLQPRVKLTPLCV TLDCQPVNSTNNTKVEVPGEMTNCSFNMTTELSDKKQKVRSLFYRIDLVQIGNNTNDSSNRSLQ YRLINCNTSTITQACPKVSFEPVPIHYCAPAGFAILKCKDQEFNGTGPCKNVSTVQCTHGIKPVVS TQLLLNGSLAEGGIKIRSANISYNAKNIIVQLDIPVKINCSRPNNNTRTSVRIGPGQTFYATGDIIGN IRQAHCNLSRTAWNDTLYNVSKALREHFPNKTIIFNKSSGGDLEVTQHMFICGGEFFYCNTSGLF NSSWAPNGSWINNIGSRENDTMTLPCRIKQIINMWQRVEQAMYAPPIKGNITCVSNITGLLLTRD GGTNETRENETFRPIGGEMRDNWRSELYKYKVVKIEPLGVAPTKAKRRVVEGREKRAVGMGAF FLGFLGAAGSTMGAASITLTVQARQLLAGIVQQQSNLLKAIEAQQHLLRLTVWGIKQLQARVLA VERYLKDQQLLGIWGCSGKLICTTTVPWNSSWSNKSQDEIWNNMTWMQWDKEINNYTEIIYGLI EESQKQQEKNEFELLELDKWANLWNWFEISNWLWYIKIFIMIVGGLIGLRIVFAVLSIVNRVRQG YSPLSFQTPIPSPEGPDRPGRIEEGGGEQGRTRSIRLVSGFLELAWDDLRSLCLFVYHRSRDFILIAA RTVELLGHSSLKGLRLGWEGLKYLWNLLVYWSQELKTSAVSAFNALAITVAEWTDRAIEVGQR IGRAFLNIPRRIRQGFERALL |
| A.TZ.08.CH0175 | A | 181 | MKVKGIQMNSQHLLRWGIMILGLIIICSAAEKLWVNVYYGVPVWKNADTTLFCASDAKAYNPE AHNIWATHACVPTDPSPQEIYLENVTEDFNMWKNNMVEQMHADIISLWDQSLKPCVQLTPLCV TLECKKINITNTTGNNVTANNDTILAREEMRNCSYNMTTELRDKKQKVHSLFYRLDIVEINNNN NGSSNNNSDNDSRPYRLINCNTSAITQACPKISFEPIPIHYCAPAGFAILKCRDKEFNGTGPCKNVS TVQCTHGIKPVVSTQLLLNGSLAEKRVMIRTENITNNAKNIIVQFNESVEINCTRPNNNTRQSVRI GPGQAFYTTEIIGDIRRAYCTVNETKWNQTLYRVAEQLRKYFNNDTTIIFANSSGGDVEITTHSFN CGGEFFYCNTSGLFNSRWENDTQPNNTQPNGTITLPCRIKQIINMWQRTGQAIYAPPIRGVISCQS NITGLLLTRDGGGKNTSDNETFRPGGGDMRDNWRSELYKYKVVKIEPLGVAPNRARRRVVERE KRAVGIGAVFLGFLGAAGSTMGAASMTLTVQARQLLSGIVQQQSNLLRAIEAQQHLLKLTVWGI KQLQARLLAVERYLRDQQLLGIWGCSGKLICATNVPWNSSWSNKSYDEIWGNMTWLQWDKEI DNYTQLIYQLIEDSQNQQEKNEQELLALDKWANLWNWFEISNWLWYIRLFVMIVGGLIGLRIVL AVLSVINRVRQGYSPLSFQTHTPSPGGLDRPRGIEEEGGEQDRGRSIRLVSGFLALAWDDLRSLCL FSYHRLRDFVLIAARIVELLGHNSLKGLRLGWEGLKYLWNLLAYWGRELKNSAINLLDTIAIAV AGWTDRVIELGQRLCRAFLHIPRRIRQGFERALL |
| A.KE.99.99KE | A | 182 | MKVKGTQRNFQHLLRWGIMVLGMIIICSATEQLWVTVYYGVPVWKDAETTLFCASDAKAHETE VHNVWATHACVPTDPNPQEIELKNVTEEFNMWKNNMVEQMHTDIISLWDQSLKPCVKLTPLCV TLNCSNFSVTNNSTNNSTNTTTIDNNMQGEIKNCSYNVTTVIRDEKQKVYSLFYRTDVVEIENNS SQYRLINCNTSAITQACPKVTFEPIPIHYCAPAGFAILKCRNETFNGTGPCNNVSTVQCTHGIRPVV STQLLLNGSLAEKEIMIRSENISDNAKSILVQLTRPVIINCTRPNNNTRKSIHIGPGQAFYATGDIIG DIRQAYCTVNRTEWNETLHNVTTQLKTYFNKTIVFTSHSGGDLEITTHTFNCGGEFFYCNTSGLF NSTWDDNIKNDTNSTQGSNSTEIILPCKIKQIVRMWQRTGQAMYAPPIPGVIRCVSNITGLILTRD GGNTSSINETFRPGGGDMRDNWRSELYKYKVVKIEPLGVAPTKARRRVVEREKRAVGIGAVLFG FLGAAGSTMGAASITLTVQARQLLAGIVQQQSNLLRAIEAQQQMLRLTVWGIKQLQARVLAVE RYLRDQQLLGLWGCSGKLICTTTVPWNSSWSNKSQNEIWNNMTWLQWDKEISNYTQIIYDLIEE SQNQQEKNEQDLLALDKWANLWSWFDISSWLWYIRIFIMIVGGLIGLRIVFAVLSIIKRVRQGYSP LSFQIHTPNPGEPDRPGEIEEEGGKQDRSRSIRLVSGFLALAWDDLRSLCLFCYHRLRDFILIAART VELLGHSSLKGLRLGWEGLKYLWNLLVYWSQELKSSAINLFDTLAITVAGWTDRVIEVGQRLCR ALLHIPRRIRQGLERALL |
| A.VI191A | A | 183 | MRVKGIQMNWQHLWKWGTLILGLGIICNAENLWVTVFYGVPVWKDAETTLFCASDAKAYSEE KHNVWATHACVPTDPDPKEINLVNVTEDFNMWKNKMVEQMQEDIISLWDQSLKPCVKLTPLC VTLECHNSTTPDNNTTQSTIIDETTNCTYNMTTELRDKVQKVHSLFYRLDVVQINGINGTSNNNS AQYRLINCNTSAITQACPKVSFEPIPIHYCAPAGFAILKCRDKEFNGTGPCRNVSSVQCTHGIKPV VSTQLLLNGSLAEEKIMIRSENFTDNGKTIIVQLVEPVKINCTRPSNNTRKGIHIGPGRAFYATGQI TGDIRQAHCNISRTEWNKTLHQVAIQLREHFITKNTTIIFNSSSGGDLEITTHSFNCGGEFFYCSTS GLFNSTWENGTQVANDTGSNDTITLPCRIKQIVRMWQRVGQAMYAPPIQGMIRCESNITGLILTR DGVGNNSTNETFRPGGGDMRDNWRSELYKYKVVKIEPLGVAPSRAKRRVVAREKRAVGLGAV FLGFLGAAGSTMGAASLTLTVQARQLLSGIVQQQSNLLRAIEAQHHLLQLTVWGIKQLQARVLA LERYLRDQQLLGIWGCSGKLICPTNVPWNYSWSNKSYDDIWNNMTWLQWDKEIDNYTQLIYGL IEESQNQQEKNEQDLLALDKWASLWSWFDISNWLWYIRIFIMIVGGLIGLRIVFAVLSIINRVRQG YSPLSFQTLPHHQREPDRPEGIEEGGGEQDRGRSIRLVSGFLALAWDDLRSLCLFSYHRLRDFILIV ARIVETLGRRGWEALKYLWNLLLYWGRELKISAITLLDATAIAVARWTDRIIEIGQRIVRAILNIP RRIRQGAEKALQ |
| A.CD.97.97D C.KMST91 | A | 184 | MRARGIQRNYQHLWKWGTLIFGMLMICNAVDNLWVTVYYGVPVWREAKTTLFCASDAKTYD QEVHNVWATHACVPTDPDPQEIDLRNVTEKFNMWKNDMVEQMHTDIISLWDQSLQPCVKLTPL CVTLNCTNITNISNNNNTDSRNITHSEDPAEMRNCSFNVATGIRDRKKKVYSLFYRLDITPINGSD DSKSSGQYILINCNTSTITQACPKVSFEPIPIHYCAPAGFAILKCKDKQFNGTGTCNNVSPVQCTHG IKPVVSTQLLLNGSLPEGGVIIRSENITNNAKTIIVQLDEPVRINCTRPNNNTRKGIHIGPGGAFYAT GEVVGNIRHAWCEVNGTAWKEALKKVVTKLKEHFKNKTIAFQPSSGGDLEITTHSFNCRGEFFY CNTSGLFNSTWPGNTSESNVNTTGNITLPCKIKQIINMWQRVGQAMYAPPIPGIIQCISNITGLLITR DGGNNDNNVTEIFSPGGGDMRDNWRSELYKYKVVEIEPLGVAPTKAKRRVVQREKRAVGIGAV FLGFLAAAGSSMGAASMTLTVQARQLLSGIVQQQSNLLKAIEAQQHLLKLTVWGIKQLQARVL AVERYLKDQQLLGIWGCSGKLICTTTVPWNTTWSNKTLTDIWDNMTWLEWDREIRQYTGIIYNL IEESQNQQEKNEQDLLALDKWATLWSWFDISNWLWYIRIFIIIVGGLIGLRIVFAVFSIVKKARQG YSPLSLQILGPNPGGPDRPERIKEEGGEQGRDRSIRLASGFLALVWDDLRSLGICSYHRLRDFVLIA TRTVELLGRSSLKGLRLGWEGLKYLWNLLQYWSQELKNSAISLLGAIAIAVAEGTDRVITVGQRI CRAILNIPRRIRQGFERALL |
| A.FI..FIN911 21 | A | 185 | MRARGIQRNYQHLLRWGTIILGMILICSTTENLWVTVYYGVPVWKDAETTLFCASDAKAYDTE MHNVWATHACVPTDPNPQEIYLENVTEEFNMWKNNMVEQMHTDIISLWDESLKPCVQLTPLCV TLNCSNANANSINANATSTNATAENEKGEIKNCSFNMTTELRDKKKKVYSLFYRLDVIPLNGTE NETYTEYRLINCNTSAITQACPKVSFEPIPIHYCAPAGFAILKCRDEKFNGTGPCTNVSTVQCTHGI RPVVSTQLLLNGSLAKEEVRIRSENITNNVKTIIVQLVKPVNITCIRPNNNTRKSIHLGPGRAFYAT GDIIGNIRKAHCIVNESEWNEALQQVATQLGKYFENKTINFTSPSGGDLEVTTHSFNCGGEFFYC NTSGLFNSTWVSNNTGKYYSNSTRENPNITLPCRIKQIINMWQRAGQAIYAPPIEGVIRCESNITGL LLTRDGGDKNNTSEIFRPGGGNMKDNWRSELYKYKVVKIEPLGVAPTPARRRVVQREKRAVFG LGAVFLGFLGAAGSTMGAASITLTVQARQLLSGIVQQQSNLLRAIEAQQHLLKLTVWGIKQLQA RVLAVERYLKDQQLLGIWGCSGKLICTTNVPWNSTWSNKSYSEIWDNMTWMQWDKEISNYTQ TIYNLIEKSQIQQEKNEQDLLALDKWTNLWTWFDISNWLWYIKIFIMIIGGLIGLRIVFAVLSAINR VRQGYSPLSFQTHTPNPEGLDRPGRIEEEGGEQGRGRSIRLVSGFLALAWDDLRSLCLFSYHRLK DFILIAARTVELLGHSSLKGLRLGWEGLKYLGNLLLYWGRELRISASDLLDTIAIAVAGWTDRVI EIGQRIGRAILNIPRRTRQGLERALV |
| A.KE.07.21020 | A | 186 | MRVMGIQRNWQHLLSWGTMVLGMIIICSAVDNLWVTVYYGVPVWKDAETTLFCASDAKAYET EQHNVWATHACVPTDPNPQEMPLKNVTEQFNMWKNNMVEQMHTDIISLWDQSLKPCVELTPL CVTLNCSTITLNNTDTHSTNNTDIDAMRNCSFNVTTELRDKTKRVYSLFYRLDIVQIDGNNSNGS GEYRLINCNTSAITQACPKVSFEPIPIHYCAPAGFAILKCNEKEFNGTGPCKNVATVQCTHGIKPV VSTQLLLNGSLAEGKVMIRSENITDNSKNIIVQFNKTVTINCTRPNNKTKIRSIHIGPGRVFQATQT GDMRKAFCTVNKTEWDKTLEEVAKQLRAHFNNKTIIFKSSSGGDIEITTHSFNCGGEFFYCNTSG LFNSTWEANSTANTSTTNITLPCKIKQLVRMWQRTGQALYAPPIEGVIKCVSNITGLILTRDGGGN NSATNETFRPGGGDMRDNWRSELYKYKVVQIEPLGVAPTEARRRVVKREKRAVPGLFFLGFLA AAGSTMGAASITLTVQARQLLSGIVQQQSNLLRAIEAQQHLLKLTVWGIKQLQARVLTVERYLR DQQLLGIWGCSGKLICTTNVPWNSSWSNKSQTEIWDNMTWLQWDKEISNYTQQIYKLLEESQN QQEQNEQDLLALDKWASLWNWFEISNWLWYIKIFIMIVGGLIGLRIVFAVLSIINRVRQGYSPLSF QTQFPNPAGPGRPGRIEEESGEQDRDRSIRLVSGFLALAWDDLRSLCLFSYHRLRDFILIVARTVE LLGHSSLKGLRLGWEGLKYLGNLLIYWGRELKLSAINLFDTIAIVVAGWTDRVIELGQRIVRGFL HIPRRIRQGLERALL |
| A.ZA.07.SO | A | 187 | MKVRGIQRNWPQWWIWGILGFWVIMMCKGVGNLWVTVYYGVPVWREANTTLFCASDAKAY DTEVHNVWATHACVPTDPNPQEIVLENVTENFNMWKNDMVDQMHEDIISLWDQSLKPCVKLT PLCVTLNCTEATFDNNVTDSMKKEMKKCSFNTTTEIRDRKQQAHALFYKLDLVPLNGSSSGYGN YILINCNTSTITQACPKVTFEPIPIHYCAPAGFAILKCKDEAFNGTGPCNNVSTVQCTHGIKPVVST QLLLNGSLAKGGIRIRSKNITNNAQTIIVQLSKPVKINCTRYNNNTRESIRIGPGQTFYTNKVIGDIR QAHCNISETEWNNTLQEVAKQLVVHFPNKTITFAPAAGGDLEITTHSFNCGGEFFYCNTSGLFND TFMPNNTANSTKNETQEKNITISCKIKQIIRIWQRVGQAMYAPPIEGLIKCESNITGILLTRDGGNN TNSTEVFRPGGGNMKDNWRSELYNYKVVKIEPLGVAPTGARRRVVEREKRAVVGLGAVFLGFL GAAGSTMGAASLTLTVQARQLLAGIVQQQSNLLQAIEAQQHMLKLTVWGIKQLQARVLALERY LQDQQLLGIWGCSGKLICTTTVPWNSSWSNKSQEEIWKNMTWLQWDKEISKYTGIIYTLLQESQ SQQEKNEQDLLALDKWASLWNWFDISNWLWYIKIFIMIVGGLIGLRIVFAVISVVNRVRKGYSPL SFQTLTPNQEGLDRLGRIEEEGGEQGRGRSIRLVSGFFSLAWDDLRSLCLFSYHRLRDLLLIAART VEHLGSSLLKGLRLAREGLNQLWNLLLYWGAEIKNSAISLLDTTAIAVANWTDRVIELGQGIGR AILHIPRRIRQGFETALL |
| A.TZ.05.6592 | A | 188 | MRVKGTQMNLQHLLKWGTMILGLIIICSAAENLWVTVYYGVPVWKDAETTLFCASDAKTYDK EVHNVWATHACVPTDPNPQEINLENVTEDFNMWKNNMVDQMHADIISLWDQALKPCVKLTPL CVTLNCSDSVTTANNSIQNEMKGEIKNCSFNVTTELRDKRQKVYSLFYRLDIVQIDNNNRNSTQY RLINCNTSAITQACPKVSFDPIPIHYCAPAGFAILKCKDENFTGVGPCKNVSTVQCTHGIKPVVST QLLLNGSLAEKKVMIRSENISNNAKNILVQLTTPVNITCIRPGNNTRKSVHIAPGQAFYATGDIIGD IRQAHCNVNRTQWNNTLKEVANQLRTYFGNETAIIFASSSGGDLEITTHSFNCRGEFFYCNTSGLF HSTWFNSTWFNSTWEDNQANSINDTIVLPCRIKQIINMWQRAGQAIYAPPIPGEIRCESNITGLML TRDGGNNNNNRTNETKNETFRPGGGDMRDNWRSELYKYKVVKIEPLGLAPTGAKRRVVEREK RAVGIGAVFLGFLGAAGSTMGAASITLTVQARQLLSGIVQQQSNLLKAIEAQQHLLKLTVWGIK QLQARVLAVERYLKDQQLLGIWGCSGKLICTTTVPWNSSWSNRSQSEIWGNMTWQQWEKEISN YTEEIYRLIENSQNQQEKNEQDLLALDKWASLWNWFDISNWLWYIRLFVIIVGGLIGLRIVLAVF SIINRVRQGYSPLSFQIHTPNPGGLDRPGRIEEEDGEQGKSRSIRLVSGFLALAWDDLRSLCLFSYH RLRDFILIATRTVELLGHSSLKGLRLGWEGLKYLWNLLACWGRELKIRAINLLDTLAIATANWTD RVIELLQALGRAFLNIPRRIRQGAERAWL |
| A.UA.AF082 486 | A | 189 | MKARGMQRNYQHLWRWGTMLFWMIIMCKAAENLWVTVYYGVPVWRDAETTLFCASDAKAY DKEVHNVWATHACVPTDPNPQEISLENVTEKFDMWKNNMVEQMQTDIISLWDQSLKPCVKLTP LCVTLNCAEPKSTSSNNSSVNSNSSDSLFEEMKNCSFNMTTELRDKRKTVHSLFYKLDIVSTGNG SGQYRLINCNTSAMTQACPKVTFEPIPIYYCAPAGFAILKCKDTNFTGTGPCKNVSTVQCTHGIKP VVSTQLLLNGSLAEKEVMIRSENITDNSKIIIVQLTEPVNITCIRPGNNTRTSIRIGPGQTFYATGDVI GDIRKAYCNVSRAAWNSTLQKISTQLRRYFNNKTIIFKNSPGGDLEVTTHSFNCGGEFFYCNTTD LFNSTWDEHGTVTNSTMANGTITLPCRIKQIINMWQRVGQAMYAPPIKGSIRCESNITGLLLTRD GGSGTNSSNETFRPIGGNMRDNWRSELYKYKVVKIEPIGLARTRAKRRVVEREKRAIGLGAAFL GFLGAAGSTMGAASMTLTVQARQLLSGIVQQQSNLLRAIKAQQHLLKLTVWGIKQLQARVLAV ERYLKDQQLLGIWGCSGKLICTTNVPWNSNWSNKSQSEIWDNMTWMQWDREVIYYTDIIYDLI EKSQNPQEKNEQDLLALDKWASLWSWFDISNWLWYIKIFIIIVGGLIGLRIVFAVLSIINRARQGY SPLSLQTLTPHPEGPDRPGRIKEEGGEQGRDRSIRLVSGFLALAWDDLRSLCLFSYRRLRDFISITA RTVELLGRSSLKGLRLGWEGLKYLGNLLGYWGQELKSSAINLIDTIAIAVAGWTDRVIEIGQRFC RAIRNIPRIRQGAEKALQ |
| A.KE..Q168. AF4071-19 | A | 190 | MKVRGIKRNLWKWGTMLLGMLMTYSVAEQLWVTVYYGVPVWKDAETTLFCASDAKAYSTE KHNIWATHACVPTDPNPQEIHLESVTEEFNMWKNNMVEQMHTDIISLWDQSLRPCVKLTPLCVT LNCTNVNNNTTNVNNNTGWDEERKNCSFNVTTELRDKRQKVYSLFYKLDVVQIDNSSYRLINC NTSAITQACPKVTFEPIPIHYCAPAGFAILKCKDEKFNGTGPCKNVSTVQCTHGIKPVVSTQLLLN GSLAEKEVMIRSENFTNSAKNILVQFKEPVKINCTRPDNNTRTSIRIGPGQAFYATGIIGDIRQAYC TVNGSEWNKALQKVVEQLRSSFENKTIIFANSSGGDLEITTHSFNCGGEFFYCNTSGLFDSTWND TDSRQENGTITLPCRIKQIINMWQRTGQAIYAPPIQGAIRCVSNITGLILTRDGGNNNSTNETFRPG GGDMRDNWRSELYKYKVVKIEPLGVAPTKARRRVVGREKRAVGIGAVFLGFLGAAGSTMGAA SITLTVQARQLLSGIVQQQSNLLKAIEAQQHLLRLTVWGIKQLQARVLAVERYLKDQQLLGIWG CSGKLICTTNVPWNSSWSNKSQSEIWENMTWLQWEKEISNYTQIIYTLIEESQNQQEKNEQDLLA LDKWASLWNWFDISKWLWHIRIFIMIVGGLIGLRIVFAVLSVVNRVRQGYSPLSFQTLLPAPRGP DRPDGIEEEGGEQGRGRSRQLVNGFSTLIWDDLRNLCLFSYHRLRDLILIAARIVELLGRRGWEAI KYLWNLLQYWIQELKNSAISLLNTTAIAVAEGTDRAIEIIQRAITAVLNIPTRIRQGFERALL |
| A.SN.01.DDJ 369.AY521631 | A | 191 | TRVMGIQRNCTPWWRWGIIFLGLIIICKATEKLWVTVYYGVPVWKDAETTLFCASDAKAYEREV HNVWATHACVPTDPNPQEIDLENVTEEFNMWKNNMVEQMHTDIISLWDQSLKPCVKLTPLCVT LNCHNVTKTNSNSNSSVKNEMPGEIKNCSYNVTTAIRDKRQNVYSLFYRVDVVPINENSNSSENS SEYRLINCNTSAITQACPKVTFEPIPIHYCAPAGFAILKCKDKDFNGTGTCRNVSTVQCTHGIKPV VTTQLLLNGSLAEGKVMIRSENLTNNAKTIIVQLDEPVYINCTRPNNNTRKSVHIGPGQAFYATG DIIGDIRQAHCNVSREDWNKTLYKVVEQLRKHFNNRTINFTAPSGGDLEITTHSFNCGGEFFYCN TSGLFNSTWYNSTWNNTDSTQKSNGTEKITLPCRIKQVIKMWQRVGQAMYAPPIQGVIKCVSNI TGILLTRDGGNLNSSFETFRPGGGNMKDNWRSELYKYKVVKIEPLGVAPTKAKRRVVEREKRA VGLGAVFLGFLGAAGSTMGAASITLTAQARQLLSGIVQQQSNLLRAIEAQQQMLKLTVWGIKQL QARVLAVERYLKDQQLLGIWGCSGKLICTTNVPWNSSWSNKSYSEIWDNMTWLQWDKEISNYT DLIYNLIEESQNQQEKNEQDLLALDKWASLWNWFNISNWLWYIRIFIMIVGGLIGLRIVFTVLSIIN RVRKGYSPLSFQTHSPSPGGLDRPRGIEEEGGEQDRDRSIRLVSGFLALAWNDLRSLCLFSYHRLR DFTLIAARTVELLGHSSLKGLRLGWEGLQYLGNLLLYWGRELKNSAISLLDAIAITVAGWTDRVI EVGQRAGRALLHIPRRIRQGLERALL |
| A.UG.07.191955 | A | 192 | MRARGTQKNCQDLWWIWGTMILGMLIICSAAGNLWVTVYYGVPVWKDAETTLFCASDAKAY STEKHNIWATHACVPTDPNPQEIDLENVTEKFNMWKNGMVEQMHTDIISIWDQSLQPCVKLTPL CVTLICIDIANDTSRNVTVEMKREIQNCSYNVTTEVRDKKQKVYSLFYKVDIVQISNNSSEYRLIN CNTSVIKQACPKVTFEPIPIHYCAPAGYAILKCKDDKFNGTGLCKNVSTVQCTHGIKPVVSTQLLL NGSLAEEEVIIRSENINNNAKTIIVQLYQPVKINCTRPGNNTRKSVHIGTGQAYFATGDIIGDIRQA YCKVNKSEWNNTLQQVVAQLRKHWNKTIIFNSSSGGDLEITTHRFNCGGEFFYCNTSSLFNSTW WSNNSNNTDSEESNSMGLNDTITLQCRIKQIINMWQRVGQAMYAPPIQGVIRCESNITGILLTRD GGDNNNESETFRPVGGDMRDNWRSELYKYKVVKIEPLGVAPTEARRRVVGRGKRAVVGMGA MFLGFLGAAGSTMGAASITLTVQARQLLSGIVQQQSNLLRAIEAQQHLLKLTVWGIKQLQARIL AVENYLKDQQLLGIWGCSGRLICTTNVKWNSTWSNKSKSEIWDKMTWLEWDREISNYTDIIYQ LIEESQNQQEKNEQDLLALDKWANLWNWFDISNWLWYIKIFIMIVGGLIGLRIVFAVLSVINRVR QGYSPLSFQTHTPSPRDLDRPGRIEEEGGEQGRDRSIRLVSGFLALAWDDLRSLCIFSYHRLRDLL LIAARTVELLGQSSLKGLRLGREKLKYLGNLLLYWGRELRISIINLYDTIAIAVAGWTDRIIEIGQR IGRAILNIPRRIRQGFERALL |
| A.GM.00.N00 6909.HQ385444 | A | 193 | MRVMGIQKNYALLWRWGTIIFWIMIICNADNNLWVTVYYGVPVWKDAETTLFCASDAKAYDQ EVHNVWATHACVPTDPNPQEIDLENVTEEFNMWKNNMVEQMHTDIISLWDQSLKPCVKLTPLC VTLNCNNITGNATYDMQREISNCSFNITTEIRDKKQKVNSLFYRLDIVPIGDNKSSEYRLINCNTS ALTQACPKVTFEPIPIHYCAPAGFAILKCKDKKFNGTGPCKNVSTVQCTHGIKPVVSTQLLLNGSL AEEGVMIRSENFTNNAKTIIVQLQEPVKINCTRPNNNTRKGVHIGPGQAFYATGGIIGNISKARQY VNTTEWIKALKEVNKTLNQQFVNKTLYFNESSGGDLEITTHSFNCRGEFFYCNTSDLFNSTWLNS TVESNETIILPCKIKQIINMWQRVGQAMYAPPIQGVINCVSNITGILLVRDGGNNNSTAETFRPGG GDMKDNWRSELYKYKVVKIEPLGVAPTRAKRRVVEREKRAVGLGAVFFGFLGAAGSTMGAAS ITLTVQARQLLSGIVQQQSNLLKAIEAQQHLLKLTVWGIKQLQARVLAVERYLKDQQLLGIWGC SGKLICTTNVPWNSSWSNKSQDEIWDNMTWLQWDREISNYTSTIYNLLEEAQNQQEKNEQDLL ALDQWASLWNWFNITKWLWYIKIFIMIVGGLIGLRIVFAVLTLINRVRQGYSPLSFQTLTHHQRE PDRPERIEEGGGEQDRDRSVRLVSGFLALAWDDLRSLCLFSYHRLRDFVLIVVRTVELLGHSSLK GLRLGWEALKYLGNLLSYWGQEIKNSAINLLNTTAIAVANWTDRVIEVGQRAGRALLHIPRRIR QGFERALL |
| A.TZ.06.3504 | A | 194 | MRVKGIQRNSQHLLRWGTIILGLIIISSAAENLWVTVYYGVPVWRDADTSLFCASDAKTYKTEV HNVWATHACVPTDPNPQEVYLKNVTENFNMWKNNMVEQMHADIISLWDQSLQPCVKLTPLCV TLDCSYNVTVRPNNTAVNYTTFMKEEMKNCSYNMTTEIRDKKKKVYSLFYRLDVVQIDEIDEN NSNNSNNTNNSLYRLINCNTSAITQACPKVSFEPIPIHYCAPAGFAILKCRDKNFNGTGPCRNVSS AQCTHGIRPVVSTQLLLNGSLAEGKEVMIRSENITSNTKNIIVQLTKPVQINCTRPSNNTRTSVHIG PGQAFYTTGEIIGDIRRAHCNVSKAKWNETLGKIAIQLEKHFKNKTIKFNPSSGGDMEITTHMFNC GGEFFYCNTTNLFNSNWTIWNGSAISNGSATANTESNDTVITLQCRIKQIINMWQRAGQAIYAPPI KGVIKCNSNITGLILTRDGGDNNSNIETFRPGGGDMRDNWRSELYKYKVVKIEPLGVAPTRARR RVVQREKRAAGLGAVFIGFLGAAGSTMGAASITLTVQARQLLSGIVQQQSNLLRAIEAQQHLLK LTVWGIKQLQARVLAVERYLKDQQLLGIWGCSGKLICTTNVPWNSSWSNKSQNEIWDNMTWL EWDKEISNYTRIIYDLIEDSQNQQEKNEQDLLALDKWASLWNWFDISKWLWYIKIFIIIVGGLIGL RIVCTVLSVINRVRQGYSPLSFQTLAPSPGDRDRPGRIEEEGGEQGRGRSIRLVSGFLALAWDDLR SLCLFSYHRLRDFILIAARTVELLGHNSLKGLRLGWEGLKYLWNLLAYWGRELKISAINLLNTIAI AVAGWTDRVIEIGQRLCRAFINIPRRIRQGFERALL |
| A.SN.96.DDJ 360.AY521630 | A | 195 | MRVMGIQRNYLPLWRWGMIFLGMMIICKATENLWVTVYYGVPVWKDAETTLFCASDAKAYD QEVHNVWATHACVPTDPNPQELDLENVTEEFNMWKNNMVEQMHTDIISLWDQSLKPCVKLTP LCVTLNCSNINSTRYDTKDKEEMKNCSFNITTELRDKRQSVYSLFYKLDVVPINNNSSNNSSNEY RLINCNTSAITQACPKVSFEPIPIHYCAPAGFAILKCKDKKFNGTGPCRNVSTVQCTHGIKPVVSTQ LLLNGSLAEEEVMIRSENLTNNAKTIIVQLVTPVQINCTRPNNNTRTSVRIGPGQAFYATGDIIGKI RQAHCTVRRAQWNTTLQQVADQLRKHFNKTINFTRPSGGDLEITTHSFNCGGEFFYCNTSGLFN STWNSTTIKQNLTNNDTGVINLPCRIRQIVHMWQRVGQAMYAPPIKGVINCTSNITGLLLTRDGG DNNSTDEKFRPGGGDMRDNWRSELYKYKVVKIEPLGVAPTRAKRRVVQREKRAVGLGAVFFG FLGAAGSTMGAASITLTAQARQLLSGIVQQQSNLLRAIEAQQHLLKLTVWGIKQLQARVLAVER YLRDQQLLGLWGCSGKLICTTAVPWNSSWSNKSQNEIWENMTWLQWDKEISNYTDIIYNLIEES QNQQEKNEQDLLALDKWANLWNWFDISKWLWYIKIFIMIVGGLIGLRIVFAVLSIINRVRQGYSP LSFQTHTPNPGGLDRPGRIEEEGGEQDRDRSIRLVSGFLALVWDDLRSLCLFSYHRLRDFTLIAAR TVELLGQGLKYLWNLLLYWGRELKSSAISLLNATAIVVAEWTDRVIEVGQRAGRALLHIPRRIR QGLERALL |
| A.KE.00.00KE | A | 196 | MRVMGTQRNWQPLLRWGTMILGMLMICSAADNLWVTVYYGVPVWKDAETTLFCASDAKAYE TEKHNVWATHACVPTDPSPQEIPLKNVTEKFNMWKNNMVEQMHTDIISLWDQSLKPCVQLTPL CVTLNCSDVTTNNTKVNTTSAPTTTATVNNDNDMKNCSYNITTELRDKKQKRYSLFYRLDVELI NKNNSNSTEYVLINCKTSTLTQACPKVSFEPIPKHYCAPAGFAILKCKDKEFNGTGLCKNVSTVQ CTHGIKPVVSTQLLLNGSLVEKRVMIRSANITDNTKNIIVQLKEPVEINCTRPNNNTRKGVHIGLG RRFYTTQVVGDIRQAYCNVSKSKWNDTLKKVVYQLRKYFNKTIIFNSSSGGDVEITTHMFTCGG EFFYCNTSGLFNSTWPVNASEQESNSTESDGIITLRCRIKQIINMWQRTGQAMYAPPIQGIIKCVSN ITGLLLTRDGGNSTVNETFRPGGGDMRDNWRSELYKYKVVKIEPLGVAPTKARRRVVGREKRA VGIGAVFLGFLGAAGSTMGAASITLTVQARQLLSGIVQQQNNLLRAIEAQQQLLKLTVWGIKQL QARVLAVERYLRDQQLLGIWGCSEKLICTTNAPWNSSWSNKSQSEIWDNMTWLQWDREINNYT DIIYRLLEDSQNQQEKNEQDLLALDKWSSLWTWFDLSNWLWYIKIFIMIVGGLIGLRIVFAVLAVI NRVRQGYSPLSFQTLTPNPGELDRPGRIEGEGGEQDRGRSIRLVSGFLALAWDDLRSLCLFSYHH LRDFILIAARIVGLLGRRGWEGLKYLWNLLIYWGQELKTSAVSLLDTIAIVVAGWTDRVLEIGQR IGRAFINIPRRIRQGFERALL |
| A.U455 | A | 197 | MRVMGIQRNYPCLWRWGTMILGLIIICNAQQLWVTVYYGVPVWKDAVTTLFCASDAKAYDAE VHNVWATHACVPTDPNPQEIDLVNVTEEFNMWKNNMVDQMHEDIISLWDQSLKPCVKLTPLC VTLDCHNITINNTNNNTNITDGVREEMKNCSFNMTTELRDKKQKVYSLFYRLDIVQINKTDNNS YRLINCNTSTITQACPKVSFEPIPIHYCAPAGFAILKCKDPEFNGKGPCRNVSTVQCTHGIKPVVST QLLLNGSLAEREIRIRSENFTNNAKTIIVQLVNPVKINCSRPYNTRKNIRRYSIGSGQAFYVTGKIIG DIRQAHCNVSRRDWNRTIQQVAEQLKKKFNNKTIIFASSSGGDIEITTHSFNCGGEFFYCNTSGLF NSIWNGSMSNDMGPNGTITLQCRIKQIINMWQRVGQAMYAPPIQGVIRCESNITGLLLTRDGGTN NTKNETFRPGGGDMRDNWKSELYKYKVVKIEPLGVAPTRAKRRVVEREKRAVGLGAIFLGFLG AAGSTMGAASITLTVQARQLLSGIVQQQSNLLRAIEAQQHLLKLTVWGIKQLQARVLAVERYLQ DQQLLGIWGCSGKLICTTTVPWNSSWSNKSQEDIWNNMTWLQWEKEISSYTGIIYQLIEESQNQQ EKNELDLLALDKWANLNWFNISNWLWYIRLFVIIVGGLIGLRIVFTVLSIINRVRQGYSPLSFQTL APIPEGLGRPGRIEEEGGEQGKDRSIRLVSGFLAIAWDDLRNLCLFSYHRLRDFALIVARAVELLG RSSLKGLRLGWEGLKYLWNLLLYWGRELKISAITLLDAVAVAVAGWIDRVIEIGQTIGRAILNIP RRIRQGLERALL |
| A.SE.SE8131 | A | 198 | MRVMGTQRNCQHLLNWGIMILGMIIICSTAENLWVTVYYGVPVWKDAETTLFCASDAKAYEKE VHNVWATHACVPTDPNPQELYLENVTEDFNMWKNNMVEQMHTDIISLWDQSLKPCVQLTPLC VTLNCSNNVTANTNSTSANLTDSVKGEMRNCSFNITTELRDKKKKVYSLFYKLDIVKINKNKSFR GKNSSGNSSSDRYRLINCNTSAITQACPKVSFEPIPIHYCAPAGFAILKCNEDEFNGTGTCRNVSTV QCTHGIRPVVSTQLLLNGSLAKEEVRIRSENISDNAKTIIVQFTKPVEIICTRPNNNTRKSIRIGPGQ AFYGMGDIIGDIRKAHCNVSRSKWNETLKKVAIQLRKYWNTTIIFTNSSGGDLEITTHSFNCGGEF FYCNTSGLFNSSWNENDTKVNYNTESNDTITLQCRIKQIINMWQRTGQATYAPPIPGVIQCRSNIT GLLLTRDGGVTNNTNNETFRPGGGDMRDNWRSELYKYKVVKLEPLGVAPTKAQRRVVKREKR AVGLAAVFFGFLGAAGSTMGAASITLTVQARQLLSGIVQQQNNLLRAIEAQQHMLRLTVWGIK QLQARVMAVERYLKDQQLLGIWGCSGKIICTTAVPWNSTWSNKSYTQIWDNMTWLQWDKEIS NYTDIIYQLIEESQYQQEKNEKELLELDKWANLWNWFDISNWLWYIKIFIMIVGGLIGLRIVFAVL TVIKRVRQGYSPLSFQIHTPSPRDPDRPGRIEEEGGEQGRDRSIRLVSGFLALAWDDLRSLCRFSY HRLRDFISIATRIVELLGQGLKYLGNLLLYWIRELKISAISLFDTIAIAVAGWTDRVIEIGQRIGRAI LHIPRRIRQGFERALL |
| A.CD.97.97C D | A | 199 | MKVRGIQRNYQHLWRWGTLIFGIIIICSAAENLWVTVYYGVPVWKDAETTLFCASDAKAYEREV HNVWATHACVPTDPNPQVMPLADVTEVFNMWMNDMVEQMHEDIISLWDQSLKPCVQLTPLC VTLHCHYVINNTAHKVSVSPEMQEKVANCSFNVTTELRDRKQKVYSLFYREDLVPIDEDKNNSN SASISTSSNSSNCSISDSNSTCINISTYRLINCNTSTITQACPKVSFEPIPIHYCVPAGFAILKCNEKEF NGTGPCKNVSSVQCTHGIKPVVSTQLLLNGSLAKETVIIRTENISDNGKNIIVQFVDPVRINCIRPN NNTRTSVRIGPGQTFYTSDIIGDIRQAYCEVNKTQWNMTLQKVADQLRKFFPNKTINFNQTSGGD LEVTTHIFNCRGEFFYCNTSSLFNSSWPINSTTSNNSTASNETIILPCRIIQIINMWQRLGQAMYAPP IKGLINCVSNITGLLLTRDGGDRNSSYETFRPIGGNMKDNWRSELYKYKVVKIEPLGVAPTKARR RVVEREKRAVGIGAFFLGILGAAGSTMGAASITLTVQARQVLSGIVQQQSNLLKAIEAQQHLLKL TVWGIKQLQARVLAVERYLKDQQLLGIWGCSGKLICTTNVPWNSSWSNKSQGEIWDNMTWMQ WDREISNYTDIIYKLIEESQEQQEKNELKLLALDQWASLWNWFDISNWLWYIRIFIMIVGGLIGLR IVFAVLSVVNRVRQGYSPLSFQTLTPSPEGLDRPGKIEEGGGEQDRTRSIRLASGFLALAWEDLRS LCLFSYHRLRDFILIAARTVELLGHSSLKGLRLGWEGLKYLWNLLTYWCQELKTSAISLFDTLAI AVAEWTDRIIEVGHRIGRAFLNIPRRIRQGFERALL |
| A.SE.UGSE8 891 | A | 200 | MRVMGTQMNWQNLWRWGTMILGIIIICSAAENLWVTVYYGIPVWRDAETTLFCASDAKAYDT EVHNVWATHACVPTDPNPQEINLENVTEEFNMWKNNMVEQMHTDIISLWDQSLKPCVKLTPLC VTLNCSSVTNSSVTNITSDMAGEIKNCSFNMTTEIRDKRQKVHALFYRLDVVPMDNNNSLYRLIN CNTSAITQACPKVSFEPIPIHYCAPAGFAILKCNDKEFNGTGPCKNVSSVQCTHGIRPVVSTQLLL NGSLAETEVMIRAENITNNIKNIIVQFNKSVEIICIRPNNNTRKSIRIGPGQAFYATGDIIGDIRQAYC DVNRTEWNEALQKVVNQLKTHFKNKTIIFNSSSGGDLEITTHSFNCGGEFFYCNTSGLFNSTWNG TDSMQKLNSTGNITLPCRIKQIINMWQRAGQAIYAPPIQGVIRCESNITGLILTRDGGNDNNESEIF RPGGGDMRDNWRSELYKYKVVKIEPLGVAPTKAKRRVVEREKRAIGIGAVFIGFLGAAGSTMG AASITLTVQARQLLSGIVQQQSNLLMAIEAQQHLLKLTVWGIKQLQAQVLAVERYLRDQQLLGI WGCSGKLICTTTVPWNSSWSDVSQSEIWENMTWLQWDKEISNYTQIIYSLIEESQNQQEKNEQD LLALDKWANLWNWFDISKWLWYIKIFIMIVGGLIGLRIVIAVISIINRVRQGYSPLSFQIHTPSPGG LDRPGRIEEEGGEQDRNRSIRLVNGFLALAWDDLRSLCLFCYRRLRDFILIVARTVELLGHSSLRG LRLGWEGLKYLKNLLSYWGRELKLSAINLLDTIAIVIAGWTDRVIEIGQGFCRAIFPRRSKQGLKR ALQ |
| A.GM.05.N33 456.HQ38545 2 | A | 201 | MRVMGTQKNCPLLWRWTLIFGLIIICSATGKLWVTVYYGVPVWRDAETTLFCASDAKAYDTEV HNVWATHACVPTDPSPQEVDLTNVTENFNMWKNNMVEQMHTDIISLWDQSLKPCVKLTPLCV TLNCSNITSTSSNNNSSNITNASSSSSNNSSNSTVDERNKVMTNCSFNMTTELRDKIQKVHSLFYR SDVVGINGNKEYRLINCNTSTITQACPKVSFEPIPIHYCAPAGFAILKCKDKEFNGTGPCKNVSSV QCTHGIKPVVSTQLLLNGSLAEEGIRIRSENLTNNAKIIIVQLTKPVRINCTRPYNNTRKSIRIGPGQ SWYATGDIIGNIRQAFCNVSKTDWNTALQQVAAQLGRHFNNKKITFKNSSGGDLEITTHSFNCR GEFFYCNTSGLFSGSWYNGTWANDSWSAGPGTNDNITLPCKIKQIIRMWQRVGQAMYAPPIQGE INCVSNITGLLLTRDGGNSSEEIFRPGGGDIRDNWRSELYKYKVVKIEPLGVAPTRARRRVVGRE KRAVGLGAVFLGFLGAAGSTMGAASITLTVQARQLLSGIVQQQSNLLRAIEAQHHLLKLTVWGI KQLQARVLAVERFLKDQQLLGIWGCSGKLICTTNVPWNSSWSNKSQEQIWNNMTWLQWDREI DNYTQIIYNLIEESQNQQEKNEQDLLALDKWASLWTWFDISNWLWYIKIFIMIVGGLIGLRIVFAV LSIMNRVRQGYSPLSFQTHTPVVPTGPDRPGRIEEESGEQDRNRSIRLVSGFLALAWDDLRSLCLF SYRHLRDFILIAARAVELLGHSSLKGLRLGWEGLKYLWNLLVYWGRELKNSAISLLDTVAVAVA GWTDRVIEIGQRVGKAIIHIPRRIRQGLERALL |
| A.CM.99.99C M | A | 202 | MRVMGTQRNYPPLWKLGIMVLGMIIICCNAADNLWATVYYGVPVWKDAETTLFCASDAKAYK TEMHNVWATHACVPTDPNPQEIKLDNVTEEFNMWKNNMVEQMHADIISLWDQSLKPCVQLTP LCVTLNCSDVKVNTTKNNGTNSSNVLVINGTENGQEELKNCSYNVTTELRDKKQRVYSLFYRL DIVPINEDNKSSDNSSGEYRLINCNTSAITQACPKVSFEPIPIHFCAPAGFAILKCRDKNYNGTGLC KNVSTVQCTHGIKPVVSTQLLLNGSLAEGEIKVRSENFTNNAKTIIVQLDQPVIINCTRPNNNTRR SVRIGPGQAYYATGEIIGDIRKAYCTVNKTAWNETKHKVMEKLRGIYHRPIKFNSSSGGDLEITT HMFNCGGEFFYCNTSGLFAQELKNDTNDNNTIILLCKIKQIINMWQRVGQAMYAPPRAGVIRCV SNITGLLLTRDGGKNDNGTNGTEIFRPGGGDMRDNWRSELYKYKVVRIEPVGVAPTRAKRRVV EREKRAVGLGAVFLGFLGAAGSTMGAASLTLTVQARQLLSGIVQQQSNLLRAIEAQQHMLRLT VWGIKQLQARVLAVERYLRDQQLLGIWGCSGKLICTTNVPWNSSWSNKSYEDIWDNMTWLQW DKEISNYTALIYDLIEESQNQQEKNEKDLLELDKWASLWNWFSISQWLWYIKIFIMVVGGLIGLRI IFAVLSLVNRVRQGYSPLSFQTHPPAPVGLDRPGGIEEEGGERGRDRSIRLVSGFLALAWDDLRN LCLFCYHRLRDFILIAARTVELLGRNSLKGLRLGWEGLKYLWNLLAYWGRELKISAINLLDTIAI AVGGWTDSLIELTQRIGRAILHIPRRIRQGFERALL |
| A.KE.09.1215 1802.HQ5406 89 | A | 203 | MRVMGIQMNYQHLLTWGTIILGMILICSPAENLWVTVYYGVPVWREADTTLFCASDAKAYETE KHNVWATHACVPTDPNPQEIHLANVTENFDMWKNNMVEQMHTDIISLWDQSLQPCVKLTPLC VTMNCSEPKFNNTNFNSTGSNSTGIQEEMRNCSFNMTTELKDKKKEVYSLFYRLDIVQIDSKKG NSSDYRLINCNTSAVKQACPKVSFNPIPIHYCAPAGFAILKCRDEDFNGTGPCKNVSTVQCTHGI MPVVSTQLLLNGSLAKENVQIRSENISNNAKIILVQLAHPVRINCTRPGNNTRKSIHMGPGQAFY ARGDVIGDIRQAYCNVSSSEWSNTLYKVAEQLRKHYGNETTIKFTNHSGGDLEVTTHSFNCGGE FFYCNTTNLFNSSIPFNASERANNTNSTDDIITLQCRIKQIVRMWQRVGQAMYAPPIPGVIRCESNI TGLMLTWDGGSKNITEGNRTETFRPGGGDMRDNWRSELYKYKVVKIEPLGVAPTKARRRVVG REKRAVGIGAVFLGFLGAAGSTMGAASVTLTVQARQLLSGIVQQQSNLLRAIEAQQQLLKLTVW GIKQLQARVLALERYLKDQQLLGIWGCSGRLICTTNVPWNSSWSNKSYNEIWDNMTWLQWDR EIENYTQIIYGLIEESQNQQEKNEQDLLSLNKWADLWSWFNITNWLWYIKIFIMIVGGLIGLRIVF AVLSVINRVRQGYSPLSFQTHLPNPGGLDRPERIEEEDGEQGRTRSIRLVSGFLALAWDDLRSLCL FSYHRLRDFILIAARTVELLGHSSLKGLRLGWEGLKYLWNLLVYWGRELKTSAISLVDTIAIVVA GWTDRAIEIGQRIGRAILHIPIRIRQGLERALL |
| B.US.85SFM HS21 | B | 204 | MRVKGIRKNYQHLWRWGTMLLGMLMICSAAEPEQLWVTVYYGVPVWKEATTTLFCASDAKA YDTEVHNVWATHACVPTDPSPQEVALENVTENFNMWKNNMVEQMHEDIISLWEQSLKPCVKL TPLCVTLNCTDELNNVTSRIGEKMKGEIKNCSFNITTSIRDKVKKYALFYKLDVVPIDNDNTTTNT TANYRLINCNTSVITQACPKVSFEPIPIHYCAPAGFAILKCKDKKFNGKGLCTNVSTVQCTHGIRP VVSTQLLLNGSLAGEEVVIRSDNFTNNAKTIIVQLKEAVEINCTRPNNNTRKSIHIQPGRAFYTTG DIIGDIRQAHCNLSKANWNNTLKQIVIKLRQQFNNKTIVFNHSSGGDPEIVMHSFNCGGEFFYCNS TQLFNSTWPFNSTLNDTSTEGNATITLPCRIKQVVNTWQEVGKAMYAPPIRGQIRCSSNITGLLLT RDGGTSENGTNEIEIFRPGGGDMRDNWRSELYKYKVVKIEPLGVAPTKAKRRVVQREKRAVGTI GAMFLGFLGAAGSTMGAASITLTVQARLLLSGIVQQQNNLLRAIEAQQHLLQLTVWGIKQLQAR VLAVERYLRDQQLLGIWGCSGKLICTTAVPWNTSWSNKSLDKIWNNMTWMEWEREIDNYTSLI YTLIEESQNQQEKNEQELLELDKWASLWNWFDITKWLWYIKIFIMIVGGLVGLRIVSTVLSIANR VRQGYSPLSFQTRLPAPRGPDRPEGIEEEGGERDRDRSGRLVNGLLALIWDDLRSLCLFSYHRLR DLLLIAARIVELLGRRGWGILKYWWSLLQYWSQELKNSAVSLLNATAIVVAEGTDRVIEVVQRI YRAFLHIPRRIRPGLERALL |
| B.US.00.WITO | B | 205 | MKVMGTKKNYQHLWRWGIMLLGMLMMSSAAEQLWVTVYYGVPVWREANTTLFCASDAKA YDTEVHNVWATHACVPTDPNPQEVVMGNVTEDFNMWKNNMVEQMHEDIISLWDQSLKPCVK LTPLCVTLHCTNVTISSTNGSTANVTMREEMKNCSFNTTTVIRDKIQKEYALFYKLDIVPIEGKNT NTSYRLINCNTSVITQACPKVSFEPIPIHYCAPAGFAILKCNNKTFNGKGPCRNVSTVQCTHGIKPV VSTQLLLNGSLAEEDIIIRSENFTNNGKNIIVQLKEPVKINCTRPGNNTRRSINIGPGRAFYATGAII GDIRKAHCNISTEQWNNTLTQIVDKLREQFGNKTIIFNQSSGGDPEVVMHTFNCGGEFFYCNSTQ LFNSTWFNNGTSTWNSTADNITLPCRIKQVINMWQEVGKAMYAPPIRGQIDCSSNITGLILTRDG GSNSSQNETFRPGGGNMKDNWRSELYKYKVVKIEPLGIAPTRAKRRVVQREKRAVTLGAVFLG FLGAAGSTMGAASLTLTVQARLLLSGIVQQQSNLLRAIEAQQHMLQLTVWGIKQLQARVLAIER YLKDQQLLGIWGCSGKLICTTTVPWNTSWSNKSYDYIWNNMTWMQWEREIDNYTGFIYTLIEES QNQQEKNELELLELDKWASLWNWFNITNWLWYIKLFIMIIGGLVGLRIVCAVLSIVNRVRQGYS PLSFQTRLPNPRGPDRPEETEGEGGERDRDRSARLVNGFLAIIWDDLRSLCLFSYHRLRDLLLIVA RVVEILGRRGWEILKYWWNLLKYWSQELKNSAVSLLNVTAIAVAEGTDRVIEIVQRAVRAILHIP TRIRQGFERALL |
| B.CN.RL42 | B | 206 | MRVTGIRKNYQHLWRWGTMLLGMLMICNAAENLWVTVYYGVPVWKEATTTLFCASDAKAY DTEVHNVWATHACVPTDPNPQEVVLGNVTENFNMWKNNMVEQMHEDIISLWDQSLKPCVKLT PLCVTLNCTNLKNATNTSSTMEGGEIKNCSFNITTSIKTKVKDYALFYKVDVVPIGNDSTSYRLIN CNTSVITQACPKVSFEPIPIHYCTPAGFAIIKCNNKKFNGTGPCTNVSTVQCTHGIRPVVSTQLLLN GSLAEEEVVIKFSNFTDNARVIIVQLNESVEIKCIRPNNNTRKSIHLGPGKAWYTTGQIIGDIRQAH CNLSSTKWNNTLKQITKKLREQFGNKTIVFNQSSGGDPEIVMHSFNCGGEFFYCNTSQLFNSTWN DTGTWNDTTGNSTITLPCRIKQIVNMWQEVGKAMYAPPIEGQIRCSSNITGLLLTRDGGNNESKP TETFRPGGGDMRDNWRSELYKYKVVKIEPLGVAPTKARRRVVQREKRAVGTIGAMFLGFLGAA GSTMGAASITLTVQARQLLSGIVQQQRNLLRAIEAQQHLLQLTVWGIKQLQARVLAVERYLKDQ QLLGIWGCSGKLICTTAVPWNASWSNKSLHEIWNNMTWMEWEREIDNYTREIYTLIEESQNQQE KNELELLELDKWASLWNWFDITKWLWYIKIFIMIVGGLVGLRIVFAVLSIVNRVRQGYSPLSLQT RFPAQRGPGRPEGIEEEGGERDRDRSERLVTGFLSLIWEDLRSLCLFSYHRLRDLLLIVARIVELLG RRGWEVLRYWWNLLQYWIQELKNSAIGLLNATAIAVAEGTDRVIEVVQRAYRAILHIPTRIRQG LERALL |
| B.CN.06.CNH LJSM06059.E U131799 | B | 207 | MTVMGIRKNCQHWLTWGTMLLGMLIICSAEDLWVTVYYGVPVWKEASTTLFCASEAKAYSTE VHNVWATHACVPTDPNPQEVVLGNVTENFNMWKNNMVEQMQEDVISLWDESLKPCAKLTPL CVTLNCTDWKSNTTNTSSTTNATQSSWENMNGEIKNCSFNVTTQIRNKVRKEQALFNSLDVVPI DNNNSMYMLTNCNTSVITQACPKISFEPIPIHYCTPAGFAIIKCNDKTFNGTGPCTNVSTVQCVHG IRPVVSTQLLLNGSLAEEDMVIRSENFTNNAKTIIVQLNETVKIECIRPNNNTRKSIQLVGGRSLYA TGDIIGDIRQAHCNVSAQNWTETLKRVAIKLRDQFEHKNKTIFLRPPSGGDPEIVMLTLNCGGEFF YCNTTKLFNHNLTENNTWQENASESTITLPCRIKQIINRWQEVGRAMYAPPISGQIKCSSNITGLL LTRDGGKTNGSENGSEIFRPGGGDMRDNWRSELYKYKVVKIEPLGIAPTKAKRRVVQREKRAV GTIGAMFLGFLGAAGSTMGAASMTLTVQARLLLSGIVQQQNNLLRAIEAQQHMLQLTVWGIKQ LQARVLAVERYLRDQQLLGIWGCSGKLICTTAVPWNASWSNKSTDEIWGNMTWMQWEREIDN YTELIYTLLEKSQNQQEKNEQELLELDKWANLWSWFDISNWLWYIRIFIMIVGGLKGLRIIFTVFS IVNRVRQGYSPLSFQTRLPAQKGPDRPEETEEGGGERDRGRSTRLVHGFLALIWDDLRSLCLFSY HRLIDLLSIVARIVELLGRRGWEALKYWWNLLQYWSQELKKSAVSLLNATAIAVAEGTDRVIAV IQAIGRAIIHIPRRIRQGAEIALL |
| B.SE.11.SE60 0034.KP4118 25 | B | 208 | MKVKETRKSCQHLRGILLLGMLMICSAEDQLWVTVYYGVPVWKEATTTLICASDAKAYDTEAH NVWATHACVPTNPNPHEVVLENVTEKFNMWKNNMVEQMHEDIISLWDQSLKPCVKLTPLCVT LHCTNLTNSTTTSISPTMSIQKIKNCSFNTTTSMRDKIEKKHALFYDLDVVQIDNDKNKTNTRYRL RHCSTSTLACAWTNIDSVPIPYCARAAFGIAIRKCKDFDVNGPCPCVNAAQWTPGLKLKVSIPLL LIVILSEGDIDIASDTISDNAKTIIVQLNETVQISCTKPNNNTRKSIHIGPGRAFYATGDIIGDIRQAY CNVSGKAWNKTLGKVVIKLREQFINKTIVFRRSSGGDPEIVLHNFTCGGEFFYWNTTSLFDSTWN TNATWDRNWNDTSGENDIITLPCRIKQIIYMGQEVGKAMYAPPITGLISCSSNITGLLLIGCGGES YNDEIFTPGGGNMKDYWRSELYKYDVQEIEPLLVAPTKAKRIVVHREKRAFRFGSMFLGFLEAP GSTMGAASITLTVQAILLLSGILHQHSNLLKAIEAHQLLLLVTVSLISNLQCRIPAEQRQLKYRQLL GICCCSGKIHYAPAAPWNTSWSNRSRDHILHNKTCRQWEKEIDNYTSLIYTLIEESQNQQEKNEL DFVELDKWASLRNWFDKSNWLWYIKIFIMIVGVLIGLRIMFAVLSIVNRVRQGSSSLSCQTRFPA PRGPDRPEGIEEDGGEQGRDRSIRLVDGFLALIWDDLRSLCLFSYHRLRDLLLIAARVVELLGRRG REILKYWWNLLQYWTQELKKSAISLFNATAIAVAEGTDRVIELLQRTGRAILHIPVRIRQGLERLL L |
| B.JP.00.117.A B428551 | B | 209 | MRVRGIRKNYRHLWRWGTMLLGMLMICSAADKLWVTVYYGVPVWKEAPTTLFCASDAKAYK TEMHNVWATHACVPTDPNPQEVLLGNMTENFNMWENNMVEQMQEDVISLWDQSLKPCVELT PLCVTLNCTDYNGNNTNSTHTNSSSSEGEKLEKGEIKNCTFNITTNIGDKQRDYAFFYKLDLEPIN KDNASYRLISCNTSVITQACPKVSFEPIPIHYCTPAGFALLKCNDKKFNGTGPCTNVSTVQCTHGI RPVVSTQLLLNGSLAEEEVVIRSANFSNNGKIIIVQLNETVEINCTRPNNNTRKGIQLGQGRALYA TGDIIGDIRQAYCTINGTKWNKTLNQIAEKLREQFGENKTIKFQPSSGGDPEVVMHSFNCGGEFF YCNTSSLFNWSTNGSNITNGSNITKTIPCRIKQIINMWQKVGKAMYAPPIRGQINCLSNITGLLLTR DGGNQTEFNNTEIFRPGGGDMRDNWRSELYKYKVVKIEPLGVAPTKARRRVVQREKRAVGTIG AMFLGFLGAAGSTMGAASLTLTVQARLLLSGIVQQQNNLLRAIEAQQHLLQLTVWGIKQLQAR VLAVERYLQDQQLLGIWGCSGKLICTTNVPWNSSWSNKTLNQIWENMTWMQWEREIDNYTREI YSLIEEAQNQQEKNEQDLLELDKWDNLWSWFSITKWLWYIKIFIMIVGGLIGLRIVFAVLSIVNRV RQGYSPLSLQTRLPAQRGPDRHEETGEEGGERDRDTSGRLVTGFLALIWDDLRSLFLFSYHHLRD LLLIVARIVELLGRRGWEVLKLWWNLLQYWSQEIKNSAVSLLNATAIIVAEGTDRIIEIAQRTYRA ILHIPTRIRQGLERALL |
| B.CN.12.2097 | B | 210 | MRVTGIRKNYQHLWRWGTLLLGMLMICSAAEKLWVTVYYGVPVWKDANTTLFCASDAKAYA TEVHNVWATHACVPTDPNPQEVVLGNVTENFDMWKNDMVEQMHEDIISLWDQSLKPCVKLTP LCVTLNCTNVRPLNCTDGKPPNCTAVEPSTSGNNSSATGGGEMKNCSFNVTTSIRTKKTYATFY NLDIVKINDDNDSTSTSYRLINCNTSVITQACPKVSFEPIPIHYCTPAGFAILKCNNKTFNGKGPCT NVSTVQCTHGIRPVVSTQLLLNGSLAEGEVVIRSSNFTDNAKIIIVQLNEPVVINCTRPNNNTRKSI PLGPGQAWYTTGDIIGNIRQAHCNLSRTEWNNTLRQITKKLREHIGNKTITFNQSAGGDPEIVMH SFNCGGEFFYCNTSQLFNSTWPWNDTSTGNDTTGNSTITLPCKIRQIVNRWQEVGKAMYAPPIAG PISCSSNITGLLLTRDGGNGSADNETFRPGGGDMRDNWRSELYKYKVVQIEPLGVAPTKAKRRV VQREKRAVGTIGAMFLGFLGAAGSTMGAASITLTVQARQLLSGIVQQQRNLLRAIEAQQHLLQL TVWGIKQLQARVLAVERYLKDQQLLGIWGCSGKLICTTAVPWNASWSNKSLSQIWDNMTWME WEREINNYTKEIYTLIEQSQNQQEKNELELLELDKWASLWNWFDITNWLWYIRIFIMIVGGLVGL RIVFAVLSIVNRVRQGYSPLSLQTRFPTQRGPGRPEGIEEEGGEQDRDRSERLANGFLTLFWEDLR SLCLFSYHHLRDLLLIVARIVELLGRRGWEALKYWWNLLQYWIQELKNRAVSLFNATAIAVAEG TDRVIEVVQRAFRAVLNIPRRIRQGLERSLL |
| B.ES.04.3571 84 | B | 211 | MRVKGIRKNYQHLWRWGTMLLGLLIICSAAERLWVTVYYGVPVWKEATVTLFCASDAKAYDT EVHNVWATHACVPTDPNPQEVVLENVTENFNMWKNNMVEQMHEDIISLWDQSLKPCVKLTPL CVTLNCTDWRNATNTTSSSGGTIEGGEIKNCSFNITAGIRDKVQKEYALFYKLDVVPIDNDNSSY RLISCNTSVITQACPKVSFDPIPIHYCAPAGFAILKCNDKKFDGKGPCTNVSTVQCTHGIRPVVSTQ LLLNGSLAEEEVVIRSDNFTDNAKTIIVQLNESIEINCTRPNNNTRKSIHIGPGRAFYTTGEITGDIR QAHCNLSRAKWDNTLKQIADKLRKQLKNKTIVFNQSSGGDPEIVMHSFTCGGEFFYCNSTKLFN STWNGTEGSNNSGTRANNSHILTLPCRIKQIINMWQEVGKAMYAPPIRGQIRCSSNITGLLLTRDG GNNNSGTEIFRPGGGDMRDNWRSELYKYKVVKIEPLGVAPTKAKRRVVQREKRAVGIGAVFLG FLGAAGSTMGAASMTLTVQARLLLSGIVQQQNNLLRAIEAQQHLLQLTVWGIKQLQARVLAVE RYLKDQQLLGIWGCSGKLICTTTVPWNTSWSNKSLAEIWNNMTWMEWEREINNYTSLIYNLIEE SQNQQEKNEQELLELDKWASLWNWFDITKWLWYIKIFIMIIGGLVGLRIVFTVLSIVNRVRQGYS PLSFQTHLPAPRGPDRPEGIEGEDGERDRDRSGRLVGGFLALFWVDLRSLCLFSYHRLRDLLLIVT RIVELLGRRGWEVLKYWWNLLQYWSQELKNSAVSLLNAAAIAVAEGTDRVIEILQRAGRAILHI PTRIRQGLERALL |
| B.US.06.7010 10068 | B | 212 | MKVMETRRSWLPLWRGGILLLGILMICSTENLWVTVYYGVPVWKEATTTLFCASDAKAYSSEK HNIWATHACVPTDPSPQEIPLENVTENFNMWKNDMADQMHEDIISLWDQSLKPCVKLTPLCVTL NCTNLNCTSSQNHNTTCRNVSKEMEGEIKNCSFYTTTNIRDRVTKNYALFYSLDIVPIDDNNNTN ISYRLTSCNTSIITQACPKVSFEPIPIHYCAPAGFAILKCNDQEFNGTGPCKNVSTVQCTHGIRPVVS TQLLLNGSLAEKDIVIRSSNISDNTKTIIVQLKEAIIIDCIRPNNNTRRSIHIGPGRAFYGTGDIIGNIR QAYCSINISKWDETLRQIAGKLGEQLNKTKIITFKNSSGGDPEITMFNFNCGGEFFYCNSTQLFNSI WTKNDNGIWTKNETNGNWTRSGSTNDNDTIILPCRIRQIINMWQEVGKAMYAPPIRGNISCSSNI TGLLLTRDGGNNNDSANETFRPGGGNMKDNWRSELYKYKVVEIEPLGLAPTRAKRRVVQREKR AALGALFLGFLGAAGSTMGAASVTLTVQARLLLTGIVQQQNNLLKAIEAQQHLLQLTVWGIKQ LQARVLSIERYLKDQQLLGIWGCSGKLICTTAVPWNTSWSNKSVDMIWHNMTWMEWEREIDN YTGLIYKLLEASQNQQEQNEQELLELDKWASLWNWFDITNWLWYIKIFIMIVGGLIGLRIVFTVL SIVNRVRKGYSPLSFQTHRPAPRGPDRPEGIEEEGGERDRDTSGPLVTGFLAIIWVDLRNLCLFSY HRLRDLLLIVARIVELLGRRGWEALKYWWNLLQYWIQELKNSAVSLYNTIAIAVAEGTDRIIEVL HRIWRAILNIPRRIRQGLERALL |
| B.CU.14.14C U005.KR9146 76 | B | 213 | MRVKGIRKNYPHWWRWGIMVLGMLMIWSAKEQLWVTVYYGVTVWKGLPVWKNATTTLFCD TELHNVRDKCACVPTDPNPQEVVMGNVTEYFNMWKNDMVEQMHEDIISLWDRSLKPCVIFTPL CVTLNCNDLKNVTNTNCSSGRGIVEKLDISNCSVSISTFITGHVVIADEVMDTDDILLVSIDSINNK LTLCNTSLMTQACRSERCKPIPIHYCAPAGFAILKCNNKTFNGKGPCKNVSTVQCTHGIKPVVST QLLLNGSLAEEEVVIRSENFTDNTKTIIVQLNESVVINCTRPSNNTRKSIRIHRGPGQAFFATGKIG DIRQAHCNLSRAEWNKTLAQIAKKLGKQFGENKTISFQPSSGGDIEIVTHSFNCGGEFFYCNTTPL FNSSWYKSNWKSQRTKEIGGRETITLQCRIKQIVNMWQEVGRAMYAPPIKGQIRCSSNITGVLLT RDGGNTTNHSEIFRPGGGNMKDNWRSELYKYKVVQIEPLGIAPTKAKRRVVQREKRAIGFGAVF LGFLGAAGSTMGAASVTLTVQARQLLSGIVQQQSNLLNAIQAQQHLLQLTVWGIKQLQARVLA VERYLKDQQLLGIWGCSGKLICTTAVPWNASWSNKSLEYIWDNMTWMEWEREINNYTRLIYHL IEESQNQQEKNEKELLELDKWASLWNWFDITKWLWYIKIFIMIVGGLIGLRIVFVVLSIINRVRQG YSPLSFQTRHPAQRGPDRPEGIEGEGGERDRGGSERLVDGFLAIIWVDLRSLCLFSYHRLRDLLSI LTRIVELLGRRSWEALKYCWNLLQYWSQELKNSAVSLLNATAVAVAEGTDRIIEIVQRACRAFL HIPRRIRQGFERLLL |
| B.US.06.CH1 06 | B | 214 | MRAKEMKKHWQRSWKGGILLLGMLMICSATAPDKWVTVHYGVPVWREAKTTLFCASDAKAY STEKHNVWATHACVPTDPNPQEVVLGNVTENFNMWKNDMVEQMHEDIISLWDQSLKPCVKLT PLCVTLNCIDEITNRTNNTHKDGQLMDKGEIKNCSFNTTTSVQDKLQKVSALFYKPDVVRIGDN NNSSNSSSYRLINCNTSIITQACPKVSFEPIPIHYCTPAGFAILKCNDKNFNGSGLCTNVSTVQCTH GIRPVVSTQLLLNGSLAENEVVIKSENFTDNAKTIIVQLKEAVQINCTRPNNNTRKSIHIGPGRAFY ATGNIIGDIRKAYCTINITKWNKTLEQIVDKLRDKFGENKTIVFNSSAGGDPEIVTHSFNCGGEFFY CNTTQLFNSTWNLNSTWNGTKNLNNTTRNDTIILPCRIRQIINLWQKVGKAMYAPPIRGQINCTS NITGLLLTRDGGNTSETNTTEVFRPGGGNMRDNWRSELYKYKVVEIEPIGIAPTKAKRRVVQRE KRAVGIGAVFLGFLGAAGSTMGAASVALTVQARQLLSGIVQQQNNLLRAIEAQQHMLKLTVWG IKQLQARVLAVERYLRDQQLLGIWGCSGKLICTTAVPWNNSWSNKSLEYIWGNMTWMEWEREI DNYTGLIYNLIEESQLQQEKNEQELLELDKWASLWNWFDITNWLWYIKIFIMIVGGLVGLRIVFA VLAIANRVRQGYSPLSLQIRPPAQRGPDRPEGIEEEGGDRDRDTSTRLVHGFLALVWDDLRSLCL FSYHRLRDLLLIVTRIVELLGHRGWEALKYWWNLLQYWIQELKSSTVSLFNYTAIAVAEGTDRII EIVGRVFRAFIHIPRRIRQGLERLLL |
| B.KR.93.93JH S5 | B | 215 | MRVKGIRKNYQHWWRWGIMLLGMWMICSADEMWVTVYYGVPVWKEATTTLFCASDAKAYD TEVHNVWATHACVPIDPNPQEVELVNVTENFNMWKNNMVEQMHEDIISLWDQSLKPCVKLTPL CVTLNCSNLRNETNIKNATNSSSGKVMEKGEMKNCSFNVTTNIGNKVQKEYALFYKLDVVQID NTSYTLINCNSSIITQACPKISFEPIPIHYCTPAGFAILQCNDKKFNGSGPCSNVSTVQCTHGIRPVV STQLLLNGSLAEEEVVIRSENFTNNAKTIIVQLNASVEINCTRPNNNTRKSIHLGPGSTIYATGDIIG DIRQAHCNISETKWNNTLRQIVIKLREQFGNKTTIVFNQSSGGDPEIVMHSFNCGGEFFYCNTAQL FNSTWIWNDTKGLNKTAENGTFTLPCRIKQIINRWQEVGKAMYAPPIRGQIRCSSNITGLLLTRD GGAESRNGTEIFRPGGGDMRDNWRSELYKYKVVRIEPLGIAPTKARRRVVQREKRAVGTIGAMF LGFLGAAGSTMGAASMTLTVQARLLLSGIVQQQNNLLRAIEAQQHLLQLTVWGIKQLQARVLA VERYLKDQQLLGIWGCSGKLICTTNVPWNTSSNKSQNEIWDNMTWMQWDREINNYTNLIYTLL EESQNQQEKNEQELLELDKWSNLWNWFSISNWLWYIRIFIMIVGGLIGLRIIFSVLSIVNRVRQGY SPLSFQTHFPSPRGPDRPEGIEGEGGERNRDGSGPLVNGFLTLIWVDLRSLCLFIYRRLRDLLLIVA RSVELLGLRGWEVLKYWWNLLHYWSQELKKSAVSLLHATARAVAEGTDRAIEILQRAYRAILH IPRRIRQGLERALL |
| B.US.NL43E9 | B | 216 | MRVKEKYQHLWRWGWKWGTMLLGILMICSATEKLWVTVYYGVPVWKETTTTLFCASDAPAY DTEVHNVWATHACVPTDPNPPEVVLVNVTENSHMWTNDMVEQMHEDIISLWDQSLKPCVKLT PLCVSLKCTDLKNDTNTNSSSGRMIMEKGEIKNCSFNIGTSIRDKVQKEYAFFYKLDIVPIDNTSY RLISCNTSVITQACPKVTFEPIPIHYCAPAGFAILKCNNKTFNGTGPCTIVSTVQCTHGIRPVVSTQL LLNGSLAGEDVVIRSANFTDNAKTIIVQLNTSVEINCTRPYNNKRKSIRIQRGPGRAFVTIGKIGN MRQAHCHISRAKWNATLKQIASKLREQFGNNKTIIFKQSSGGDPEIVTHSFNCGGEFFYCNSTQL FNSTWFNSTWSTEESNNTEGSDTITLPCRIKQFINMWQKVGKAMYAPPISGQSRCSSNITGLLLTR DGGNNNNGSQIFRPGGGDMRDNWRSELYKYKVVKIDPLGVAPTKAKRRVVQREKRAVGIGAL FLGFLGAAGSTMGAASMTLTVQARQSLSGIVHQQNNLLRAIEAQQHLLQLTVWGIKQLQARILA VERYLKDQQLLGIWGCSGKLICTTAVPWNASWSNKSLEQIWNNMTWMEWDREINNYTSLIHSL IEESQNQQEENEQELLELDKWASLWNWFNITNWLWYIKLFIIIVGGLVGLRIVFAVVSIVNRVRQ GYSLLSFQTHLPIPRGPDRPEGIEEEGGERDRGRSIRLVNGSLALIWDDLRSLCLFSYHRLRDLLLI VTRIVELLGRRGWEALKYWWNLLQYWSQELKNSAVNLLNATAIAVAEGKDRVIEVLQAAYRAI RHIPRRIRQGLERILL |
| B.SE.11.SE60 0023.KP4118 24 | B | 217 | MIVMGIKKNYQHLWTWGMMLLGMLMICSTAPSLWVTVYYGVPVWKEATTTLFCASDAKAYE KEAHNIWATHACVPTDPNPQEVVLENVTEDFNMWKNNMVEQMHEDIISLWDQSLKPCVKLTPL CVTLNCTDPWYDNTANRTTERMIMEKGEIKKCSIEVNTCLIDVWMEHCALLSKLHLVRLENTTF IYMQTSRHSSKRTKGCPRYSIEPIPLQYSAPAGSAILIRKNDMVHGTGTCTHVPPVQCSNGIRPVV SSQHPSHDISGAAVLTKDNFKDTVRTIIVQLNDSVPINCTRPNNNTRKSIHIGPGRAFYATGDIIGDI RQAHCNISLVKWNNTLEKIVTKLREQFKNKTIVFSPPSGGDPEIVSHSFNCGGEFFYCNSTPLFNS TWDTNGTCKGSNSADWNVTVPCISIPITTMGQKLEQAMDVRPIAGKAICSSSLSKGILLANSHNSI IVPRLVMPKGKDKKFNWNRKMYQCQVQCQIEPLGVAPTMAKRRVVQREKRDLGLGALFLGFL GAAGSTMGAASITLTVQARQLLSGIVQQQNNLLRAIEAQQHLLQLTVWGIKQLQARVLAVERYL KDQQLLGIWGCSGKLICTTTVPWNISWSNKTLEQIWDNMTWMQWDKEIHNYTELIYTLIEESQI QQEKNEQELLELDKWSNLWNWFDITNWLWYIKIFIMIVGGLIGLRIVFVVLSIINRVRKGYSPLSF QTLLPVPRGPDRRFPIGEGDGERDRDRDSRSANGLLAIINVDMRILSLCIFLYRRDLLLIVMMTVE LLGRRGWETLIYCLNLNLYLSQNLKHKAISIINVTATAVAVAADTVIDTVPAAGRGILLILRRIRQ GLERDLL |
| B.US.09.9036 | B | 218 | MRVKGIRKNWQHWWWKWGIMLLGMWMICSTEEEKLWVTVYYGVPVWKEASTTLFCASDAK AHKTEVHNVWATHACVPIDPDPQEVRLENVTENFNMWKNDMVEQMHEDIISLWDQSLKPCVK LTPLCVTLNCSSNVTIITNSTTANSSGKGIRDELEMKNCSFNVSTNTNRKGFKEEYALFYKLDLNG TDNTSYTLINCDSSVIKQACPKVTFEPIPIHYCTPAGFAILQCNDKKFNGTGPCSNVSTVQCTHGIR PVVSTQLLLNGSLAEEEIVIRSENLTDNAKNIIVQLNASVEINCTRPNNNTRKGIGIGPGSIFYATG AIIGDIRQAHCNISRAKWNNTLRQLAGELEKRFNKTTIIFEQPLPGGDQEIVMHSFNCGGEFFYCN TSQLFNNTWNSTDKTLNNTEGNDTITLPCRIRQIINRWQEVGKAMYAPPIAGIITCSSNITGLILTR DGGTENGTNTTTETFRPGGGNMKDNWRSELYKYKVVRIEPLGIAPTRARRIVVQREKRAVGTIG AMFLGFLGAAGSTMGAASVTLTVQARLLLSGIVQQQNNLLRAIEAQQRMLQLTVWGIKQLQAR VLAVERYLKDQQLLGIWGCSGKLICTTNVPWNTSWSNKSMNDIWNNMTWMEWDREINNYTGL IQTLLEESQIQQEKNEQELLELDKWTSLWNWFDITKWLWYIRIFIMVVGGLVGLRIIFFVLSIVNR VRQGYSPLSLQTHLPSPRGPDRPGGTEGEGGEEDRGGSGRSVNGFLALIWVDLRSLLLFSYRHLR DLLLILARSVELLGLRRWEALKYCWNLLQYWSQELKNSAVSLLNAIAIAVAEGTDRIIEFLQGVF RAFIHIPRRIRQGLERALL |
| B.KR.04.04C WS5.JQ31613 3 | B | 219 | MRVKGIMKNYQHWWRWGMMLLGMWMICSANKSWVTVYYGVPVWKEATTTLFCASDAKAY DTEVHNVWATHACVPIDPNPQEVVLGNVTENFNMWKNNMVDQMHEDIISLWDQSLKPCVKITP LCVTLNCSNLKNETANSTSANSNETANSTTASNSTSNSSGEVMEEVDMKNCSFTVTTELKNTVS KQYALFYKCKPYLKNGTGPCSNVSTVQCTHGIRPVVSTQLLLNGSLAEEEVVIRSENFTNNAKNI IVQLNASVEINCTRPDNTIKNIRIGPGIGLGSTLHATKSITGRIRPHCNISEAKWNNTLKQIAIKLRE QFRNKTIIFNHSSGGDPEIEMHSFNCGGEFFYCNTTKLFDSTWNGTKEWNDTKESDKNITLQCRI KQIINRWQEVGQAMYAPPISGLIRCSSNITGLILTRDGGANETHTNTETFRPAGGDMKDNWRSEL YKYKVVRIEPLGIAPTKARRRVVQREKRAVGILGAMFLGFLGAAGSTMGAASMTLTVQARQLL SGIVQQQNNLLRAIDAQQHLLRLTVWGIKQLQARVLAVERYLKDQQLLGIWGCSGKLICTTNVP WNTSWSNKTQEDIWTNLTWMQWEREIDNYTNVIYSLLEESQNQQEKNEQELLQLDKWASLWN WFDITSWLWYIKIFIMIVGGLIGLRIIFTVLSIVNRVRQGYSPLSFQTHFPSPRGPDRPGEIEGEGGE EDRGGSDRLVTGFLTLVWVDLRNLCLFSYRSLRDLILIVARSVELLGLRGWEVLKYCWSLLQYW SQELKKSAVSLLQATARAVVEGTDRVIETLQRAYRAILHIPRRIRQGLERALL |
| B.DK.04.PM VL | B | 220 | MRVKGIRRNWQHWWRWGTMLLGILMICSATDLWVTVYYGVPVWKEANTTLFCASDAKAYDT EVHNVWATHACVPTDPSPQEVELKNVTEDFNMWKNNMVEQMHEDIISLWDQSLKPCVKLTPL CVTLNCTDWNGTATSPNDNSYRGNKMEKGEMKNCSFNITTGIRDKIQKEYALFYKLDMVPIDD DKTNSSPTTTTSSTLTSSSVTNSNSNSSNTTSNSSQYRNYRMISCNTSVITQACPKVSFEPIPIHYCA PAGFAILKCNDKNFTGKGPCSNVSTVQCTHGIRPVVSTQLLLNGSLAEEGIVIRSDNFSNNAKNIL VQLKEAVVINCTRPNNNTRRGIHIGPGRAFYTTGDVIGDIRQAHCNISGTKWNNTLYQIAKKLKE QYNETNKIIFNHSSGGDPEIVMHSFNCGGEFFYCNTAKLFNSTLNNTEWSNITAENNEDKIILQCRI KQIINMWQEVGKAMYAPPISGPINCSSSITGLILTRDGGNINNNSMRDNDTFRPGGGDMRDNWR SELYKYKVVRIEPLGVAPTKAKRRVVQREKRAVGIGAVLLGFLGAAGSTMGAASVMLTVQARQ LLSGIVQQQNNLLRAIEAQQHLLQLTVWGIKQLQARVLAIERYLQDQQLLGIWGCSGRLICTTAV PWNASWSNRSLDQIWNNMTWMEWEREIDNYTNIIYSLIQESQNQQDKNEQELLELDKWASLW NWFSITQWLWYIKIFIMIVGGLIGLRIVFAVLSIVNRVRQGYSPLSLQTLLPAPRGPDRPEGTEEEG GERDRDRSGQLADGLLTLIWVDLRSLCLFSYHRLRDLLLIVARIVELLGRRGWEALKYWWNLL QYWGREIKNSAVSLLNATAIAVAEGTDRVLELLQRIGRGILHIPRRIRQGLERSLL |
| B.US.x.CR03 17N.FJ469719 | B | 221 | MRVKGIRRNYQHLWWKWGIMLLGTLMIYSAAEQWWVTVYYGVPVWKEANTTLFCASDAKA YDTEVHNVWATHACVPTDPSPQEINMTNVTEYFNMWKNNMVDQMHEDIINLWDQSLKPCVEL TPLCVTLECTDNITTSSNTTRGSNSSNRSRWENMTIETGEIKKCSFNVTTGIRDRVNTEYAYFNRF DIVQTERDNKSYTLRHCNTSVIKQACPKISFEPIPIYYCAPAGFAILKCNNKTFSGKGECDNVSTV QCTHGIRPVVSTQLLLNGSLAEEGVVIRSDNFSDNAKTIIVQLTKAVQINCTRPNNNTRKGIHMGP GKAFFTTGQIIGDIRQAYCNISHTKWNNTLKQVVYQLRKQYGGNKTINFTQPSGGDLEIVTHSFN CGGEFFYCNTTQLFNTTWYNNETVDNKTWTEDDTNLTDKDIIILPCRIKQIINMWQKVGKAMYA PPIQGMISCSSNITGLLLTRDGGNDTDTDPNGTETFRPIGGDMRDNWRSELYKYKVVRIEPLGIAP SKAKRRVVQREKRAALGAVFLGFLGAAGSTMGAASITLTVQARQLLSGIVQQQSRLLRAIEAQQ HMLQLTVWGIKQLQARVLALERYLQDQQILGIWGCSGKLICTTSVPWNASWGNTSLGYDYIWT NWTWMKWEKEIANYTHIIYDLLEESQIQQEQNELELMKLDKWASLWDWLGITKWMKYIKIFIII VVGLVGLRIIFAVLSIVNRVRQGYSPLSFQIRPPAAREPDRPEEIEEGGGGRDRGRSNQLVDGFLA LIWVDLWSLCLFSYQRLRDLLLIVTRIVELLGRRGWEILKYWWSLLQYWGQELKKSAVSLLNAT AIAVAEGTDRIIEAAQRVFRAIIHIPRRIRQGLERALL |
| B.KR.12.12K YY10 | B | 222 | MRVKGIRKNCQHWWRWGIMLLGMWMICSAATGGLWVTVYYGVPVWREAITTLFCASDAKAY ETEMHNVWATHACVPIDPNPLEVDLPNVTENFNMWKNNMVEQMHEDIISLWDQSLKPCVKITP LCVVLNCSDAKNSTSTNNSTEKVMEKGEMKNCSFNVTTNIENKVKEARAIFDKLDVVQIDDNDT NTMIGIQYYKCYSSSITQACPMVSFEPIPIHYCTPAGFAILQCKDKKFNGTGPCTNVSTVQCTHGIR PVVSTQLLLNGSLAEEEIVIRSENFTNNAKTIIVQLNASVEINCTRPNNNTRKSIPIGPGRAFYATG DIIGDIRRAHCNISKATWNNTLRQLAIKLREQLEDNTTTIIFKNSSGGDPEIEAHSFNCREEFFYCN TTPLFNSIWYSNNTEKSYNTAENDTITLQCRIRQIIKRWQEVGKARYAPLITCLIRCYSNITGLLLA GDGGEAGKDNGNKNETFRPGGGDMRDNWRSELYKYKVVEIEPLRIAPTRARRIVVQREKRAVG TLGAMFLGFLGAAGSTMGAASLTLTVQAKQVLSGIVRQQNNLLRAIEAQQRLLQLTVWGIKQL QARVLAVERYLQDQRLLGIWGCSGKLICTTSVPWNTSWSNKSHDEIWNNMTWMEWDREINNY TQVIYDLLEESQIQQEKNEQELLKLDSWASLWKWLDISNWLWYIQIFMMMVGGLVGLRIILCVL SVVNRVRQGYSPLPFQTHLPSPRGLDRPGGIEGEGGEGDRSGSSGLAGGFLTLVWVDLRSLCLFS YHSLRDLLLIVARSVELLGLRGWEILKYWWSLLQYWSQELKKSAVSLLNATAIAVAEGTDRVIEI LQRIYRAILHIPTRIRQGLERALL |
| B.GB.x.M268 64.U36875 | B | 223 | VRGTRKNYHHYLWRWGTMLLGMLMICSAGEQLWVTVYYGVPVWKEATTTLFCASDAKSYDT EVHNMWATHACVPTDPNPQEVVLGNVTENFNMWKNNMVEQMHEDIISLWDQSLKPCVKLTPL CVTLNCTDNLDNGTISNATHTTNSSSVNNTTGNSTRKLEIDRGEIKNCSFNITTNIKDKMQKAHA LFYKLDVVPIDNSTTSYRLISCNTSVITQACPKVSFEPIPIHFCAPAGFAILKCNNKTFNGKGPCKN VSTVQCTHGIRPVVSTQLLLNGSLAEDEVVIRSENFTDNAKTIIVHLNESVEINCTRPNNKTKRSF HIGPGRAFLTEIVGDIRQAYCNISRAKWNNTLHQVVNKLREQFGNRTIVFKNSSGGDPEIVTHMF NCAGEFFYCNTTKLFNSIWMPNGTWNSTDTSNSTEIRLPCRIKQIVNMWQEVGKAMYAPPISGRI RCVSNITGLLLTRDGGNNNETTEIFRPGGGDMRDNWRSELYKYKLIKIEPLGIAPTKAKRRVVQR EKRAVGIGAVLLGFLGAAGSTMGAASLTLTVQARLLLSGIVQQQNNLLRAIEAQQHMLQLTVW GIKQLQARILAVERYLRDQQLLGIWGCSGKLICPTTVPWNASWSNKNLSQIWDNMTWMEWERE IDNYTSLIYTLIEESQNQQEKNEQDLLELDKWASLWNWFSITNWLWYIKIFIIIVGGLIGLRMVFT VLSIVNRVRQGYSPLSFQTRHPAPRGPDRPEGIEEEGGERDRDTSGFLVDGFLAIIWVDLRSLCLF SYHRLRDLLLIVARIVELLGRRGWEALKYWWNLLQYWGKELKSSAISLFNATAIAVAEGTDRVI EVIQRACRAILHIPRRIRQGLERALQ |
| B.US.13.IQA 267.PBMC.S GA1.1.KR182 196 | B | 224 | MRAKGIRKNCQHWWRGVTLLLGMLMICNAAQQLWVTVYYGVPVWRDADTILFCASDAKGYD TEVHNVWATHACVPTDPNPQEIRLENVTENFNMWKNKMVEQMHEDIISLWDQSLKPCVKLTPL CVTLNCTDLNKNSTQGNATAEEQEEIRNCSFNITTSIRDRMKKDYALFYSNDIVKLDNNTFRLTS CNTSVITQACPKISFNPIPIHYCAPAGFAILKCKDTTFNGSGLCRNVSSVQCTHGIKPVVSTHFLLN GSLAEKEVVIRSENFTDNAKTIIVQLKDPVRINCTRPNNNTRKGIHIGPGRAFYATGDIIGDIRQAH CNISRTEWNKTLNQVVEKLRAQFNNKTIVFKNASGGDPEIVMHIFNCRGEFFYCNTTQLFNSTW NANSTWNDTSSNIGESNDTITLPCKIRQIVNMWQEVGKAMYAPPIAGQLNCSSNITGILLTRDGG ESNTTNSNETFRPGGGNMRDNWRSKLYKYKVIKIEPLGLAPTKAKRRVVQRKARAAGLGAVIF GFLGAAGSTMGAASVALTVQARQLLSGIVQQQNNLLRAIEAQQHMLQLTVWGIKQLQARVLA VERYLRDQQLLGIWGCSGKLICTTAVPWNDSWSPRWSKENMSKIWDNMTWNQWEREIDNYTG YIYTLLEESQNQQEKNEQDLLSLDKWADLWSWFDITNWLWYIKIFIMIVGGLVGLRIIFSVLAIVN RVRQGYSPLSFQTHLPARRGPEGPEEIEEGGGERDRDRSRPLVDGFLTLIWIDLRSLCLFSYHRLR DLLLIVTRTLELLGRRGWEALKYCWNLLQYWGQELRSSAVSLFNATAIAVAEGTDRIIEIIQRIFR AYSFSAPTLKNDMALALG |
| B.US.81.81NJ .AY247221 | B | 225 | MRVKEIRKNYQHLWRWGTMLLGILMICSAAEKLWVTVYYGVPVWKEATTTLFCASDAKAYDT EVHNVWATHACVPTDPNPQEVVLENVTENFNMWKNNMVEQMHEDIISLWDQSLKPCVKLTPL CVTLNCTDLRNATNTTSSSGGTMERGEIKNCSFNITTSIRDKVQKEYALFYKLDVVPIDNDNTTTS YRLISCNTSVITQACPKVSFEPIPIHYCTPAGFAILKCKDKKFNGTGPCTNVSTVQCTHGIRPVVST QLLLNGSLAEEEVVIRSDNFTDNAKTIIVQLKESVEINCTRPNNNTRKSIHIGPGRAFYTTGEIIGDI RQAHCNLSRAKWDNTLKQIVRKLREQFGNKTIVFNQSSGGDPEIVTHSFNCGGEFFYCDSTQLFN STWNVTEGSNNTEGNITLTLPCRIKQIINMWQEVGKAMYAPPIRGQIRCSSNITGLLLTRDGGNNE SETEIFRPGGGDMRDNWRSELYKYKVVKIEPLGVAPTKAKRRVVQREKRAVGIGAVFLGFLGA AGSTMGAASMTLTVQARLLLSGIVQQQNNLLRAIEAQQHLLQLTVWGIKQLQARVLAVERYLK DQQLLGIWGCSGKLICTTTVPWNASWSNKSLDKIWNNMTWMEWEREINNYTSLIYTLIEESQNQ QEKNEQELLELDKWASLWNWFDITKWLWYIKIFIMIVGGLVGLRIVFAVLSIVNRVRQGYSPLSF QTRLPAPRGPDRPEGIEEEGGERDRDRSGRLVDGFLALFWVDLRSLCLFSYHRLRDLLLIVTRIVE LLGRRGWEVLKYWWNLLQYWSQELKNSAVSLLNATAIAVAEGTDRVIEVLQRTCRAILHIPTRI RQGLERALL |
| B.JP.x.DR177 7.AB604948 | B | 226 | MRVKEMRKHWQHLWTGGILLLGMLMICSTAQDAWVTVYYGVPVWKEATTTLFCASDAKAYK TEVHNVWATHACVPTDPNPQEVVLENVTENFNMWKNNMVEQMHEDIISLWDESLKPCVKLTP LCVTLNCTDELIVTNSTNGNNTNSHSTRGNDTIGNSTSWKEMKGEIKNCSFNIPTSVKDKMQKQ YALFYKLDVVAINDDNNKNSSNYNSSKLSSSNSNCKSDNNSSCNCSSSNNNCSSSNHSSNYSSYI LISCNTSTLTQACPKVSFEPIPIHYCTPAGFAILKCNDKRFNGTGPCKNVSTVQCTHGIRPVVSTQL LLNGSLAEEEVVIRSENISNNAKTIIVQLNESVAINCTRPNNNTRKGIRIGPGRTFYAAEKIIGDIRK AYCIINGTKWNETLRLIVAKLREQEQIGENTTIIFKPSSGGDPEIENHIFNCRGEFFYCNTTQLFNST WYSNGTWIGKNFTGSNITLPCRIKQIVNMWQEVGKAMYAPPIRGQINCISNITGLLLTSDGGFRK TNETTNMTETLRPGGGDMRDNWRSELYKYKVVRIEPLGIAPTQAKRRVVQREKRAVGIIGAVFL GFLGAAGSTMGAAALTLTVQARQLLSGIVQQQNNLLRAIEAQHQLLQLTVWGIKQLQARILAVE RYLRDQQLLGIWGCSGKLICTTTVPWNTSWSNKSLTEIWNNMTWMEWEREIENYTGLIYNLLEK SQNQQEKNEQELLELDKWANLWNWFDITNWLWYIRIFIMIVGGLIGLRIVFAVLSIVNRVRQGYS PISLQTHLPVPRGPDRPEGIEGEGGERDGDTSRRLVIGLLPLIWDDLRSLCLFSYHRLRDLLLIVAR IVELLGRRGWEILKYWWNLLQYWSQELKNSAVSLLNATAIAVAEGTDRIIEIARTIFRAFYHIPRR IRQGFERALL |
| B.TH.x.3141 A16.B2.KJ95 3175 | B | 227 | MRVKETQMNWPNLWKWGTLILGLVIICSASDKLWVTVYYGVPVWRDADTTLFCASDAKAHVT EVHNVWATHACVPTDPNPQEIHLENVTEYFNMWKNNMVEQMHEDIISLWDQGLKPCVKLTPL CVTLTCTNASLTKINTTTSDPKIGNITDEVRNCSFNVTTEIEDKKQRVSALFYKLDIVPMEDNSNS SEYRLINCNTSVIKQACPKVSFDPIPIHYCTPAGYAILKCNDKNFTGTGQCKNVSSVQCTHGIRPT VSTQLLLNGSLAEEEIMIRTESLTNNAKTIIVHLNKSVEINCTRPSNNTRTSITIGPGRVFYRTGDIIG DIRRAYCEINETNWYKVLKQVTGKLKEHFNKTINFGPPSGGDLEITMHHFTCRGEFFYCNTTKLF INNCTRNETKGGCNSTINGTIILPCRIKQIINMWQGAGQAMYAPPISGRINCVSNITGILLTRDGGIT SNGTNGTNGTNGTETFRPGGGDIRDNWRSELYKYKVVQIEPLGVAPTKARRRVVEREKRAVGIG AMIFGFLGAAGSTMGAASITLTVQARQLLSGIVQQQSNLLRAIEAQQHLLQLTVWGIKQLQARV LAVERYLKDQKFLGLWGCSGKIICTTAVPWNSSWSNKSYDQIWHNMTWIEWEREISNYTNQIYE ILTESQNQQDRNEKDLLELDKWASLWNWFDITRWLWYIKIFIMIVGGLIGLRIIFAVLSIVNRVRQ GYSPLSFQTPTLHQREPDRPERIEEGGGEQDRDRSVRLVSGFLALAWDDLRSLCLFSYHRLRDFIS IAARTVELLGHSSLKGLRLGWEGLKYLGNLLLYWGQELKISAISLLDATAIAVAGWTDRVIEGA QRAWRAFLHIPRRIRQGLERALQ |
| B.BR.03.BRE PM1023EF63 7057 | B | 228 | MKVRVIRMSYQQLWKGGTLLLGMLMICSAVGPLWVTVYYGVPVWKEANTTLFCASDAKAYD TEAHNVWATHACVPTDPDPREVVLENLTEDFNMWKNDMVEQMHQDIISLWDQSLKPCVKLTP LCVTLNCSDFNGTVTNNSSGNATDNSSGSGLERETTGEIKNCSFNITTDMKDKVQEKYALFYKL DIVSIDESRNGSSYRLTSCNTSVLTQACPKVSFEPIPIHFCAPAGFAILKCNNKRFNGAGPCTNVST VQCTHGIRPVVSTQLLLNGSLAEEKVIIRSKNFSDNTKIIIVQLNESVVINCTRPNNNTRKSIHIGPG RAFYGTEIIGDIRKAHCTLNRTQWNNTLQRVATELREQFKNKTIGFTNSSGGDLEITMHSFNCGG EFFFCNTTKLFGGTWDGSTWNWSDNENNTITITLPCRKKLIINMQQEEEKATYANTIRGPIKSASN ITGLLKTRDGGEEDNASETFRPGGGDMRDNWRSELYKYKVVKVVPLGVAPTRAKRRVVQREK DLGLGALFLGFLGAAGSTMGAASLTLTVQARQLLSGIVQQQNNLLRAIEAQHQLLQLTVWGIKQ LQARVLAVERYLRDQQLLGIWGCSGKLICTTSVPWNASWSNKSLDDIWNNMTWMQWEKEIEN YTVEIYKLIGEAQNQQEKNEKDLLELDNWASLWNWFSITNWLWYIKIFIMIIGGLIGLRIVFAVLS IVKKVRQGYSPLSLQTRLPTQRGPDRPEGIEEEGGEQDRGSSIRLVDGFLALIWDDLGSLGPFSYP RLEDLPPIVTRIGELLGRRGWELLKLWWSLLQYWSQELKKSAVSLLNATAIAVAEGTDRILEVV QRFCRAILHIPVRIRQGLERALL |
| B.US.96.5155 | B | 229 | MRVKGIRKNYQRLWRWGTMLLGMLMICTATGKLWVTVYYGVPVWKEATTTLFCASDAKSYD TEAHNVWATHACVPTDPNPQELELKNVTEEFNMWKNNMVEQMQEDIISLWDQSIKPCVKLTPL CVTLNCTGLRNNTNATSASSTHPPTTSSSSRNIMEGEMANCSFNITTTMNKVQKEYALFYTHDV VQIVDNDNNNASNSAKKRISYRLISCNTSVVTQACPKVNFEPIPIHYCAPAGFAILKCNEKEFSGK GPCKNVSTVQSTHGIRPVVSTQLLLNGSLAEKEIVIRSENFTDNTKTIIVQLNEPVKIECTRPNNNT RESITMGPGKAFYATGDIIGNIRKAYCTLRGTEWTNTLKRIAEKLGEKFENKTIKFTQSSGGDPEI VAHSFNCGGEFFYCETTQLFNSTWIRDNGNWTRAENITEGNNITLPCRIRQIVNLWQQVGKAMY APPISGPINCLSNITGLLLTWDGGNNSTSNNNTEIFRPGGGDMRDNWRSELYKYKVVKIEPLGVA PTKAKRRVVQREKRAVGLGALFLGFLGTAGSTMGAASLTLTVQARQLLSGIVQQQNNLLRAIEA QQHLLQLTVWGIKQLQARVLAVERYLKDQQLLGIWGCSGKLICTTAVPWNASWSNKSLNDIWD NMTWMQWEREIINYTGLIYTLLVESQIQQEKNVQELLELDTWASLWNWFNITNWLWYIKLFIMI VGGLIGLRIVFTVLSIVNTVTQGYSPLSFQTRLPSQEGTDRPEGIQEGGGETDRSGPIRLVDGFLAFI WVDLRSLCLFSYHRLRDLLLIVTRTVELLGRRGWELLNYLWNLLQYWSQELLNTAQRLFNTTAI VVAEGTDRVIEVVQRAYRAILHIPTRIRQGLERLLL |
| B.US.SC141 | B | 230 | MRVKGIRKNCQHLWKWGTMLLGMLIICSAAEQLVTVYYGVPVKEATTTLFCASDAKAYNTEV HNVWATHACVPTNPNPQKVVLKNVTKNFNMKNNMVKQMHKNIISLWDQSLKPCVKLTPLCVT LNCTDYLKNNTNTTSSSEKEMKGGKIKNCSFNITTKIENKVQKKYALFYKLNVVPINNTTTSYRL ISCNTSVITQACPKVSFKPIPIHYCTPAGFALLKCKNKKFNETGPCTNVSTVQCTHGIKPVVSTQLL LNKSLAEKKVVIKSKNFTDNAKTIIVQLNKSVKINCTKPNNNTRKSIHIGPGQALYATEAIIENIRQ AHCNISKAKNNTLKQIVKKLKVQFRNKTIIFNQSSGGDPKIVMHSFNCGGEFFYCNTTKLFNSTM FNNTNNTKNTKENGTITLPCRIKQIINRWQKVRKAMYAPPIRGQIKCSSNITGILLTKNGSTNNST NKTFRPKEENIRNNKSKLYKYKVVKIKPLKVAPTKAKRRVVQREKKAVETKALFLGFLRAAGST MGAAAVTLTVQARQLLSGIVQQQNNLLRAIKAQQHLLQLTVGIKQLQAKVLAVKKYLRNQQLL GIGCSEKLICTTTVPNASSNKSLNKINNITMKKKKINNYTSLIYSLIKKSQNQQVKNRQKLLKLNK ASLSFNITKWLWYIKIFIIIVKGLIGLKIVFLVLSIVNKVRQKYSPLSFQTRFPAPKEPDRPEGIEEKG KKDKDRSGQLVNKFLTLIINLRSLCLFSYHRLKNLLLIVAKIVKLLKRRGWEALKYWWNLLQYS QKLKNSAVSLLNATAIAVAEGTDRVIEVVQRTCRAILHIPARIRQGLERALL |
| B.US.02.F762 P.FJ469735 | B | 231 | MRAKEMRKHWQQWWKGGILLLGMLMICSTAENLWVTVYYGVPVWKEATTTLFCASDAKAY DTEAHNVWATHACVPTDPNPQEVFLKNVTENFNMGKNNMVEQMHEDIISLWDQSLKPCVKLT PLCVTLNCTDNIKLKNATENATATKANKEEIINCSFLVTTDRKDKEQNEYALFYRLDICTNKSWL LLVYTNNTDYRLDSCNTSVITPSGPRRCPCESISIHPCTLDDCAIFKCNEKKCNGTGVCKTVSTVH CIHGLSAVGSSPLLLNGSLAEEEVVIRSENFTNNAKTIIVQLKEAVTINCTRPNNNTRKGIHIGPGR AFYATESIIGDIRQAHCNLSRAQWNNTLRQIVQKLKEQFKNATTIKFNSSSGGDPEIVTHSFNCGG EFLYCNTSHLFNSTWMCNSTWNSTNTEGNNTITLPCRIKQIVNMWQEVGKAMYAPPIKGQIKCL SNITGLLLTRDGGVGDDSSNDTEIFRPGGGDMRDNWRSELYKYKVVRIEPLGIAPTKAKRRVVQ REKRAVGTLGAMFLGFLGAAGSTMGAASVTLTVQARQLLSGIVQQQNNLLRAIEAQQHLLQLT VWGIKQLQARVLAVERYLWDQQLLGIWGCSGKLICPTTVPWNTSWSNKNYTEIWNKMTWMQ WEREIDNYTSLIYSLIEESQNQQEKNEQELLELDKWDSLWSWFSITHWLWYIKIFIMIVGGLVGLR IVFTVLSIVNRVRQGYSPLSFQTHLPAQRGPDRPEGIEEEGGERDRERSTRLVHGLLALIWDDLRS LCLFSYHRLRDLLLIVARTVELLGRRGWEALKYLWNLLQYWIQELKKSAISLLNATAIAVAEGT DRIIEIAQRIFRAFLHIPRRIRQGFERALQ |
| B.CN.12.2019 | B | 232 | MKVTGIRRNYQKFWRWGALLLGMLMISSAAEKLWVTVYYGVPVWKDANTTLFCASDAKGYK TEAHNVWATHACVPTDPNPQEVVLANLTENFNMWESDMVEQMHEDVINLWDQSLKPCVKLTP LCVTLNCTNLNITSNNTSSSNTSSSNTSSITGGGEMKNCSFNITTSINTKVKDYAFFYNLDIVQINN DKTSDNTSYRLINCNTSVITQACPKISFEPIPIHYCTPAGFAIIKCNNETFNGTGPCTNVSIVQCTHGI RPVVSTQLLLNGSLAERDVAIRSKNFSNNAENIIVQLNEAVQINCTRPNNNTRKSINIGPGRAWYT TGEIIGNIRQAHCNISGSKWNDTLKQVVKKLREQFKNKTITFEQPPPGGDPEIVMHNFNCGGEFFY CNTSQLFNSRWDDVDENETSTSNGTTGNDTITLPCRIKQIINMWQEVGKAMYAPPIAGLIRCSSNI TGLLLTRDGGNINATGNNTNKNNNTEIFRPAGGNMKDNWRSELYRYKVVKVEPLGVAPTRAQR RVVQREKRAVGMIGAMFLGFLGTAGSTMGAASLTLTVQARQLLSGIVQQQRNLLRAIEAQQHL LQLTVWGIKQLQARVLAVERYLKDQQLLGLWGCSGKLICTTNVPWSGNWSSKSYNDIWYNMT WMQWEKEIENYTGVIYTLIEESQNQQEKNEQELLELDKWASLWNWFDISNWLWYIRIFIMIVGG LIGLRIIFAVLSIVNRVRQGYSPLSLQTHFPTQRGPGRPEGTEEEGGERDRDRSERLVTGFLTLFWE DLRSLCLFSYHRLRDLLLIVARIVELLGRRGWEVLRYWWNLLQYWIQELKNSAISLLNAIAIAVG EGTDRVIEVAQRIYRAILNIPRRIRQGLERALL |
| B.SE.03.005S E.MF373127 | B | 233 | MRVKGIRKNCQHSWKWGIMLLGMLMICNASDQLWVTVYYGVPVWKEANTTLFCASDAKAYD TEVHNVWATHACVPTDPNPQEIGLGNVTENFNMWKNNMVEQMHEDIISLWDQSLKPCVKLTPL CVTLNCTDCKKDNDTLQCDCNNERLIIILSLVTYTWERGEIRNCSVTATTDIEDRRKNEDASYHN RGIIQINNINNTYTLMLSSCTTSIIAGPCPSVSLDPIPNCYCAAAGFPIRNGNDNNVAGPGPSDIVTP GPCALGIMPVAPPLLLVSGPLAEEEVITTSDIFTNNAKTIIVQLNETVKINCTRPSNNTRKSITIGPG RAFYATGDIIGDIRQAHCNISQAKWNNTLKQVVVKLREQFGMNKTIIFNQSSGGDPEIVMHSFNC GGEFFYCNTTRLFNTTWNNTEKSNPTEGNGTITLQCRIKQIINLWQEVGKAMYAPPIRGQINCSSN ITGLLLTTDGGMNTSNNKTFIPAGGDMRDYWRSGVLKYDVVIVEPLGIAPTTDMRRVVQIQKRA ELLRALLLVFLGAAGRTMSAASMTLTVQARQLLSGIVQQQNNLLQAIEAQQHMLKLTVWGIKQ LQARILAVERYLQDQQFLGFWGCSGKRICTTAVPWNASWSNKSLDEIWNNMTWMEWEKEIDN YTGLIYNLLIESQNQQEKNEQELLALDKWASLWNWFDISNWLWYIRIFIMIVGGLVGLRIVFTVL SIVRRVRQRYSPISVQTPRSAAQRGHDRPDGIEEEGGERDRDRFDQSVNGFLTIIWVDVRSLCLFS YHRLRDLLSIVSRIVELMGRRGWGILKYWCNLLQYRSQELKKSAVSLFNATAIVVAEGTDRVIE AIQRAFRAILHIPRRIRQGAERALI |
| B.SE.10.SE60 0012.KP4118 23 | B | 234 | MRVKGIKEELSSSWWKWGIMLLGMLMMICSALDQLWVTVYYGVPVWKEATTTLFCASDAKA YDTEVHNVWATHACVPTDPNPQEVVLENVTENFNMWKNNMVDQMHEDIISLWDQSLKPCVKL TPLCVTLNCTDLRNNTESNDTTSGMILGKDKIKMILFNCSFNITTSRRDKWQQEYAFFYKLDIMPI DEENNTNTYTLISCNTSVITQACPKVSFEPIPIHYCTPAGFALLKCNDKKFNGTGLCKNVSTVQCT HGIRPVVSTQLLLNGSLAEEEVVIRSENFTDNIKTIIVQLNESVEINCTRPNNNTRRSIRNHRGPGR AFHTTGEIIGNIRQAHCNISRAKWNNTLKQIVAKLREQFGKNKTIVFNHSSGGDPEIVMHSFNCG GEFFYCNTTQLFNSTWNITGGLNNTEGNGTITLQCKIKQFINMWHEVGKAMYAPPIRGQITCSSNI TGLLLTRDGGENPGNDTDTFTPGGGDMKDNWSSELDKYRVIGIAPLPVAPTKAKTRLLQRDKRA VGIGSVFLVFLVAAGSTMSAMSMTMTLQAQELLYVTERMQKNLLKAIEAQQHLLQLTVWGIKQ LQARVLAIEGYLKDQQLLGLWGCSGKLICTTAVPWNVSWSNKSLDKIWNNMTWREWEREIDN YTGLIYNLLETSQNQQEKNEQELLELDKWASLWNWFDITNWLWYIKIFIMIVGGLVGLRIVFTVL SIVNRVRQGYSPISLQTPRPAQRGLDRPEAWDEKAGEKCRGHFHRCVNRIMAIFGAICGAFCSSSF SCDPLGFYWGFWKFLPGGGKPLKNAWNFLPYLVPELNQGANEVFNCPVNATGESTGRGIETFQ RTFKSIFQILSQITPGQTGAKKGWV |
| B.SE.10.SE60 0046.KP4118 27 | B | 235 | MRVQENWQNLWKGGIWFLGIWMICSAVEQLWVTVYYGVPVWKDATTTLFCASNAKAYDTEV HNVWATHACVPTDPNPQEVVLQNVTEPFNVWKNGMVEQMHEDIISLWDQSLKPCVKLTPLCV TLHCTDLKETIKEEKGKMKNCSFNTTTSIRDKTQTAYALFSTLDLVQIKDGKINANSKHSYRIIHC NTSTITQACPKVSFEPIPIHYCAPAGFAILKCNDKQFNGTGECRNVSSVQCTHGIRPVVSTQLLLN GSLAEEDIVIRSENLTNNAKTIIVQLNETVRINCTRPNNIVSRRIHIGPGRTFHATKSITGSIKQAFCN LSRPQWEDTLGKIVKKLRQNFGTNTTIVFDHSSGGDPEIVKDRINCGGKFFYCYSIKLFKSTWNM EWSTQKASNTENENITLPCKIKQVINMWQEVGKAMYAPPIEGQIKCSSNITGLLLTRDGGKHNSS DTNKTEIFRPGGGDMRDNWRSELYKYKVVKIEPLGIAPTRAKRRVVQREKRAVGLGAMFLGFL GAAGSTMGAASLTLTVQARQLMSGIVQQQNNLLRAVEAQQHMLQLTVWGIKQLQARVLAVER YLKDQQLLGIWGCSGRLICTTAVPWNTSWSNRTQDDIWTNLTWMKWEKEIDNYTDVIYRLLEE SQNQQEKNEQELLELDKWASLWSWFDITNWLWYIKIFIMIVGGLIGLKIVFTVFSIVNRVRQGYS PVLLQTHHPGRRGRPRPVGMEEEVGEGDRDSSCPLAVELLSIFEVVFRILFVFNCLRLRDLILIILR MVVLLGRGGWEIFKYWSRVLQFRIQELKNSGFILFPSTPITVEEETNGITEPFRSPCNPIVIIPIRKKQ GSER |
| C.MW.96.C12 0 | C | 236 | MRVRGILRNCQQWWIWGILGFWMLMICNVVGNLWVTVYYGVPVWKEAKTTLFCASDAKAYE KEVHNVWATHACVPTDPNPQEMVLENVTENFNMWKNDMVDQMHEDIISLWDQSLKPCVKLTP LCVTLNCSNITSIVSRNVSNNDNMRNCSFNITTEIRDKEKKEYALFYRLDIIPLEKNSSEYRLINCN TSAVTQACPKVSFDPIPIHYCAPAGYAILKCNNKTFNGTGPCNNVSTVQCTHGIKPVVSTQLLLN GSLAEEEIVIRSKNLTDNAKIIIVQLKDPVTIVCTRPGNNTRKSVRIGPGQTFYATGEIIGNIRQAHC NISKEDWNKTLQQVGKKLAEHFPNKTIEFKEHSGGDLEITTHSFNCRGEFFYCYTSTLFNSTYNA EYNSNSTNSSSTITLQCRIKQIINMWQGVGRAIYAPPIEGNITCESNITGLLLTRDGGKDGNNTEIF RPGGGDMRDNWRSELYKYKVVEIKPLGIAPTKAKRRVVEREKRAVGIGAVFLGFLGAAGSTMG AASITLTVQARQLLSGIVQQQSNLLRAIEAQQHMLQLTVWGIKQLQARVLAIERYLKDQQLLGI WGCSGKLICTTAVPWNSSWSNRSQDDIWQNMTWMEWDREINNYTNTIYRLLEDSQNQQEKNE QDLLALDSWKNLWSWFNITNWLWYIKIFIMIVGGLIGLRIIFAVLSIVNRVRQGYSPLSFQTLIPNP RGPDRLRRIEEEGGEQDKDSSIRLVSGFLALAWDDLRSLCLFSYHRLRDFILIVVRAVELLGHSSL RGLQRGWEALKYLWSLVQYWGLELKKSAISLLDTIAIAVAEGTDRIIELVQRIFRAICNIPRRIRQ GFEAALQ |
| C.CN.06.CNE 88 | C | 237 | MRVRGTLRNCQQWWMWGVLGFWMLMICNGGKNLWVTVYYGVPVWKEAKTTLSCASDAKA YDTEVHNVWATHACVPTDPNPQEMVLENVTENFNMWKNDMVDQMHEDVISLWDQSLKPCVK LTPLCVTLECRDVTNGTSGNDTLANDTSECKNGTNNETYDESVKELRNCSFNATTLVRDKKKTA YALFYRLDIMPLNEKKNSSENSSEYVLINCNSSAITQACPKVTFDPIPIHYCAPAGYAILKCNNKTF NGTGPCNNVSTVQCTHGIKPVVSTQLLLNGSLAEGEIIVRSENLTNNVKTIIVHLNQSVEIVCTRP NNNTRKSIRIGPGQTFYATGEIIGDIRQAHCIINASNWHKTLQEVSKELAGYFPNKTIIFNSSSGGD LEITTHSFNCRGEFFYCNTSGLFNSTYMTNGTYMFNGTYRLNNITSNSNITIPCRIKQIINTWQEVG RAMYANPIAGNITCKSNITGLLLVRDGGTNNATTETFRPGGGNMRDNWRSELYKYKVVEIKPLG IAPTEAKRRVVEREKRAVGIGAVFLGFLGAAGSTMGAASITLTVQARQLLSGIVQQQSNLLRAIE AQQHMLQLTVWGIKQLQTRVLAIERYLKDQQLLGIWGCSGKLICTTAVPWNSSWSNKSHEEIW NNMTWMQWDREISNYTNTIYRLLEDSQNQQERNEKDLLALDSWKNLWSWFDITNWLWYIKIFI MIVGGLIGLRIIFAVLSIVNRVRQGYSPLPFQIRTPNPGGPDRLGRIEEEGGEQDKDRSIRLVSGFLA LAWDDLRSLCLFSYHRLRDFILVAARVVELLGRSSLRGLQKGWEALKYLGSLVQYWGLELKKS ATSLLDTIAIAVAEGTDRIIELGLSICRAIRHIPRRIRQGFEAALQ |
| C.ZA.04.04Z ASK182B1.A Y878054 | C | 238 | MRVRGIPRNWPQWWIWGILGFWIIMMCRVMGNLWVTVYYGVPVWKEAKTTLFCASDAKAYE KEVHNVWATHACVPTDPNPQEMELKNVTENFNMWKNDMVDQMHEDIISLWDQSLKPCVKLTP LCVTLNCTDVKKANNTTNIGNTTSNRNTTYNATYNEYMKNCSFNITTEIRDKKRKEYALFYRPDI VPLENSSSEYILINCNSSTITQACPKVSFDPIPIHYCAPAGYAILKCNNKTFNGTGPRHNVSTVQCT HGIKPVVSTQLLLNGSLAEEEIIIRSENLTDNTKTIIVHLNKSVAINCTRPGNNTRRSIRIGPGQAIYT NNIIGDIRQAYCSISKEKWNKTLEQVKEKLKEYYKKTIEFQQPVGGDLEITTHSFNCRGEFFYCDT TKLFSPKNETDNNTITLPCRIKQIINMWQEVGRAMYAPPIAGNITCNSNITGILLTRDGGDTHEKK RKHKRNKVTEIFRPAGGNMKDNWRSELYKYKVVEIKPLGVAPTGAKRRVVEREKRAVGMATM LFGFLGAAGSTMGAASITLTVQVRQLLSGIVQQQSNLLRAIEAQQHLLQLTVWGIKQLQARVLAI ERFLQGQQLLGLWGCSGKLICTTNVPWNSSWSNKSEEDIWGNLTWMQWDREISNYTDIIYRLLE DSQTQQEQNEKDLLALDSWKNLWSWFDISNWLWYIRIFIIIVGGLIGLRIIFGVLSIVNRVRQGYS PLSFQTLTPNPRELDRLGRIEEEGGEQGRDRSIRLVSGFLTIAWDDLRSLCLFLYHQLRDFILIAAR AAELLGRSSLRGLQRGWEALKYLGNIVQYWGLELKKSAISLLDTIAIAVAEGTDRIIRVIQSICRAI RNAPRRIRQGFEAALL |
| C.ZA.09.GU0 80186.GU080 186 | C | 239 | MRVRGTLRNYQQWWTWGILGFWMLIIYSVAGNLWVTVYYGVPVWTNAKTTLFCASDAKAYE KEVHNVWATHACVPTDPHPQELVLANVTENFNMWKNDMVEQMHEDIINLWDESLKPCVKLTP LCVTLRCTKVNGTANATSEGTANATSKDEEMTMYNCSFNTTTGIRDKTQQEYALFHKHDVVPS NIDNSEYILIHCNTSTITQACPKVSFDPIPIHYCAPAGYAILKCNNKTFNGTGPCTNVSTVQCTHGI KPVVSTQLLLNGSLAEKEIIIRSENLTDNSKTIIVHLNESVDIVCTRPGNNTRRGIRIGPGQTFFATR TIGNIRQAHCNISESKWNTTLQRVSAKLKEYFNKTIIYNSSSGGDPEITTHSFNCRGEFFYCNTSRL FNRNSTDLSTNTGNNSTITLPCRIKQIINMWQGVGRAMYAPPIEGNITCKSIITGLLLVRDGGPNNS TETRPEPGREGNMKGNKFGRKSEIIIKYKVIENLSPLGEIHPTKAKRRAVEREKRAVVGIGAMILG FLGAAGSTMGAASITLTVHARQLLSGIVQQQSNLLRAIEAQQHMLQLTVWGIKQLQARVLAIER YLKDQRLLGLWGCSGKLICTTSVPWNSSWSNKSEADIWNNMTWMQWEKEINNYTYTIYQLLE DSQSQQEQNEKDLLELDKWQNLWSWFSITNWLWYIKIFIMIVGGLIGLRIIFAVLSIVNRVRQGYS PLSFQTLTPSPRELDRRERIEEEGGEQDRDRSVRLVSGFLSLAWDDLRSLCLFIYHRLRDFILIATR VVELLGQRGWEALKYLGNLVLYWGLEIKKSAINLLDTIAIAVAEGTDRIIEVVHRACRAIRYIPRR IRQDFEADLQ |
| C.IN.94.94IN | C | 240 | MRVRGIRRNYQQWWIWGVLGFWILMICNGGGNLWVTVYYGVPVWKEAKTTLFCASDAKAYE KEVHNVWATHACVPTDPDPQEMVLENVTENFNMWKNDMVNQMHEDVISLWDQSLKPCVKLT PLCVTLECRNVSTCNETYNESVKEIKNCSFNATTEIRDRKQTVYALFYRLDIVPLDKKNSSRNSSD YYRLINCNTSAITQACPKVTFDPIPIHYCTPAGYAILKCNDKTFNGTGPCHNVSTVQCTHGIKPVV STQLLLNGSLAEREIIIRSENLTDNVKTIIVHLNESVDIVCTRPNNNTRKSIRIGPGQTFYATGDIIGD IRQAHCNISEDKWNETLQRVSKNLAEHFPNKTIKFASSSGGDLEITTHSFNCRGEFFYCNTSGLFN GTYMSNGTESNSSSIITIPCRIKQIINMWQEVGRTMYAPPIKGNITCKSNITGLLLVRDGGTEPNDT ETFRPGGGDMRNNWRSELYKYKVVEIKPLGIAPTAAKRRVVEREKRAVGLGAVFLGFLGAAGS TMGAASITLTAQARQLLSGIVQQQSNLLRAIEAQQHLLQLTVWGIKQLQTRVLAIERYLRDQQLL GIWGCSGKLICTTAVPWNSSWSNRSEKEIWDNMTWMQWDREISNYTNTIYRLLEDSQNQQERN EKDLLALDSWKNLWSWFDITNWLWYIKIFIMIVGGLVGLRIIFAVLSIVNRVRQGYSPLSFQTLTP NSGGPDRLGRIEEEGGEQDKDRSIRLVNGFLALAWDDLRNLCLFSYHRLKDCILVTARVVELLG RNSLRGLQKGWEALKYLGSLVQYWGLELKKSTTSLLDTLTIAVAEGTDRIIELGQSICRAIRNIPR RIRQGLEAALQ |
| C.IN.09.T125 | C | 241 | MRVMEILRNYQQWWIWGILGFWMLMICNVGGNLWVTVYYGVPVWKEAKTTLFCASDAKAHE TEVHNVWATHACVPTDPKPQEIVLGNVTENFNMWKNDMVDQMHEDVISLWDQSLKPCVKLTP LCVTLECGNVSVSNVSVSNTTQGDANVTHNEGANEIKNCSFKIATELRDKEQKVYALFYRLDIV QLNDSSNSSSSNNSSKYSKYRLINCDTSTVTQACPKVTFDPIPIHYCAPAGYAILKCNNETFNGTG PCTNVSTVQCTHGIKPVVSTQLLLNGSLAKGGVIIRSENLTENAKIIIVHLNESVEINCTRPNNNTR KSVRIGPGQTFYATGEIIGDIRKAYCEINEQKWNKTLREVAKSLAKHFPNREIRFAPSSGGDLEIVT HSFNCRGEFFYCNTSGLFNRTYTTDLFNKTYNDNDTEDNSGPVIIIPCRIKQIINMWQEVGRAMY APPIAGNITCNSSITGLLLTRDGGNDNKTSTNETFRPLGGNMRDNWRNELYKYKVVEIKPLGIAP TTAKRRVVEREKRAVGMMGAMFLGFLGAAGSTMGAASITLTVQARQLLSGIVQQQSNLLRAIE AQQHLLQLTVWGIKQLQARVLAIERYLKDQQLLGIWGCSGKLICTTAVPWNSSWSNKSYDEIW ENMTWMQWDKEINNYTDTIYRLLEDSQIQQERNEKDLLALDNWKNLWNWFNISNWLWYIKIFI MIVGGLIGLRIIFAVLSIVNRVRQGYSPLSFQTLIPSPGGPDRLERIEEEGGEQDRDRSIRLVNGFLA LAWDDLRNLCLFSYHRLRDFILVTARAVELLGHHSLRGLRKGWEALKYLGSLVQYWGLELKKG AISLFDTIAIAVAEGTDRIIEFLQRLCRAIYNIPRRIRQGLEAALQ |
| C.ZA.99.Dul 23.DQ411850 | C | 242 | MRVKGIQRNWPQWWIWGILGFWMIIICRVVGNLWVTVYYGVPVWTEAKTTLFCASDAKAYER EVHNVWATHACVPTDPNPQEIVLGNVTENFNMWKNDMVDQMHEDIISIWDQSLKPCVKLTPLC VTLNCTDVKVNATSNGTTTYNNSIDSMNGEIKNCSFNITTEIRDKKQKVYALFYRPDVVPLNENS SSYILINCNTSTTTQACPKVSFDPIPIHYCAPAGYAILKCNNKTFNGTGPCHNVSTVQCTHGIKPVV STQLLLNGSLAEEEIIIRSENLTNNAKTIIVHLNESIEIVCTRPNNNTRKSIRIGPGQTVYATNDIIGDI RQAHCNISKTKWNTTLEKVKEKLKEHFPSKAITFQPHSGGDLEVTTHSFNCRGEFFYCDTTKLFN ESNLNTTNTTTLTLPCRIKQIVNMWQGVGRAMYAPPVEGNITCNSSITGLLLVRDGGNTSNSTPEI FRPGGGNMKDNWRSELYKYKVVEIKPLGVAPTKAKRRVVEREKRAVGIGAVLFGFLGAAGST MGAASITLTVQARQLLSGIVQQQSNLLRAIEAQQHMLQLTVWGIKQLQARVLAIERYLKDQQLL GLWGCSGKLICPTTVPWNSSWSNKSQTDIWDNMTWMQWDREISNYTGTIYKLLEESQNQQEKN EKDLLALDSWKNLWSWFDITNWLWYIKIFIMIVGGLIGLRIIFGVLSIVKRVRQGYSPLSFQTLTP NPRGLDRLGRIEEEGGEQDKDRSIRLVNGFLALAWDDLRSLCLFSYHRLRDFILVAARAVELLGR SSLRGLQRGWEALKYLGNLVQYGGLELKRRAISLFDTIAIAVAEGTDRILEVILRIIRAIRNIPTRIR QGFEAALL |
| C.NP.11.11N P041.KJ15843 0 | C | 243 | MKVRGTQRNYPQWWIWGILGFWMLMICNVERNLWVTVYYGVPVWREAKTTLFCASDAKAYD QEVHNVWATHACVPTDPNPQERSMENVTENFNMWENDMVDQMHEDVISLWDQSLKPCVKLT PLCVTLNCTDAKVNITADNSKTYNESMKEIKNCSFNATTVIKDKKQTVYALFYKLDIVPLDNEEQ ENNGNENYTYRLINCNTSTITQACPKVTFDPIPIHYCAPAGFAILKCNDKTFNGTGPCSNVSTVQC THGIKPVVSTQLLLNGSLAEEEIIIRSKNLSQNHRTIIVHLNESVGIECTRPSNNTRKSIRIGPGQTFF ATGEIIGNIRQAHCNISKEKWDKTLQMVGKKLAEHFPNKTIIFAPASGGDLEITMHSFNCRGEFFY CDTKKLFNATYMNDDTANNSRSNGNDTVTLPCKIKQIINMWQEVGRAMYAPPIAGNITCKSNIT GLLLVRDGGKMNQNIETNKTETFRPEGGNMKDNWRSELYKYKVVEIKPLGIAPTKAKRRVVER EKRAVGLGAVFLGFLGVAGSTMGAASITLTAQARQLLSGIVQQQSNLLKAIEAQQHLLQLTVWG IKQLQTRILAIERFLQDQQLLGIWGCSGKLICTTAVPWNNTWSNKSESEIWNNMTWMQWDREIS NYTFTIYQLLEDSQIQQEKNEKDLLALDSWNSLWSWFNITSWLWYIKIFIMIVGGLIGLRIIFAVLS IVNRVRQGYSPLSLQTLIPTPREPDRPRGIEEGGGEQDNATSIRLVNGFLALAWDDLRNLCLFSYR HLRDLLSVIARVVGLLGRVGGGGLQRGGKAPKLGGLGAYWGLEIKKSVFSCLIRLAIAVLKGTV RFLKLVQDFGKGINNYPRRIPGFETGL |
| C.SE.09.035Z A.MF3731-18 | C | 244 | MRVMGTLRSCQRWWIGGILGFWMLFMCSVGEDLWVTVYYGVPVWKEAKTTLFCASDARAYE KEVHNVWATHACVPTDPNPQEMILKNVTENFNIWKNDMVDQMHEDIISLWDQGLKPCVKLTPL CVTLNCSNVNSSSVANSTNSTNSGNITNANTTNVNITDSDMKNCSFNITTELRDKTRKEYALFYR PDIVPLSNSNSSEYILINCNSSTITQACPKVSFEPIPLHYCAPAGYALLKCRDTKFNGTGPCKNVST VQCTHGIKPVVSTQLLLNGSLAEDEIVIRSENITNNAKTIIVQLKEPVTIMCTRPNNNTRKSMRIGP GQTFYATGAIIGNIRQAHCNISRTNWTKTLQEVGEKLQEQELFKNKTIKFTEHSGGDIEITTHSFN CGGEFFYCNTSELFNGTYSNGTYTPNNNGSDSNLTITIPCRIKQVVNLWQGVGQAMDAPPIAGIS WGSIRGLLLPGGGPNPSTAEIFRPGGGDMRDNWRSELYKYKVVEVNPLGIAPTRAKRRVVEREK RAVGIGAVFLGFLGAAGSTMGAASVTLTVQARQLLSGIVQQQSNLLRAIEAQQHMLQLTVWGI KQLQARVLALERYLRDQQLLGIWGCSGKLICTTAVPWNSSWSNKSYDAIWDNMTWMQWEKEI DNYTDTIYRLLEVSQTQQEQNERDLLALDKWNNLWSWFNISNWLWYIKIFIMIVGGLIGLRIIFA VLSIVNRVRQGYSPLSLQTLIPSPRGPDRPGGIEEEGGEQDRSSSIRLVSGFLALAWDDLRSLCLFS YHRLRDLVLIAARGVNLLGQRGWEALKLLWSLVQYWGLELKKSAISLFDTLAITVAEGTDRIIEI VQRTWRAICNIPRRIRQGFEAALQ |
| C.SE.03.004Z M.MF373126 | C | 245 | MSVKDILENYQHWWIWGLLAFWMQMVCMGDNLWVMVYYGAPVWREAKPTLFCASDAKGY NKESQSVWATCACVPTDPPPQELVLGIVTEYFNVWKMDGIHCEGYNLLGYQPKPVCKGNICVL KLYICYCYQQFWLVGEMKNCTLNATTELRDNRKKESALFYRLDVLPLNSSDNNNNEYRLINCST SAITQACPKVSFDPIPIHYCAPAGYAILKCNDRTFNGTGPCKNVSTVQCTHGIKPVVSTQLLLNGS LTKEIIISSENLTKNTKTIIVHLNESVGIVCIRPNNNTRKSVRIGPGQTFFATGAIIGEIRQAYCTVNG SAWNNTLQRVRKKLRRYFSKAIRFEPSSGGDLLLTTHSFTCRGGYFYCNTSNLINNNINIASASTI QRRIKQIINMWQGVGRAMYAPPIAGNITCKSNITGLLLTRDGGENNRTETFRPGGGDMKDNWRS ELYKYKVVESKPLGIAPTKAKRRVVEREKRAVGLGAVFLGFLGAAGSTMGAASITLTVQARQLL SGIVQQQSNLLKAIEAQQHMLQLTVWGIKQLQARVLAIERYLEDQQLLGMWGCSGRRICTTALS WNSSWSNKTYDDIWNNMTWMQWDREISNYTDTIFRLLEDSQNQQEKNEKDLLALDSWKNLW SWFNITKWLWYIKIFIMIVGGLIGLRIIFAVLSIVNRVRQGYSPVSLQTLIPAPRGPDRPGGIEEGGG EQDKDRSIRLLNGFLTLVWDDLRSLCLFSNHRLRDFILQSQRAVEVVGRRGWEAKKYAGGGLQ YWGEELKQSGFSSNDTIAIVVEEGTDRIIQVLQWIGRASNNIPGRIRQGFEVAGQ |
| C.SE.12.076S O.MF373179 | C | 246 | MRVMGMQRNCQKWWVWGILGFWMFMIYNVMGNLWVTVYYGVPVWKEANPTLFCASDAKG YEREVHNVWATHACVPTDPNPQELFLENITENFNMWKNEMVEQMHQDIISLWDQSLKPCVKLT PLCVTLRCSNVTYNETIREMTNCSFNVTTELRDRRQEVYALFYKLDVVSLNKSGDYRLINCNTST ITQACPKVSFDPIPIHYCAPAGYAILKCNDEEFNGTGPCGNVSTVQCSHGIKPVVSTQLLLNGSLA ENVTIRSENLTNNAKTIIVQLREPVYINCTRPNNNTRKSIRIGPGQALYATGEVIGDIRHAHCNISK EKWKTALTGVRERLRELFNRTILFNKSSGGDLEVTTHSSNCRGEFVYCDTSDLLTTSVSVQKVLV ITSTDAIILRCQIQHIISMWQVRRALYAPPMKGMRSSTCTITGTTDLRDGRTDYHSMYIVRPGGGD MRDNWRSELYKYKVVEVKPIGIAPTGAKRRVVEREKRAALGAVFLGFLGAAGSTMGAASVTLT VQARQLLSGIVQQQNNLLRAIEAQQHMLQLTVWGIKQLQTRVLALERYLRDQQLLGIWGCSGK LICTTNVPWNGSWSNRSQQEIWENMTWMQWEREIDNYTNIIYELLEVSQNQQETNERDLLALD KWNNLWSWFNISNWLWYIRLFIMIVGGLIGLRIIFAVLSIVNRVRQGYSPLSFQTLIPNPRGPDRP GGIEEEGGEQDRDRSIRLVSGFLALAWDDLRSLCLFSLLHLRDLIEIVARAVELVEHSSLRGLQRG WDTLNYLRSLVQYRSKEIKKSATNMLDTTAIAVDEVTDSIIGFAQRIWRGICNIRARIRQGYEAG GQ |
| C.NP.11.11N P007.KJ15842 3 | C | 247 | MRVRGILRNYQQWWIWGILGFWMFMICNGVGNLWVTVYYGVPVWREAKTTLFCASEAKSYE KEVHNVWATHACVPTDPNPQEVLDNVTENFNMWENDMVEQMHEDVISLWDQSLKPCVKLT PLCVTLNCTDINITGGGAECMASNERIREVRNCSFNATTEIRDKKQKVYALFYKLDIVSLYNLDIV AIQASSGHYRLINCNTSAITQACPKVSFDPIPIHYCVPAGYAILKGNNKTFKGPGHGNKVSTVQTH GIKPVVSTQLLLNGSLAEEEIIIRSKNLSDSTKIIIVQFNQSVKIVCARPNNNTRKSIRIGPGQVFYAT GDILGDIRQAHCNISKNKWKDTLHNVSKILAEQFSIKTIIFTSSSSSTLNFNRHSFNCRGAFLYCNS CYLFNRLYTAANTKSNSWIDINCTVAIPCRIKQIINMWQEVGRAMYANPIEGNITCKSDITGMLLE RDCGKERDKTISTTKEIFRPIGGNMRDNWRSELYKYKVVEIKPLGVAPTEAKRRVVEREKRVAG LGAVFLGFLGAAGSTMGAASITLTVQARQLLSGIVQQQSNLLRAIEAQQHLLQLTVWGIKQLQT RVLAIERYLKDQQLLGLWGCSGKLICTTAVPWNSNWSSKSQTEIWNHTTWMQWDREINNYTNT IYQLLEDSQNQQEKNEKDLLALDRWKNLWNWFDISNWLWYIKIFIMIVGGLIGLRIIFAVLSIVNR VRQGYSPLSFQTLTPNPRELDRLGRIEEEGGEQDRDRSIRLVNGFFSLAWDDLRSLCLFCYHRLT DLISVTARAVELLGRSSLRGLQRGWKVLKYLGSLVQYWGLELKKSAIRLLDITAIAVAEGTDRII EFFQRLCRAICNIPRRIRQGFEAALQ |
| C.ZA.09.704 MC003N.GU 080161 | C | 248 | HQWWIWGILGFWMIYNVVGNLWVTVYYGVPVWKEAKTTLFCASDAKAYEKEAHNVWATHA CVPTDPNPLEIVMGNVTENFNMWDNSMVEQMHEDIISLWDESLKPCVKLTPLCVTLECDNVTYT GNEDSITEKEMKNCSFNATTEIEDKKRKERVLFHSLDIVPLENNNSSNNGSSNKKYRLINCDTSTT TQACPKVSFDPIPIHYCAPAGYAILKCNNETFNGTGPCHNVSTVQCTHGIKPVVSTQLLLNGSLAE KEIIIRSENLTENAKIIIVHLNESIEIMCIRPGNNTRKSVRIGPGQTFYANDIIGDIRKAHCKVNRSKW NETLQKVGIKLKEHFNKTIIFESSSGGDPEITTHSFNCGGEFFYCNTSGLFNGTFNGTYTNSTQNET TITLPCRIKQIINMWQGVGRAMYAPPIAGNITCTSNITGLLLLRDGGNTTNSKEEEIFRPGGGNMR DNWRSELYKYKVVEVKPLGVAPTGAKRRVVEREKRAVIGAVVLGFLGAAGSTMGAASIALTA QARKVLSGIVQQQSNLLRAIEAQQHLLQLTVWGIKQLQARVLAVERYLQDQQLLGIWGCSGKLI CTTNVPWNSSWSNKSLDDIWGNMTWMQWDKEVSNYTHTIYSLLEESQIQQERNEKELLALDK WQSLWNWFDITNWLWYIKIFIMIVGGLIGLRIIFAVLSIVNRVRQGYSPLSFQTLTPTSRGPERLGG IEEEGGEQGKDRSIRLVSGFLTIVWDDLRSLCLFSYHLLRDFVLIVARGVELLGRRGWETLKYLG SLVQYWGLELKKSAISLLDTIEIIVAEGTDRIINFLQSICRAIRNIPRRIRQGFEAALL |
| C.ZA.05.05Z AFV26.DQ38 2378 | C | 249 | MRVRGIQRNWPQWWIWGILGFWIILSCRGVGDLWVTVYYGVPVWREAKTTLFCASDAKAHVR EVHNIWATHACVPTDPNPQEMVLENVTENFNMWKNDMVDQMHEDIINLWDQGLKPCVKITPL CVTLRCTNATFTANDSNDNNVTMSEWTEMTTCSFSNTTQIGDALQTAYALLYLTDSKPLFERCY TSIHLPYLTHGTLGDPKASCYRAPIHYCAPAGYAIRKCNDRTFNGTGPCNRVSTVQCTHGIKPVV STQLLLNGSLAEEEIMIRSENLTDNTKTIIVQLKEPVEINCTRPGKGKRTRVRIGPGRTFYATGAVT GDIRKAHCTVNGSRWNSTLEQVREKLKKYYDNRTIKFEQPSGGDPEVTTHSFNCRGEFFYCDTT NLFNISNINSTSNITLQCKIKQIINMWQKVGRAMYAPPIEGTITCISNITGLLLTRDGGSSNETSSIN KTEIFRPGGGNMKDNWRSELYKYKVVEIKPLGIAPTGAKRRVVEREKRAVGIGAVFLGFLGAAG STMGAASITLTVQARQLLSGIVQQQSNLLRAIEAQQHMLQLTVWGIKQLQARVLALERYLTDQQ LLGLWGCSGKLICTTNVPWNSSWSNKSRADIWDNMTWMQWDREINNYTDTIYRLLEESQTQQE KNEKDLLALDSWKNLWNWFNISNWLWYIKIFIMVVGGLIGLRIILGVLSIVKRVRQGYSPLSFQT LIPNPRDPDRLGGIEEEGGEQDRDRSIRLVSGFLSLAWDDLRSLCLFSYHQLRSFILVTARAVQLL GRSSLRGLQGGWEALKYLGNLVQYWGLELKRSAISLLDTIAITVAEGTDRIIELIQGICRAICNIPR RIRQGFERALL |
| C.ET.08.ET11 9KU319532 | C | 250 | MRVMGTSRNCQQGWIWGILGFWMFMICNVLGNLWVTVYYGVPVWKEAKTTLFCASDAKAYD TEVHNVWATHACVPTDPNPQELVLENVTENFNMWKNDMVDQMHQDVISLWDESLKPCVKLTP LCVTLNCSDATINGTIVEKMKNCSFNVTSTLKPTKLKAYALIYRTELVPLIENQINGNYMITGYLN NLNITKSGQTVSFGPFRPHPWPHAGQASLTVKCNNISFKGTGPCNKVSSVQCTQGIKLVEWTRL MLYGSIAEEQIEIRCENLTIKTGLIIVQLKESVAIRCERPKNNTRGSMRVGPGQTFYATGDIIGDIRQ DHSNISEKEWNETKQKVVEKLIELLPTTRKLTRSSGGDPEITTHSFNCRGEFFYCDTTNLFNSTYN GTSTNSTSSGTITLSCSIKQIIQMWQGLRKVLNAPVCAPLDGNIKCTSNFTGLLLPRAGGSGLNDT DAEVFRPAGGDMRDNWRSELYKYKVVEIKPLGVAPTTAKRRVVEREKRAVGIGAVFLGFLGAA GSTMGAASITLTVQARQLLSGIVQQQSNLLRAIEAQQHLLQLTVWGIKQLQTRVLAVERYLQDQ QLLGIWGCSGKLICTTAVPWNSSWSNRTQDEIWNNMTWMQWEKEINNYTGTIYRLLEDSQNQQ EKNEKDLLALDKWQNLWSWFSISKWLWYIKIFIMIVGGLIGLRIIFAVLSIVNRVRQGYSPLSFQT LIPVPRTDRLSMCLFSYRHLRDVLLIAARILNLLGQRRWETIKYLGSIVQYWSIELKKSAINLLDAI AIVVAEGTARILEAIQRIWRAIFNIPRRIRQGLEAALQ |
| C.ES.x.MH08 .EF531335 | C | 251 | MRVTGTRKNCQQWWIWGILGFWMLMIYNVKGLWVTVYYGVPVWREANTTLFCASDAKAYD KEVHNVWATHACVPTDPKPQEQWLKNVTEDFNMWKNNMVEQMHEDIISLWDQSLKPCVKLT PLCVTLNCTNATSIVNMTDVASNRSRSNDTVENVNGTSVENMTIVNSTDEIKNCSFNITTGIGDKP KKESALFYSLDIVPLNKSANNSEYILITCKTSKITQACPKVSFEPIPIHYCAPAGYAILRCDNKTFNG TGPCNNVSSVQCTHGIRPVVTTQLLLNGSLAEEDIIIRSVNMSNNAKTIIVHLNESVEIICTRPNNN TRKSVGMGPGQAIYTTGAIIGNIRQAHCNISGRNRSRTLQRVSEKLQKYFYNKTIKFQPHSGGDPE ITTHSFNCGGEFFYCNTSDLFNSTYFNNTYFNGVYNGTRDNGTITLPCRIKQFINMWQRVGRAM YAPPIQGLITCRSNITGLLLTRDGGNTINNTNNNTEIFRPGGGDMRDNWRSELYKYKVVEIRPLG VAPTDARRRVVGREKRAVMGALFLGFLGAAGSTMGAASMALTVQTRQLLSGIVQQQSNLLRAI EAQQHMLQLTVWGIKQLQAGVLALERYLQDQQILGIWGCSGKRICTTDVPWNSSWSNKTKEYI WGNMTWMQWDKEISNYTGIIYDLLEESQNQQEKNEQELLALDSWKNLWNWFNITNWLWYIKI FIMIIGGLIGLRIIFAVLSIVHRVRQGYSPLSFQTLIPNQREPDRPGRIEEEGGGQDRDRSIRLVSGFL ALAWGDLRSLCLFSYHRLRDCISIAARAGELLGRSSLKGLQRGWETLKYLGSLVQYWGLELKKS AISLLDTIAIAVAEGTDRIIEFIQRICRAICNIPRRIRQGLERILL |
| C.ZA.09.704 MC019F.GU0 80178 | C | 252 | MRVTGIPRNWPQWWIWGILGFWIMVMCNKTENLWVTVYYGVPVWTDAKTTLFCASDAKAYV KEVHNVWATHACVPTDPNPQELELKNVTEDFNMWKNDMVDQMHEDVISLWDQSLKPCVKLT PLCVTLNCTEVTPNCTKVNNSSSNNTTVAPTTATCNNHTKDNASIIGEREMRNCSFNITTEIRDKK QKAYALFYRPDIVPLNGNPNTTEYILINCNTSTITQACPKVTFDPIPIHYCAPAGFAILKCNNKTFN GTGPCNNVSTVQCTHGIKPVVSTQLLLNGSLAEEDIIIRSENLTSNTKTIIVHLNESVKIECLRPGN NTRKSIRIGPGQTFYATGAIIGDIRQAHCNISTLAWNATLERVRGKLKEHFPNKTINFTSPAGGDL EVITHSFNCQGEFFYCNTTNLFDKFNSSGPNITTIPCRIKQIINMWQEVGRAMYAPPIAGNITCNSN ITGLLLTHDGINITYNGTGNTTIIFRPGGGNMKDNWRSELYKYKVVEIKPLGVAPTKAKRRVVER EKRAVGLGAMILGFLGTAGSTMGAASITLTVQARQLLSGIVQQQSNLLRAIEAQQHMLQLTVW GIKQLQARVLAIERYLKDQQLLGLWGCPGKLICTTNVPWNISWSNKSYNKIWDEMTWMQWDK EISNYTDTIYRLIEDSQSQQEKNEKDLLALDSWNNLWNWFNITNWLWYIRIFIMIVGGLIGLRIIFC VLSVVRRVRQGYSPLSFQTLIPNPRGPDRPERIEEGGGEQDRRKSIRLVSGFLALAWDDLRSLCLF SYHHLRDFILIVLRAVELLGRSSLRGLQRVWEALKYLGNLVLYWGQELKKSAISLLDTIAIAVAE GTDRILEVLQGIWRGIRNIPRRIRQGFEASLL |
| C.ZM.02.ZM2 11M | C | 253 | MRVMGILRSYQQWWIWGILGFWMVMTCNVGGNLWVTVYYGVPVWKEAKTTLFCASDAKSY EKEVHNIWATHACVPTDPDPQELVLENVTENFNMWKNDMVDQMHKDIISLWDQSLKPCVKLT PLCVTLNCTDVKGNVTYSDKGNLAHSNSTDEMKNCTFNVTTELRDKSKKESALFYRLDIETLDP KGNSSNNNSSYKEYILINCNTSTIAQSCPKITFDPIPIHYCAPAGYAILKCNNKAFNGTGPCNNVST VQCTHGIKPVVSTQLLLNGSLAEGGIIIRSANLTDNAKTIIVHLNESVGITCVRPGNNTRKSVRIGP GQTFYATGQIIGDIRQAHCNITGSEWNRTIDRVGKKLQEHFPNKTINFTQPSGGDLEITMHSFNCG GEFFYCNTSGLFNTSGLFNDTEGWINPINKTSNITIQCRIKQFINMWQEVGRAMYAPPIAGNITCTS NITGLLLTRDGGNSSSNTEIFRPGGGDMRDNWRSELYKYKVVEIKPLGIAPTTAKRRVVEREKRA ALGAMFLGFLGAAGSTMGAASVTLTVQARQLLSGIVQQQSNLLRAIEAQQHLLQLTVWGIKQL QTRVLAIERYLKDQQLLGIWGCSGKLICTTAVPWNSSWSNKTQNEIWNNMTWMQWDKEVSNY TDQIYRLLEKSQNQQEKNERDLLSLDSWNSLWSWFSITKWLWYIKIFIMIVGGLIGLRIIFAVLSV VNRVRQGYSPLSFQTLIPSPRGPDRPGRIEEEGGEQDRNRSTRLVSGFLALVWDDLRSLSLFSYHR LRDFILIATRAAELLGRSSLRGLQRGWEALKYLGNLVQYWGQELKRSAISLFDTIAIIVAEGTDRII ELAQRICRARQGETALL |
| C.SE.06.018S E.MF373138 | C | 254 | MRVMGIQRNCQQWWIWGILGFWMLLICNGMGNLWVTVYYGVPVWKEASPTLFCASDAKAYE SEVHNVWATHACVPTDPSPQELVLKNVTENFNMWKNDMVEQMHQDIISLWDQSLKPCVKLTP LCVTLNCSNVDNSTTINGTIHPNEEGKNCSFYITTKLKLKIRQKFALFYDPDNLILIDNDNSMYLL NCNTSTITQTCPWVSFDFDPIPYCDRAAYGILIRNNNKFNVTGPCPSVNAGQCTRGIGTMPSTPLL LNGNITKEDIVIISEYLDNNANVIIIQLNKTVPVNCTRPRHNTRTRVRMRPGQRLYVSADIIGNIRL AHCHCIGSGRYDTLELVKLNTLEHCLYHKTIMFTHPSGGALPTTTHTLNCGGELFYCSTSSLLNF TWRSLFCHAQAPFQETHNNSTNNIPFPCRIKQFINMWQTVGRAMYAPPIAGVITCNSNITGLLLTR DGGNPTNSTEGNNETEIFRPGGGDMRDNWRSELYKYKVVEIKPLGVVPTKAKRRVVEREKRAA LGAMFLGFLGAAGSTMGAASIALTVQARQLLSGIVQQQNNLLRAIEAQQHMLQLTVWGIKQLQ TRVLALERYLRDQQLLGIWGCSGKLICTTAVPWNSSWSNKSQEEIWDNMTWMQWEREIDNYT DIIYNLLEVSQIQQETNEKDLLALDKWHNLWSWFDISNWLWYIKIFIMIVGGLIGLRIIFAVLSIVN RVRQGYSPLSFQTLIPSPRGPDRPGGIEEEGGENDRGRSIRLVSGFLGLAWDDLRSLCLFCYHRLR DLLLIAARVVELLGQRGWEILKYLGSLVQYWGLELKKSAISLLDTVAIVVAEGTDRIIELIQRAW RTICNIPVRIRQGFEAALL |
| C.SE.07.SE60 0119.KP4118 31 | C | 255 | MTVMGIQMNCPQWWRWGILSLWMFMIWNVWGKGNLRVTVYYGVPVWVEAKTTLFASDANA HSEEVHNVRATHACVCAHPNPQEINVTENFNMCKNDMEKQMHEDIVSLCHQSLKPCVRVTPLC VTCHLTSSVITDTDTISSSLIINDDDSNMNCCSFITTAVNRDKKKDDALYYRLVVVPDDEEKKN NCSECRLINCNSSAVPQACTKVSDEPILIHYCAPAGYAIIKCNDTTFNATAPCNNVTALQCPRGITP VVSHQLLLNGSAAERGDSIRSENITNNATTIIVHLKNPVEISRNYCTRPHINTRKSMRIGPGQIFYA TGDIIGNIRQAHCNISAKEWNETLLMVSEKLLKHFPNKTTINFPSSSGEDQTITKRSFCRGEQFFYC NTSILSNTTYSYNSTSNTTCSTNNTTTSTFTLQCRRIHIIIMCLEPGLAIDASPTAGEIISKSNTTDLL LTRAGGSNRTEIFRPGGGDMRDNWRSELYKYKVVEIKPLGIAPTKAKRRVVEREKRAVGIGAVF LGFLGVAGSTMGAASITLTVQARQLLSGIVQQQSNLLRAIEAQQHMLQLTVWGIKQLQTRVLAI ERYLKDQQLLGIWGCSGKLICTTNVPWNKSWSNKTQKDIWDNMTWMEWDREISNYTDIIYSLL VDSQTQQEKNEKELLALDSWKNLWNWFNITNWLWYIKIFIMIVGGLIGLRIIFAVLPIINRVRQGY SPLSFQTIIPNPRGPDRLGRIEEEGGEQDRNRSIRLVNGFLALAWDDLRSLCLFSYHRLRDFILIVV RAVELLGHSSLRGIQRGWEALKYLGSLVQYWGLEIKKSAISLFDTLAIVVAEGTDRIIELVQRIFR AICNIPRRIRQGSEASLL |
| C.CY.07.CY2 05.JF683757 | C | 256 | MRVRGILRNWQQWGIWGILGFWMLMICNVVGNKDLWVTVYYGVPVWKEAKTTLFCASNAKA YEREVHNVWATHACVPTDPNPQKIDLENVTENFNMWKNDMVDQMHADVISLWKESLKPCVK LTPLCVTLNCTEVTVIKNTGVNMTNNSTSTSIDTSILNDTSNADLKNCSFNTTTKISDNEIFEFAVN NTLDVEPIDGTYTSITFRLTNSNSSSIPQACPKVSFDPLPIPYCAPAGYTGLRCSNNTFTGTGPCRN VSTVQCTHGIKPVVSTQLLLNGSLAEGEIMIRSENLTNNAKTIIVHLNESVNIVCTRPNNNTRKGV GIGPGQAIYATGAIIGDIRQAHCNITKRGWNGLPQKVKGKLREHLPGKLIIFQPHSGGDLQITMHT FNWGGEFFYCNTSVLSNSTHNSGTGWHLQSSDINSYATIAVSYRIGQIINMWRQVGRAIYGPPVA RKMSAHPSTTGLLLVCDGGEKSLNSTNGTGITNATEIFRPQGGDMKDNWRSELYKYKVVEIKPL GIAPTEARRRVVEREKRAVGIGAVILGFLGAAGSTMGAASIALTAQARTVMAGIVQQQSNLLRAI DAQQHMLQLTVWGIKQLQARVLALERYLKDQQLLGIWGCSGKLICTTTVPWNTSWSNRSKEEI WNNMTWMQWEREIDNYTEIIYQLLGESQIQQEQNEKDLLALDSWKNLWSWFSISNWLWYIRIFI MIVGGLIGLRIIFAVLSIIHRVRQGYSPLSLQTLIPNSREPLDRLGRIEEGGGEQDRSRSIRLVNGFL ALAWDDLRSLCLFCYHLLRDFILIVARVGELLGHSSLRGLQKGWEALKYLGSLVQYWGLELKK SAISLLDTVAIAVAGRTDRIIEVIQEICRAIYHIPRRIRQGFEAALL |
| C.SE.06.SE60 0516.KP4118 39 | C | 257 | MKGRRNTENLSPGWIWGILGFWMLMMHKGEGNLWFTVYYGLPVWTEPKPTLFGASNAKGYK TEVHMVWATHACVPTNPNQQDLVLENVTENFSMWKNDMLDQMHEHVVIFSDQGLKPYAKVT SLTVTVTCTDLKNSTFTSTSSNDTSMVTESEEMKNCSFNITTEIRDKKKKEYALFYRLDIVPLKEK EDNNSDYRLINCNTSAVTQACPKVSFDPIPIHYCAPAGYAILKCNNKTFNGTGPCNNVSTVQCTH GIKPVVSTQLLLNGSLAEKEIVIRSENLTNNAKIIIVHLNESVEIVCTRPNNNTRKSMRIGPGQTFY ATGDIIGNIRQAYCNISEKRWNETLQKVSKKLREHFPNKTIKFKPSSGGDLEITTHSFNCGGEFFY CNTSKLFNSTYPPNSTDTYMNSANTSNIILQCRIKQIINMWQGVGQAMYAPPIAGNITCESNITGL LLTRDGGNNQTEEETFTPGGGDMRDNWRSELYKYKVVEIKPLGIAPTKAKRRVVEREKRAVGIG AVFLGVLGAAGSTMGAASITLTVQARQLLSGIVQQQSNLLRAIEAQQHMLQLTVWGIKQLQTRV LAVEGYLKDQQLLRIWAASGKLICTTNVPWTPSWSIKSQEEIWNNMTWMQCDREITYTENLIQC LAPQPRKWKDTTCNGDLDLDKWQNLWKWFNIPKWLWYIELFIMIVGGLIGLRIIFAVLSIVNRV RQGYSPLSFQTLLPSPRGPDRLGGTEEESGEQDRDRSVRLVNGFLPLVWNDLRSLCLFCYHQLRT FILVTARAVELLGRSILRGLQTGWEALKYLGGIVQYWGLELKKSAISLFDTLAIVVAEGTDRIIEV VRGFFRAIYNIPRRIRQGLEAALQ |
| C.ET.08.ET12 4.KU319534 | C | 258 | MRVMGIHEELSTMGWIWGILGFWMLLICNVMGNLWVTVYYGVPVWTEANPTLFCASDAKSYE PEVHNVWATHACVPTDPNPQELVLENVTENFNMWKNGMVEQMHQDIISLWDQSLKPCVKLTP LWVTLNCSDVNVTAIIDADILTMYEPVINRSFSTTPKLKKKTETDLFHYVEVEALDMTATDDGYI NCNTSTLTITCPCVSFAPVPIPYCAPAGYAIPIRNGNNFTGPGPCPSVNAFQGTRGLEPVASTPLLLI GNVAKNEIVIISETLTHNVKMVMVQLNETVTINCTRPNNNTRKSIRIGPGQAFPATATIEGDIRQA HCNINKTEWNNTLQKVREKLRKHFNKTIKFDSSSGGDPEITTHSFNCRGEFFYWDTTKLFNETYN NTDANGTIIMPCRIKQIINMWQKVGRAMYAPPIEGPITCISNITGLLLTHDGEENDTETFRPWGGD MRDNWRSELYKYKVVEIKPLGIAPTKAKRRVVQREKRAALGAMFLGFLGAAGSTMGAASITLT VQARQLLSGIVQQQSNLLRAIEAQQHLLQLTVWGIKQLQTRVLAIERYLKDQQLLGIWGCSGKLI CTTAVPWNSSWSNKSQEEIWDNMTWMQWDREINNYTGIIYNLLEVSQNQQEKNEEDLLALDK WQNLWNWFDITQWLWYIKLFIMIVGGLIGLRIIFAVLSIVNRVRQGYSPLSFQTLIPNPRGPDRPG GIEEEGGEQDRDRSIRFVNGFLALAWDDLRSLCLFIYRQLRDLILIAARAVEILGQRGWETLKYLG SLVQYWGLELKKRAISMLDTTAIVVAEGTDRIIEVVQRTWRIICNILTRIRQGFEAALL |
| C.SE.08.SE60 0213.KP4118 34 | C | 259 | MRVMGIQRNYQRWWIWGILGFWMFMICNVRGNLWVTVYYGVPVWKEADPTLFCASDAKAYE REVHNVWATHACVPTDPSPQELVLENVTENFNMWKNGMVDQMHQDIISLWDQSLKPCVKLTP LCVTLSCNNTIVVNATIRMIQKDCSFNTTTELRDKRQTAYALFYKLDIVALSEKSSEYRLINCNTS AITQACPKVSFDPIPIHYCAPAGYAILKCRDANFNGTGPCKNVSTVQCTHGIKPVVSTQLLLNGSL AENETIIRSENLTNNAKIIIVQLKTPVEINCTRPANNTRKSIRIGPGQTFYATEEIIGDIRQAHCNISG KTWNATLKDVGDKLKRHFPNGTIKFLPSSGGDIQITTHSFNCGGEFFYCCTSYLLNNTLDNNTIN DTTNNGGTNDSIILQCRIKQIINVGQEVGRAMYAPPIKGNITCRSNITGILLAEDGGPINETETFRRG GGDMRDNWRSELYKYKVVEIKPLGIAPTKAKRRVVGREKRAALGAMFLGFLGAAGSTMGAASI TLTVQARQLLSGIVQQQSNLLRAIEAQQHMLQLTVWGIKQLQTRVLALERYLRDQQLLGIWGCS GKLICTTAVPWNSSWSNRSQEEIWENMTWMQWDREINNYTNIIYELLEVSQNQQEKNEKDLLEL DKWQNLWNWFDITHWLWYIKIFIMIVGGLIGLRIIFAVLSIVNRVRQGYSPLSFQTLIPNRRGPTSP GGLEEEGEDQDRGRSFRLVRGFLALPWDVLRTLCLFSSHRWRDLFWIAARAVELLDQRGWKTF NFLGTLVQYGGRKLKKSVISLFNTPPILVAEETDRFMKLFQRFGRAFSNIPGRLSQGLEAAFQ |
| C.ZA.99.LT2 5.AY522727 | C | 260 | MRVRGILRNYPQWWIWGILGFWMIYNVMGNLWVTVYGVPVWKEAKTTLFCASDAKAYDREV HNIWATHACVPTDPNPQELVLKNVIENFNMWKNDMVDQMHEDIISLWDESLKPCVKLTPLCVT LNCTKVTVNGTASNSTATNDTATNGTNTVNSTDATKTKIGNSTDDMKKCFFNVTTEIRDKEKKE HALFYKLDIVPLGNSSRNNYSEYRLINCNTSAITQACPKVNFDPIPIHYCAPAGYAILKCNDETFN GTGPCNNVSTVQCTHGIKPVVSTQLLLNGSLAEKETIIRSENLTENTKIIIVQLNRSVEINCTRPGN NTRTSIRIGPGQSFYATGEIIGDIRQAHCNISAKKWNETLQEVGKKLQEHFENKTIIFANSSGGDLE ITTHSFNCRGEFFYCNTTSLFNNIYSNKTGSPNEKEIITLPCRIKQFINMWQKVGRAMYAPPIAGNI TCKSNITGLLLLRDGGNFSRDNETFRPGGGDMRDNWRSELYKYKVVEIKPLGVAPTKAKRRVV EREKRAALGAVLIGFLGAAGSTMGAASMALTVQARQLLSGIVQQQSNLLKAIEAQQHLLQLTV WGIKQLQARVLAIERYLKDQQLLGIWGCSGKLICTTAVPWNSSWSNNRTHDYIWENMTWMQW DREVNDYTDTIYRLLEKSQNQQEENEKDLLALDSWNSLWNWFSITKWLWYIKIFIMIVGGLIGLR IIFTVLSLVNRVRQGYSPLSFQTLTPSPGGPDRLERIEEGGGEQDRSRSIRLVSGFLSLAWEDLRSL CLFSYHRLRDFILVTARAVELLGRSSLKGLQRGWEALKYLGSIVQYWGLELKKSAISLLDTIAITV AEGTDRIIEFIQSICRAIRNIPRRIRQGFETALL |
| C.ET.08.ET16 5.KU319549 | C | 261 | MRVMGISRNCQQGWILGILGFWMLLMCNGTGNLWVTVYYGVPVWKEANPTPPLFCASDAKAY ETEVHNVWATHACVPTDPDPQELELKNVTEDFNMWKNGMVDQMHQDVISLWEQSLKPCVKM TPLCVTLNCTYVTSKNTTDNSTSTMLLSVNATDSDMKNCSFNVTTELKDKQKKVYALFYLLDIR QLNDSSNTHTGNSSNTGNYSDYILINCNTSTIAQACPKVSFDPIPIPYCAPAGYAILKCNDKNFNG TGTCTNVSTVQCTRGIKPVVSTPLLLNGSISEGDIVIISKNLTDNAKTIIVQLNQTVAVNCTRPTNN TRTGIRIGPGRAFYATGDIIRDIRQAHCNISGGDCNRTLEEVEKKLNESFSGNETIQFEPTSGGDPEI TTRSFNCGGEFFYCKTSKLFIKTWKSWNHRNDGTIITIPCRIKQIIHMWQGVGRAMYAPPISGPITC RSNITGLLLTRDGGISDNNSTNTTEIFRRQGGDMKDNWRSELYKYKVVEIKPLGIAPTKAKRRMV QRERGEKRHGAMFLGFLGAAGSTMGAASITLTVQARQLLSGIVQQQSNLLRAIEAQQHMLQLT VWGIKQLQTRVLALERYLRDQQLLGIWGCSGKIICTTAVPWNSSWSNKTHDQIWKNMTWMQW EREIDNYTGIIYNLLEVSQIQQEQNEQDLLALDKWQNLWSWFDITNWLWYIKIFIMIVGGLIGIRII FAVLSIVNRVRQGYSPLSFQTLIPNPRGPDRPGGIEEEGGEQDRDRSVRLVNGFLALAWDDLRSL CLFCYHRLRDLILIAARVLELLGQRGWEALKYLKNIVQYWGLELKKSAVSLLDTVAIAVAEGTD RIIEVIQRAWRAFCNIPTRIRQGFEIALL |
| C.ZA.02.02Z APS015MB1. DQ369995 | C | 262 | MKVKGTRRNCQQWWIWGILGFWMVYNVVGNLWVTVYYGVPVWREAKATLFCASDAKAYEK EVHNVWATHACVPTDPNPLERVLENVTENFNMWKNDMVDQMHEDIISLWDESLKPCVKLIPLC VTLNCTTAKNKSEEMNCSFKVTTELRDKKRKEYALFYRLDVVPLNETNESSNDTNGDFREYRLI NCNTSTLTQACPKVTFDPIPIHYCAPAGYAILKCNNKTFNGTGPCNNVSTVQCTHGIKPVVSTQL LLNGSLAEEEIIIKSENITDNAKTIIVQLNKAIEIECIRPSNNTGRSVRIGPGQTFYTTGEIIGDIRQAH CNISGNWDRTLYNISKKLGEHFPNKNISFKPSSGGDPEITTHSFNCGGEFFYCDTSQLFNKTYNVT DDSNTTDTNNTIQCRIKQIINMWQEVGRAMYAPPIAGSITCTSNITGLLLTRDGGGGTNSTSNTEE IFRPGGGNMKDNWRSELYKYKVVEVQPLGIAPTKAKRRVVEREKRAIGIGAVFLGFLGAAGSTM GAASITLTVQARQLLSGIVQQQSNLLRAIEAQQHMLQLTVWGIKQLQARVLAIERYLKDQQLLGI WGCSGRQICTTNVPWNTSWSNKSLNDIWNNMTWMQWDKEISNYTNIIYQLLELSQNQQEQNEK DLLALDKWQNLWSWFDITHWLWYIKIFIMIIGGLIGLRIIFAVLSIVNRVRQGYSPLSFQTLIPSQR EPDRLGRIEEEGGEQDKDRSIRLVHGFLAIAWDDLRSLCLFSYHRLRGFILVTTRVVGLLGQRGW EALKYLGSLVQYWGLELKKSAISLLDTIAIVVAEGTDRIIELILGICRAIRNLPRRIRQGFEAALL |
| C.ZA.09.707P KE02N3.HM6 23589 | C | 263 | MRVRGTQMNCQGWWRWGIMIIGLIIICRAKEDLWVTVYYGVPVWKDAETTLFCASDAKAYDT EKHNVWATHACVPTDPNPQEIDLDNVTENFNMWKNGMVDQMHKDIINLWDQSLKPCVKLTPL CVTLNCTNININNNSTDNGTSITNEIGDEIKNCSYNITTEFRDKRRRVSSLFYRHDIMPINEKNDSRT YILINCNTTTITQACPKVSFEPIPIHYCAPAGYAILKCKDKDFNGTGPCKNVSTVQCTHGIKPVVST QLLLNGSLAEGGIKIRSENITNNAKTIIVQLDTPVKINCSRPNNNTRDRIGIGPGQAFFASNRIIGNIR QAHCNISASIWNMILQKVAEQLAKQFPNKNISFTNHSGGDLEITSHTFNCGGEFFYCNTSSLFNRT FPANYTYNRTADSTQGNTTITIPCRIKQIINMWQRVGQAMYAPPIEGVIRCESNITGILLTRDGGNE TNVEVFRPEGGNMRDNWRSELYNYKVVRIEPLGVAPTRARRRVVEREKRAVGLGAVFLGFLGA AGSTMGAASITLTVQARQLLAGIVQQQSNLLQAIEAQQHLLKLTVWGIKQLQARVLALERYLRD QQLLGIWGCSGKTICTTTVPWNSSWSNKSQEEIWGNMTWLQWDKEISNYTDLIYVLLQESQNQ QDRNEQELLALDKWANLWNWFDISNWLWYIRIFIMVVGGLVGLRIVFAVISIVNRVRQGYSPLS LQTLTPNQEGLDRLGRIEEGGGEQGRGRSIRLVSGFFSLAWDDLRSLCLFSYHRLRDLVLIAARIV ELLGSSLLKGLRLGLEGLNYLWNLLLYWGGELKTSAISLFDTTAIAVANWTDRAIELGQRICRAI RNIPRRIRQGFETALL |
| C.ET.08.ET15 4.KU319545 | C | 264 | MRVRGISRNCQQGWIWGILGFWMLMICNVWGNLWVTVYYGVPVWKEAKTTLFCASEAKAYE TEVHNVWATHACVPTDPNPQEMTLENVTENFNMWKNDMVDQMHEDIISLWDQSLKPCVKLTP LCVTLNCTNATITGDGNNTDGTNGTARLLIYHGDIANCSFNETTELKDKVSKEHDLFYRLDVKPL NEEKNNSTHNTSILINCSSSKVTHPCPKVSFDPIPIPYCAPAGYAILECRDKAFSGTGPWPYVVTLQ CPHGNWPVVSTQLLMYGFLSENRRDNTNNITVHLSESVDIMCTRPSNNTRRRIIGIRPGRVSMMR GDMIIYLRATYCNIRDTRAACNITLRDVVDELHLQFDETITIAPSSGGPPEDNTNSSTCGEDLCCC NTSQLIHSSICHNNPTSEPSKNRAANPTFIIPCRVKQVLSMRLVLREAMYAHPISGDVLLTSDVGG LISTRDNANSANEIFRPGGGDMRDNWRSELYKYKVVEIKPLGIAPTKAKRRVVEREKRAVGIGA VFLGFLGTAGSTMGAASITLTVQARQLLSGIVQQQSNLLKAIEAQQHMLQLTVWGIKQLQARVL AIEKYLQDQQLLGIWGCSGKLICTTTVPWNSSWSNKSLEEIWDNMTWMQWEREVGNYTEIIYKL LEDSQSQQEKNEKDLLALDNWNNLWSWFNISNWLWYIKIFIMIVGGLIGLRIIFAVLSMVRRVRQ GYSPLSLQTLIPNPRGPDRLGGIEEEGGEQDRDRSVRLVSGFFSLAWDDLRSLILFLYHHLRDLLLI AARTVEILGQRGWEALKYLGSLVQYWGLELKKSAISLLDTIAISVAEGTDRIIEIVQRIWRAIYNIP RRIRQGFEAALL |
| C.ET.08.ET16 7.KU319550 | C | 265 | MRVKGTQQELSTLGWIWGILGFWMLLMNNVGGGKWGVTYYEGVAGRKEATTTLCGAYDAK YDNDVQHVGWTTAGGVPTHPPPQEMLLNVVEDFFMMRNDEVEHMMDDDIILWQRLLPCCVT MTLCGAHSTCSNINSTATINDSDGGMTMCDFIRGEIKNCSFNITTDFRYKLDKEPALFYKPDLVPL NNDNSSYRLTNCNTSAGTQACPKASFDPIPAPYCAPAGYAILECNNGTFNGTRACNNVSTLECTH GIKPLVSTHLLLNENLANDIVIISDNLTIIAVIIIVHLKNDGTIVCTRTSNSTRIRLRIGSGPVCYATG EIIQDIRHAHDHISRSDWNDALRNVREHFRKHFPKFNTMRFTQSVGGNLQITTRRFNCVGEFFHC STTGLFTDTYGTSTCGTNSTGNTSGIHTITLHCKIKQFINMWQGVGLAMYAPSLAGNIICKSNFTG LVLTCDGGEGNNVTNLTETFRPGGGDMRDNWRSELYKYKVVEIKPLGVAPTKAKRRVVEREKR AVGIGAVFLGFLGAAGSTMGAASITLTVQARQLLSGIVQQQNNLLRAIEAQQHMLQLTVWGVK QLQARVLALERYLRDQQLLGIWGCSGKLICTTAVPWNTSWSNKSQEEIWNNMTWMQWEKEID NYTDIIYKLLEVSQTQQEKNEKDLLALDSWQNLWNWFDISKWLWYIKIFIMIVGGLIGLRIIFAVL AIVNRVRQGYSPLSFQTLTPSPEGPDRLRGIEEEGGEQDRTRSIRLVSGFLALAWDDLRSLCLFSY HRLRDFILIVVRAVELLGHRGWWETLKHLGSLELYKGVEIKKNAISLLKPTATAGAEGTVQIIEIV LRIIRSIFNLPRRVSQGFKAAQQ |
| C.ZA.06.C.x.0 6.CF01 | C | 266 | MRVMAIQRNCQHLLSWGTMILGILMICSAANNLWVTVYYGVPVWRDAETTLFCASDAKAYDT EVHNVWATHACVPTDPDPQEIDLKNVTEEFNMWKNNMVEQMHTDIISLWDQSLKPCVKLTHL CVTLNCSTANVNVTDYNITTGDKEEIKNCSFNMTTELSDKKQKVHSLFYRLDVVPIKPDNSDDN RSNSSYSNYRLINCNTSAITQACPKVTFDPIPIHYCAPAGFAILKCKDDGFNGTGPCKNVSSVQCT HGIKPVVSTQLLLNGSLAGKGIRIRSENITDNTKTIIVQLDKPVRINCTRPNNNTRKSMRIGPGQTF FATGDIIGDIRKAHCNISISEWNETLYKVAEQLGGIIGNKTVKFANSSGGDLEITTHSFNCGGEFFY CNTTDLFKGTWHPDNKTWSDPWNASMKSNNTSNVNITILCKIKQIVRMWQRVEQAMYAPPIQG VISCSSNITGLLLTSDGGRDNSNNTGGQKNTETFRPGGGNMRDNWRSELYKYKVVKIEPLGVAP TPARRRVVQREKRAVGLGAVFIGFLGAAGSTMGAASVTLTVQARQLLTGIVRQQSNLLKAIEAQ QHLLRLTVWGIKQLQARVLAVERYLKDQQLLGIWGCSGKLICTTNVPWNSSWSNKNQSEIWDN MTWLQWDKEINNYTQIIYDLLEESQNQQEKNEQELLALDKWTNLWNWFNITNWLWYIKIFIMIV GGLIGLRIVFAVLSIINRVRQGYSPLSFQIHNPNPGGPDRLGRIEEEGGEQDRDRSIRLVSGFLALA WDDLRSLCLFSYHHLRDFILIAARTVEILLRRAWEGLKYLWNLLLYWSRELKISAISLFDTIAIAV AGWTDRVLEIGQAICRAILHIPRRIRQGLERLLL |
| C.ET.08.ET15 9.KU319547 | C | 267 | MMVMGTLKNLSWGRIWGIGFFWMLMICNGEDTLWVTVYYGAAVWKEATTTLCCAYDAIPYE TELHVGWATPACVPTDPHPQEHVFENVLEPFIMWNDDMEQMMHDSIIYLCHRRLPPLVPLCVSQ LCHEVDIYKNTTTANATSSMDVTVTEELKNCSFSTTTDIKDKKQKAYALFYKLDLAPLLIGDDIV INMSCTASTVCRAGAFVPFPPIHCAPCAPAGFASRECKDNEVRGTRHGVTVHRGHGTHAMVPSP PLLLVGNGKQEKEDISDKSKNYANKVIILLLQLRTCEEIKCTKNTTKTRRSVRRGPFFASLEIRGEI RQAPRTNSASNGSAGITLIGEELLQHLDHNTIRSVPSSPCERETLTHTTPSCGWEVFYCVSIQICNG VYFTDTWTTYNSNDADYTDILTLTCRIKLIVNLSQELGRARSAPPIDGSVKCTSNFTGLVLTRAG GNATGDVTAENNTEIFRPGGGEMRDNWRSELYKYKVVEIKPLGVAPTEAKRRVVEREKRAVGI GAVFLGFLGAAGSTMGAASITLTVQARQLLSGIVQQQSNLLRAIEAQQHMLQLTVWGIKQLQTR VLAIERYLKDQQLLGIWGCSGKLICTTTVPWNASWSNKSYNEIWDNMTWMQWDREINNYTDTI YRLLEDSQNQQEKNERDLLALDSWNSLWNWFSISNWLWYIRIFIMIVGGLIGLRIIFAVLAIVNRV RQGYSPLSFQTLIPNPRGPDRPGGIEEEGGEQDRGRSIRLVHGFLALIWDDLRSLCLFCYRRLRDL TLIVARAVELLGQRGWEILKYWGNLVQYWVLQLKNSAISLLDTVAIPLAEGPDRILEVVQRFCR AILNIPRRIRQGFEAALQ |

Notwithstanding the appended claims, the disclosure set forth herein is also defined by the following clauses:
1. A vaccine comprising a set of antigens that are representative of at least 60% of fifth order clades of a microbe.
2. A vaccine comprising a set of antigens of a microbe wherein the smallest pairwise edit distance between two of the antigens is at least 10% of the average size of the antigens and the largest pairwise edit distance between two of the antigens is no more than 98% of the average size of the antigens.
3. A vaccine comprising a set of antigens that are representative of at least 60% of all operational taxonomic units (OTUs) of the microbe.
4. The vaccine of clause 1 or clause 3, wherein an antigen is representative of a clade if its sequence is up to 40% of the average size of antigens of the represented clade different than other members of the represented clade.
5. The vaccine of clause 1 or clause 3, wherein an antigen is representative of a clade if the edit distance is up to 5% of the size of another strain of the represented clade.
6. The vaccine of clause 1 or clause 3, wherein an antigen is representative of a clade if its sequence is up to 20% of the average size of antigens of the represented clade different than at least 95%, 96%, 97%, 98%, or 99% of all strains of the represented clade.
7. The vaccine of clause 1 or clause 3, wherein an antigen is representative of a clade if its sequence is up to 10% of the average size of antigens of the represented clade different than at least 95%, 96%, 97%, 98%, or 99% of all strains of the represented clade.
8. The vaccine of clause 1 or clause 3, wherein the antigen is representative of a clade if the edit distance is up to 5% of the size of 95%, 96%, 97%, 98%, or 99% of all strains of the represented clade.
9. The vaccine of clause 1 or clause 3, wherein an antigen is representative of a clade if its sequence is up to 25 edit distance from all strains of the represented clade.
10. The vaccine of clause 1 or clause 3, wherein an antigen is representative of a clade if its sequence is up to 100 edit distance from all strains of the represented clade.
11. The vaccine of clause 1 or clause 3, wherein an antigen is representative of a clade if its sequence is present in the clade.
12. A vaccine composition comprising a set of antigens, wherein the antigens are derived from a library of variants of a microbe wherein:
   a. two antigens of the set with the greatest edit distance have an edit distance S;
   b. two antigens of the library with the greatest edit distance have an edit distance L; and
   c. S is at least 60% of L.
13. A vaccine composition comprising at least 4 influenza virus hemagglutinin antigen polypeptides, each represented by a sequence that is at least 20% identical, but not more than 95% identical, to the other influenza virus hemagglutinin antigen polypeptides, wherein each polypeptide comprises an antigen that is at least 90% identical among the 4 influenza virus hemagglutinin antigen polypeptides.
14. A vaccine composition comprising at least 4 influenza virus neuraminidase antigen polypeptides, each represented by a sequence that is at least 20% identical, but not more than 95% identical, to the other influenza virus neuraminidase antigen polypeptides, wherein each polypeptide comprises an antigen that is at least 90% identical among the 4 influenza virus neuraminidase antigen polypeptides.
15. A vaccine composition comprising at least 4 HIV gp160 antigen polypeptides, each represented by a sequence that is at least 20% identical, but not more than 95% identical, to the other HIV gp160 antigen polypeptides, wherein each polypeptide comprises an antigen that is at least 90% identical among the 4 HIV gp160 antigen polypeptides.
16. A vaccine composition comprising at least 4 HIV gp120 antigen polypeptides, each represented by a sequence that is at least 20% identical, but not more than 95% identical, to the other HIV gp120 antigen polypeptides, wherein each polypeptide comprises an antigen that is at least 90% identical among the 4 HIV gp120 antigen polypeptides.
17. A vaccine composition comprising at least 4 HIV gp41 antigen polypeptides, each represented by a sequence that is at least 20% identical, but not more than 95% identical, to the other HIV gp41 antigen polypeptides, wherein each polypeptide comprises an antigen that is at least 90% identical among the 4 HIV gp41 antigen polypeptides.
18. A vaccine composition comprising a set of antigens that activate an immune response in a subject to at least 6 strains identified in Table 1.
19. The vaccine composition of clause 13 or clause 18, wherein the immune response is detectable using head-specific antibodies in a hemagglutinin inhibition assay.
20. The vaccine composition of clause 19, wherein the immune response is at least 2 fold greater using the antigens than H1N1+H3N2+HAB, when tested using the hemagglutinin inhibition assay.
21. The vaccine composition of clause 14, wherein the immune response is at least 10 fold greater using the antigens than H1N1+H3N2+HAB, when tested using the hemagglutinin inhibition assay.
22. The vaccine composition of clause 19, wherein the immune response is at least 100 fold greater using the antigens than H1N1+H3N2+HAB, when tested using the hemagglutinin inhibition assay.
23. The vaccine of clause 1, clause 2, clause 3, or clause 12, wherein the microbe is a bacterium.
24. The vaccine of clause 1, clause 2, clause 3, or clause 12, wherein the microbe is a virus.
25. The vaccine composition of clause 24, wherein the virus is influenza.
26. The vaccine composition of clause 25, wherein the influenza is type A.
27. The vaccine composition of clause 25, wherein the influenza is type B.
28. The vaccine of clause 26, wherein the type A influenza is H1N1, H1N2, H3N1, H3N2, or H2N3.
29. The vaccine of clause 28, wherein the type A influenza is H1N1.
30. The vaccine of clause 28, wherein the type A influenza is H3N2.
31. The vaccine composition of clause 24, wherein the virus is human immunodeficiency virus (HIV).
32. The vaccine composition of clause 31, wherein the HIV is HIV-1.
33. The vaccine composition of clause 32, wherein the HIV-1 is of subclade A, subclade B, or subclade C.
34. The vaccine of clause 1, clause 2, clause 3, or clause 12, wherein the antigen is a broadly neutralizing antigen of surface exposed residues adjacent in tertiary space.
35. The vaccine composition of clause 34, wherein the broadly neutralizing antigen is in a stem of hemagglutinin.
36. The vaccine composition of clause 34, wherein the broadly neutralizing antigen is in a head of hemagglutinin.
37. The vaccine composition of clause 34, wherein the broadly neutralizing antigen is in a neuraminidase.
38. The vaccine composition of clause 34, wherein the broadly neutralizing antigen is in a gp160.
39. The vaccine composition of clause 34, wherein the broadly neutralizing antigen is in a gp120.
40. The vaccine composition of clause 34, wherein the broadly neutralizing antigen is in a gp41.
41. The vaccine of clause 1, clause 2, clause 3, or clause 12, wherein the antigen is a broadly conserved fragment of hemagglutinin.
42. The vaccine of clause 41, wherein the antigen is a broadly conserved fragment of a head of hemagglutinin.
43. The vaccine of clause 41, wherein the antigen is a broadly conserved fragment of a stem of hemagglutinin.
44. The vaccine of clause 1, clause 2, clause 3, or clause 12, wherein the antigen is a broadly conserved fragment of neuraminidase.
45. The vaccine of clause 1, clause 2, clause 3, or clause 12, wherein the antigen is a broadly conserved fragment of gp160.
46. The vaccine of clause 1, clause 2, clause 3, or clause 12, comprising at least one antigen selected from SEQ ID NOs: 1-87.
47. The vaccine of clause 1, clause 2, clause 3, or clause 12, comprising at least one antigen that is at least 60% identical to any one of SEQ ID NOs:1-87.
48. The vaccine of clause 1, clause 2, clause 3, or clause 12, comprising at least one antigen that is at least 70% identical to any one of SEQ ID NOs:1-87.
49. The vaccine of clause 1, clause 2, clause 3, or clause 12, comprising at least one antigen that is at least 80% identical to any one of SEQ ID NOs:1-87.
50. The vaccine of clause 1, clause 2, clause 3, or clause 12, comprising at least one antigen that is at least 90% identical to any one of SEQ ID NOs:1-87.
51. The vaccine of clause 1, clause 2, clause 3, clause 12, comprising at least one antigen selected from SEQ ID NOs:88-127.
52. The vaccine of clause 1, clause 2, clause 3, or clause 12, comprising at least one antigen that is at least 60% identical to any one of SEQ ID NOs:88-127.
53. The vaccine of clause 1, clause 2, clause 3, or clause 12, comprising at least one antigen that is at least 70% identical to any one of SEQ ID NOs:88-127.
54. The vaccine of clause 1, clause 2, clause 3, clause 12, comprising at least one antigen that is at least 80% identical to any one of SEQ ID NOs:88-127.
55. The vaccine of clause 1, clause 2, clause 3, or clause 12, comprising at least one antigen that is at least 90% identical to any one of SEQ ID NOs:88-127.
56. The vaccine of clause 1, clause 2, clause 3, or clause 12, comprising at least one antigen selected from SEQ ID NOs:128-171.
57. The vaccine of clause 1, clause 2, clause 3, or clause 12, comprising at least one antigen that is at least 60% identical to any one of SEQ ID NOs:128-171.
58. The vaccine of clause 1, clause 2, clause 3, or clause 12, comprising at least one antigen that is at least 70% identical to any one of SEQ ID NOs:128-171.
59. The vaccine of clause 1, clause 2, clause 3, or clause 12, comprising at least one antigen that is at least 80% identical to any one of SEQ ID NOs: 128-171.
60. The vaccine of clause 1, clause 2, clause 3, or clause 12, comprising at least one antigen that is at least 90% identical to any one of SEQ ID NOs:128-171.
61. The vaccine of clause 1, clause 2, clause 3, or clause 12, comprising at least one antigen selected from SEQ ID NOs: 172-267.
62. The vaccine of clause 1, clause 2, clause 3, or clause 12, comprising at least one antigen that is at least 60% identical to any one of SEQ ID NOs:172-267.
63. The vaccine of clause 1, clause 2, clause 3, or clause 12, comprising at least one antigen that is at least 70% identical to any one of SEQ ID NOs:172-267.
64. The vaccine of clause 1, clause 2, clause 3, or clause 12, comprising at least one antigen that is at least 80% identical to any one of SEQ ID NOs:172-267.
65. The vaccine of clause 1, clause 2, clause 3, or clause 12, comprising at least one antigen that is at least 90% identical to any one of SEQ ID NOs:172-267.
66. The vaccine of clause 1, clause 2, clause 3, or clause 12, wherein the set of antigens comprise at least 5, 6, 7, 8, 9, 10, 15, 20, 30, 40, 50, 60, 70, 80, 90, 100, 200, 300, 400, 500, 600, 700, 800, 900 or 1000 different antigens.
67. The vaccine composition of clause 1, clause 2, clause 3, or clause 12, wherein the set of antigens comprises at least 30 antigens.
68. The vaccine composition of clause 1, clause 2, clause 3, or clause 12, wherein the set of antigens comprises at least 50 antigens.
69. The vaccine of clause 1, clause 2, clause 3, clause 12, wherein the antigens have an average edit distance from each of the other antigens that is at least 5% of the average size of antigens in the clade.
70. The vaccine of clause 1, clause 3, or clause 12, wherein a smallest pairwise edit distance between two or more of the antigens in the set is no more than 1, and the largest pairwise edit distance is at least 5, 10, 20, 30, 40, 50, 60, 70, 80, 90, or 100.
71. The vaccine of clause 1, clause 3, or clause 4, wherein a smallest pairwise edit distance between two or more antigens in the set is no more than 5% of the size of the antigens, and the largest pairwise edit distance is at least 75% of the size of the antigens.
72. The vaccine of clause 1,wherein the clades are clades of a phylogenetic tree is a neighbor joining clustering tree or maximum parsimony tree.
73. The vaccine of clause 1, wherein a first order clade is a clade that is not subsumed in whole or in part within another higher level clade.
74. The vaccine of clause 1, wherein each X-order clade is phylogenetically below X-1 number branch nodes in a phylogenetic tree of the microbe.
75. The vaccine of clause 1, further comprising antigens that are representative of at least 60% of each of third, fourth, fifth, sixth, seventh, eighth, ninth, tenth, eleventh, twelfth, thirteenth, fourteenth, or fifteenth order clades of the microbe.
76. The vaccine of clause 75, wherein a represented clade is a clade Y that is subsumed by a clade Y-1.
77. The vaccine of clause 1, wherein a representative clade is a node-based clade.
78. The vaccine of clause 1, wherein a representative clade is a stem-based clade.
79. The vaccine of clause 1, wherein a representative clade is apomorphy-based clade.
80. The vaccine of clause 1, wherein an average number of branches between each of the antigens in the set is at least 1, 3, 5, or 10.
81. The vaccine of clause 1, wherein a smallest number of branches between any two antigens in the set is no more than 1 and a largest number of branches between any two antigens in the set is at least 5, 10, or 15.
82. The vaccine of clause 3, wherein each OTU comprises sequences that are at least 95% homologous of one another.
83. The vaccine of clause 3, wherein each OTU comprises of at least 3 different sequences.
84. The vaccine composition of clause 1, clause 2, clause 3, or clause 12, wherein each of the antigens in the set shares at least 90%, 95, or 99% sequence identity to at least one other antigen in the set.
85. The vaccine composition of clause 1, clause 2, clause 3, clause 12, wherein each of the antigens in the set shares at least 90%, 95, or 99% % sequence identity to at least one other antigen in the set over a length of at least 100 amino acids.
86. The vaccine composition of clause 1, clause 2, clause 3, or clause 12, wherein each of the antigens in the set differs from each of the other antigens in the set by at least 5% sequence identity.
87. The vaccine composition of clause 1, clause 2, clause 3, clause 12, wherein each of the antigens in the set differs from each of the other antigens in the set by no more than 75% sequence identity.
88. The microbial vaccine composition of clause 1, clause 2, clause 3, or clause 12, wherein each antigen is a peptide comprising at least 10, 15, 20, 25, 30, 35, 40, 45, 50, 55, 60, 65, 70, 75, 80, 85, 90, 85, 100, 110, or 120 amino acids.
89. The vaccine composition of clause 1, clause 2, or clause 3, wherein the antigens are selected from Table 2, Table 3, Table 4, Table 5, or a fragment or homologue thereof.
90. The vaccine composition of clause 89 comprising 2 or more antigens from Table 2, Table 3, Table 4, Table 5, or fragments or homologues thereof.
91. The vaccine composition of clause 90, comprising 3 or more antigens from Table 2, Table 3, Table 4, Table 5, or fragments or homologues thereof.
92. The vaccine composition of clause 91, comprising 5 or more antigens from Table 2, Table 3, Table 4, Table 5, or fragments or homologues thereof.
93. The vaccine composition of clause 92, comprising 10 or more antigens from Table 2, Table 3, Table 4, Table 5, or fragments or homologues thereof.
94. The vaccine composition of clause 89, wherein the fragment comprises at most 10, 15, 20, 25, 30, 35, 40, 45, 50, 55, 60, 65, 70, 75, 80, 85, 90, 95, 100, 110, or 120 amino acids.
95. The vaccine composition of clause 89, wherein the fragment comprises at least 10, 15, 20, 25, 30, 35, 40, 45, 50, 55, 60, 65, 70, 75, 80, 85, 90, 95, 100, 110, or 120 amino acids.
96. The vaccine composition of clause 89, wherein the homologue comprises a sequence having at least 90% sequence identity to the antigen of Table 1.
97. The vaccine composition of clause 12, wherein the library comprises at least 1x10⁴, 1x10⁵, 1x10⁶ different variants of the microbe.
98. The vaccine composition of clause 12, wherein the library comprises at least 90% of all known sequences of the microbe.
99. A pharmaceutical composition comprising the vaccine composition of any of the preceding clauses and a pharmaceutically acceptable diluent, adjuvant, excipient, or any combinations thereof.
100. The vaccine composition of clause 99, wherein the vaccine is in a form of an aerosol formulation.
101. The vaccine composition of clause 99, wherein the vaccine is in a form of an injectable formulation.
102. The vaccine composition of any of the preceding clauses, wherein each of the antigens is at a concentration that alone does not provide a significant prophylactic immune response to the broadly neutralizing antigen in a subject; and collectively, the antigens have a combined concentration that provides an immune response to the broadly neutralizing antigen in the subject.
103. The vaccine composition of clause 102, wherein the subject is a bird.
104. The vaccine composition of clause 102, wherein the subject is a mammal.
105. The vaccine composition of clause 103, wherein the subject is a human.
106. The vaccine composition of clause 103, wherein the subject is a pig.
107. A virus like particle (VLP) comprising the vaccine composition of any of the preceding clauses.
108. A recombinant expression vector comprising a nucleic acid molecule encoding:
   (a) a set of antigens that are representative of at least 60% of each of first order clade and second order clades of a microbe;
   (b) set of antigens wherein the smallest pairwise edit distance between two of the antigens is no more than 25 and the largest pairwise edit distance between two of the antigens is at least 300;
   (c) set of antigens that are representative of at least 60% of all operational taxonomic units (OTUs) of the microbe; or
   (d) set of antigens that are representative of at least 60% of all operational taxonomic units (OTUs) of the microbe.
109. A recombinant expression vector comprising a nucleic acid molecule encoding:
   (a) a set of antigens that are representative of at least 60% of each of first order clade and second order clades of a microbe;
   (b) set of antigens wherein the smallest pairwise edit distance between two of the antigens is no more than 5% of the size of the antigens, and the largest pairwise edit distance is at least 75% of the size of the antigen;
   (c) set of antigens that are representative of at least 60% of all operational taxonomic units (OTUs) of the microbe; or
   (d) set of antigens that are representative of at least 60% of all operational taxonomic units (OTUs) of the microbe.
110. A method for treating or reducing the likelihood of an infection in a subject, comprising administrating to the subject a vaccine composition of any of the preceding clauses.
111. A method for treating or reducing the likelihood of a flu infection in a subject, comprising administrating to the subject a vaccine composition that results in an immune activation effective against seasonal flu for at least 3, 4, 5, 6, 7, 8, 9, or 10 years.
112. The method of clause 110 or clause 111, wherein the subject is a human.
113. The method of clause 110 or clause 111, wherein the subject is a domesticated animal.
114. A method for making a vaccine composition, comprising: selecting a set of antigens that are (a) representative of at least 60% of each of first order clade and second order clades of a microbe;
   (b) set of antigens wherein the smallest pairwise edit distance between two of the antigens is no more than 5% of the size of the antigens, and the largest pairwise edit distance is at least 75% of the size of the antigens;
   (c) representative of at least 60% of all operational taxonomic units (OTUs) of the microbe; or
   (d) representative of at least 60% of all operational taxonomic units (OTUs) of the microbe.
115. The method of clause 114, further comprising:
   obtaining a plurality of antigen sequences from a library of strains of the microbe; and
   aligning the plurality of antigen sequences to create a phylogenetic tree of the antigen.

The term "about," as used herein, generally refers to a range that is 2%, 5%, 10%, or 15% greater than or less than (±) a stated numerical value within the context of the particular usage. For example, "about 10" would include a range from 8.5 to 11.5. As used herein, the terms "about" and "approximately," when used to modify a numeric value or numeric range, indicate that deviations of up to about 0.2%, about 0.5%, about 1%, about 2%, about 5%, about 7.5%, or about 10% (or any integer between about 1% and 10%) above or below the value or range remain within the intended meaning of the recited value or range.

As used in this specification and the appended claims, the singular forms "a," "an," and "the" include plural references unless the context clearly dictates otherwise. Thus, for example, references to "a method" include one or more methods, and/or steps of the type described herein and/or which will become apparent to those persons skilled in the art upon reading this disclosure.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

## Claims

1. A vaccine composition comprising a set of antigens that activate an immune response in a subject to at least 6 strains identified in Table 1, wherein the virus is type A influenza, and wherein the type A influenza is H1N1 or H3N2.

2. The vaccine of claim 1, wherein the vaccine activates an immune response against A/California/7/2004.

3. The vaccine of claim 1, wherein the vaccine activates an immune response against A/Memphis/1/68.

4. The vaccine of claim 1, wherein the vaccine activates an immune response against A/New York/1/1918.

5. The vaccine of claim 1, wherein the vaccine activates an immune response against A/California/07/2009.

6. The vaccine of claim 1, wherein the vaccine activates an immune response against A/Beijing/262/1995.

7. The vaccine of claim 1, wherein the vaccine activates an immune response against A/Puerto Rico/8/1934.

8. The vaccine of claim 1, wherein the vaccine activates an immune response against A/Brisbane/59/2007.

9. The vaccine of any one of claims 1 to 8, wherein the antigens are further selected from Table 2, Table 3, Table 4, Table 5, or a fragment or homologue thereof.

10. The vaccine of claim 9, wherein the antigen is from A/Wisconsin/28/2011.2011/12.

11. The vaccine of claim 9, wherein the vaccine further comprises an antigen from A/Denver/1957.

12. The vaccine of claim 9, which further includes an antigen from A/Indiana/11/2018.

13. The vaccine of claim 9, which further includes an antigen from A/Bilthoven/17938/1969.

14. A vaccine comprising at least three H1 hemagglutinin antigens and at least three H3 hemagglutinin antigens, wherein the at least three H1 hemagglutinin antigens comprise the antigens for A/California/07/2009, A/Puerto Rico/8/1934, and A/Brisbane/59/2007 and wherein the at least three H3 hemagglutinin antigens comprise the antigens for A/California/07/2004, A/Nanchang/933/1995, and A/Memphis/1/1980.

15. A vaccine comprising a set of antigens of a microbe wherein the smallest pairwise edit distance between two of the antigens is at least 10% of the average size of the antigens and the largest pairwise edit distance between two of the antigens is no more than 98% of the average size of the antigens.
